# EUROPEAN PATENT APPLICATION

(11) **EP 3 211 097 A1**
(43) Date of publication of application: **30.08.2017**
(21) Application number: 17154875.3
(22) Date of filing: 24.03.2011
(51) Int. Cl.: C12Q 1/68

(54) **GENES AND GENES COMBINATIONS PREDICTIVE OF EARLY RESPONSE OR NON RESPONSE OF SUBJECTS SUFFERING FROM INFLAMMATORY DISEASE TO CYTOKINE TARGETING DRUGS (CYTD)**

(30) Priority: 24.03.2010 US 282732 P; 19.01.2011 US 201161457158 P
(62) Divisional of application: 11711830.7
(71) Applicant: TC LAND Expression, 44200 Nantes (FR)
(72) Inventor: CERVINO, Alessandra, 44100 Nantes (FR); POPA-NITA, Oana Ruxandra, 44200 Nantes (FR); BRAUD, Christophe, 44980 Sainte-Luce-sur-Loire (FR)
(74) Representative: Regimbeau

(57) **Abstract**

This invention refers to the field of human medicine and specifically to the diagnosis/prognosis of the responsiveness to a cytokine targeting drug (CyTD) treatment of a subject suffering from an inflammatory disease. More precisely, the present invention concerns a method for the *in vitro* diagnosis/prognosis of a CyTD responsive or non-responsive phenotype, comprising: (a) determining from a subject biological sample an expression profile comprising the gene MAPK14; or the genes MAPK14 and S100A9; or the genes MAPK14 and GNLY; or the 61 genes of Table 2, or the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or Equivalent Expression Profile of anyone of the expression profiles of (i) and (ii), and optionally one or more housekeeping gene(s), (b) comparing the obtained expression profile with at least one reference expression profile, and (c) determining the responsive or non-responsive phenotype from said comparison. The present invention also relates to kits and nucleic acid microarrays for performing said method. The present invention also relates to methods of treatment of inflammatory disease-suffering patients.

SEQUENCE LISTING

<110>  TC LAND EXPRESSION

<120>  Genes and genes combinations predictive of early response or non
       reponse of subjects suffering from inflammatory disease to
       cytokine targeting drugs (CyTD)

<130>  B73373-D28552

<150>  US61/282,732
<151>  2010-03-24

<150>  US61/457,158
<151>  2011-01-19

<160>  106

<170>  PatentIn version 3.5

<210>  1
<211>  4045
<212>  DNA
<213>  Homo sapiens

<400>  1
gcagccagag ctcagcaggg ccctggagag atggccacgg tcccagcacc ggggaggact      60

ggagagcgcg cgctgccacc gccccatgtc tcagccaggg cttccttcct cggctccacc     120

ctgtggatgt aatggcggcc cctgctctgt cctggcgtct gcccctcctc atcctcctcc     180

tgcccctggc tacctcttgg gcatctgcag cggtgaatgg cacttcccag ttcacatgct     240

tctacaactc gagagccaac atctcctgtg tctggagcca agatggggct ctgcaggaca     300

cttcctgcca agtccatgcc tggccggaca gacggcggtg gaaccaaacc tgtgagctgc     360

tccccgtgag tcaagcatcc tgggcctgca acctgatcct cggagcccca gattctcaga     420

aactgaccac agttgacatc gtcaccctga gggtgctgtg ccgtgagggg gtgcgatgga     480

gggtgatggc catccaggac ttcaagccct ttgagaacct tcgcctgatg gcccccatct     540

ccctccaagt tgtccacgtg gagacccaca gatgcaacat aagctgggaa atctcccaag     600

cctcccacta ctttgaaaga cacctggagt tcgaggcccg gacgctgtcc ccaggccaca     660

cctgggagga ggcccccctg ctgactctca agcagaagca ggaatggatc tgcctggaga     720

cgctcacccc agacacccag tatgagtttc aggtgcgggt caagcctctg caaggcgagt     780

tcacgacctg gagcccctgg agccagcccc tggccttcag gacaaagcct gcagcccttg     840

ggaaggacac cattccgtgg ctcggccacc tcctcgtggg cctcagcggg gcttttggct     900

tcatcatctt agtgtacttg ctgatcaact gcaggaacac cgggccatgg ctgaagaagg     960

tcctgaagtg taacacccca gacccctcga agttcttttc ccagctgagc tcagagcatg    1020

gaggagacgt ccagaagtgg ctctcttcgc ccttcccctc atcgtccttc agccctggcg    1080

gcctggcacc tgagatctcg ccactagaag tgctggagag ggacaaggtg acgcagctgc    1140

tcctgcagca ggacaaggtg cctgagcccg catccttaag cagcaaccac tcgctgacca 1200

gctgcttcac caaccagggt tacttcttct tccacctccc ggatgccttg gagatagagg 1260

cctgccaggt gtactttact tacgacccct actcagagga agaccctgat gagggtgtgg 1320

ccggggcacc cacagggtct tccccccaac ccctgcagcc tctgtcaggg gaggacgacg 1380

cctactgcac cttcccctcc agggatgacc tgctgctctt ctcccccagt ctcctcggtg 1440

gccccagccc cccaagcact gcccctgggg gcagtggggc cggtgaagag aggatgcccc 1500

cttctttgca agaaagagtc cccagagact gggaccccca gcccctgggg cctcccaccc 1560

caggagtccc agacctggtg gattttcagc caccccctga gctggtgctg cgagaggctg 1620

gggaggaggt ccctgacgct ggccccaggg agggagtcag tttcccctgg tccaggcctc 1680

ctgggcaggg ggagttcagg gcccttaatg ctcgcctgcc cctgaacact gatgcctact 1740

tgtccctcca agaactccag ggtcaggacc caactcactt ggtgtagaca gatggccagg 1800

gtgggaggca ggcagctgcc tgctctgcgc cgagcctcag aaggaccctg ttgagggtcc 1860

tcagtccact gctgaggaca ctcagtgtcc agttgcagct ggacttctcc acccggatgg 1920

cccccaccca gtcctgcaca cttggtccat ccatttccaa acctccactg ctgctcccgg 1980

gtcctgctgc ccgagccagg aactgtgtgt gttgcagggg ggcagtaact ccccaactcc 2040

ctcgttaatc acaggatccc acgaatttag gctcagaagc atcgctcctc tccagccctg 2100

cagctattca ccaatatcag tcctcgcggc tctccagggc tccctgccct gacctcttcc 2160

ctgggttttc tgccccagcc tcctccttcc ctcccctccc cgtccacagg gcagcctgag 2220

cgtgctttcc aaaacccaaa tatggccacg ctccccctcg gttcaaaacc ttgcacaggt 2280

cccactgccc tcagccccac ttctcagcct ggtacttgta cctccggtgt cgtgtgggga 2340

catccccttc tgcaatcctc cctaccgtcc tcctgagcca ctcagagctc cctcacaccc 2400

cctctgttgc acatgctatt ccctggggct gctgtgcgct ccccctcatc taggtgacaa 2460

acttccctga ctcttcaagt gccggttttg cttctcctgg agggaagcac tgcctccctt 2520

aatctgccag aaacttctag cgtcagtgct ggagggagaa gctgtcaggg acccagggcg 2580

cctggagaaa gaggccctgt tactattcct ttgggatctc tgaggcctca gagtgcttgg 2640

ctgctgtatc tttaatgctg gggcccaagt aagggcacag atccccccac aaagtggatg 2700

cctgctgcat cttcccacag tggcttcaca gacccacaag agaagctgat ggggagtaaa 2760

ccctggagtc cgaggcccag gcagcagccc cgcctagtgg tgggccctga tgctgccagg 2820

cctgggacct cccactgccc cctccactgg aggggtctcc tctgcagctc agggactggc 2880

acactggcct ccagaagggc agctccacag ggcagggcct cattattttt cactgcccca 2940

gacacagtgc ccaacacccc gtcgtatacc ctggatgaac gaattaatta cctggcacca 3000

cctcgtctgg gctccctgcg cctgacattc acacagagag gcagagtccc gtgcccatta 3060

ggtctggcat gccccctcct gcaaggggct caacccccta ccccgacccc tccacgtatc 3120

tttcctaggc agatcacgtt gcaatggctc aaacaacatt ccaccccagc aggacagtga 3180

ccccagtccc agctaactct gacctgggag ccctcaggca cctgcactta caggccttgc 3240

tcacagctga ttgggcacct gaccacacgc ccccacaggc tctgaccagc agcctatgag 3300

ggggtttggc accaagctct gtccaatcag gtaggctggg cctgaactag ccaatcagat 3360

caactctgtc ttgggcgttt gaactcaggg agggaggccc ttgggagcag gtgcttgtgg 3420

acaaggctcc acaagcgttg agccttggaa aggtagacaa gcgttgagcc actaagcaga 3480

ggaccttggg ttcccaatac aaaaatacct actgctgaga gggctgctga ccatttggtc 3540

aggattcctg ttgcctttat atccaaaata aactcccctt tcttgaggtt gtctgagtct 3600

tgggtctatg ccttgaaaaa agctgaatta ttggacagtc tcacctcctg ccatagggtc 3660

ctgaatgttt cagaccacaa ggggctccac acctttgctg tgtgttctgg ggcaacctac 3720

taatcctctc tgcaagtcgg tctccttatc cccccaaatg gaaattgtat ttgccttctc 3780

cactttggga ggctcccact tcttgggagg gttacatttt ttaagtctta atcatttgtg 3840

acatatgtat ctatacatcc gtatctttta atgatccgtg tgtaccatct ttgtgattat 3900

ttccttaata ttttttcttt aagtcagttc attttcgttg aaatacattt atttaaagaa 3960

aaatctttgt tactctgtaa atgaaaaaac ccattttcgc tataaataaa aggtaactgt 4020

acaaaataag tacaatgcaa caaaa 4045

<210> 2
<211> 586
<212> DNA
<213> Homo sapiens

<400> 2
aaacactctg tgtggctcct cggctttgac agagtgcaag acgatgactt gcaaaatgtc 60

gcagctggaa cgcaacatag agaccatcat caacaccttc caccaatact ctgtgaagct 120

ggggcaccca gacaccctga accaggggga attcaaagag ctggtgcgaa aagatctgca 180

aaattttctc aagaaggaga ataagaatga aaaggtcata gaacacatca tggaggacct 240

ggacacaaat gcagacaagc agctgagctt cgaggagttc atcatgctga tggcgaggct 300

aacctgggcc tcccacgaga agatgcacga gggtgacgag ggccctggcc accaccataa 360

gccaggcctc ggggagggca ccccctaaga ccacagtggc caagatcaca gtggccacgg 420

ccacggccac agtcatggtg gccacggcca cagccactaa tcaggaggcc aggccaccct 480

gcctctaccc aaccagggcc ccggggcctg ttatgtcaaa ctgtcttggc tgtggggcta 540

ggggctgggg ccaaataaag tctcttcctc caagtcaaaa aaaaaa 586

<210>  3
<211>  1593
<212>  DNA
<213>  Homo sapiens

<400>  3
   actttcaatt ctagatcagg aactgaggac atatctaaat tttctagttt tatagaaggc      60
   ttttatccac aagaatcaag atcttccctc tctgagcagg aatcctttgt gcattgaaga     120
   ctttagattc ctctctgcgg tagacgtgca cttataagta tttgatgggg tggattcgtg     180
   gtcggaggtc tcgacacagc tgggagatga gtgaatttca taattataac ttggatctga     240
   agaagagtga tttttcaaca cgatggcaaa agcaaagatg tccagtagtc aaaagcaaat     300
   gtagagaaaa tgcatctcca ttttttttct gctgcttcat cgctgtagcc atgggaatcc     360
   gtttcattat tatggtaaca atatggagtg ctgtattcct aaactcatta ttcaaccaag     420
   aagttcaaat tcccttgacc gaaagttact gtggcccatg tcctaaaaac tggatatgtt     480
   acaaaaataa ctgctaccaa ttttttgatg agagtaaaaa ctggtatgag agccaggctt     540
   cttgtatgtc tcaaaatgcc agccttctga aagtatacag caaagaggac caggatttac     600
   ttaaactggt gaagtcatat cattggatgg gactagtaca cattccaaca aatggatctt     660
   ggcagtggga agatggctcc attctctcac ccaacctact aacaataatt gaaatgcaga     720
   agggagactg tgcactctat gcctcgagct ttaaaggcta tatagaaaac tgttcaactc     780
   caaatacgta catctgcatg caaaggactg tgtaaagatg atcaaccatc tcaataaaag     840
   ccaggaacag agaagagatt acaccagcgg taacactgcc aactgagact aaaggaaaca     900
   aacaaaaaca ggacaaaatg accaaagact gtcagatttc ttagactcca caggaccaaa     960
   ccatagaaca atttcactgc aaacatgcat gattctccaa gacaaaagaa gagagatcct    1020
   aaaggcaatt cagatatccc caaggctgcc tctcccacca caagcccaga gtggatgggc    1080
   tgggggaggg gtgctgtttt aatttctaaa ggtaggacca acacccaggg gatcagtgaa    1140
   ggaagagaag gccagcagat cactgagagt gcaaccccac cctccacagg aaattgcctc    1200
   atgggcaggg ccacagcaga gagacacagc atgggcagtg ccttccctgc ctgtgggggt    1260
   catgctgcca cttttaatgg gtcctccacc caacggggtc agggaggtgg tgctgcccca    1320
   gtgggccatg attatcttaa aggcattatt ctccagcctt aagtaagatc ttaggacgtt    1380
   tcctttgcta tgatttgtac ttgcttgagt cccatgactg tttctcttcc tctctttctt    1440
   ccttttggaa tagtaatatc catcctatgt ttgtcccact attgtatttt ggaagcacat    1500
   aacttgtttg gtttcacagg ttcacagtta agaaggaatt ttgcctctga ataaatagaa    1560
   tcttgagtct catgcaaaaa aaaaaaaaaa aaa                                 1593

<210>  4

<211>  2168
<212>  DNA
<213>  Homo sapiens

<400>  4
ggagttagcc tcgctcaggg cgcggctaag gcgcccagat ggcctgcggg cgccaccacg      60

tccctggtcc cagctcggga gcacatcaga ggcttagagg cgagtgggaa gggactcaga     120

cagtgcagga cgagaaacgc ccgcggcacc aaagcccctc agagcgtcgc ccccgcctct     180

agttctagaa agtcagtttc ccggcactgg caccccggaa cctcaggggc tgccgagctg     240

ggggggcgct caagctgcga ggatccgggc tgcccgcgag acgaggagcg ggcgcccagg     300

atggggtgca tgaagtccaa gttcctccag gtcggaggca atacattctc aaaaactgaa     360

accagcgcca gcccacactg tcctgtgtac gtgccggatc ccacatccac catcaagccg     420

gggcctaata gccacaacag caacacacca ggaatcaggg aggcaggctc tgaggacatc     480

atcgtggttg ccctgtatga ttacgaggcc attcaccacg aagacctcag cttccagaag     540

ggggaccaga tggtggtcct agaggaatcc ggggagtggt ggaaggctcg atccctggcc     600

acccggaagg agggctacat cccaagcaac tatgtcgccc gcgttgactc tctggagaca     660

gaggagtggt ttttcaaggg catcagccgg aaggacgcag agcgccaact gctggctccc     720

ggcaacatgc tgggctcctt catgatccgg gatagcgaga ccactaaagg aagctactct     780

ttgtccgtgc gagactacga ccctcggcag ggagataccg tgaaacatta caagatccgg     840

accctggaca acgggggctt ctacatatcc ccccgaagca ccttcagcac tctgcaggag     900

ctggtggacc actacaagaa ggggaacgac gggctctgcc agaaactgtc ggtgccctgc     960

atgtcttcca agccccagaa gccttgggag aaagatgcct gggagatccc tcgggaatcc    1020

ctcaagctgg agaagaaact tggagctggg cagtttgggg aagtctggat ggccacctac    1080

aacaagcaca ccaaggtggc agtgaagacg atgaagccag ggagcatgtc ggtggaggcc    1140

ttcctggcag aggccaacgt gatgaaaact ctgcagcatg acaagctggt caaacttcat    1200

gcggtggtca ccaaggagcc catctacatc atcacggagt tcatggccaa aggaagcttg    1260

ctggactttc tgaaaagtga tgagggcagc aagcagccat tgccaaaact cattgacttc    1320

tcagcccaga ttgcagaagg catggccttc atcgagcaga ggaactacat ccaccgagac    1380

ctccgagctg ccaacatctt ggtctctgca tccctggtgt gtaagattgc tgactttggc    1440

ctggcccggg tcattgagga caacgagtac acggctcggg aaggggccaa gttccccatc    1500

aagtggacag ctcctgaagc catcaacttt ggctccttca ccatcaagtc agacgtctgg    1560

tcctttggta tcctgctgat ggagatcgtc acctacggcc ggatccctta cccagggatg    1620

tcaaaccctg aagtgatccg agctctggag cgtggatacc ggatgcctcg cccagagaac    1680

tgcccagagg agctctacaa catcatgatg cgctgctgga aaaaccgtcc ggaggagcgg    1740

ccgaccttcg aatacatcca gagtgtgctg gatgacttct acacggccac agagagccag 1800

taccaacagc agccatgata gggaggacca gggcagggcc agggggtgcc caggtggtgg 1860

ctgcaaggtg gctccagcac catccgccag ggcccacacc cccttcctac tcccagacac 1920

ccaccctcgc ttcagccaca gtttcctcat ctgtccagtg ggtaggttgg actggaaaat 1980

ctctttttga ctcttgcaat ccacaatctg acattctcag gaagccccca agttgatatt 2040

tctatttcct ggaatggttg gattttagtt acagctgtga tttggaaggg aaactttcaa 2100

aatagtgaaa tgaatattta aataaaagat ataaatgcca aagtctttac caaaaaaaaa 2160

aaaaaaaa 2168

<210> 5
<211> 751
<212> DNA
<213> Homo sapiens

<400> 5
gtatctgtgg taaacccagt gacacggggg agatgacata caaaaagggc aggacctgag 60

aaagattaag ctgcaggctc cctgcccata aaacagggtg tgaaaggcat ctcagcggct 120

gccccaccat ggctacctgg gccctcctgc tccttgcagc catgctcctg ggcaacccag 180

gtctggtctt ctctcgtctg agccctgagt actacgacct ggcaagagcc cacctgcgtg 240

atgaggagaa atcctgcccg tgcctggccc aggagggccc ccagggtgac ctgttgacca 300

aaacacagga gctgggccgt gactacagga cctgtctgac gatagtccaa aaactgaaga 360

agatggtgga taagcccacc cagagaagtg tttccaatgc tgcgacccgg gtgtgtagga 420

cggggaggtc acgatggcgc gacgtctgca gaaatttcat gaggaggtat cagtctagag 480

ttacccaggg cctcgtggcc ggagaaactg cccagcagat ctgtgaggac ctcaggttgt 540

gtataccttc tacaggtccc ctctgagccc tctcaccttg tcctgtggaa gaagcacagg 600

ctcctgtcct cagatcccgg gaacctcagc aacctctgcc ggctcctcgc ttcctcgatc 660

cagaatccac tctccagtct ccctcccctg actccctctg ctgtcctccc ctctcacgag 720

aataaagtgt caagcaagat tttaaaaaaa a 751

<210> 6
<211> 1517
<212> DNA
<213> Homo sapiens

<400> 6
aaagtgcggg gtcggccggg tgctgggccg aggccgaggc cggggcggga tccagagcgg 60

gagccggcgc gggatctggg actcggagcg ggatccggag cgggacccag gagccggcgc 120

ggggccatgg cgaggcgcgg gccagggtgg cggccgcttc tgctgctcgt gctgctggcg 180

ggcgcggcgc agggcggcct ctacttccgc cggggacaga cctgctaccg gcctctgcgg 240

ggggacgggc tggctccgct ggggcgcagc acataccccc ggcctcatga gtacctgtcc 300

ccagcggatc tgcccaagag ctgggactgg cgcaatgtgg atggtgtcaa ctatgccagc 360

atcacccgga accagcacat cccccaatac tgcggctcct gctgggccca cgccagcacc 420

agcgctatgg cggatcggat caacatcaag aggaagggag cgtggccctc caccctcctg 480

tccgtgcaga acgtcatcga ctgcggtaac gctggctcct gtgaaggggg taatgacctg 540

tccgtgtggg actacgccca ccagcacggc atccctgacg agacctgcaa caactaccag 600

gccaaggacc aggagtgtga caagtttaac caatgtggga catgcaatga attcaaagag 660

tgccacgcca tccggaacta caccctctgg agggtgggag actacggctc cctctctggg 720

agggagaaga tgatggcaga aatctacgca aatggtccca tcagctgtgg aataatggca 780

acagaaagac tggctaacta caccggaggc atctatgccg aataccagga caccacatat 840

ataaaccatg tcgtttctgt ggctgggtgg ggcatcagtg atgggactga gtactggatt 900

gtccggaatt catggggtga accatggggc gagagaggct ggctgaggat cgtgaccagc 960

acctataagg atgggaaggg cgccagatac aaccttgcca tcgaggagca ctgtacattt 1020

ggggacccca tcgtttaagg ccatgtcact agaagcgcag tttaagaaaa ggcatggtga 1080

cccatgacca gaggggatcc tatggttatg tgtgccaggc tggctggcag gaactggggt 1140

ggctatcaat attggatggc gaggacagcg tggcactggc tgcgagtgtt cctgagagtt 1200

gaaagtggga tgacttatga cacttgcaca gcatggctct gcctcacaat gatgcagtca 1260

gccacctggt gaagaagtga cctgcgacac aggaaacgat gggacctcag tcttcttcag 1320

cagaggactt gatattttgt atttggcaac tgtgggcaat aatatggcat ttaagaggtg 1380

aaagagttca gacttatcac cattcttatg tcactttaga atcaagggtg ggggagggag 1440

ggagggagtt ggcagtttca aatcgcccaa gtgatgaata aagtatctgg ctctgcacga 1500

aaaaaaaaaa aaaaaaa 1517

<210> 7
<211> 3227
<212> DNA
<213> Homo sapiens

<400> 7
attagagcgt gtctgggagc aggttaaact ttctcctagg tggtgatgtg cctccaggtg 60

agcgcacagg ctttgcagaa accccagcat gacaagcaag taggaaggtg acattgttgt 120

ttgagaaaag ccagtgaccc ggttgggacg gagacacgat ggggagccgg tctccaacag 180

catgcccttc ctgaaggcca acgaggtgac ggacagcgcg tacatgggct ccgagagcac 240

ctacagtgag tgtgagacct tcacggacga ggacaccagc accctggtgc accctgagct 300

gcaacctgaa ggggacgcag acagtgccgg cggctcggcc gtgccctctg agtgcctgga 360

cgccatggag gagcccgacc atggtgccct gctgctgctc ccaggcaggc ctcaccccca 420

tggccagtct gtcatcacgg tgatcggggg cgaggagcac tttgaggact acggtgaagg 480

cagtgaggcg gagctgtccc cagagaccct atgcaacggg cagctgggct gcagtgaccc 540

cgctttcctc acgcccagtc cgacaaagcg gctctccagc aagaaggtgg caaggtacct 600

gcaccagtca ggggccctga ccatggaggc cctggaggac ccttcccccg agctcatgga 660

gggcccagag gaggacattg ctgacaaggt tgtcttcctg gaaaggcgtg tgctggagct 720

ggaaaaggac acggcagcca ccggtgagca acacagccgc ctgaggcagg agaacctgca 780

gctggtgcac agagcaaacg ccctggagga gcagctgaag gagcaggagc tgagagcctg 840

cgagatggtc ctggaagaga cccggcgtca gaaggagctc ctgtgcaaga tggagaggga 900

gaagagcatt gagatcgaga acctgcagac caggctacag caactggacg aggagaacag 960

tgaactccgg tcctgcacgc cctgtctgaa ggccaacatt gagcgtctgg aggaggagaa 1020

gcagaagctg ttggatgaga tagagtcgct gacgctgcgg ctcagtgaag agcaggagaa 1080

caagaggaga atgggggaca ggctgagtca cgagaggcac cagttccaga gggacaagga 1140

ggccacccag gagctgatcg aggacctccg aaagcagctg gagcacctgc agctcctcaa 1200

gctggaggcc gagcagcggc ggggccgcag cagcagcatg ggcctgcagg agtaccacag 1260

ccgcgcccgg gagagcgagc tggagcagga ggtccgcagg ctgaagcagg acaaccgcaa 1320

cctgaaggag cagaacgagg agctgaacgg gcagatcatt accctcagca tccagggcgc 1380

caagagcctc ttctccacag ccttctctga gtccctggct gcagagatca gctccgtctc 1440

ccgagatgag ctcatggagg cgattcagaa gcaggaggag atcaacttcc gcctgcagga 1500

ctacatcgac aggatcatcg tggccatcat ggagaccaac ccgtccatcc tggaggtcaa 1560

gtagaggcag gaaggtccag cctgagctgg attcgggact ccaacaccct ggagtggttc 1620

cgtcagacca tgaggagcca agaccagcag gtcccacagc cgacagtgcc cagagcatgc 1680

agggaaccct cgtgcagctg agctggggcc gccaaagacc ggggctgcca aaggggcaga 1740

gggtggtgga gaggagaggg agaaagggaa gtcccagggc ccggggtcca cagaggatga 1800

gggttgtggc agggccgtcc atcagcgctg accttccggg ggcccagagc ttcccagccc 1860

tgagtcaagc tggccatgaa cgcgtacact tcagttcagc aggatgggct ggagagcctc 1920

tctgtgcagc ggtgtggggt gagccctgct gtggcctcct tgtggtggtc cctcttccca 1980

cgtgcagccc tgttgggaag aaaggaagaa aacaggtccc tccaggggtg ctgctgccta 2040

agccacccac ataagtacgc tggtgccgtg tcacccatgt tgagccgctc ctgatggctg 2100

acgggctccc agaccctcac ctcggacatg gtggtggggg aaggacgggt gggcaaggct 2160

ggtgcgttcc ccagctctcc ctacgctgct cgggccattg cccagccaga tgtggtcacc 2220

tcagtccagc tctggggcct ccaggccatg tggctgttcc cacggcccag tcctcgctgc 2280

agtaacccct gggggctctg accacctatg ggggccgggc aggagcctct ggggcctcca 2340

ctccgacatc aggacctgag atgaccgctg tgtggcgctc tctccctggg cagggtggat 2400

gccacaggcc cctctggctc ccaggtgctg cttctccaca ggtgcggcct ggcccggcct 2460

cctaaaggcc acaccctccc cacgcacttc ccaggccaga atccaaacat cgggaaccct 2520

gttttcttct gggtgtgtct cacttagaaa tcgtggttct tccccgaggg tgcatgttgc 2580

aggagggaga gggcagggaa gactcacagc agagcaggag ggggcctgtg cttctcgggg 2640

tctgcacccc aggcacagcg gtgtcacccc gcaggaccgc gggcctgccc caacccccag 2700

cattcccggg tgggcccaga ccccatcacc aagactggcc acccgctgcg tgtgtgtgcg 2760

cgcgcgtgta cgtgtggccc cacatccgcc gccttccacg ctaggatgta agaggtcgcc 2820

tcctattgta catttgggga aagccttggg tgtaaatcag tgtaaacttg gaggagagat 2880

ttttctatca tgtagagtag gtatttttta tagattgaag gttgatcaat tttttaatac 2940

tttcaagaga aaactgtgta tacacatgaa atatatatat atatatatat atatatatgt 3000

ataatatata aagactggca ccctgcctct ctgtgcccag gcccagccct ggtgacatgg 3060

caccactcag cagtgctgtc actgtaagca tggactccca ggagacagtg tgggaaacgc 3120

tcctgcttta attccccgag aaacggctct tcctgcctgg atgcaggagg gcaggggcca 3180

ccacagatta aagctgttac tgcacaaaaa aaaaaaaaaa aaaaaaa 3227

<210> 8
<211> 5537
<212> DNA
<213> Homo sapiens

<400> 8
gcctttgcta aattgctgaa taagaagatg gttgagtcac ctccttcgct taaacttgtc 60

ctcattggag gttgtcgtaa caaagatgat gaacttaggg taaaccaact gagaaggctg 120

tctgaggatt taggagttca agaatatgtg gaatttaaaa taaacattcc atttgatgaa 180

ttaaagaatt atttgtctga agcaacaatt ggtctgcata ccatgtggaa cgagcatttt 240

gggattggag ttgtggagtg tatggcagct ggcacaatta tccttgcaca caattcgggg 300

ggcccaaagc ttgacattgt ggttcctcac gaaggagata taactggctt tctggctgag 360

agtgaagaag actatgctga aactatcgct cacattcttt ccatgtctgc agaaaagaga 420

ctccaaatca gaaaaagtgc tcgtgcatct gtaagcagat tctctgatca ggaatttgaa 480

gtgacattcc tatcatctgt ggaaaagtta tttaagtaat gccatatctg taaaattaaa 540

gatattttat ataaactggt taaacacctt catatgtaaa tatttttcta aattcaatct 600

catttgtcaa atcattttac tttagaaaac agacaaaatt tccttttaga ataaaaggaa 660

gtgttgaaaa gaaaatggat gactagcctt cggcttccat tcttggtata catgagagag 720
gctggctgct gagatgaatg tgaaccaggt tgcagagaat ctggctttga gccaccagga 780
agaactagtg gatttgccaa aaaactaccc cttgagtgaa aatgaagatg agggggacag 840
tgatggagag agaaagcatc aaaagcttct ggaagcaatc atttcccttg atggaaagaa 900
taggcggaaa ttggctgaga ggtctgaggc tagtctgaaa gtgtcagagt tcagtgtcag 960
ttctgaagga tcaggagaaa agctgggcct tgcagatctg cttgagcccg ttaaaacttc 1020
atcttctttg gccactgtaa aaaagcaact gaatagagtc aaatcaaaga aggtggtgga 1080
gttacctctt aacaaagaaa aaattgaaca gatccacaga gaagtagcat tcagtaaaac 1140
ctcacaggtc ctctccaaat gggaccctat catcctgaag aaccagcagg cagagcagct 1200
ggtttttccc ctggggaagg agcagccagc cattgctccc attgaacatg cgctcagtgg 1260
ctggaaggca agaactcccc tggagcagga aatttttaac ctcctccata agaacaagca 1320
gccagtgaca gatcctttac tgactcccat ggaaaaggcc tctctccaag ccatgagcct 1380
ggaagaggca aagatgcacc gagcagagct tcagagggct cgggctctgc agtcctacta 1440
tgaggccaag gctcgaaaag agaagaaaat caaaagtaaa aagtatcaca aagtcgtgaa 1500
gaaaggaaag gccaagaaag ccttaaaaga gtttgagcag ctacagaagg ttaatccaac 1560
tgtggcactg gaagaaatgg aaaaaattga aaatgccaga atgatggaaa gaatgagcct 1620
taagcaccaa aacagtggga aatgggccaa gtcaaaggca attatggcca aatatgacct 1680
ggaggctcgc caagctatgc aggaacagtt ggccaagaac aaagaactga cacagaaact 1740
ccaggtagcc tctgagagtg aggaagagga gggaggcaca gaagtggaag aactccttgt 1800
ccctcatgta gcgaatgaag tgcagatgaa tgtggacgga ccgaatccct ggatgttcag 1860
gagctgcacc agtgacacca aagaggctgc aacacaggag gaccctgagc aagtgccaga 1920
gcttgcagct catgaggttt ctgcaagtga ggcagaagaa agaccagtgg cagaggaaga 1980
aattttgttg agagaatttg aggaaaggca atcccttaga aaaagatctg agctcaacca 2040
ggatgctgag ccagcaagca gtcaagaaac aaaagattct agcagccagg aggtgctgtc 2100
cgaattgagg gcactatctc agaaattgaa ggaaaaacat cagtccagga agcaaaaagc 2160
aagttcagag gggactgttc cccaggtcca gagagaggaa cctgccccag aagaagcgga 2220
acccctattg ctacagaggt cagagagagt acaaactctg gaagagctag aagagctggg 2280
aaaagaagat tgttttcaaa ataaggagct tcccagacct gtgttagaag gacagcagtc 2340
agagaggacc ccaaataatc ggcctgatgc ccctaaggag aagaaagaga aggagcaact 2400
gatcaaccta cagaacttcc tgaccacaca gtctccttcc gtgaggtctt tggcagttcc 2460
cacaataata gaggagctgg aagatgaaga ggagagagac caaaggcaga tgataaagga 2520
agcttttgct ggggatgatg tcatcagaga tttcttgaaa gagaagaggg aagctgtgga 2580

ggcgagtaag ccaaaggacg tggacctgac actacctggc tggggcgagt ggggtggtgt 2640

gggcctaaag cccagtgcca agaaaagacg ccagtttctc attaaagccc ctgagggtcc 2700

tccaagaaaa gataagaatt tgccaaatgt gattatcagt gagaagcgca acatccacgc 2760

agcagctcat caggtacaag tgcttccata tccatttacc caccatcggc aatttgaaag 2820

gaccatccag acccctatag gatccacatg gaacacccag agggctttcc aaaagctgac 2880

tactcccaag gtcgtcacca agccaggcca tatcattaag cccataaaag cagaggatgt 2940

gggctaccag tcttcctcaa ggtcagacct gcctgtcata cagaggaatc caaaacgaat 3000

caccacacgt cacaataaag aagaaaaact gtaggttgtg tagctggaga agtgacagtc 3060

aggggccctg attccacttc ctttggtcca gttttactct gctacagggt ggattccaaa 3120

actggctcag cacattgcat gtagttgagc cacatttttt aaaaaaagaa aatggatgac 3180

cattaattga ctagcatttt agaattgatc agacattaga acacagaaaa attctagtac 3240

atttaaattc taaacaatac agtggatgac ccttttgaat atacctaatg atttccttaa 3300

aaaagaaatt ttaaacagac ttgtttaatc gtgttctcaa agcatacagt caagaggtgg 3360

gactgactga tgctttatag gtgtgtgtag ggtggtagag gccaaggtgc tgccagcaat 3420

cctttccata ctaggtactg gtgaaaattg tttttgttta tgctgtcagc acatttgtgt 3480

gggtctctca ttgtccctta acagtgccgc atctcagcct ggaagtcagc tttaagtcat 3540

tcaagagaac ctcaggctgt ttttctgaca gtgatgatat gatatacaga tacatccaca 3600

gggtatctat taccagcata atgcattgta agatggcaag gtggcatttt gaaagagcgc 3660

tgggcaagca gttagcacat ctgggcctac cttcagcttc ttcatttaca aacttctgac 3720

cttttgacac taaagctgct cctttatctt tctgagtctc agattcttca tctgtaatct 3780

ggagttgtta attccagtcc ttactacctt ttagagttgg aatgagatgg aaagtagatg 3840

aaactacttt gaaaattacg acagttaagg gctgggtgcg gtgtctcaca cctgtaatcc 3900

cagcactttg ggaggccgag atgggtggat catgaggtca gcagttgaga ccagcctgaa 3960

caacatggtg aaaccctgtc tgtactaaaa atacaaaaaa attagctggg cctggtggca 4020

ggcacctgta atcccagcta cttgggagac tgaggcagga gaattgcttg aaactggaag 4080

gcagaggttg cagtgagcca agattgtgcc actgcactct agcctgggca ataagcaaaa 4140

ctccatctca aaaaagaaaa aaaggaaaaa gaaaattatg agagttactt aaaggtaaca 4200

tcacatacta aatgtcttct ataatcctat atttattaat gcattacaac tctgtagatt 4260

gttagttact aggccagtag ctaggaattg gtataaattt aatgcacctt ctatcctgaa 4320

taactagcat ggaaaagtga atatatgtgt gagcagatat ggctataaag acctatagct 4380

tttgcacttt atgcatatat aatcaatcct ttctagttca gtgaattgac cccatccaca 4440

ggctgattca tctttgtgtt aaggggcaaa tgaaacggta tattatttct ttgcagtctc 4500

ctctcagtca ttcatcaatg tggccagctt atctactccc aattatgttg ttgatacatc 4560

tccaagccat ctgtcatcag atcaaaaagc agcaaacaga gggtcagtca caggatgttc 4620

tgacacacca ttgtaacttt ttgttagaga tgatcccatt tagaaaaaga ctggtagaaa 4680

ttggagtgaa aggaacccta cagattagcc cagttctctc ttattttcag ctttacagac 4740

aagaacaatt taaatctaaa gaatttagta gattccttca gtgtcacaaa gctgtttcat 4800

gaaagaatca agattataac ctggatattc tgactcctgg cccagtgctt tttcttactt 4860

tgtagctaca ctttgaagta agattcaaac tgttatccac tcaattgcct tattcctgag 4920

gatgtagtga aggaagaaaa agttttctgg aattccgtaa attatatttt aagcttattt 4980

cttcaaaatt attttcatat atcacagata tatcattgga agatataatt tgcatatatg 5040

ttcattatca gtgttcctaa tttggtatta catgtattct atttttttct gaatgatagc 5100

atgaaaagtg tcaaagtggt ttgtccgcta gcgtctgtct gcagaacttt caggatgact 5160

attaattcct ctcagatgtc atttttgagt ggtccaagcc tgctgttttg aacccacagc 5220

agtggagatt tgtattctta tttacagttg tgtactataa agtgtgtgtt acataggttt 5280

tgtgtaataa ttatttgtaa atattattta gatttgtatt tagacatgat ttatatctaa 5340

tatagataca aagtctgtgt ctaaatatta tttaaagaag tgatttttca ttctcttgga 5400

ttctttccag tgtggtgcct tttatatgcc tcacatagtc tccttgttct cctactaata 5460

ttcccaagct ccatatgcca attaaagaag aaacaaaaat aaaagtttgt cttgcttgtg 5520

aaacattaaa aaaaaaa 5537

<210> 9
<211> 1022
<212> DNA
<213> Homo sapiens

<400> 9
gggcgggagc ggcggtccag actggggagg gacgcgcacc ggccaggagg cttcaagagg 60

agggcactag ggccctgcga gcggcgtctt aaccggcggc gctaggactc cgcgggaaac 120

ggcgggggcg gagcgggcgg caccaggacc caggggaacc gcgacgggcg ggcggcgagc 180

aggcccggga gccgggaggc tgcgggcggc ggcgctggac ccgacgcggc gagagaggcc 240

ccgagatgcc gagcaagaag aagaagtaca acgcgcggtt cccgccggcg cggatcaaga 300

agatcatgca gacggacgaa gagattggga aggtggcggc ggcggtgcct gtcatcatct 360

cccgggcgct cgagctcttc ctagagtcgc tgttgaagaa ggcctgccag gtgacccagt 420

cgcggaacgc gaagaccatg accacatccc acctgaagca gtgcatcgag ctggagcagc 480

agtttgactt cttgaaggac ctggtggcat ctgttcccga catgcagggg gacggggaag 540

acaaccacat ggatggggac aagggcgccc gcaggggccg gaagccaggc agcggcggcc 600

ggaagaacgg tgggatggga acgaaaagca aggacaagaa gctgtccggg acagactcgg 660

agcaggagga tgaatctgag gacacagata ctgatgggga agaggagaca tcacaacccc 720

caccccaggc cagccacccc tctgcccact ttcagagccc cccgacaccc ttcctgccct 780

tcgcctctac tctgcctttg cccccagcgc ccccgggccc ctcagcacct gatgaagagg 840

acgaagaaga ttacgactcc tagcgccttc tgccccccag accatagccc cttttagttg 900

gttttagttg ctctgggggg aggagagaag gtagagctgt tcttaaattt attaaaaaaa 960

aaaataaaag ggaatctcag tgtctgttcc aggctctgcg caggaaaaaa aaaaaaaaaa 1020

aa 1022

<210> 10
<211> 2379
<212> DNA
<213> Homo sapiens

<400> 10
gggtgcacac ccggaagtgg gtgcgggcca gccggctcgc ccgggggcca tggcagcagc 60

ggctactgca gccgaggggg tccccagtcg gggcctcccc ggggaagtca ttcatctgaa 120

tgtgggaggc aagagattca gtacctctcg ccagactctc acctggatcc cagactcctt 180

cttctccagt cttctgagcg gacgcatctc gacgctgaaa gatgagaccg gagcaatctt 240

catcgacagg gaccctacag tcttcgcccc catcctcaac ttcctgcgca ccaaagagtt 300

ggatcccagg ggtgtccacg gttccagcct cctccatgaa gcccagttct atgggctcac 360

tcctctggtt cgtcgcctgc agcttcgaga ggagttggat cgatcttctt gtggaaacgt 420

cctcttcaat ggttacctgc cgccaccagt gttcccagtg aagcggcgga accggcacag 480

cctagtgggg cctcagcagc taggaggacg gccagcccct gtccgacgga gcaacacgat 540

gccccccaac cttggcaatg cagggctgct gggccgaatg ctggatgaga aaacccctcc 600

ctcaccctca ggacaacctg aggagccggg gatggtgcgc ctggtgtgtg gacaccataa 660

ttggatcgct gtggcctata cccagtttct agtctgctac aggttgaagg aagcctctgg 720

ctggcagctg gtgttttcca gcccccgcct ggactggccc atcgaacgac tggcgctcac 780

agcccgggtg catggtgggg ctttgggtga acatgacaag atggtggcag cagccaccgg 840

cagcgagatc ctgctatggg ctctgcaggc ggaaggcggt ggctccgaga taggggtctt 900

tcatctgggg gtgcctgtgg aggccttgtt cttcgtcggg aaccagctca ttgctacaag 960

ccacacaggg cgcatcgggg tgtggaatgc cgtcaccaag cactggcagg tccaggaggt 1020

gcagcccatc accagttatg acgcggcagg ctccttcctc ctcctgggct gcaacaacgg 1080

ctccatttac tacgtggatg tgcagaagtt ccccttgcgc atgaaagaca acgacctcct 1140

tgtcagcgag ctctatcggg acccagcgga ggatggggtc accgccctca gtgtctacct 1200

cacccccaag accagtgaca gtgggaactg gatcgagatc gcctatggca ccagctcagg 1260

gggcgtgcgg gtcatcgtgc agcacccgga gactgtgggc tcggggcctc agctcttcca 1320

gaccttcact gtgcaccgca gccctgtcac caagatcatg ctgtcggaga agcacctcat 1380

ctcagtctgt gccgacaaca accacgtgcg gacatggtct gtgactcgct tccgcggcat 1440

gatttccacc cagcccggct ccaccccact cgcttccttt aagatcctgg ctctggagtc 1500

ggcagatggg catggcggct gcagtgctgg caatgacatt ggcccctacg gtgagcggga 1560

cgaccagcaa gtgttcatcc agaaggtggt gcccagtgcc agccagctct tcgtgcgtct 1620

ctcatctact gggcagcggg tgtgctccgt gcgctccgtg gacggctcac ccacgacagc 1680

cttcacagtg ctggagtgcg agggctcccg gcggctcggc tctcggcccc ggcgctacct 1740

gctcactggc caggccaacg gcagcttggc catgtgggac ctaaccaccg ccatggacgg 1800

cctcggccag gcccctgcag gtggcctgac ggagcaagag ctgatggaac agctggaaca 1860

ctgtgagctg gccccgccgg ctccttcagc tccctcatgg ggctgtctcc ccagcccctc 1920

accccgcatc tccctcacca gcctccactc agcctccagc aacacctcct tgtctggcca 1980

ccgtgggagc ccaagccccc cgcaggctga ggcccggcgc cgtggtgggg gcagctttgt 2040

ggaacgctgc caggaactgg tgcggagtgg gccagacctc cgacggccac ccacaccagc 2100

cccgtggccc tccagcggtc tcggcactcc cctcacacct cccaagatga agctcaatga 2160

aacttccttt tgaacaacgc agctgccatg atgccttggg atgccctggt cctgggggac 2220

tcaggtgcct ccctgattcc tgtgggaacc ccgggttcag ggccagggcc tccttggaat 2280

aaatggttat tgttactagg tccccacctt ccctcttttc tggaagccaa agtcaccctc 2340

cccaataaag tcctcactgc caacaaaaaa aaaaaaaaa 2379

<210> 11
<211> 4406
<212> DNA
<213> Homo sapiens

<400> 11
cggtgcggag cctgtctcct tcactggatc ccgcattttc agcgcgttgc atcacctccg 60

tgcgcccggt tgcagcgtgg acgccggatg agttgctttt aggcttgctg gcccgcgggg 120

ctgtccaggc acgcgaggcc cctcagcaac aaaatgcttc aacaagttcc agaaaacata 180

aattttcctg ctgaagaaga gaaaatcttg gagttttgga ctgaatttaa ttgttttcag 240

gaatgcttaa agcaatcaaa acataaacca aaatttacct tctatgatgg tcctcctttt 300

gcaactggac tgcctcacta tggacatata cttgcgggta caattaaaga tatagttaca 360

agatatgctc accagagtgg gtttcatgtt gacagaagat ttggatggga ttgccatggc 420

ttacctgtgg aatatgaaat tgataagaca ctgggaatca gaggaccaga ggatgtggcc 480
aaaatgggga ttacagagta taacaatcag tgccgagcaa ttgtgatgag atattctgct 540
gagtggaagt ctactgttag cagacttggc cgatggattg actttgacaa tgactataaa 600
actctgtatc cacaattcat ggaatcagtc tggtgggtct tcaaacaact ctatgataaa 660
ggccttgttt atagaggtgt gaaagtcatg cccttctcta cggcatgtaa cactccactt 720
tccaacttcg agtcacacca gaattataag gatgttcaag atccttcagt atttgtaact 780
ttccctttgg aagaagatga aactgtatct ttagttgctt ggacaaccac tccctggact 840
ctacctagta accttgctgt gtgtgttaat ccagaaatgc aatatgtgaa aattaaagat 900
gttgccagag gacgattact cattttaatg gaagccagat tgtcagccct ctataaattg 960
gagagtgact atgagatcct tgaaagattt cctggtgcct atcttaaagg caagaagtac 1020
aggcccctgt ttgactattt cctgaagtgt aaagagaatg gcgctttcac tgtgcttgtt 1080
gacaactatg tgaaggaaga agaaggcaca ggggttgtcc accaagctcc ttacttcggt 1140
gctgaggact atcgggtctg tatggacttt aacattattc ggaaagactc actccctgtt 1200
tgccctgtgg atgcttcagg ctgcttcaca acggaggtga cagatttcgc aggacagtat 1260
gtgaaggatg ctgacaaaag tatcatcagg actttgaagg aacaaggccg acttctggtt 1320
gccaccacct tcactcacag ctaccctttt tgctggagat cagacactcc tctaatttac 1380
aaagcagtgc ccagctggtt tgtgcgagtg gagaacatgg tggaccagct cctaaggaac 1440
aatgacctgt gctactgggt cccagagttg gtacgagaaa aacgatttgg aaattggctg 1500
aaagatgcac gtgactggac aatttccaga aacagatact ggggcacccc catcccactg 1560
tgggtcagcg atgactttga ggaggtggta tgcattgggt cagtggcgga acttgaagaa 1620
ctgtcaggag caaagatctc agatctccac agagagagtg ttgaccacct gaccattcct 1680
tcacgctgtg ggaagggatc cttgcaccgc atctctgaag tgtttgactg ttggtttgag 1740
agtggcagca tgccctatgc tcaggttcat tacccgtttg aaaacaagag ggagtttgag 1800
gatgcttttc ctgcagattt cattgccgag ggcatcgacc aaaccagagg atggttttat 1860
accctgctgg tgctggccac ggccctcttt ggacaaccgc ctttcaagaa cgtaattgtg 1920
aatgggcttg tcctggcaag tgatggccaa aaaatgagca aacggaaaaa gaattatcca 1980
gatccagttt ccatcatcca gaagtatggt gctgatgccc tcagattata tctgattaac 2040
tcccctgtgg tgagagcaga aaacctccgc tttaaagaag agggtgtgcg ggacgtcctt 2100
aaggatgtac tgctcccatg gtacaatgcc tatcgcttct taatccagaa cgttctgagg 2160
ctccagaagg aggaagaaat agaatttctc tacaatgaga acacggttag agaaagcccc 2220
aacattacag accggtggat cctgtccttc atgcagtctc tcattggctt ctttgagact 2280
gaaatggcag cttataggct ttatactgtg gtgcctcgcc tggtcaagtt tgtagatatt 2340

ctgaccaatt ggtatgttag aatgaaccgc agaagattaa agggtgaaaa tgggatggag 2400

gattgtgtca tggccctaga aaccttgttt agtgttctgc tttctctttg cagacttatg 2460

gctccctaca caccttttct cactgaattg atgtaccaga atctaaaggt gctgattgac 2520

cctgtttctg ttcaggacaa ggacacactc agcattcact acctcatgct gccccgtgtt 2580

cgagaagaat tgattgacaa gaaaacagag agtgcagtat ctcagatgca gtctgtgatt 2640

gaacttggaa gagtgatcag agaccgaaaa actattccca taaagtatcc tttgaaagaa 2700

attgtggtta tccatcaaga tccagaagct cttaaagata tcaagtcttt ggagaagtat 2760

atcattgagg aactcaatgt tcgaaaagtt acactgtcta cagataaaaa caagtatggc 2820

attcggctaa gggcagaacc agatcacatg gtcctgggga agcgtctgaa gggagccttt 2880

aaggcagtga tgacgtccat caagcagttg agcagtgagg agctggagca gttccagaag 2940

actgggacca ttgttgtgga aggccatgaa ttgcacgatg aagacatccg cctcatgtac 3000

acctttgatc aggccacagg tgggactgcg caatttgaag cacactcaga tgctcaggct 3060

ttggtcctct tagatgtcac tcctgaccag tcaatggtag atgaaggaat ggctcgggaa 3120

gtcatcaatc gcatacagaa acttcgcaaa aagtgcaatc tggttccaac tgatgaaatc 3180

acagtgtact ataaagcaaa gtctgaagga acatatctga atagtgttat tgaaagccac 3240

acagagttca tatttaccac cataaaggct cccttgaaac catatccagt ttctccatcg 3300

gataaagtcc ttattcaaga aaaaacacag ttgaagggat ctgaactgga aattacactc 3360

accagaggat cttcccttcc tggtcctgct tgtgcatatg tcaatcttaa catttgtgca 3420

aatggcagtg aacaaggtgg agtattgctc ctggaaaatc caaaaggtga caataggttg 3480

gaccttttaa agctgaagag tgttgtcact agcatttttg gtgtgaaaaa tacagagctg 3540

gctgtcttcc atgatgaaac agaaatacaa aaccaaactg acttactgag tcttagtgga 3600

aaaacacttt gtgtgactgc aggatcggct ccctctctga tcaacagttc tagtactctt 3660

ctttgtcagt atatcaacct acagctcctg aatgcaaagc cacaagagtg tttaatgggg 3720

acagtgggca ctctcctgct tgaaaaccca cttgggcaga atggactcac ccaccaaggt 3780

cttctgtatg aagcagccaa ggtgtttggc cttcggagca ggaagctaaa gctgtttctg 3840

aatgagaccc aaacgcagga aattacagaa gacatccccg tgaagacttt gaatatgaag 3900

actgtgtatg tttctgtgtt accaacaaca gcagacttct agcatgtact tatcaatgtt 3960

gttcggtcag cccttcccta attacaccta tcccctacac atacatgcac atagacacac 4020

acatgaacac actgaagata tttccttcag gtgtgtgtaa aatatgctgc ttggattgaa 4080

attcaaatgg gattgattag tcaagtaact tgagacctca cagtaatctt cacacttaac 4140

cttagacacc tatgcagtca tgttgggagc aggttacaat gttacttcag cccacagttt 4200

atttctatac ttgagttctt aagtacagaa gatagaagtg atttaaatgg catagtatat 4260

atatcatttt ctggcctttt aaaatttatt tgagacctct tgatgaaatg gacatattat 4320

atatttctgc cacctggatt ttcctggata atttgatgga atattttaag tttcagtaaa 4380

tcagaacaat aaacaaactc agatat 4406

<210> 12
<211> 1316
<212> DNA
<213> Homo sapiens

<400> 12
ctcttcccat tggctgagaa gggtcatgag gatgaactgt gcagtcacct ggcaggaacc 60

ggaacccgcg gttataaagt aaaggaaccc gagatctgcg caggggttcc ctttgccgat 120

ttctctcacc tcacctttca aacctaaact cgagcctact gttcaccggc ctagcattgc 180

tctcgccatg gctctcagcg atgctgacgt gcaaaagcag ataaagcata tgatggcttt 240

cattgaacaa gaagccaatg agaaagcaga agaaatagat gcaaaggcag aagaagagtt 300

caacatagag aaaggtcggc ttgtgcaaac ccaaagacta aagattatgg aatattatga 360

gaagaaagag aaacagattg agcagcagaa gaaaattcag atgtccaatt tgatgaatca 420

agcgagactc aaagtcctca gagcaagaga tgaccttatc acaggtttgt accagttgct 480

ggagccccga atgattgttc gttgcaggaa acaagatttc cctctggtaa aggctgcagt 540

gcagaaggca attcctatgt acaaaattgc caccaaaaac gatgttgatg tccaaattga 600

ccaggagtcc tacctgcctg aagacatagc tggtggagtt gagatctata atggagatcg 660

taaaataaag gtttccaaca ccctggaaag ccggctggat ctcatagccc agcagatgat 720

gccagaagtc cggggagcct tgtttggtgc aaatgccaac aggaagtttt tggactaagc 780

cttcaggagg tggagctcgt cgtcagctct cctgctgtga tgtggaagct tctgatattt 840

gaagaaacac gaatgtctct gtagcttcct cttcactgcc ccagtattgc tctgtattta 900

tcagcgatgc ccctctgtca ctcatgcctt gcctaattgt tcacaatggt ggaaagcttc 960

atgtaatatg atcaggaccc acctccagtt cttctgaaag tgtgacagtg tccagccggt 1020

tctgcagcac taggggaggg ggcagatggt ggttgcatgg gcttcctggg tctccactct 1080

ccgtctggcc taaaggtgat gtatttggtg tttggccctg cagtccccac tcttgaggct 1140

taaggcgcat gtggcacacc actccttcca gcagtagtcg ctttactgtt acctgtttag 1200

gcctagaagt tttccctcat ctgtaaatgt gatttaaaat ctaagccatg aatatgcttt 1260

atttattaaa agagttatgc ggatttaatg tgatttctag tgtaaggcac tacaaa 1316

<210> 13
<211> 541
<212> DNA

<213>  Homo sapiens

<400>  13
agcgcggcgg tgatggcggg gccggtgaag gaccgcgagg ccttccagag gctcaacttc      60

ctgtaccagg ccgcccattg tgtccttgcc caggaccccg agaaccaggc gctggcgagg     120

ttttactgct acactgagag gaccattgcg aagcggctcg tcttgcggcg ggatccctcg     180

gtgaagagga ctctctgccg aggctgctct tccctcctcg tcccgggcct cacctgcacc     240

cagcgccaga gacgctgcag gggacagcgc tggaccgtac agacctgcct aacatgccag     300

cgcagccaac gcttcctcaa tgatcccggg catttactct ggggagacag gcctgaggcc     360

cagctcggga gccaagcaga ttccaaacca ctacaaccct tgccaaacac agcccactcc     420

atttcagacc gccttcctga ggagaaaatg cagactcagg gttccagtaa ccagtgatgg     480

attcacccca tctcccaaat aaagtttact tgttttacat tcaaaaaaaa aaaaaaaaaa     540

a                                                                     541


<210>  14
<211>  1651
<212>  DNA
<213>  Homo sapiens

<400>  14
attttgtgga gcgccagagc tgctaagtgc gtcagttgtg gagtggcgta gacgagttaa      60

gtcctggtct gcgtggaggt cgacgactcc gtcgcagact acggacctgt ctgggtctca     120

gccgccaaag accccgtccg gtaggtgagt ggctcacttt gagggcaagc cttctcggat     180

cgaggcttct tcatggccgc tcagatcgtg agcggccggg gctgctctct ttgcggagga     240

tggcgtctaa tgagcgcagt tgattcgagg aagtactagc cggacatcat gagtggctgt     300

cgggtattca tcgggagact aaatccagcg gccagggaga aggacgtgga aagattcttc     360

aagggatatg gacggataag agatattgat ctgaaaagag gctttggttt tgtggaattt     420

gaggatccaa gggatgcaga tgatgctgtg tatgagcttg atggaaaaga actctgtagt     480

gaaagggtta ctattgaaca tgctagggct cggtcacgag gtggaagagg tagaggacga     540

tactctgacc gttttagtag tcgcagacct cgaaatgata gacgaaatgc tccacctgta     600

agaacagaaa atcgtcttat agttgagaat ttatcctcaa gagtcagctg gcaggatctc     660

aaagatttca tgagacaagc tggggaagta acgtttgcgg atgcacaccg acctaaatta     720

aatgaagggg tggttgagtt tgcctcttat ggtgacttaa agaatgctat tgaaaaactt     780

tctggaaagg aaataaatgg gagaaaaata aaattaattg aaggcagcaa aaggcacagt     840

aggtcaagaa gcaggtctcg atcccggacc agaagttcct ctaggtctcg tagccgatcc     900

cgttcccgta gtcgcaaatc ttacagccgg tcaagaagca ggagcaggag ccggagccgg     960

agcaagtccc gttctgttag taggtctccc gtgcctgaga agagccagaa acgtggttct    1020

tcaagtagat ctaagtctcc agcatctgtg gatcgccaga ggtcccggtc ccgatcaagg 1080

tccagatcag ttgacagtgg caattaaact gtaaataact tgccctgggg gccttttttt 1140

aaaaaacaaa aaccacaaaa attcccaaac catacttgct aaaaattctg gtaagtatgt 1200

gcttttctgt gggggtggga tttggaaggg gggttgggtt gggctggata tctttgtaga 1260

tgtggaccac caaggggttg ttgaaaacta attgtattaa atgtcttttg ataagccttc 1320

tgctcacatt tttgtgaatg tctgaagtat atagtttgtg tatattgaca gagctctttt 1380

ataactaaag caaatttaat ttttttgtac tagaaaaaaa tttgaacatt ttagttcttg 1440

gttataaaaa tgttaattca gaattagttt aatgccttaa ttaaactaat taatagcttt 1500

ggacacttaa aagagctcta aatttgcttg tacataaagg cttaatttgt tttccttgtt 1560

agggtcaagg gtgtcctcca ctctttaaca gctgctggac agacacatta gagcagctgt 1620

ttgttattga taataaaata ttataaaact a 1651

<210> 15
<211> 5250
<212> DNA
<213> Homo sapiens

<400> 15
   atggcggccg cagtctctag tgtggtgaga cgagtggaag agctcgggga tctggctcag 60
   gcccacatac agcaacttag cgaagctgcc ggtgaagatg atcacttttt aattcgggcc 120
   tctgcagcat tagaaaaatt gaaactcctg tgtggagaag agaaagaatg ttcaaatcca 180
   tcaaatcttc tagaacttta cacacaggct attttggaca tgacatattt tgaggagaac 240
   aagctagtag atgaagattt tcctgaagac tcttcttcac agaaagtaaa agagctgatt 300
   agttttcttt cagaaccaga aattttagta aaggaaaata atatgcatcc aaaacactgc 360
   aatttgcttg gggatgagct actggaatgt ctctcttgga gacgaggagc cctgctgtat 420
   atgtattgtc attctcttac caaaagaaga gagtggctct taagaaaatc cagtttgctt 480
   aaaaagtacc ttcttgatgg aatcagttac ttgctacaga tgctaaatta tcgatgtcct 540
   atccagttaa atgaaggagt ttctttccaa gatctagaca cagctaaatt actgagtgca 600
   ggaatattta gtgacattca tctgctggct atgatgtaca gtggagaaat gtgttactgg 660
   ggatcgaagt attgtgctga tcagcaacca gaaaatcatg aagtggatac tagtgtgtct 720
   ggagcgggct gcactacata caaggaaccc ttggatttcc gagaagtagg agaaaaaatc 780
   ttgaaaaagt atgtatctgt gtgtgaagga cccctgaaag aacaagaatg gaatacaacg 840
   aatgcaaaac aaattttaaa cttctttcat catcgctgta actagtaagt tcatctagtc 900
   cttttcaaat agaaaaacaa caaaacccat gaaatggaaa agagagttcc aaaaaagaag 960
   acctgttgat actgaatatg agaacattac actgcataaa gatatccagc ttgggtaccc 1020
   tactaaaaca acatcaccat ctttttcatt atatattagt aaacctatga agggatgatt 1080
   ctttaatttc taaagtgttg ttcaaactca ccttaaaata cagatgttat gtcactaata 1140
   atgagtagta acccaattta gctttacaaa gattttttct attacttttt ttttttattg 1200
   tggtatatgt aacacaagtt taccattttt atcatcttta gattattttt aaatctcact 1260
   accttgaagt ttgccatgct tcagaaatta aagtcaagtt acttcactgc tttgaataca 1320
   tactatggat ataaatgtag gcaaaatgaa agattatgac taatattcag attccatttt 1380
   caataaggta aataatgaaa gatctcacaa aggaatcatt gtgtttaatt attttgccat 1440
   gtattactaa ttgtatttaa aatttctact tcccttaggc attttataaa aatttaaaaa 1500
   ttatttttct attttgttta aaagacaatg catggctttt caagtgagta atgaagataa 1560
   aaaccaagct aggttagtct accattagtt catatgtttt tgtagcacat cttttcataa 1620
   catactgaaa ttttaatgtt ttttgccttt aataataggg gtgagtgtta aaggccagag 1680
   agcctaactt gatatcttgc tcatggtctt tctgtctacc tcaactcctg ttgtcatctt 1740
   tggatgatct ttctgatacc ttgctgatac cttggacttc tgcccagtga ctttattaca 1800
   ttccagcagc ttactcccgt ggccgcactt tgaatgtcac caccttgagt ttctctttct 1860
   ggtaaaattt cagtaactga taactgccct cttttcagtt ttttcacttc cttattttat 1920
   tgacttttct ctcctttatg ctcattccac attcttcagt tccttcttac ccccttttct 1980
   tgactgctcc ccttcctcct aaactgtcag cccctttcag gctttcttcc ctatccaccc 2040
   tcagctttat gggatactat atgaactaat ctcaccagtt attcttcttt ccctggtctt 2100
   ttagtctagt cctttggcaa gccctcaacc tcagattaat cctatttatt atctaccttt 2160
   tcggggatgc tgagttcttt gtgatgggta gtccttcaaa gtagaatcac tacaagtgtc 2220
   tcacttcagc cttagaaaag ccctcaatgt aaaccttatg tttgttacta gggtggcctc 2280
   tctcccattt cccgctgtgg ctgagccaaa cttcattact ttccttaggc cctctgcccc 2340
   tcccctagca tatggctctt tctcctattg aacagaaaat tgagactatg aagaggtcaa 2400
   cttgtattta ccaactttac tcattttccc attttagagg aaaagaggtt gcctgcttcc 2460
   tatcaaaggc gactctgtgc tacatgttgg tttcatcttc gctgtctctc tcatttcccc 2520
   acaaagtgtt gtctcacttt ctgcctacct tttaggtatt gatcttcact atattttttt 2580
   ctctctctct cttttttttt tttactttac atattttata caatgtcatt cataactatg 2640
   gcttctttta ccaccagtaa taacagactg agtctcaaac ctgtaacttg tatcctgact 2700
   tatctcttgc tttctaaacc ttgtttctaa ctgtctatca aatatcttca ttagacatcc 2760
   cacagtaatt ttaagctcag tgtttccaaa cgtcaagtcc tcatctgtta tataaaatcc 2820
   tttctgcttt gtaagttttc tctttttggg attttccgca gatcagggca acacagaatg 2880
   actaccttcc cagccatgtc tctgcagtca tcctgtgtaa tcatgttcta gtccagtgtt 2940
   gctcaaacag ttaaaatcca ggctgctttt ggtaaacata caaattctga tatagagcct 3000
   ctgtggcaaa gggaatttct cagttcctgc cccagccccc ttgtttatca ttacacagct 3060
   aagattttta tttaacttag cattttagca agtttcttta tgaaaatacc tttgtgcttt 3120
   ctaagtataa agatttaaat tatttttaat acggttttta gaaccacaga ctatccctct 3180
   ttttagagca tcctgatcaa ttgcaagtac ttccacttct tccctaaatg cgctttgtgt 3240
   gttttccacc tcagtagcct tgcacacatc attacttctg catccttctt tgtgaccaag 3300
   tgaataccta cctacctctt aggactcagc ttcagtgatt ctgttttcat aggcttttta 3360
   aagccctttt cagtcaaatt agatgctcct ttgttctggt cctttactct tcccctccca 3420
   tgttgtcgca ctgatcacac tgctttgtaa ttattttctt tattgttggt aaactccata 3480
   ttttaaaaat ttacctgtat acatagtgct taatgcatgg taggccctta gtaatgtttt 3540
   tgaattaatg aggcatgatt ctaaatgcca aactttgcac aaaaagttgt tgctattgga 3600
   ttccaaactg ctatgccctc tataccagtg gctgacaagg catataggga tatatgccat 3660
   cctgagagga gtaaagtgtt tagattctga acctgagaaa agagaacttt ctcttaagaa 3720
   gttggaaact tcattttacc ttcgtgaccc tggtagacca cttggtctca ctggttggta 3780
   caattatgag ggtagtactg ggtgcttttt atacaacttt ccatccccca cacagttgga 3840
   gaataatttg tagaccatgg cagttaaaat gctttttact ctggtgtatt atagaataat 3900
   gaacacattt ttatatatgt taaagcctgc taaatcattc acttagaatg aagtaacctc 3960
   aaggtaccaa ataccccaca taaggaaagt actacactaa attcactcaa ctattgtatg 4020
   cctaccatat gccagatttt atcctagacc ctgaggatat agctttttaa aaccccctcc 4080
   tgtttcagag cttatattct agtggaggaa gttagacaag cattttaagt aataaatgta 4140
   tagagagtgt caagtgatga gtaccatgaa gaagaataaa tgagggtaag gggctagtgt 4200
   gatagggaga ggggtgggat gccatcatat ttaggggtgg ttgggaactg tctactgctt 4260
   tagcatttgt gtcttcaaat ttctctcctt tggttatata ccttgcgtat tccgcacatt 4320
   gataaagttt ctttcttaca gaagttctga tattgaatta aggaatgggt cactacttaa 4380
   gactttatca tttcagctac acataaaagg tttctctccc ctatggattt tgctaatggt 4440
   tgagtgatac ctaaaggcct tgttgcattt tttacacttg gggtttctct tcggtttgaa 4500
   ttctctcatg gttaccaaaa ctctgttaaa ggacttttca cagtcgttaa atgtgtatgg 4560
   ctctttccca atatgaattt ttttgatgtt aactaggctt ttagacttag ttaaagacct 4620
   gtgtgctctg ctgcatttat agggttttgg gctgctgaga ataattgatg ctgattaagg 4680
   tgcaaatcat gactaaagac ttttctttct tcttttgcat atagtagagt gaggtgaggg 4740
   tcgtggctaa aaccccacaa taactttaca ttcctagctt tacagtcttg ctgtgaattc 4800
   tctggtgttc attaaagtgt gagctgtgtt aaaaggcatt cttagattca ttttaaccac 4860
   atttttaaaa actcacagtt ggttaacatg atggtttcag attgcttgcc tcttttctct 4920
   ttctacctta cagggctcct tttctagctt aaataagggt actccatctg ccacagaatc 4980
   caggattcta tagtttttta gtttcacttc cctatgttct tttttttttt ttaattttta 5040
   acaatgagat gataccatgt tacagcggta ttacattaag atacatttaa tatagccaca 5100
   attaaagtat gtattatgta tttatgctgg tgttctttca tgttatttct tctagtgaaa 5160
   atttccaagt aaactgttat tgagcatata catttttaaa aaaataaaac catacacccc 5220
   taaaaaaaaa aaaaaaaaaa aaaaaaaaaa 5250

<210> 16
<211> 3113
<212> DNA
<213> Homo sapiens

<400> 16
cgctcagccc gggcgggcga tgcgggcggc gcgggcggcc ccctcccccg gcccgcgtct 60

ccgggacggc tgcgggcggc ccccccggcg gccggagggc tccctggccc cgatctgacg 120

gcggcggcgg cggcggccac agcggcggga gcggcgcggg gaaggagcag cggctcgcag 180

ccctcggccc gcgcccccac ccagcgccag cccgaggggg gaggcgcagc gccggagggt 240

ggcggtcctc ggccctccca ggtctccgcg ccgggaagcc gctccgagcc ggggctggag 300

ggttgttttg ccgttgtgtt gagcacgtca cccattaaga gccctttaaa gacctggatt 360

gattggaagg acaaaaatta aaagcaatct gatccagcct catgcaggat ccctgcggat 420

tttctcctta tcccatttcc atccactgtc acaatttgag aatctgcctg atttgatcag 480

attcacctcc aggggaggtg tgataccagg gttaggagga cgtgaagtta tgggcaactt 540

tctgatctgt ccatcagcag tctgagaaac gctggctctg aattttccgt gtcggccttt 600

tggaaacaac aagttcctcg ctgtttgcaa agcttcagtg ctcgggtccc tgggacaccc 660

cggccaccct cgcctggtag atgtggcatt tccatgctga ggccgcgagt cccgcctgac 720

cccgtcgctg cctctccagg gcttctctgg gccgcgcctc tgcagactgc gcagccatgc 780

tgcatctgct ggcgctcttc ctgcactgcc tccctctggc ctctggggac tatgacatct 840

gcaaatcctg ggtgaccaca gatgagggcc ccacctggga gttctacgcc tgccagccca 900

aggtgatgcg cctgaaggac tacgtcaagg tgaaggtgga gccctcaggc atcacatgtg 960

gagacccccc tgagaggttc tgctcccatg agaatcccta cctatgcagc aacgagtgtg 1020

acgcctccaa cccggacctg gcccacccgc ccaggctcat gttcgacaag gaggaggagg 1080

gcctggccac ctactggcag agcatcacct ggagccgcta ccccagcccg ctggaagcca 1140

acatcaccct ttcgtggaac aagaccgtgg agctgaccga cgacgtggtg atgaccttcg 1200

agtacggccg gcccacggtc atggtcctgg agaagtccct ggacaacggg cgcacctggc 1260

agccctacca gttctacgcc gaggactgca tggaggcctt cggtatgtcc gcccgccggg 1320

cccgcgacat gtcatcctcc agcgcgcacc gcgtgctctg caccgaggag tactcgcgct 1380

gggcaggctc caagaaggag aagcacgtgc gcttcgaggt gcgggaccgc ttcgccatct 1440

ttgccggccc cgacctgcgc aacatggaca acctctacac gcggctggag agcgccaagg 1500

gcctcaagga gttcttcacc ctcaccgacc tgcgcatgcg gctgctgcgc ccggcgctgg 1560

gcggcaccta tgtgcagcgg gagaacctct acaagtactt ctacgccatc tccaacatcg 1620

aggtcatcgg caggtgcaag tgcaacctgc acgccaacct gtgctccatg cgcgagggca 1680

gcctgcagtg cgagtgcgag cacaacacca ccggccccga ctgcggcaag tgcaagaaga 1740

atttccgcac ccggtcctgg cgggccggct cctacctgcc gctgccccat ggctctccca 1800

acgcctgtgc cactgcaggt tcctttggca actgcgaatg ctacggtcac tccaaccgct 1860

gcagctacat tgacttcctg aatgtggtga cctgcgtcag ctgcaagcac aacacgcgag 1920

gtcagcactg ccagcactgc cggctgggct actaccgcaa cggctcggca gagctggatg 1980

atgagaacgt ctgcattgag tgtaactgca accagatagg ctccgtgcac gaccggtgca 2040

acgagaccgg cttctgcgag tgccgcgagg gcgcggcggg ccccaagtgc gacgactgcc 2100

tccccacgca ctactggcgc cagggctgct accccaacgt gtgcgacgac gaccagctgc 2160

tgtgccagaa cggaggcacc tgcctgcaga accagcgctg cgcctgcccg cgcggctaca 2220

ccggcgtgcg ctgcgagcag ccccgctgcg accccgccga cgatgacggc ggtctggact 2280

gcgaccgcgc gcccggggcc gccccgcgcc ccgccaccct gctcggctgc ctgctgctgc 2340

tggggctggc cgcccgcctg ggccgctgag ccccgcccgg aggacgctcc ccgcacccgg 2400

aggccggggg tcccggggtc ccggggcggg gccggcgtcc gaggccgggc ggtgagaagg 2460

gtgcggcccg aggtgctccc aggtgctact cagcagggcc ccccgcccgg cccgcgctcc 2520

cgcccgcact gccctccccc cgcagcaggg gcgccttggg actccggtcc ccgcgcctgc 2580

gatttggttt cgtttttctt ttgtattatc cgccgcccag ttcctttttt gtctttctct 2640

ctctctcttt tttttttttt ttttctggcg gtgagccaga gggtcgggag aaacgctgct 2700

cgccccacac cccgtcctgc ctcccaccac acttacacac acgggactgt ggccgacacc 2760

ccctggcctg tgccaggctc acgggcggcg gcggaccccg acctccagtt gcctacaatt 2820

ccagtcgctg acttggtcct gttttctatt ctttattttt cctgcaaccc accagacccc 2880

aggcctcacc ggaggcccgg tgaccacgga actcaccgtc tgggggagga ggagagaagg 2940

aaggggtggg gggcctggaa acttcgttct gtagagaact atttttgttt gtattcactg 3000

tcccctgcaa gggggacggg gcgggagcac tggtcaccgc gggggccgat ggtggagaat 3060

ccgaggagta aagagtttgc tcactgctgc caaaaaaaaa aaaaaaaaaa aaa 3113

<210> 17
<211> 2525
<212> DNA
<213> Homo sapiens

<400> 17
aaagtttccg agtccattcc gggagcggga gcccatcttg ctggctgccg aggccctcgc 60

tggaggagga gggtcagaac tcgggtgcag ccaatcgagg gcaacgctgc tacttatcag 120

agcagaatgg gctgtagttt agtgaaatag gaaagctgca aaacactgtg gagtgctccc 180

gtgtaaataa aaagaggaaa aaagtttctc aagtcgccgc tgcacgacgt ctggccggcg 240

ctggagcggg ggtctgcgct ctcccgagcg gccgcgcgct ggactttatt gtgccgcaac 300

cagccccagt tcccattgtt tgtgtttttt tcaaaatatg gcaaaggttc aggtgaacaa 360

tgtagtggtg ctggataacc cttctccttt ctacaacccg ttccagttcg agatcacctt 420

cgagtgcatc gaggacctgt ctgaagactt ggaatggaaa attatctatg tgggctctgc 480

agaaagtgaa gaatacgatc aagttttaga ctctgtttta gtgggtcctg ttcccgcagg 540

aaggcatatg tttgtatttc aggctgatgc acctaatcca ggactcattc cagatgcaga 600

tgcagtaggc gtaactgttg tgctaattac ttgtacctat cgaggacaag aatttattag 660

agttggctat tatgtaaata atgaatatac tgagacagaa ttaaggaaaa atccaccagt 720

aaaaccagac ttttctaagc ttcaaaggaa tattttggca tctaatccca gggtcacaag 780

attccacatt aattgggaag ataacacaga aaaactggaa gatgcagaga gcagtaatcc 840

aaatctacag tcacttcttt caacagatgc attaccttca gcatcaaagg gatggtccac 900

atcagaaaac tcactaaatg tcatgttaga atcccacatg gactgcatgt gaccacctac 960

catcccttta gtacaaatta agctattaaa aatacacaga actatttccc tgaaattccg 1020

taagtacata gtcaaaacac aatgtgaaga atttgtttaa aaacatcctg tagaaagttt 1080

ataagaaaac cagtatttga acaaattgtg gaatataaat acaactattt ttaagtaatt 1140

tttttctcta atgtgttatt ttatttgttc tgaaactaat ctgattaaag catatatatt 1200

attttcttct cctttatatg taatgaaagc acttataaag aaacaggaat cattagacca 1260

ggttgtaaag atgtcttggc ctcccagaca gtctttggac cactatttta ctggcctttg 1320

aaagaacaaa gtttacttca actaaaatgt ttcttcctgt gaagacatat ggtatcattt 1380

taatttaagg ggcagatttc cattcttttt tggcatgtcc ttaaaaagag gatttgaaat 1440

caactatatg ctaccaagaa ctttaggatc caattttcca agccaccgtg aagcctctta 1500

tggctactat tatagttcat gagatgttgg acagcatttg gtttgtttgg taagagaaga 1560

acaaatatgg ctaattgtta ataaggtccc ctggctatgg tttttgttct tataacagag 1620

tttagaatat cagagcattt cttgaatcat acatcattat tgtccagtga attcaagacc 1680

aaatacaata tcgggagaaa atacaaactc cctgtgttta agaatagggg attacaatgg 1740

ctcaaattgg ttcatttgat ttctaaaaat accatgacct ttaaaaattc ttttaataga 1800

tcatgttatt ggcagtattt atataaaaac catggattta gaaaggtata tttagcttta 1860

ttataataga tacgttacta ttttggaaat atatatattt tcacacctgt agtactcttc 1920

cccatttcct ctgacactca tgcagaatga gatcaggcat atttgtggtt gacatactct 1980

agatagcttt tccaacaaat ctttgaaaag caatcttgga aaggaattca ttaactaaat 2040

ggggtaaaag gtttcttttt ctttgagagt taagtccttc ctcaaaaaat tttcttatgc 2100

tccataaaat tgaggtagaa tattttcatt attcttgcag ctaagcaaga cagccatatg 2160

atgcaggtta tgcagtgcct tcatatctaa aacttttata ctgcactttt taaagctttt 2220

atgttgagga aaggaaaagg gcatttgtct aaacatggat tctgagttgt atatatttcc 2280

tatcattcta aaaaagtgaa tttgtgaagc aaagttttgc cagatgttaa actttgaatt 2340

taatatacca gattattaaa atattgtcca ttaactagtt catagatttt aaaagtaaaa 2400

tacttctgac tgttaaaact atataaagaa aatctcattt gtctaattgc aattaaaaga 2460

aaaatgttaa aatattaaaa tgaaaataaa agcattactt tccaacaaaa aaaaaaaaaa 2520

aaaaa 2525

<210> 18
<211> 6682
<212> DNA
<213> Homo sapiens

<400> 18
tctccgcgga ggagcagcga aggcgacagc tctcttggcg cggctgcctg ggagccgggc 60

gcttgctggg tgggtgctcg ggcccggtgc tcccgctccc gccctgactg cgcgcctgtg 120

tgccgccggg gctgcccagc catgctgtgc tgcctgctgg tgagggccag caacctcccc 180

agtgcgaaga aggaccggcg cagcgaccct gtcgcaagcc tgactttccg aggggtgaag 240

aagagaacca aagtcatcaa gaacagcgtg aaccctgtat ggaatgaggg atttgaatgg 300

gacctcaagg gcatccccct ggaccagggc tctgagcttc atgtggtggt caaagaccat 360

gagacgatgg ggaggaacag gttcctgggg gaagccaagg tcccactccg agaggtcctc 420

gccaccccta gtctgtccgc cagcttcaat gcccccctgc tggacaccaa gaagcagccc 480

acaggggcct cgctggtcct gcaggtgtcc tacacaccgc tgcctggagc tgtgcccctg 540

ttcccgcccc ctactcctct ggagccctcc ccgactctgc ctgacctgga tgtagtggca 600

gacacaggag gagaggaaga cacagaggac cagggactca ctggagatga ggcggagcca 660

ttcctggatc aaagcggagg cccgggggct cccaccaccc caaggaaact accttcacgt 720

cctccgcccc actaccccgg gatcaaaaga aagcgaagtg cgcctacatc tagaaagctg 780
ctgtcagaca aaccgcagga tttccagatc agggtccagg tgatcgaggg gcgccagctg 840
ccgggggtga acatcaagcc tgtggtcaag gttaccgctg cagggcagac caagcggacg 900
cggatccaca agggaaacag cccactcttc aatgagactc ttttcttcaa cttgtttgac 960
tctcctgggg agctgtttga tgagcccatc tttatcacgg tggtagactc tcgttctctc 1020
aggacagatg ctctcctcgg ggagttccgg atggacgtgg gcaccattta cagagagccc 1080
cggcacgcct atctcaggaa gtggctgctg ctctcagacc ctgatgactt ctctgctggg 1140
gccagaggct acctgaaaac aagcctttgt gtgctggggc ctggggacga agcgcctctg 1200
gagagaaaag acccctctga agacaaggag gacattgaaa gcaacctgct ccggcccaca 1260
ggcgtagccc tgcgaggagc ccacttctgc ctgaaggtct tccgggccga ggacttgccg 1320
cagatggacg atgccgtgat ggacaacgtg aaacagatct ttggcttcga gagtaacaag 1380
aagaacttgg tggacccctt tgtggaggtc agctttgcgg ggaaaatgct gtgcagcaag 1440
atcttggaga agacggccaa ccctcagtgg aaccagaaca tcacactgcc tgccatgttt 1500
ccctccatgt gcgaaaaaat gaggattcgt atcatagact gggaccgcct gactcacaat 1560
gacatcgtgg ctaccaccta cctgagtatg tcgaaaatct ctgcccctgg aggagaaata 1620
gaagaggagc ctgcaggtgc tgtcaagcct tcgaaagcct cagacttgga tgactacctg 1680
ggcttcctcc ccacttttgg gccctgctac atcaacctct atggcagtcc cagagagttc 1740
acaggcttcc cagaccccta cacagagctc aacacaggca aggggaaggg tgtggcttat 1800
cgtggccggc ttctgctctc cctggagacc aagctggtgg agcacagtga acagaaggtg 1860
gaggaccttc ctgcggatga catcctccgg gtggagaagt accttaggag gcgcaagtac 1920
tccctgtttg cggccttcta ctcagccacc atgctgcagg atgtggatga tgccatccag 1980
tttgaggtca gcatcgggaa ctacggaaac aagttcgaca tgacctgcct gccgctggcc 2040
tccaccactc agtacagccg tgcagtcttt gacgggtgcc actactacta cctaccctgg 2100
ggtaacgtga aacctgtggt ggtgctgtca tcctactggg aggacatcag ccatagaatc 2160
gagactcaga accagctgct tgggattgct gaccggctgg aagctggcct ggagcaggtc 2220
cacctggccc tgaaggcgca gtgctccacg gaggacgtgg actcgctggt ggctcagctg 2280
acggatgagc tcatcgcagg ctgcagccag cctctgggtg acatccatga gacaccctct 2340
gccacccacc tggaccagta cctgtaccag ctgcgcaccc atcacctgag ccaaatcact 2400
gaggctgccc tggccctgaa gctcggccac agtgagctcc ctgcagctct ggagcaggcg 2460
gaggactggc tcctgcgtct gcgtgccctg gcagaggagc cccagaacag cctgccggac 2520
atcgtcatct ggatgctgca gggagacaag cgtgtggcat accagcgggt gcccgcccac 2580
caagtcctct tctcccggcg gggtgccaac tactgtggca agaattgtgg gaagctacag 2640

acaatctttc tgaaatatcc gatggagaag gtgcctggcg cccggatgcc agtgcagata 2700

cgggtcaagc tgtggtttgg gctctcagtg gatgagaagg agttcaacca gtttgctgag 2760

gggaagctgt ctgtctttgc tgaaacctat gagaacgaga ctaagttggc ccttgttggg 2820

aactggggca caacgggcct cacctacccc aagttttctg acgtcacggg caagatcaag 2880

ctacccaagg acagcttccg cccctcggcc ggctggacct gggctggaga ttggttcgtg 2940

tgtccggaga agactctgct ccatgacatg gacgccggtc acctgagctt cgtggaagag 3000

gtgtttgaga accagacccg gcttcccgga ggccagtgga tctacatgag tgacaactac 3060

accgatgtga acggggagaa ggtgcttccc aaggatgaca ttgagtgccc actgggctgg 3120

aagtgggaag atgaggaatg gtccacagac ctcaaccggg ctgtcgatga gcaaggctgg 3180

gagtatagca tcaccatccc cccggagcgg aagccgaagc actgggtccc tgctgagaag 3240

atgtactaca cacaccgacg gcggcgctgg gtgcgcctgc gcaggaggga tctcagccaa 3300

atggaagcac tgaaaaggca caggcaggcg gaggcggagg gcgagggctg ggagtacgcc 3360

tctctttttg gctggaagtt ccacctcgag taccgcaaga cagatgcctt ccgccgccgc 3420

cgctggcgcc gtcgcatgga gccactggag aagacggggc ctgcagctgt gtttgccctt 3480

gagggggccc tgggcggcgt gatggatgac aagagtgaag attccatgtc cgtctccacc 3540

ttgagcttcg gtgtgaacag acccacgatt tcctgcatat tcgactatgg gaaccgctac 3600

catctacgct gctacatgta ccaggcccgg gacctggctg cgatggacaa ggactctttt 3660

tctgatccct atgccatcgt ctccttcctg caccagagcc agaagacggt ggtggtgaag 3720

aacaccctta accccacctg ggaccagacg ctcatcttct acgagatcga gatctttggc 3780

gagccggcca cagttgctga gcaaccgccc agcattgtgg tggagctgta cgaccatgac 3840

acttatggtg cagacgagtt tatgggtcgc tgcatctgtc aaccgagtct ggaacggatg 3900

ccacggctgg cctggttccc actgacgagg ggcagccagc cgtcggggga gctgctggcc 3960

tcttttgagc tcatccagag agagaagccg gccatccacc atattcctgg ttttgaggtg 4020

caggagacat caaggatcct ggatgagtct gaggacacag acctgcccta cccaccaccc 4080

cagagggagg ccaacatcta catggttcct cagaacatca agccagcgct ccagcgtacc 4140

gccatcgaga tcctggcatg gggcctgcgg aacatgaaga gttaccagct ggccaacatc 4200

tcctccccca gcctcgtggt agagtgtggg ggccagacgg tgcagtcctg tgtcatcagg 4260

aacctccgga agaaccccaa ctttgacatc tgcaccctct tcatggaagt gatgctgccc 4320

agggaggagc tctactgccc ccccatcacc gtcaaggtca tcgataaccg ccagtttggc 4380

cgccggcctg tggtgggcca gtgtaccatc cgctccctgg agagcttcct gtgtgacccc 4440

tactcggcgg agagtccatc cccacagggt ggcccagacg atgtgagcct actcagtcct 4500

ggggaagacg tgctcatcga cattgatgac aaggagcccc tcatccccat ccaggaggaa 4560
gagttcatcg attggtggag caaattcttt gcctccatag gggagaggga aaagtgcggc 4620
tcctacctgg agaaggattt tgacaccctg aaggtctatg acacacagct ggagaatgtg 4680
gaggcctttg agggcctgtc tgacttttgt aacaccttca agctgtaccg gggcaagacg 4740
caggaggaga cagaagatcc atctgtgatt ggtgaattta agggcctctt caaaatttat 4800
cccctcccag aagacccagc catccccatg cccccaagac agttccacca gctggccgcc 4860
cagggacccc aggagtgctt ggtccgtatc tacattgtcc gagcatttgg cctgcagccc 4920
aaggacccca atggaaagtg tgatccttac atcaagatct ccatagggaa gaaatcagtg 4980
agtgaccagg ataactacat cccctgcacg ctggagcccg tatttggaaa gatgttcgag 5040
ctgacctgca ctctgcctct ggagaaggac ctaaagatca ctctctatga ctatgacctc 5100
ctctccaagg acgaaaagat cggtgagacg gtcgtcgacc tggagaacag gctgctgtcc 5160
aagtttgggg ctcgctgtgg actcccacag acctactgtg tctctggacc gaaccagtgg 5220
cgggaccagc tccgcccctc ccagctcctc cacctcttct gccagcagca tagagtcaag 5280
gcacctgtgt accggacaga ccgtgtaatg tttcaggata aagaatattc cattgaagag 5340
atagaggctg gcaggatccc aaacccacac ctgggcccag tggaggagcg tctggctctg 5400
catgtgcttc agcagcaggg cctggtcccg gagcacgtgg agtcacggcc cctctacagc 5460
cccctgcagc cagacatcga gcaggggaag ctgcagatgt gggtcgacct atttccgaag 5520
gccctggggc ggcctggacc tcccttcaac atcaccccac ggagagccag aaggtttttc 5580
ctgcgttgta ttatctggaa taccagagat gtgatcctgg atgacctgag cctcacgggg 5640
gagaagatga gcgacattta tgtgaaaggt tggatgattg gctttgaaga acacaagcaa 5700
aagacagacg tgcattatcg ttccctggga ggtgaaggca acttcaactg gaggttcatt 5760
ttccccttcg actacctgcc agctgagcaa gtctgtacca ttgccaagaa ggatgccttc 5820
tggaggctgg acaagactga gagcaaaatc ccagcacgag tggtgttcca gatctgggac 5880
aatgacaagt tctcctttga tgattttctg ggctccctgc agctcgatct caaccgcatg 5940
cccaagccag ccaagacagc caagaagtgc tccttggacc agctggatga tgctttccac 6000
ccagaatggt ttgtgtccct ttttgagcag aaaacagtga agggctggtg gccctgtgta 6060
gcagaagagg gtgagaagaa aatactggcg ggcaagctgg aaatgacctt ggagattgta 6120
gcagagagtg agcatgagga gcggcctgct ggccagggcc gggatgagcc caacatgaac 6180
cctaagcttg aggacccaag gcgccccgac acctccttcc tgtggtttac ctccccatac 6240
aagaccatga agttcatcct gtggcggcgt ttccggtggg ccatcatcct cttcatcatc 6300
ctcttcatcc tgctgctgtt cctggccatc ttcatctacg ccttcccgaa ctatgctgcc 6360
atgaagctgg tgaagccctt cagctgagga ctctcctgcc ctgtagaagg ggccgtgggg 6420

tcccctccag catgggactg gcctgcctcc tccgcccagc tcggcgagct cctccagacc 6480

tcctaggcct gattgtcctg ccagggtggg cagacagaca gatggaccgg cccacactcc 6540

cagagttgct aacatggagc tctgagatca ccccacttcc atcatttcct tctcccccaa 6600

cccaacgctt ttttggatca gctcagacat atttcagtat aaaacagttg gaaccacaaa 6660

aaaaaaaaaa aaaaaaaaaa aa 6682

<210> 19
<211> 2486
<212> DNA
<213> Homo sapiens

<400> 19
gacggcaaat ggcggacttc gacacctacg acgatcgggc ctacagcagc ttcggcggcg 60

gcagagggtc ccgcggcagt gctggtggcc atggttcccg tagccagaag gagttgccca 120

cagagccccc ctacacagca tacgtaggaa atctaccttt caatacggtt cagggcgaca 180

tagatgctat ctttaaggat ctcagcataa ggagtgtacg gctagtcaga gacaaagaca 240

cagataaatt taaaggattc tgctatgtag aattcgatga agtggattcc cttaaggaag 300

ccttgacata cgatggtgca ctgttgggcg atcggtcact tcgtgtggac attgcagaag 360

gcagaaaaca agataaaggt ggctttggat tcagaaaagg tggaccagat gacagaggct 420

tcagggatga cttcttaggg ggcaggggag gtagtcgccc aggcgaccgg cgaacaggcc 480

cccccatggg cagccgcttc agagatggcc ctcccctccg tggatccaac atggatttca 540

gagaacccac agaagaggaa agagcacaga gaccacgact ccagcttaaa cctcgaacag 600

tcgcgacgcc cctcaatcaa gtagccaatc ccaactctgc tatcttcggg ggtgccaggc 660

ctagagagga agtcgttcaa aaggagcaag aatgagcctg cggttgggag ggaatggggc 720

gtggggggtt agagcaggac cacagcctgg tgagtccccg ggcagccgtc ctgcagccgc 780

cactcctgcg cctgccattg gcctcctcac agcggaaaca cagcttgtga gtgcatgtca 840

gctgttaaca agtggttttt agtacattct gggctttgct gtatctatct agtgcctgtt 900

tgtgcgtttt tttctttctt ccgctgcttc cccattttcc ttctgtcctt tttctcctgc 960

tccttgtttt cccagcagca catggggttc ctcggaggag cagaggtggc cgccgtgggg 1020

gggcgtttgg gctgcggtgc tgcgtcattt ttcctttgct ttctctttac tttagacact 1080

ggcccaactc caggcgtttc ctttcattcc ctcagtgctt ctcttctgac ctgcatgttg 1140

agttctgtat tgctggggct tccaacaaaa accagagtca ctgacagagg gaacagcaga 1200

gaccttgttg gtattcagct gtgatggata tagagaatca gaggcacctt gttttcacaa 1260

ctaggataaa aatatctgca gggtcctttc cattcctatt tagagggagt cctggctcca 1320

tgaccccctc ccgagtggac tgtccaagca gataggctca cacgagaaac agtgaggctg 1380

aaaggggggg ctatggaaga gcggtaggga gtccacggag aagatgcagt gaatgcttgc 1440

atgcattcac acgtgtgtgt gtcccagcta gttcactcct ttcgccgtgc gtggtggagg 1500

ctggcctctc tggctgggtg cagtgaatgg ccagcgggtt tcttttctgc tgggccaagg 1560

cgctttgggg gtggaggggg tggtgctggt gctgcactgg gctgactgcg gcgctgacgc 1620

agcgtttccc cccatccctg ttgcctgtgt gttgtgtgga tctgttccta gtataggcaa 1680

cataatgaga tactgtgctt cccacctccc cttcagttca gagccaaaat gggtctagaa 1740

tctggcactt tactcatttc ctttgataaa ttgtactatg cagagctgtc aggaaccttc 1800

agatagcagt agaggactgc agctgtctag gtctgcggcc acatcttggg gacacactgg 1860

actgttccca tgtgcagggt tcagcagtta tgtgggagtg ctaggggtta ggcttttgag 1920

cttgaacgcc tgcgtgtgaa cagatgaaaa atccttcagt acccaagtcc cagtctgtcc 1980

tatggggagc agtttggggg cggccggcag caggagcctg ggaaagaggc cctcgccagg 2040

tgatggcagg gccagggtgg cctggggcac ccagcggaat gtgcttagta tttggtcacc 2100

agccgtcatc ctgggctttt cctactgtgt cttgttacaa ggcctcagca atccacagaa 2160

ctctctctcc ttccttccac ctgtcagctt ctctgcttct gagataagaa ccatttgtgt 2220

aacaccaaca cttaacttca gaaagacatg cattatgtgg tgtaatcaaa cccgatgctt 2280

tcagatgacc tacttacatc ttcaatgtgg ataagataaa gaacaaaaca catgcatcta 2340

aactgctggg caatccagtt gacttttaaa tgtaagaatg gaattccaaa cacttaacac 2400

attcagctat atgacagaaa gtaaatctat ggatatggta ttttgtgaat gatcttttaa 2460

ataaaagaaa accttacgta atattt 2486

<210> 20
<211> 2234
<212> DNA
<213> Homo sapiens

<400> 20
tggcgcctgc gctggacgac tcggccggta gtggagatgt ccggccggtc taagcgggag 60

tctcgcggtt ccactcgcgg gaagcgagag tctgagtcgc ggggcagctc cggtcgcgtc 120

aagcgggagc gagatcggga gcgggagcct gaggcggcga gctcccgggg cagccctgtg 180

cgcgtgaagc gggagttcga gccggcgagc gcgcgcgagg ccccggcttc tgttgtcccg 240

tttgtgcggg tgaagcggga gcgcgaggtc gatgaggact cggagcctga gcgggaggtg 300

cgagcaaaga atggccgagt ggattctgag gaccggagga gccgccactg cccgtacctg 360

gacaccatta acaggagtgt gctggacttt gactttgaga aactgtgttc tatctccctc 420

tcacacatca atgcttatgc ctgtctggtg tgtggcaagt actttcaagg ccggggtttg 480

aagtctcacg cctacattca cagtgtccag tttagccacc atgttttcct caacctccac 540

accctcaagt tttactgcct tccagacaac tatgagatca tcgattcctc attggaggat 600

atcacgtatg tgttgaagcc cactttcaca aagcagcaaa ttgcaaactt ggacaagcaa 660

gccaaattgt cccgggcata tgatggtacc acttacctgc cgggtattgt gggactgaat 720

aacataaagg ccaatgatta tgccaacgct gtccttcagg ctctatctaa tgttcctcct 780

ctccggaact actttctgga agaagacaat tataagaaca tcaaacgtcc tccaggggat 840

atcatgttct tgttggtcca gcgttttgga gagctgatga gaaagctctg gaaccctcga 900

aatttcaagg cacatgtgtc tccccatgag atgcttcagg cagttgtact ttgcagtaag 960

aagacttttc agatcaccaa acaaggagat ggcgttgact ttctgtcttg gtttctgaat 1020

gctctgcact cagctctggg gggcacaaag aagaaaaaga agactattgt gactgatgtt 1080

ttccaggggt ccatgaggat cttcactaaa aagcttcccc atcctgatct gccagcagaa 1140

gaaaaagagc agttgctcca taatgacgag taccaggaga caatggtgga gtccactttt 1200

atgtacctga cgctggacct tcctactgcc cccctctaca aggacgagaa ggagcagctc 1260

atcattcccc aagtgccact cttcaacatc ctggctaagt tcaatggcat cactgagaag 1320

gaatataaga cttacaagga gaactttctg aagcgcttcc agcttaccaa gttgcctcca 1380

tatctaatct tttgtatcaa gagattcact aagaacaact tctttgttga gaagaatcca 1440

actattgtca atttccctat tacaaatgtg gatctgagag aatacttgtc tgaagaagta 1500

caagcagtac acaagaatac cacctatgac ctcattgcca acatcgtgca tgacggcaag 1560

ccctccgagg gctcctaccg gatccacgtg cttcatcatg ggacaggcaa atggtatgaa 1620

ttacaagacc tccaggtgac tgacatcctt ccccagatga tcacactgtc agaggcttac 1680

attcagattt ggaagaggcg agataatgat gaaaccaacc agcagggggc ttgaaggagg 1740

cgtctagggc tttgctccca agggctgtgg ctgatgatgg taaataagaa cacagaagct 1800

gtagctgaac acaggctggc tggtgggctt cctaggccag cccagcttgt atgggttctg 1860

gctacaccag agcaccaaga gcccacttgc ctgggatggc cccacactgt cactcagctg 1920

ttctttgatc atttttttct agattgatgc tcctttctcc catgcattga gctcccatct 1980

agcttcagca gggcagaacc cttctccaga tgtgtgtaac ttatgtcttg agtatctggg 2040

agtagttgaa gaacagataa ttccttccaa acatcaagcc ttgggattct tggagcaagc 2100

agaaagccag taacttcgct ctgttagagg tggaggattt tcctatggtt ccccccattt 2160

cctgatttgt atttttagat ggattaaata gtctcctgtt tttaaaccaa aaaaaaaaaa 2220

aaaaaaaaaa aaaa 2234

<210> 21
<211> 2701
<212> DNA

<213>  Homo sapiens

<400>  21
gtgcctggag cgcgcgacag cggcggggcg gggcggcctg gaggctgtgg cgcgcggccg      60

gcagagggag gggagaggcc actggggccg tgttagtctg ccggtgggga ctcttgcagg     120

gccgtcccca tgttgcgttt tccgacctgt ttcccatcct tccgggtggt gggagagaag     180

cagctcccgc aggagattat tttcctggtc tggtcgccca agcgggatct cattgctttg     240

gccaacacag ctggcgaggt tttacttcat cgactggcaa gttttcatcg agtttggagt     300

tttccaccaa atgaaaatac aggaaaggag gtgacgtgtc tggcatggag accagatggc     360

aaacttttgg cctttgctct tgctgatacc aagaaaattg ttttgtgtga tgtagaaaaa     420

cctgagagct tacactcttt ttctgtggag gctccagttt cctgtatgca ttggatggaa     480

gtgacagtag aaagcagtgt tctcacatca ttttataatg ctgaggatga atcaaatctt     540

ctcttaccta aactacctac actgccaaaa aactatagca acacctcaaa aatatttagt     600

gaagaaaatt ctgatgaaat tattaagctc ttgggagacg tcaggcttaa tattctcgtc     660

cttggaggaa gctctggatt tattgagctt tatgcttatg gaatgtttaa aattgctcga     720

gtcacaggga ttgctggtac ttgtcttgca ttatgtttat caagtgattt gaaatcatta     780

tcagtggtca cagaagtctc taccaatggt gcttcagaag tttcatactt tcagcttgaa     840

actaatctgt tgtactcttt cttacctgaa gtaactcgga tggccagaaa gtttactcat     900

atttcagctc tgttacagta tataaatttg tcactaacat gtatgtgtga agcatgggaa     960

gaaatactaa tgcagatgga ttctcgtctc accaagtttg tgcaggaaaa gaacacaacc    1020

acatcagtgc aagatgagtt catgcacttg ctattatggg ggaaagcaag tgctgaactt    1080

cagactctct tgatgaatca gttaacagta aagggcttga aaaagcttgg ccagtctata    1140

gagtcatcat actccagtat acaaaaattg gtcataagtc atttacagag tggctcggag    1200

tctttattat accatttgag tgaattgaaa ggaatggctt catggaagca aaaatatgaa    1260

cctcttggac tagatgctgc aggaatcgaa gaagctataa ctgctgtggg ttcttttata    1320

ctcaaggcaa atgaacttct tcaagttata gatagtagta tgaaaaactt caaagcattt    1380

tttcggtggc tttatgtggc aatgttgaga atgacagaag accatgtgct tcccgagctg    1440

aataagatga ctcagaaaga tatcacattt gttgctgaat ttcttactga acatttcaat    1500

gaggctccag acctttataa tcgaaaagga aaatacttta acgttgaaag agttggtcag    1560

tacttgaaag atgaagatga tgatcttgtg tcacccccta acacagaagg aaaccagtgg    1620

tatgactttc ttcaaaatag cagccacctt aaagaaagtc ctttgctgtt tccttattat    1680

cctcgaaaat cattgcattt tgtgaaaagg cggatggaga atattattga tcagtgtttg    1740

caaaagccag cagatgtaat tggaaaatcg atgaatcaag caatctgtat tccattgtat    1800

agagatacca gaagtgagga ttctacacgt agattgttca aatttccttt tctgtggaat 1860

aataaaactt caaatctaca ttatcttctt tttactattc tagaagattc actttataaa 1920

atgtgcatct taaggagaca tactgatatt tctcaatctg tgagtaatgg actaattgct 1980

attaaatttg ggagctttac atatgccaca acagaaaaag tcagaagaag catctacagt 2040

tgtttagatg cacagtttta tgatgatgaa actgtaacag tagttcttaa agacactgta 2100

ggacgtgaag gaagagatag actcttggtc cagctgcctt tgtctttagt atataacagt 2160

gaagattctg cagaatatca gttcactggg acttattcta caaggctaga tgaacagtgt 2220

agtgctattc ccacccgtac catgcatttt gagaagcact ggagattact ggaaagtatg 2280

aaagcacagt atgttgctgg gaatggtttt cgaaaagtgt cctgtgtgtt aagctcaaat 2340

cttcgtcatg tgagagtatt tgaaatggac atagatgatg aatgggagct cgatgagtct 2400

tcagatgaag aggaggaggc cagtaataag cctgtaaaaa taaaggaaga agtgttgtcg 2460

gagtcagagg cagagaacca acaagctggt gctgccgctt tagctccaga gatagtcatt 2520

aaagtggaaa aacttgaccc tgagctagac tcctaatcta gcttgccatt attgtgtgtg 2580

taattatggc caaaaggaca taggagatgg actaagatgt cttggaccac ctttgtgtaa 2640

caaagaaata aacagtaaat tttatttttt caaaaaaaaa aaaaaaaaaa aaaaaaaaaa 2700

a 2701

<210> 22
<211> 2022
<212> DNA
<213> Homo sapiens

<400> 22
attccccaac ctgatagccc tccgcgacgc attacgcacc gcggacagct ggagaggccg 60

aggcgctctc gctttgattt cggcgcctcc gccctcgcgg ggagagattg gctgcggccg 120

cgggacgggg tagtgagcgc gtcacttcct gccgctgcca ggcgcgtcct cccgcgcgct 180

atgacggcca gcgcacagcc gcgcgggcgg cggccaggag tcggagtcgg agtcgtggtg 240

accagctgca agcatccgcg ttgcgtcctc ctggggaaga ggaaaggctc ggttggagct 300

ggcagtttcc aactccctgg aggtcatctg gagttcggtg aaacctggga agaatgtgct 360

caaagggaaa cctgggaaga agcagctctt cacctgaaaa atgttcactt tgcctcagtt 420

gtgaattctt tcattgagaa ggagaattac cattatgtta ctatattaat gaaaggagaa 480

gtggatgtga ctcatgattc agaaccaaag aatgtagagc ctgaaaaaaa tgaaagttgg 540

gagtgggttc cttgggaaga actacctccc ctggaccagc ttttctgggg actgcgttgt 600

ttaaaagaac aaggctatga tccatttaaa gaagatctga accatctggt gggatacaaa 660

ggaaatcatc tctaggtggc cgagaagatt tgattttctt taaaaagaca agaataaggt 720

ctggttaggg aatgaaaaat gtatacattt cggaacaact ccattttatc taaaaaagtt 780

cttgtgattg ccagtttatt tgcagtctct taatgtatcc cccactcttt cagccagtac 840

ttgagaaaat ttttctgaaa tatgtcattg aattgtattc cagacacaga atacatgata 900

aatactgata ttatgggtaa tctgctttcc atatttacct atgatattta ctgtgcagtt 960

tgtcattact agcttgcatg gagtaggatg cagtcaaatt tgccttagtg tttctgttca 1020

aatagagacc tgaattcaaa tattgtagtt taggttcaaa cagagtatgg ctgttgcaaa 1080

atttaccaaa tttgttgatt acctctttta ttaaagaaat gttggggaga gggtaatata 1140

ttgtgacaat ttttgcaact tacagggaat aggacaggtc attagggtgt atttcccagg 1200

tttccaattg agaatctaaa ccaagagaag tgagacaaac catctaatat ctgtatcatg 1260

ctattaatag gctgtgatgt tgagtttctc acttactctt tctgaggtcc agggtcctta 1320

tatgaagaga ttcatatata tgaagagatt cgtctagagc agtggttgtc aaagtgtggt 1380

ctgggggggc ctcaagagcc tttcaagggt attctaggtc aaagctgttt ttataacact 1440

aagacattat ttcactattt atagcttttc ctttttttta tttgagaagg agtcttgctc 1500

tgttgcccac attggagtgc agtggcacga tctcagctca ttgcagcctc cgccacctgg 1560

gttcaagcaa ttctcctacc tcagcttccc gagtagctgg gattataggc acccaccacc 1620

acacctggct aatttttgta tttttagtgg agacggggtt tcaccatgtt ggccaggctg 1680

gtctggaact cctgagctca agtgatctgc ccatctcagc ctcccaaagt gctgggatta 1740

caggtatgag ccacaacgcc cagccatcat ttatcttttt cactcatttt ttcatgagta 1800

tgcagaggag ttttcaagag gctatctggt gtgatattgt aataggctga atgcaaaagc 1860

agatacaaga ttccagcttt cgtctgtcat cagacattga agagatttgg agaaatgtaa 1920

agcagcagta ctcttctcac taatttttat tgtggttagt tagaaaaatg gttattttta 1980

gtgggtttta atatgtgaaa aaaataaaat tagctatttg cc 2022

<210> 23
<211> 1278
<212> DNA
<213> Homo sapiens

<400> 23
gctgaggggc agcggcttag gctccggcgt ctgcaggggt cgccgagcta acccgtggct 60

aggcgagtgg ggcggggcgg ccggcaccat gtcgaggcag gcgaaccgtg gcaccgagag 120

caagaaaatg agctctgagc tcttcaccct gacctatggt gccctggtca cccagctatg 180

taaggactat gaaaatgatg aagatgtgaa taaacagctg gacaaaatgg gctttaacat 240

tggagtccgg ctgattgaag atttcttggc tcggtcaaat gttgggaggt gccatgactt 300

tcgggaaact gcggatgtca ttgccaaggt ggcgttcaag atgtacttgg gcatcactcc 360

aagcattact aattggagcc cagctggtga tgaattctcc ctcattttgg aaaataaccc 420

cttggtggac tttgtggaac ttcctgataa ccactcatcc cttatttatt ccaatctctt 480

gtgtggggtg ttgcggggag ctttggagat ggtccagatg gctgtggagg ccaagtttgt 540

ccaggacacc ctgaaaggag acggtgtgac agaaatccgg atgagattca tcaggcggat 600

tgaggacaat cttccagctg gagaggaata accatcccta caactcgagg atagccatca 660

ggagcactgt tggaatcagc aggcctctgt gctccctctg ccctccagaa ctcagtgact 720

cttgaacatg gatgttatat attcttataa cctgtttcca ttctccattc aaataaagag 780

cagactgcga tatagtccat ttaccccatg tgtgcacatt caggagcgac agtctctgcc 840

cccattccct tgagaggggc tggatgtaat cacctttggt tggactagaa agagctcaaa 900

ccattttaca ttcctgtttg aatttttcca aagcaaaact cactttgacc ccattaagag 960

gcaagcctgg cacatctatc cctgggcctt tagaaagcca tttgcctcaa atggctatag 1020

ggttgtgggg tggagggagg aagggctggg agggagtggg gaggaattgc tagctgtagt 1080

gtgacacatt gtagtgtttg ccaggaaagg agccagtcat gccggaaaca ctgacttctg 1140

ggaagccacc caggtctcat tcctccctgc tgttggaggc aacatctcct ctttttacag 1200

agggtacatc cttttttctt acaaattctt caataaagac acattcttga gtgaaatccc 1260

aaaaaaaaaa aaaaaaaa 1278

<210> 24
<211> 2880
<212> DNA
<213> Homo sapiens

<400> 24
gtaggccatg gagccgagca gatccgggtc ttcgggtggc cgcaatgtcc caagtggacg 60

acacccacct agtcccgagg acaaccgaca aagtccttaa tgggcccaga aggaggacgg 120

agtcccagag aactacaact cccatcaagc acttggggga tcgccgcggc tgagggagag 180

actatggctt aggaaccacc attcgcgtga ggctctgctc cccgcgcctg cgcagagtgc 240

agggccacgt cgcttttgct gtaccgggga ccacgcgtct catccatggc ttccgcggac 300

tcgcgccggg tggcagatgg cggcggtgcc gggggcacct tccagcccta cctagacacc 360

ttgcggcagg agctgcagca gacggaccca acgctgttgt cagtagtggt ggcggttctt 420

gcggtgctgc tgacgctagt cttctggaag ttaatccgga gcagaaggag cagtcagaga 480

gctgttcttc ttgttggcct ttgtgattcc gggaaaacgt tgctctttgt caggttgtta 540

acaggccttt atagagacac tcagacgtcc attactgaca gctgtgctgt atacagagtc 600

aacaataaca ggggcaatag tctgaccttg attgaccttc ccggccatga gagtttgagg 660

cttcagttct tagagcggtt taagtcttca gccagggcta ttgtgtttgt tgtggatagt 720

gcagcattcc agcgagaggt gaaagatgtg gctgagtttc tgtatcaagt cctcattgac 780

agtatgggtc tgaagaatac accatcattc ttaatagcct gcaataagca agatattgca 840

atggcaaaat cagcaaagtt aattcaacag cagctggaga aagaactcaa caccttacga 900

gttacccgtt ctgctgcccc cagcacactg gacagttcca gcactgcccc tgctcagctg 960

gggaagaaag gcaaagagtt tgaattctca cagttgcccc tcaaagtgga gttcctggag 1020

tgcagtgcca agggtggaag aggggacgtg ggctctgctg acatccagga cttggagaaa 1080

tggctggcta aaattgcctg agaggcagct ctaaagcaca agacctggat gtgtgacaca 1140

cagttttgga aaaaggtctg tggtagtctg gagttgatga ggaaggggta caagatgtgg 1200

ttagaaacat ttctttgttc tggaaacaaa gtactgttga aaccagcttg gaattttttt 1260

tttttttttt tttaagttca gttctccctt atggctgcct ttcaaacaag taccttttat 1320

ctgatgcctg tatcttccct ttgttaaggt gtaacttgat gtagggtcaa ggtttttgtg 1380

acaacaggca gactccacac agagaggata tgatgagaat atggccatca cctgaaaagt 1440

tttcttatct tctgtgcttt tggtccctgg aaacaaatcc gcctatgtat gaagctagtt 1500

gatttccagt tgcactattt ccagttgcct ctgaagttca caggcaatac attgtctagt 1560

cctttgcgaa tttctctgat ttgtgggcac agttatgaag tttccccaca tgtgaagaca 1620

ggtacaaaat agcagagcca agcagacagt gggtctattc ttcattagct cagtgacttg 1680

tccacactcg tcttagcact tacgtttcaa aagcttgtca caaacccttg gagtcattcc 1740

cagataatag aactggaaat gataaatccc ctaatgccaa gggtctagtg tgttcttagt 1800

ggttatactg ggaagtgtgt ggagatttag gtgctgctct gctgctctgg atggctgaag 1860

gctcctgggc catcttcatg tgctgcttga agagctccta ttttgtactc ctggctagaa 1920

tgctgtggaa caaatacaaa gtgaaaaaag ttctctgtag atttctgaag tgcatattca 1980

ttgatgccaa gaaaaaaaaa aagttgcctt tttgaagtga tgttttttgc tgtcttctta 2040

aacacaaggc ttttttgaat gattagtata tttcatggta aagaaaacag cctgtctggc 2100

tcaaagcaat taaatagaat gtaatggtga gtacaaatga gtgcacatgt caggactcag 2160

gtctaactcc ttgtctcctg agcctaaaga ttgcaacata cacaagaaca cactcctatt 2220

cctaccccac acactcaggg acaagcccaa ctaaagctta caaggagacc agggtggctc 2280

tgtccagggg agaagccagt tatggaacag tgcattgaga gccatggtag gagaggccca 2340

cagttctctg gagcatgcag caggggcacc ccacctggcc ttgaggatca gggggagtca 2400

aaggataaag catggggctg atgacgtctg agggagtgtg atcctccatg tatggcctct 2460

gcctgctgtc tcacatgtcc cttctggtgg tcacttgggc tctaggagta tacgtcacct 2520

cagaccatct ggcagaaata ctccaggctc ctaccccaaa gcacatgtca gccttgctgc 2580

tggagcacga agacaatgta aatgaaacat gaaatggagg agttgtgaga ccctgaccct 2640

gagtccttac ttgaaagctg ctgctggtgt tctgagtgtc ttttggactc ttatttcttg 2700

cccttttcct tattaggcaa gcagtaactt aggaagtagg taagagcaat aaatgtgaca 2760

tgttatgtca tcatagtagg agctcatggg aataaaagtc agtggcttga tgcttctgtt 2820

agaggcaaaa aaaaaaaaaa aaaaaaaaaa aaaaaaaaaa aaaaaaaaaa aaaaaaaaaa 2880

<210> 25
<211> 3101
<212> DNA
<213> Homo sapiens

<400> 25
acactctggc ccggttctcg gtggtgcggg agcgggcggg agcagcggcc gctctggtcg 60

gcggacgtgc tgccgagtag tcccggaagc gaagcagcga tggcggagag tccgactgag 120

gaggcggcaa cggcgggcgc cggggcggcg ggccccgggg cgagcagcgt tgctggtgtt 180

gttggcgtta gcggcagcgg cggcgggttc gggccgcctt tcctgccgga tgtgtgggcg 240

gcggcggcgg cagcgggcgg ggccgggggc ccggggagcg gcctggctcc gctgcccggg 300

ctcccgccct cagccgctgc ccacggggcc gcgctgctta gccactggga ccccacgctc 360

agctccgact gggacggcga gcgcaccgcg ccgcagtgtc tactccggat caagcgggat 420

atcatgtcca tttataagga gcctcctcca ggaatgttcg ttgtacctga tactgttgac 480

atgactaaga ttcatgcatt gatcacaggc ccatttgaca ctccttatga agggggtttc 540

ttcctgttcg tgtttcggtg tccgcccgac tatcccatcc acccacctcg ggtcaaactg 600

atgacaacgg gcaataacac agtgaggttt aaccccaact tctaccgcaa tgggaaagtc 660

tgcttgagta ttctaggtac atggactgga cctgcctgga gcccagccca gagcatctcc 720

tcagtgctca tctctatcca gtccctgatg actgagaacc cctatcacaa tgagcccggc 780

tttgaacagg agagacatcc aggagacagc aaaaactata atgaatgtat ccggcacgag 840

accatcagag ttgcagtctg tgacatgatg gaaggaaagt gtccctgtcc tgaaccccta 900

cgaggggtga tggagaagtc ctttctggag tattacgact tctacgaggt ggcctgcaaa 960

gatcgcctgc accttcaagg ccaaactatg caggaccctt ttggagagaa gcggggccac 1020

tttgactacc agtccctctt gatgcgcctg ggactgatac gtcagaaagt gctggagagg 1080

ctccataatg agaatgcaga aatggactct gatagcagtt catctgggac agagacagac 1140

cttcatggga gcctgagggt ttagaccctg ctcccatctc cccttccccc actcaagagt 1200

cccagcagaa tcccttcccc ccaccccagg gatggagagg cactgtgtat ctccctccag 1260

actcgaagtc atcctgcaag atggcaagaa ccaagcaagc tccgatccca gggtgtggga 1320

gtgggggcct gttcccggtc tgacctcctt ggcactggag catctggggc ttcgttcatc 1380

cattcatccc gtatcagggg ccaaggtacc tttacaggag cacctagagc gagggccttt 1440

ggcaaaaaca aaacaaccaa cacacctctc cacagggcca gctccttagg gataagtgga 1500

agatggaaat tgcaattcca agagggagtg tgcccaaatg atttatgggg atacctggaa 1560

gggagcttgg ggtgggggct gtctgtgaca cttaagcagt ctgggtggtt gtctatttgt 1620

ctgtcttcag tcttgaagca gggcttccca atgccctttt cctccctgcc ttccttcccc 1680

cattatttcc cacaggccag cataattttg tttttcctaa tttatagtca ctgttctaga 1740

cagaccaaag agaaggaaca gtggtggagt ctaggctgct gatcagtaag ctttacctag 1800

cacctgagca cctttctccc ctcccctctt tcctcaccct tttctagatg taagacagaa 1860

agtaaatgtg actgggactt aaccaaggtc ttggtaaagc ctgcatggca ccgtaagaag 1920

ctgaaaatac tgtttgttcc cgcaatcact gatttgaaaa gttcccaaca caggcagctg 1980

ctgtgtatat gggattagag ccactacata gaatagtctc ttacagattt tcataaatac 2040

tagtcacaat aagggtattt ttcttggggg tggagtaagg gggagactga tgctagtcct 2100

tgttgtattt tgttgggctg tccttgtgta ttttcacccc agcctgtagt cctcctcact 2160

tcaaccccag ggatttttgg ggagcaaggg tagccaatgg cagagggggt tggggctggg 2220

actctggagg ctcctcccct tctttctctt ccttccgcct cccccgtgcc cccagctgct 2280

cttgtcactg tctctgatgg gtatttgcct ggctttgttg cttctctatc tgtatttagc 2340

tgcagtgatc ctttagctgg ttggctcaga aaaaaaaaaa tgtgctttag gtgccctgta 2400

atcctgggca tcaagggaat ccatccttcc cctttttgat atgttctccc cgtacttcca 2460

gatttattgt tatggctccc agtgggtatt ggcgattctt gtgatgcagg gcctcagtca 2520

gtgtccagcc atgcataagg gagaggatag tgtgtacctg ccctgccctc tgctatgaag 2580

gtctctgcct tgtggatcat gggactcccc ttggaggatc tgtgcaaagg ggggctgggc 2640

acaaaggaga atgtcctatt tgggagggca ggaagcaaag gaactggaca gggattggtg 2700

ggcttgggga acggaagttt atcttggata cccttgaaga ggctgggtct cttcacatga 2760

agatcgaaaa gggaccctgc ttccaatttc cctcttccat tcctcgagct actccagggc 2820

ttagaagaat gctcttggtc tgtgggtcca gtgttgtctg tcatccattt aagtgttccc 2880

actttcaagt gacaatcctc tccttggccc tgccataggg cagagcatgt ctggcatagc 2940

agcctgactt ttatgcccta atcttgagtt gaggaaatat atgcacagga gtcaaagaga 3000

tgtctttata tctgactgta tataaatgaa gtttttttgt tttttttgtt ttcctttttg 3060

gtgcaataaa gtttgttttg gcagaaggag aaaaaaaaaa a 3101

<210> 26
<211> 2213
<212> DNA
<213> Homo sapiens

<400> 26
ataggcctcc tcctggcgcg cctccgcacc gcattggtgg accggccccc agcccgtcca 60

tgggcgttgt ggccgccacc gcaggttccg cctcccgttc cctccgccct caacgtacgt 120

cgcaccgcct ctctgtagcc gcccgcggag catcgcagcc ggcccgggcc cccgccagcc 180

tccctcctcg cgtccctcgg tgtcctccgc gggccggcgc gatgcggctg ggcccgagga 240

ccgcggcgtt ggggctgctg ctgctgtgcg ccgccgcggc cggcgccggc aaggccgagg 300

agctgcacta cccgctgggc gagcgccgca gcgactacga ccgcgaggcg ctgctgggcg 360

tccaggaaga tgtggatgaa tatgttaaac tcggccacga agagcagcaa aaaagactgc 420

aggcgatcat aaagaaaatc gacttggact cagatggctt tctcactgaa agtgaactca 480

gttcatggat tcagatgtct tttaagcatt atgctatgca agaagcaaaa caacagtttg 540

ttgaatatga taaaaacagt gatgatactg tgacttggga tgaatataac attcagatgt 600

atgatcgtgt gattgacttt gatgagaaca ctgctctgga tgatgcagaa gaggagtcct 660

ttaggaagct tcacttaaag gacaagaagc gatttgaaaa agctaaccag gattcaggtc 720

ccggtttgag tcttgaagaa tttattgctt ttgagcatcc tgaagaagtt gattatatga 780

cggaatttgt cattcaagaa gctttagaag aacatgacaa aaatggtgat ggatttgtta 840

gtttggaaga atttcttggt gattacaggt gggatccaac tgcaaatgaa gatccagaat 900

ggatacttgt tgagaaagac agattcgtga atgattatga caaagataac gatggcaggc 960

ttgatcccca agagctgtta ccttgggtag tacctaataa tcagggcatt gcacaagagg 1020

aggcgcttca tctaattgat gaaatggatt tgaatggtga caaaaagctc tctgaagaag 1080

agattctgga aaacccggac ttgtttctca ccagtgaagc cacagattat ggcagacagc 1140

tccatgatga ctatttctat catgatgagc tttaatctct gagcctgtct cagtagagta 1200

ctggctcctt ttataatttg ttaccagctt tacttttgtg ataaaatatt gatgttgtat 1260

tttacactct taagtcttaa ccacagtcag aattatctta atgtagatta taattttggt 1320

cttttaggaa aaaaaaacaa aaatctgata tttatttcaa aatgtattga agcaacaaaa 1380

tattaatatt gtgccatatg acaacaaagt ctttcctaaa tactccatct gtttagtact 1440

gtattgtgga atatttgagt tctatttcca tacttgaaaa catggaggat tttagagatg 1500

cctgaacaat attatttaag tagtatgtga ccgagctata aatttttgtt tttgttctaa 1560

gtagatttaa tttgggaact gacaggacaa tgtttttagg tttagcattt tgtttaaaaa 1620

cctttaaaga aacctttaga aggacttaga cctcacatat taatgttgag aagttctgct 1680

taattttaaa atggtttcta taaagggttt tattgtatga aatagaactt tatatttttg 1740

catatgtata gatagtaatt atatttaatg tataactata gcattatggt gagtggaatt 1800

tgacattgtc caaacctttt tcatttttga gtgattaaaa atgaaatgtc ctttgtatat 1860

gtttgtttgc ttttctttaa ctttcagata aatattgaat ttagcatagg ttttgtggta 1920

ttgtacatta tcttgcctca tccattcctt tatgtggata gttcccaaaa ctgtcaaatt 1980

attcttgctg cattttctta tttttttttt aaataagaga atcatgttat aatataattg 2040

aatgtgcacc tgacacattt taataattgg tgttgtagca aatgtggttt tgctctttgg 2100

ggctataaat aatcttgagt aacgcaggtt ggaattacca atagagctgg tcagttatca 2160

acagtagagc tcagtatcaa ttttactgaa attaaaatca tacttgataa gca 2213

<210> 27
<211> 1097
<212> DNA
<213> Homo sapiens

<400> 27
   ctctgtcctc attgcgccca gacgggccgg cccagagctc ccgggtcgtc tttcgtgtgg 60
   ccgcgagaca ctcttgcact cctgtaatga gcctggcact gtgatgaaac acttttcccg 120
   tgtcgtttga gtgcatcttc tcaacaaccc taggagggtt cttgaagctt ttgagattaa 180
   caatggcagg aaaatcatca ctttttaaag taattctcct tggagatggt ggagttggga 240
   agagttcact tatgaacaga tatgtaacta ataagtttga tacccagctc ttccatacaa 300
   taggtgtgga atttttaaat aaagatttgg aagtggatgg acattttgtt accatgcaga 360
   tttgggacac ggcaggtcag gagcgattcc gaagcctgag gacaccattt tacagaggtt 420
   ctgactgctg cctgcttact tttagtgtcg atgattcaca aagcttccag aacttaagta 480
   actggaagaa agaattcata tattatgcag atgtgaaaga gcctgagagc tttccttttg 540
   tgattctggg taacaagatt gacataagcg aacggcaggt gtctacagaa gaagcccaag 600
   cttggtgcag ggacaacggc gactatcctt attttgaaac aagtgcaaaa gatgccacaa 660
   atgtggcagc agcctttgag gaagcggttc gaagagttct tgctaccgag gataggtcag 720
   atcatttgat tcagacagac acagtcaatc ttcaccgaaa gcccaagcct agctcatctt 780
   gctgttgatt gttagattgt tgatgcattc taaccaactc acacatatac acaaaatcaa 840
   catggggatg gagaagagaa ttagcgtttg cagcagtgta tcatctacta ataaaattaa 900
   actaatgttg ctgcttcatt agttggtggg agaagggaca catccactct tggaggaata 960
   tatttactca ataatggcac cttacattta taaattgtaa cagttgtcta ataacgtttc 1020
   tttaatttaa atatgtaagt tgcagagcta ataaatgaaa tgaccaagac tttaattata 1080
   aaaaaaaaaa aaaaaaa 1097

<210> 28
<211> 1495
<212> DNA
<213> Homo sapiens

<400>  28
gcttcttccc tggtaggttc cggaagagcc gcgcactcct tgggcgttaa gggttcgcgc      60

gccgcagggt cgtttcagcc gagcacttgg cgtcccctcg agctcgagat ctgtgaacag     120

ccaccatgtc gatcttcacc cccaccaacc agatccgact gaccaatgtg gccgtggtgc     180

ggatgaagcg gggagggaag cgcttcgaaa tcgcctgcta taaaaacaag gtcgtcggct     240

ggcggagtgg cgtggaaaaa gaccttgatg aagttctgca gacccattca gtgtttgtaa     300

atgtttccaa aggtcaggtt gccaagaagg aagacctcat cagtgcattt gggacagacg     360

accagactga aatctgcaag cagattttga ctaaaggaga agttcaagtg tcagataaag     420

aacggcacac acagctggag cagatgttta gggatatcgc caccattgtg gcagacaagt     480

gtgtgaaccc agaaacaaag agaccttaca ccgttatcct catcgagaga gccatgaagg     540

acatccacta ctccgtgaaa cccaacaaga gcacaaagca acaggctttg gaagtgataa     600

agcagctgaa agagaagatg aagatagagc gggcccacat gcgattgcgc ttcatcctgc     660

cagtgaacga agggaagaag ctgaaggaga agctgaagcc actgatgaag gtggtggaga     720

gtgaggacta cagccagcag ctggagatcg tgtgcctcat cgacccaggc tgcttcagag     780

aaattgatga gctaataaaa aaggaaacga aaggcagggg ttctctggaa gtgctcagtc     840

tgaaggacgt ggaggaaggc gatgagaagt ttgaatgaca ccgcccggct cctcaactgg     900

agcacgaccg aggacgcttg ttcctcacag cagcagctcg ttctgtgacc tgccaaacgc     960

cctgctcacg cgacgtgcca ctttccatct tgtgttaaac atttacccag gtacctgggt    1020

atttttgttg tcaattgggg tttccagcaa aaatgaaaaa taacctaaaa tacagagtcc    1080

agaacagctg ctcactgctg cgtctgcctt tctagttcca ggggaccaga gacagcattg    1140

gtggataaga aggtagagtt agtccatgac agatcattgg agaggggtct gaataacaaa    1200

gggggtacgc ctgctggaaa gaagatgggg tgtttctgaa taatgaagtg caggtatggg    1260

gtgtgagcat ggagagaaga gttcctgggt ccctcccaat agatttataa tgactaggga    1320

gaatttgact ttctaatttt caaccaacat gctaccaaaa ctgacttaga ttattcttgg    1380

gaaaatatat acagtcattt aatactaatt cttaaaggtt tataatatat gttagtatag    1440

ttaaaattct atgtaatcaa taaaacttat ttttactaaa aaaaaaaaaa aaaaa         1495

<210>  29
<211>  10835
<212>  DNA
<213>  Homo sapiens

<400>  29
   caaaaatcag tctgatctcg ggaaacctgg agaaatttat tttctgtact ctaatgttct      60
   ttcattttgg tgaccatcaa ggtgctggga gaggaattag atggctgtaa ttcaaagtta     120
   atggaattag atgcagcagt acagaaattc ttggaacaga atggccaact gggtaagcca     180
   ctggccaaga agataggaaa actgactgaa cttcaccagc agaccattag acaagctgag 240
   aatcggctct ccaagctcaa tcaggcagca tcacatttag aagaatacaa tgaaatgctt 300
   gaattaattt tgaagtggat tgaaaaagct aaagtcttgg ctcatggaac tattgcatgg 360
   aattctgcaa gccagcttcg ggaacaatat attttgcatc agaccctgct agaagaatcc 420
   aaagaaattg acagtgagct ggaagcaatg actgagaaat tacagtacct cactagcgtg 480
   tactgtacag aaaaaatgtc tcagcaagtg gcagaactgg gacgggagac tgaggagttg 540
   cgacagatga tcaaaattcg tttgcagaac ctccaagatg cagctaagga tatgaaaaaa 600
   tttgaagcag agttgaaaaa gttacaagct gccttggagc aagcccaggc aacactgact 660
   tctccagaag ttggacgtct cagtctcaag gagcagctct ctcatcggca gcatttgttg 720
   tctgagatgg agtcactgaa gccgaaggtg caagcagtgc agctctgcca gagtgccctc 780
   cggatccccg aggatgtggt tgccagctta cctctctgtc atgctgctct gcggctgcag 840
   gaagaggcca gccggctgca gcacaccgcc atccagcagt gtaacatcat gcaggcagct 900
   gtggtacaat atgaacaata tgagcaagaa atgaaacatc tccagcaact gatagaagga 960
   gctcacagag agattgagga taaacctgtt gccaccagta acatacagga gctgcaggct 1020
   cagatttctc ggcatgagga gctggcgcag aaaattaagg gctaccagga gcagatcgct 1080
   tctttgaatt ccaagtgcaa gatgctgacg atgaaagcca agcacgccac catgctgctg 1140
   acggtgaccg aggtcgaggg gctggcggaa gggacagagg acctggatgg ggagctcctc 1200
   cccacgcctt cggcccaccc ctctgtggtc atgatgactg caggtcgctg tcacactttg 1260
   ctgtcaccgg tcactgagga gtctggggag gagggaacca acagtgagat ttcctctcca 1320
   cctgcctgtc gctccccttc acctgtggct aatacagatg cttctgttaa ccaggacatt 1380
   gcatattacc aagccttgtc tgctgagagg ttgcagacag atgctgcaaa aattcacccc 1440
   agcacatccg catcccagga gttctatgaa ccgggattgg agccatccgc tactgccaaa 1500
   ctgggtgatt tgcagcgttc ttgggaaacc ttaaagaatg tgatcagtga gaagcagcgc 1560
   acactctatg aagctttgga acgccagcag aagtaccagg actccctcca gtccatctct 1620
   acgaagatgg aggccattga gctgaaactc agtgagagcc cagagcctgg caggagtcca 1680
   gaaagccaga tggctgaaca tcaggcattg atggatgaga ttctcatgct ccaggatgaa 1740
   atcaatgagc tccagtcctc tctcgcagag gagctggtat ccgagtcttg tgaggccgac 1800
   cctgcggagc agctggcctt gcagtccacg ctcactgtct tagccgagcg aatgtccacc 1860
   atcaggatga aagcctcggg gaaacggcag cttttggagg agaagttgaa tgatcagctg 1920
   gaggaacaaa ggcaggaaca ggccctgcag aggtatcgct gtgaagccga tgagctggac 1980
   agctggctct tgagtaccaa ggccactctg gacactgcgc tgagtccacc caaggagccc 2040
   atggacatgg aggcccagct tatggactgc cagaatatgc tggtggaaat agagcagaag 2100
   gtggtggctt tatcagaact gtcagtccac aatgagaacc tgctgctgga gggcaaagct 2160
   cacaccaagg acgaggccga gcagctggct ggaaagctga gaaggctcaa ggggagcctg 2220
   ctggagctgc agagagccct gcatgataag cagctcaaca tgcagggaac agcacaggag 2280
   aaggaggaga gcgatgttga cctaacagcc acgcagagcc ccggcgtcca ggaatggctg 2340
   gcccaagctc gcaccacatg gacccagcag cggcagagca gtctccagca acaaaaagag 2400
   ttagaacagg aattagccga gcagaagagt ctccttcgct cagtagccag tcgtggagag 2460
   gagattctaa ttcaacattc ggcggcagag acctctggtg atgctggcga aaaacctgat 2520
   gtgttatccc aggagttggg gatggaaggg gagaaatcat ccgctgaaga ccagatgaga 2580
   atgaaatggg aaagcctaca tcaagaattt agtaccaagc agaaactact acagaatgtt 2640
   ctggaacagg aacaagagca agtgctttat agcaggccaa atcgactctt gtctggtgtg 2700
   ccactgtaca aaggggacgt gccaacccaa gataaatctg cagttacatc tttgctggat 2760
   ggactgaacc aagccttcga ggaggtttca tcccagagtg gaggggcaaa gaggcagagt 2820
   atacacttgg agcagaagtt gtatgatgga gtctcagcca cctctacttg gttggatgac 2880
   gttgaagaac gtttatttgt tgccacagca cttttaccag aagaaacaga gacttgtctc 2940
   ttcaaccaag agattcttgc caaagacatt aaggaaatgt ctgaagaaat ggataagaac 3000
   aaaaacttgt tttcccaagc ttttccagag aatggtgata atcgagatgt tattgaagat 3060
   actttgggtt gtcttttggg caggttatcc ttgctagact cagtagtgaa tcaacgatgt 3120
   catcagatga aagaaagact tcagcaaata ctaaatttcc agaatgatct gaaagtgctg 3180
   tttacatcac tggctgacaa caaatacatc attctgcaaa aactggcaaa tgtgtttgaa 3240
   cagcccgtag cagaacaaat agaggcaata caacaggctg aagatggact caaagaattt 3300
   gatgcaggaa tcattgaatt aaagaggcgt ggtgacaagc tacaggtcga gcagccgtcc 3360
   atgcaagaac tctccaagct ccaggacatg tatgatgagc tgatgatgat cattggctcc 3420
   cggaggagtg gtctgaatca gaaccttaca ctcaagagtc agtatgagag ggccctacaa 3480
   gatctggctg acctgctaga aactggtcag gagaagatgg caggagacca gaaaatcatc 3540
   gtgtcttcca aagaggaaat ccagcaacta cttgacaaac ataaggaata ctttcagggc 3600
   ctggaatctc atatgatctt gactgaaaca ctcttcagaa agataatcag ctttgcagtc 3660
   caaaaggaaa cccagttcca tacagagctg atggctcagg cttctgctgt actgaaacgg 3720
   gctcacaaga ggggtgtgga gctggagtac attctagaga cgtggtccca tctggatgag 3780
   gaccagcagg agctcagcag acagctggag gtggtggaaa gcagcatccc aagcgtgggt 3840
   ctggtggagg agaacgagga caggcttatt gaccgcataa cactctacca gcatttaaaa 3900
   tctagcctta atgaatacca gcccaaatta tatcaagtat tagatgatgg gaaacgactt 3960
   ctgatatcca tcagctgctc agatctagaa agccaactaa atcaacttgg agagtgctgg 4020
   ctaagtaaca ccaataaaat gtctaaggaa cttcacagac tggaaacaat attgaaacac 4080
   tggaccagat atcaaagtga atctgcagat ctaattcact ggttacaatc tgcaaaagac 4140
   cggctagaat tttggactca gcaatctgtg acagtcccac aagagctgga aatggtccgt 4200
   gatcatctaa atgctttcct ggagttttct aaagaagtgg atgcccaatc ttccctgaaa 4260
   tcatctgttc tgagtactgg aaatcagctc cttcgactaa aaaaggtgga cacagccacg 4320
   ctgcgctctg agctgtcgcg cattgatagc cagtggactg acctgctaac caatatccca 4380
   gccgtccagg agaagctcca ccagctccag atggataaac tgccttcccg ccatgccatt 4440
   tctgaagtca tgagttggat ttctctaatg gaaaatgtta ttcagaagga tgaagataat 4500
   attaaaaatt ccataggtta caaggcaatt catgaatacc ttcagaaata taagggtttt 4560
   aagatagaca ttaactgtaa acagctgaca gtggattttg tgaaccagtc cgtgctacaa 4620
   atcagcagtc aggatgtgga aagtaagcgt agtgataaga ctgattttgc tgagcaactt 4680
   ggagcaatga ataaaagttg gcaaattctg caaggtctag taactgagaa gatccagctg 4740
   ttggaaggct tattggaatc ttggtcagaa tatgaaaata atgtacaatg tctgaaaaca 4800
   tggtttgaaa cccaggaaaa gagactaaaa caacagcatc gaattggaga tcaggcttct 4860
   gttcaaaatg cactgaaaga ctgtcaggat ctggaagatt tgattaaagc aaaagaaaaa 4920
   gaagtagaga aaattgagca gaatggactt gctttgattc agaacaagaa agaagacgtc 4980
   tctagcattg tcatgagcac actgcgagag ctcggccaaa cctgggcaaa tttagatcac 5040
   atggttggac aattaaagat actgctgaaa tcagtgcttg accaatggag tagtcacaaa 5100
   gtggcctttg acaagataaa cagttacctc atggaggcca gatactctct ttcccgattc 5160
   cgtctgctga ctggctcctt agaagctgtg caagttcagg tggacaatct tcagaatctc 5220
   caagatgatc tggaaaaaca ggaaaggagc ttacagaaat ttggctctat caccaaccaa 5280
   ttattaaaag agtgtcaccc acccgtgaca gaaactctta ccaatacact gaaagaagtc 5340
   aacatgagat ggaataactt gctggaagag attgctgagc agctacagtc cagcaaggcc 5400
   ctacttcagc tttggcaaag atacaaggac tactccaaac agtgtgcttc gacagttcag 5460
   cagcaggagg atcgaaccaa tgagctgttg aaggcagcca caaacaagga cattgccgat 5520
   gatgaggttg ccacatggat tcaagattgc aacgacctcc tcaaaggact gggcacagtt 5580
   aaagattccc tcttttttct ccatgagctg ggagagcaac tgaagcaaca agtggatgct 5640
   tccgcagcat cagctattca atcggatcaa ctctctttga gtcaacactt gtgtgccctg 5700
   gagcaagctc tctgcaaaca gcagacttca ttacaggctg gagttcttga ttatgaaacc 5760
   tttgccaaga gtttagaagc tttggaggcc tggatagtgg aagctgaaga aatactacaa 5820
   gggcaggacc ctagccactc atctgacctc tccacaatcc aggaaaggat ggaagaactt 5880
   aagggacaga tgttaaaatt cagcagcatg gctccagatt tagaccgtct aaatgagctt 5940
   ggatataggt tacccttgaa tgataaggaa atcaaaagaa tgcagaatct gaaccgccat 6000
   tggtctctga tctcctctca gactacagaa agattcagca agttgcagtc atttttgcta 6060
   caacatcaga ctttcttgga aaaatgtgaa acatggatgg aattcctagt tcagacagaa 6120
   caaaagttag cagtagagat ttcaggaaat tatcagcacc ttttggaaca gcagagagca 6180
   cacgagttgt ttcaagccga gatgttcagt cgtcagcaga ttttgcactc aatcattatt 6240
   gatgggcaac gtcttctaga acaaggtcaa gttgatgaca gggatgaatt caacctgaaa 6300
   ttgacactcc tcagtaatca atggcaggga gtgattcgca gggcccagca gaggcggggg 6360
   atcattgaca gccagattcg ccagtggcag cgctataggg agatggcaga aaagcttcgt 6420
   aaatggttgg ttgaagtgtc ctacctcccc atgagtggtc tcggaagtgt tcctatacca 6480
   ctgcaacaag caaggaccct ctttgatgaa gtgcagttca aagaaaaagt gtttctgcgg 6540
   caacaaggca gctacatcct gactgtggag gctggcaagc aactccttct ctcggcggac 6600
   agtggcgctg aggccgcctt gcaggccgaa ctcgctgaaa tccaagagaa atggaaatca 6660
   gccagcatgc ggctggaaga acagaagaaa aaactagcct tcttgttgaa agactgggaa 6720
   aaatgtgaga aaggaatagc agattccctg gagaaactac gaactttcaa aaagaagctt 6780
   tcgcagtctc tcccggatca ccatgaagag ctccatgcag aacaaatgcg ttgcaaggaa 6840
   ttagaaaatg cagttgggag ctggacagat gacttgaccc agttgagcct gctgaaggac 6900
   accctctctg cctatatcag tgctgatgat atctccattc ttaatgaacg cgtagagctt 6960
   ctgcaaaggc agtgggaaga actatgccac cagctctcct taaggcggca gcaaataggt 7020
   gaaagattga atgaatgggc agtcttcagt gaaaagaaca aggaactctg tgagtggttg 7080
   actcaaatgg aaagcaaagt ttctcagaat ggagacattc tcattgaaga aatgatagag 7140
   aagctcaaga aggattatca agaggaaatt gctattgctc aagagaacaa aatacagctc 7200
   caacaaatgg gagaacgact tgctaaagcc agccatgaaa gcaaagcatc tgagattgaa 7260
   tacaagctgg gaaaggtcaa cgaccggtgg cagcatctcc tggacctcat tgcagccagg 7320
   gtgaagaagc tgaaggagac cctggtagcc gtgcagcagc ttgataagaa catgagcagc 7380
   ctgaggacct ggctcgctca catcgagtca gagctggcca agccaatagt ctacgattcc 7440
   tgtaactcgg aagaaataca gagaaagctt aatgagcagc aggagcttca gagagacata 7500
   gagaagcaca gtacaggtgt tgcatctgtc ctcaacctgt gtgaagtcct gctgcacgac 7560
   tgtgacgcct gtgccactga tgccgagtgt gactctatac agcaggctac gagaaacctg 7620
   gaccggcggt ggagaaacat ttgtgctatg tccatggaaa ggaggctgaa aatcgaagag 7680
   acgtggcgat tgtggcagaa atttctggat gactattcac gttttgaaga ttggctgaag 7740
   tcttcagaaa ggacagctgc ttttcccagc tcttctgggg tgatctatac agttgccaag 7800
   gaagaactaa agaaatttga ggctttccag cgacaggtcc acgagtgcct gacgcagctg 7860
   gaactgatca acaagcagta ccgccgcctg gccagggaga accgcactga ttcagcatgt 7920
   agcctcaaac agatggttca cgaaggcaac cagagatggg acaacctgca aaagcgtgtc 7980
   acctccatct tgcgcagact caagcatttt attggccagc gtgaggagtt tgagactgcg 8040
   cgggacagca ttctggtctg gctcacagag atggatctgc agctcactaa tattgaacat 8100
   ttttctgagt gtgatgttca agctaaaata aagcaactca aggccttcca gcaggaaatt 8160
   tcactgaacc acaataagat tgagcagata attgcccaag gagaacagct gatagaaaag 8220
   agtgagccct tggatgcagc gatcatcgag gaggaactag atgagctccg acggtactgc 8280
   caggaggtct tcgggcgtgt ggaaagatac cataagaaac tgatccgcct gcctctccca 8340
   gacgatgagc acgacctctc agacagggag ctggagctgg aagactctgc agctctgtcg 8400
   gacctgcact ggcacgaccg ctctgcagac agcctgcttt ctccacagcc ttcctccaat 8460
   ctctccctct cgctcgctca gcccctccgg agcgagcggt caggacgaga caccccggct 8520
   agtgtggact ccatccccct ggagtgggat cacgactatg acctcagtcg ggacctggag 8580
   tctgcaatgt ccagagctct gccctctgag gatgaagaag gtcaggatga caaagatttc 8640
   tacctccggg gagctgttgg cttatcaggg gaccacagtg ccctagagtc acagatccga 8700
   caactgggca aagccctgga tgatagccgt tttcagatac agcaaaccga aaatatcatt 8760
   cgcagcaaaa ctcccacggg gccggagcta gacaccagct acaaaggcta catgaaactg 8820
   ctgggcgaat gcagtagcag tatagactcc gtgaagagac tggagcacaa actgaaggag 8880
   gaagaggaga gccttcctgg ctttgttaac ctgcatagta ccgaaaccca aacggctggt 8940
   gtgattgacc gatgggagct tctccaggcc caggcattga gcaaggagtt gaggatgaag 9000
   cagaacctcc agaagtggca gcagtttaac tcagacttga acagcatctg ggcctggctg 9060
   ggggacacgg aggaggagtt ggaacagctc cagcgtctgg aactcagcac tgacatccag 9120
   accatcgagc tccagatcaa aaagctcaag gagctccaga aagctgtgga ccaccgcaaa 9180
   gccatcatcc tctccatcaa tctctgcagc cctgagttca cccaggctga cagcaaggag 9240
   agccgggacc tgcaggatcg cttgtcgcag atgaatgggc gctgggaccg agtgtgctct 9300
   ctgctggagg agtggcgggg cctgctgcag gatgccctga tgcagtgcca gggtttccat 9360
   gaaatgagcc atggtttgct tcttatgctg gagaacattg acagaaggaa aaatgaaatt 9420
   gtccctattg attctaacct tgatgcagag atacttcagg accatcacaa acagcttatg 9480
   caaataaagc atgagctgtt ggaatcccaa ctcagagtag cctctttgca agacatgtct 9540
   tgccaactac tggtgaatgc tgaaggaaca gactgtttag aagccaaaga aaaagtccat 9600
   gttattggaa atcggctcaa acttctcttg aaggaggtca gtcgtcatat caaggaactg 9660
   gagaagttat tagacgtgtc aagtagtcag caggatttgt cttcctggtc ttctgctgat 9720
   gaactggaca cctcagggtc tgtgagtccc acatcaggaa ggagcacccc aaacagacag 9780
   aaaacgccac gaggcaagtg tagtctctca cagcctggac cctctgtcag cagtccacat 9840
   agcaggtcca caaaaggtgg ctccgattcc tccctttctg agccagggcc aggtcggtcc 9900
   ggccgcggct tcctgttcag agtcctccga gcagctcttc cccttcagct tctcctgctc 9960
   ctcctcatcg ggcttgcctg ccttgtacca atgtcagagg aagactacag ctgtgccctc 10020
   tccaacaact ttgcccggtc attccacccc atgctcagat acacgaatgg ccctcctcca 10080
   ctctgaacta agcagatgcc atctgcagaa gtgctggtag cataaggagg atcgggtcat 10140
   aagcaatccc aaactaccaa caagaggacc ttgatcttgg cgaaagccct cggtgtggca 10200
   gctttagccc tcctccagat cacatgtgtg caaattatgg cttcagaggt ggaagataaa 10260
   cagtgacggg ggaacaaaca gacaacaaga aggtttggaa gaaatctggt ttgagactct 10320
   gaaccttagc actaaggaga ttgagtaagg acctccaaag ttccccggac tcatgaattc 10380
   tgggcccttg gcccattctg tgcacagcca aggacttcag tagaccatct gggcagcttt 10440
   cccatggtgc tgctccaacc atcagataaa tgaccctccc aagcaccatg tcagtgtcgt 10500
   acaatctacc aaccaaccag tgctgaagag atttagaacc cttgtaacat acaattttta 10560
   agagcttata tggcagcttc ctttttacct tgttttcctt tggggcatga tgttttaacc 10620
   tttgctttag aagcacaagc tgtaaatcta aaaggcactt ttttttagag gtataaagaa 10680
   aaactagatg taataaataa gatcatggaa ggctttatgt gaaaaaagtt gaatgttata 10740
   gtaaaaaaaa aaagatattt atgtatgtac agtttgctaa agccaagttt tgtttgtatt 10800
   gatttctttg catttattat agatattata aaata 10835

<210> 30
<211> 27436
<212> DNA
<213> Homo sapiens

<400> 30
cggccgccgg ccgcagcggg ctgagattgt tgtcctctgt caccagggcg gctgggctcc 60

cgcagtcctg cagaccgcgc ccgatcccgg cgacagggcg ggcggacagc cgcgcatccc 120

cggggtcccg ccgagcctgg gcgcagagag ccgggaggaa gcgttcgctc gcttcgcctt 180

gctgctggga aactgaacga ggccgagaga gaaggttctt gagttcatgt aagaggacag 240

tcttaaaacg gaagaagaaa aagaagcagt tcagtctttg ggagagctgc ctccttgttg 300

agtgctgcaa aggcctggaa ttcatttatg acagaataga tctagaaaag tccaagcatg 360

ttttctagag tggtgtagcc ctgtgctgcc tccagtgaag agtctcttgg tgttggcttc 420

gtgcttccgg agggaccatg gcaacctcca gaggggcctc ccggtgtcct cgggatatcg 480
ccaatgtgat gcagaggctg caagatgagc aagagatagt acaaaaacga actttcacaa 540
aatggatcaa ctctcatctg gccaagcgga aacctccaat ggtggtggac gatctttttg 600
aagacatgaa agatggtgtt aaactgcttg cccttctgga ggtcctgtct gggcagaaac 660
tgccttgtga acaaggacgc cggatgaagc gaatccatgc tgtggctaac attggcacgg 720
cactcaagtt cctcgaagga agaaagtcca tgcacagagg atcaccgatt aaattagtca 780
acattaactc caccgatata gctgatggcc gaccctcaat agttcttgga ttgatgtgga 840
ccattattct atatttccag attgaagagt tgaccagcaa cctgccccag ctccagtctt 900
tgtccagcag cgcatcctcc gtggacagca tagttagctc tgagactccc agcccaccaa 960
gtaaacggaa ggtgaccacc aagatccaag gaaatgctaa gaaggcttta ttaaagtggg 1020
ttcagtacac agctggcaag cagactggaa tagaagtaaa agattttggg aagagttgga 1080
gaagcggggt tgcctttcat tcagttattc atgccattcg accggaattg gtggacttgg 1140
agacagtgaa aggcagatcc aaccgagaaa atttggagga tgctttcact atcgccgaaa 1200
cagaactggg gatcccaaga ctgctagatc ctgaagacgt tgatgtggat aaaccagatg 1260
agaaatctat tatgacctat gtagcccagt ttctgaaaca ttatcctgac atccacaatg 1320
caagcactga tgggcaagag gatgatgaaa tacttccagg tttcccatct tttgcaaatt 1380
ctgtacaaaa ttttaagaga gaagacagag taatttttaa ggaaatgaaa gtttggatag 1440
aacaatttga gagagatttg acaagagcac agatggtgga atcaaattta caggataaat 1500
atcagtcatt taagcacttc agagttcaat atgaaatgaa gaggaaacag attgaacatt 1560
taatacaacc attacacaga gacggtaaat tgtcacttga ccaagcattg gtaaaacaat 1620
cttgggatag agtgacctcc aggctctttg actggcatat acagcttgat aaatctcttc 1680
ctgcacctct gggcaccata ggtgcctggc tgtacagagc ggaggtggcc ctgagagagg 1740
aaataaccgt tcaacaggtc cacgaggaaa cagcaaacac gatacaacgg aaacttgagc 1800
aacataagga tctgcttcaa aacacggatg cccacaaaag agcattccat gaaatctacc 1860
ggaccaggtc tgttaacggg attccagtgc cacctgatca attagaggac atggccgaga 1920
ggtttcattt tgtttcctcc acatcagagc tacacctaat gaaaatggaa tttttagaat 1980
taaagtaccg tctgctctca ctgctggttc ttgcagagtc aaagctgaag tcttggatca 2040
ttaagtacgg gaggagagag tcagtggagc agcttctaca aaactacgtg tcttttatag 2100
aaaatagcaa gttctttgaa caatatgagg tgacatacca gatcttgaaa cagacagctg 2160
agatgtatgt caaagcagat ggttcagtgg aagaagctga gaatgtgatg aaattcatga 2220
atgaaaccac cgctcagtgg aggaatctct cagtagaagt gaggagtgtg aggagcatgc 2280
tggaagaagt gatctctaac tgggatcgct atggcaatac agtggctagt ctgcaagcct 2340

ggctagagga tgctgaaaaa atgctcaatc aatcagaaaa tgccaaaaag gatttttttc 2400

gaaatttacc tcattggatt cagcagcata ctgccatgaa cgatgctggc aattttctaa 2460

ttgaaacctg tgatgagatg gtttcccgtg acctgaagca gcaattactg ttgctaaatg 2520

ggcggtggag ggagttgttt atggaagtca agcaatatgc tcaagctgat gagatggaca 2580

gaatgaagaa ggaatacaca gactgtgttg ttaccctgtc tgcttttgca acggaagccc 2640

ataagaaact ttctgaaccc ttagaagtct cttttatgaa tgtcaagcta ttaattcaag 2700

acttggagga tattgagcag agggtgcctg tgatggatgc ccaatacaag ataattacaa 2760

agacagcaca cctcattacc aaagaaagcc cccaagaaga aggaaaagaa atgtttgcga 2820

ccatgtcaaa gctcaaagag cagctaacca aggtcaaaga atgttactcc ccactccttt 2880

atgagtctca gcagctgttg attccgttgg aggaattaga aaagcagatg acgtcctttt 2940

atgactcact tgggaaaatc aatgaaatta tcacagttct tgagcgtgag gcacaatcga 3000

gtgccctttt taaacaaaaa catcaggaac tgttagcttg tcaagaaaac tgtaagaaaa 3060

ccttgacact tattgagaaa ggcagtcaaa gtgttcaaaa gtttgtgacc ttgagcaacg 3120

tgttaaagca ttttgatcag acgaggctac aaagacagat tgcagatatt catgttgctt 3180

ttcagagtat ggtaaagaaa actggagatt ggaagaagca tgtggaaacc aacagtcgct 3240

tgatgaagaa gtttgaggag tctcgagcag agttggagaa ggtactgcgg attgctcagg 3300

agggcctgga ggaaaagggg gatccagagg agctcctgcg gagacacact gagtttttca 3360

gtcagctgga tcagagggtg ctcaatgctt tcctgaaagc ttgtgatgaa ctcaccgaca 3420

tccttccaga gcaggagcag caggggctgc aggaagctgt tcgaaagctc cacaaacaat 3480

ggaaggatct tcaaggagaa gccccttatc atttgcttca tctgaagatt gatgtggaga 3540

agaataggtt cttagcctct gtagaagaat gcagaactga gctggatcga gagaccaagc 3600

tgatgcccca ggaaggcagt gaaaagataa ttaaagagca cagggttttc ttcagtgaca 3660

aaggtcctca tcatctctgt gagaaaaggt tacagctcat cgaggaactc tgtgtgaaac 3720

tcccagtgcg ggacccagta agggacacac ctggaacctg tcacgtgact ctcaaagagc 3780

tcagagctgc cattgacagc acctacagga agctcatgga agacccagac aagtggaagg 3840

actacactag cagattctct gagttctcat cttggatatc tacaaatgag acacaattaa 3900

aggggatcaa gggtgaggcc atcgatactg ccaaccacgg agaggttaaa cgtgccgttg 3960

aagagatcag aaatggtgtt accaaaaggg gtgagaccct cagctggctg aaatccaggc 4020

tgaaagtttt gacagaagtt tcttctgaga atgaagccca aaagcaggga gatgagctgg 4080

caaaattatc cagctctttc aaggctcttg tgacgctgct gtcagaggtt gaaaagatgc 4140

taagcaattt tggggactgt gtccagtaca aagaaatagt caaaaattct ctcgaagaat 4200

taatttctgg ctctaaagaa gtccaggaac aagctgagaa gatcttggat actgaaaatc 4260

tgtttgaagc acagcagtta cttcttcatc accagcaaaa gacaaagcgg atctcagcaa 4320

agaagagaga tgtgcagcag cagatcgcgc aggcgcagca gggagaaggg gggctgcctg 4380

accgaggcca cgaggagctg cggaagctgg agagcacact ggatggcctg gagcgcagcc 4440

gggagaggca ggaacgccgc atccaggtca cattaagaaa atgggagcga tttgaaacaa 4500

acaaagaaac agtagtaaga tacctttttc aaacaggttc cagtcatgaa cgcttcttga 4560

gttttagcag tttggaaagt ttatcttcag aactggaaca aacaaaggag ttttctaaac 4620

ggacagaaag tattgcagtc caggctgaga accttgtaaa ggaagcttca gagataccgc 4680

ttgggcccca aaataagcag ctgcttcaac agcaggccaa gtcaatcaaa gaacaagtca 4740

aaaaattaga agacacgctt gaagaagata ttaaaaccat ggaaatggtg aaaaccaagt 4800

gggatcattt tggcagtaat tttgagactc tgtccgtctg gataactgag aaagaaaaag 4860

aactcaatgc cttggaaact tcgtcatctg ccatggacat gcaaatcagc caaattaagg 4920

tcacaattca ggaaatagaa agtaagctca gcagcattgt aggattagaa gaagaagccc 4980

agtcttttgc tcagtttgtt accactggag aatctgctcg aattaaagcc aagttgacac 5040

aaataagaag atacggggaa gagcttcgag agcatgcaca gtgtctggaa ggaacaatcc 5100

tgggacattt atctcagcag caaaagtttg aagagaacct tagaaagatc cagcaatctg 5160

tgtctgaatt tgaagataaa cttgctgttc caattaaaat atgttcttca gctacagaaa 5220

catacaaagt tcttcaagaa catatggatc tctgccaggc cctggagtca ctgagcagcg 5280

cgatcactgc cttctcagcc agtgccagga aggttgtgaa cagagattcc tgtgttcagg 5340

aggctgcggc tctacagcag caatacgagg acatcctaag gagggcgaag gagagacaga 5400

cggcgctgga gaatctgctg gcccactggc agaggctaga gaaagaacta tcatcctttt 5460

tgacctggtt agagcggggt gaagctaaag ccagttcccc agaaatggac atttctgcag 5520

acagagtcaa agtggaaggt gaacttcagt taatacaggc actgcaaaat gaagttgtat 5580

cccaggcctc attctatagc aaacttttgc aattgaagga atcattgttc tcagtagcct 5640

ccaaagatga tgtgaaaatg atgaaactac atttggagca gttggatgag agatggagag 5700

atttaccaca gatcattaac aaaaggatta attttcttca gtctgtggtt gctgaacacc 5760

agcaatttga tgagctgctg ctttcctttt ctgtctggat taaactgttt ctcagtgaat 5820

tacaaactac ctctgagatt agcataatgg accatcaagt agcccttact cggcataagg 5880

accacgcagc agaagtagag agcaaaaagg gcgaattgca gagtctgcag ggtcacttag 5940

caaagttggg ttctctgggc cgtgctgagg acctccacct cctgcaggga aaggctgagg 6000

actgcttcca gctgtttgag gaggccagcc aggttgtgga gaggcggcag cttgccctgt 6060

cccatttggc agaattcctc cagagccatg cctctctgtc cggcattctc cgccagctga 6120

ggcaaacagt ggaagcaacc aacagtatga ataagaacga gtctgatttg atagaaaagg 6180

acctcaatga tgctcttcaa aatgctaaag cattagaatc tgctgccgtc agtctggatg 6240

gcattctttc caaagcccaa taccatctga aaatcgggag ctctgagcaa aggacttcct 6300

gcagagccac ggctgatcag ctctgtggag aggtagagag gatccagaac cttctgggaa 6360

ccaagcagag tgaggcagat gctctggcag tgttgaaaaa agcattccaa gaccagaaag 6420

aggagcttct gaaaagcatt gaggacattg aagaaaggac tgacaaagag cgattgaaag 6480

aacctacccg ccaagctctt cagcagaggt taagagtgtt taatcagcta gaagatgaat 6540

tgaattctca cgagcatgaa ctatgttggt tgaaagacaa agccaagcaa attgcccaga 6600

aagatgtagc ttttgcacct gaagttgaca gggagataaa ccgcttagag gtcacctggg 6660

atgataccaa aagactaatt catgaaaatc agggtcagtg ctgtggactt attgacttaa 6720

tgagagaata tcagaacctg aaatcagctg tatctaaagt cttagaaaat gccagcagtg 6780

tgattgtaac cagaactacc ataaaagatc aggaggatct taaatgggct ttttccaagc 6840

atgaaactgc caagaacaaa atgaattaca aacagaaaga cttggataac tttaccagca 6900

aaggaaaaca cttgttatct gagctgaaga aaattcacag tagtgatttc agcttggtga 6960

aaacagacat ggagagcacc gtggacaaat ggctggatgt atcagagaaa cttgaagaaa 7020

acatggatag gctgagagta agcctgtcca tttgggatga tgtactgtca actagagatg 7080

agattgaggg atggtcaaac aactgcgttc cacagatggc agaaaacatc agcaacctgg 7140

ataaccacct cagagctgaa gaactgctta aagaatttga gtctgaagtt aaaaacaaag 7200

cattgagatt ggaagaactg cattccaaag ttaatgatct gaaagaatta actaaaaatc 7260

tagaaacacc gccagacctt cagtttatag aagcagactt aatgcagaaa ctggagcatg 7320

ccaaagaaat aactgaagta gcaaaaggaa ccctgaagga tttcacggct caaagtacac 7380

aagtggagaa gtttattaat gacataacaa catggttcac aaaagtggaa gaatcgttga 7440

tgaactgtgc ccaaaatgag acttgtgaag cattgaaaaa agtcaaggat atacaaaaag 7500

aacttcaaag tcaacaaagc aacatcagct ctacccaaga aaatctcaat agcttgtgcc 7560

gcaagtacca ctcagctgag ttggagagcc tgggccgtgc aatgactggt ctgataaaga 7620

aacatgaagc cgtgagccag ttgtgctcca aaacccaggc cagcctgcag gaatctctgg 7680

aaaaacactt cagtgagtct atgcaggaat tccaagaatg gtttttggga gcaaaggcag 7740

cagcaaaaga atcatcagat cgcaccggtg acagcaaagt tctagaagca aagctccatg 7800

atcttcagaa cattttggac tcagtcagtg atgggcagag caaacttgat gcagtgactc 7860

aagaaggaca aactttgtat gcacatttgt ctaaacaaat tgtcagtagc attcaagaac 7920

aaatcacaaa ggccaatgaa gagtttcaag catttctgaa acaatgcctt aaagataagc 7980

aggctcttca agactgtgct tcagaacttg gaagctttga agatcagcac agaaaactga 8040

acttatggat ccatgaaatg gaagaaaggt tcaatacgga aaacttggga gagagtaaac 8100

agcacattcc tgagaagaaa aatgaagttc ataaagttga aatgtttttg ggagaactgc 8160

tggctgcaag agagtctctt gataagcttt cccagagagg gcagcttctg agtgaagaag 8220

gccacggtgc tgggcaggag ggccgcctgt gttcccagct cctcacaagc caccagaacc 8280

tacttagaat gaccaaagag aaactccgga gctgccaggt ggcccttcag gagcacgaag 8340

ccctggagga agcactgcaa agcatgtggt tctgggtgaa ggccattcag gacagactgg 8400

cctgtgcaga gagcactctt gggagcaaag acaccctgga gaaacggctg tcacaaatac 8460

aggatattct cctgatgaaa ggtgaagggg aagttaagtt gaatatggcc attggcaagg 8520

gggaacaggc cttgagaagt agcaacaaag aaggtcagag ggtgattcag actcagttag 8580

agacccttaa agaagtgtgg gctgacatca tgagctcctc cgtccacgct caaagcactt 8640

tagagtctgt gattagccaa tggaatgact atgtagagag gaaaaaccag ttggagcagt 8700

ggatggaatc agtggatcaa aaaatagaac atcccttaca accacagcca ggtctgaaag 8760

agaagttcgt cctgcttgac cacctccagt ccatcctgtc tgaggcagaa gatcacacga 8820

gagcccttca ccgtctaatt gcgaagtcca gggagctcta cgaaaagaca gaggatgagt 8880

ctttcaagga cacagctcaa gaggagctga aaacacagtt taatgatata atgactgttg 8940

ccaaggaaaa aatgaggaaa gtggaagaga ttgtgaaaga tcatctaatg tatttagatg 9000

cggtccacga gttcacagat tggctccatt cagcaaagga agaacttcac cggtggtcag 9060

atatgtctgg agattcatca gccacccaga aaaagttatc aaaaattaag gagctgatag 9120

attccagaga gattggtgca agccgtctca gcagagtgga gtcgctggct cccgaagtga 9180

aacagaacac aactgccagt gggtgtgagc tcatgcacac ggagatgcag gccctgcgtg 9240

ccgactggaa gcagtgggaa gacagtgtat tccaaacgca gagctgtttg gagaacctgg 9300

tcagccagat ggccctttcg gagcaggaat tctcaggcca agtggctcaa ctggagcagg 9360

ccctggaaca gttcagtgcc cttctgaaaa cctgggctca gcagttaacc ctcctggaag 9420

gcaagaacac ggatgaggag atagtggaat gctggcacaa aggacaagag atactggatg 9480

ctttgcaaaa agcagagcct agaacagagg atctcaagtc tcagctgaat gaactttgtc 9540

gattttccag agacctgagt acctacagtg gaaaagtttc tggcttgatt aaagagtata 9600

attgtctttg tttgcaagca tccaagggct gccagaataa agaacagatt ttacagcaaa 9660

gatttcgaaa ggccttcagg gatttccagc agtggttggt taatgcaaaa atcactaccg 9720

ccaagtgttt tgatatacct caaaatataa gtgaagtttc aactagtctt cagaaaatac 9780

aggagttttt gtcagaaagt gaaaatggac agcacaagct aaacatgatg ctgtctaaag 9840

gggaacttct gagtaccctg ctgaccaaag agaaagcgaa agggatccag gccaaagtta 9900

cagctgcaaa agaagattgg aaaaattttc attcaaatct ccaccaaaaa gaatctgctc 9960

tagagaatct aaagatccaa atgaaggact ttgaagtaag tgctgagcct atccaggact 10020

ggctgagtaa aactgagaag atggtccatg aaagcagcaa tcgcctctat gatctgccag 10080

caaagaggag ggagcagcag aagctccagt ctgtccttga ggaaatacac tgctacgagc 10140

ctcagctcaa caggctgaaa gagaaagctc agcagctgtg ggaaggacaa gctgccagca 10200

agagctttag gcacagagtg tcgcagctgt cttctcagta tctagcgcta agcaatttaa 10260

caaaggagaa agtgtcaaga ctggatagaa tcgttgcaga acacaatcag ttctctcttg 10320

ggattaaaga attacaagac tggatgacgg atgcgattca catgctggat tcatactgcc 10380

acccgacatc cgacaaaagt gtgctggaca gcaggacgct caagctcgag gctctattat 10440

cagtcaaaca ggaaaaagag attcagatga aaatgatagt gaccagggga gaatctgtcc 10500

ttcagaatac ttctccagaa ggcattccca ctattcagca gcagctgcag agtgtgaagg 10560

atatgtgggc atcccttttg tctgcaggga ttcgttgtaa aagccaactc gaaggagctc 10620

tctccaagtg gacaagttat caggatggcg ttcgacagtt ctccggttgg atggatagta 10680

tggaagccaa cctgaatgaa tcagaaaggc agcatgcgga gctgcgggat aaaacaacga 10740

tgctcggaaa agccaagtta ttaaatgaag aagtgctgag ttacagcagc ttgctggaga 10800

ccatcgaagt caaaggggct ggcatgacag aacactatgt cacccagcta gaactccagg 10860

atctacagga acgatacaga gccatccaag agagggccaa ggaagccgta accaagtctg 10920

aaaaacttgt ccgcctgcac caagagtatc agagagacct aaaggcattt gaagtttggt 10980

tggggcaaga acaagaaaag ctcgaccagt attcagttct tgaaggtgat gcccacactc 11040

atgagacaac attgcgtgat cttcaggagc tacaggtaca ctgtgcagag gggcaggccc 11100

tgttgaactc agtgctgcac accagagagg atgtgatccc atcaggtatc ccacaggcag 11160

aggaccgggc tttagagtct ctccggcaag actggcaggc ttaccagcac aggctgtccg 11220

agactcgaac tcagttcaat aacgtggtga acaaattgag gctaatggag caaaagtttc 11280

agcaagtaga tgaatggctc aaaacagcag aggagaaaaa gtggcacgaa gaagtgactg 11340

catacagaga tgaagttgag gaagtgggag ctagagctca ggagatactg gacgagagcc 11400

acgtgaacag cagaatgggt tgccaggcca cccagctgac ttccagatac caggccctgc 11460

ttctccaagt gctggaacaa ataaaattcc tggaggagga gattcagagt ttggaggaat 11520

cagaatcatc cctcagttcc tattctgatt ggtatggctc tactcataaa aacttcaaga 11580

atgtggctac caagattgac aaagtagata cagtaatgat ggggaagaaa ttgaagacgt 11640

tggaggtttt gctcaaagac atggagaaag gtcacagttt gctgaaatca gcccgggaga 11700

aaggagagag ggctgttaaa tacttggagg aaggcgaggc agagaggtta agaaaggaga 11760

ttcatgatca catggagcag ttgaaggaac tgaccagcac tgtccggaaa gaacacatga 11820

cgctggaaaa aggtcttcat ttagcaaagg aattctcaga taaatgcaaa gcactgacac 11880

agtggatagc agaataccag gaaattctac atgttcctga agaacccaaa atggaattat 11940

atgagaaaaa agctcagtta tctaaataca agtcacttca acaaacggtg ctgtcccatg 12000

aaccatcagt aaagtcagtg agagagaagg gtgaagctct tttggaactg gtgcaggacg 12060

tcactttaaa ggacaaaata gatcaacttc aaagtgatta ccaggacctg tgcagcatag 12120

gaaaggcaat gatggaagaa attgctggtt ttgaagaccg tttgaacaat cttcaaatga 12180

aaggtgatac tttgattggc caatgtgcag accacctgca agcgaaactt aaacaaaacg 12240

tgcatgctca tctgcagggc acaaaggaca gctactcagc gatctgcagc acagctcaga 12300

ggatgtacca gagtttggaa cacgaacttc agaagcacgt cagccgacaa gacaccctgc 12360

agcagtgcca ggcctggctt tctgcagtcc agccggattt agagccaagt cctcaaccac 12420

ctcttagtag ggcagaagcc attaagcagg tcaaacactt cagagctttg caagagcagg 12480

caaggaccta cctagatctc ctttgctcca tgtgtgacct gtcaaatgct tcggtgaaaa 12540

ccacagcaaa agacattcaa caaacagagc aaacgattga acaaaagctt gtccaggccc 12600

agaacttaac tcagggctgg gaagagatca agcacctgaa gtctgagctc tggatttacc 12660

tgcaagatgc tgatcagcaa ctgcagaaca tgaagaggag gcactctgag ctggagctga 12720

acattgcaca gaacatggtt tcacaagtta aggattttgt taagaaacta cagagcaaac 12780

aggcatccgt gaacaccata atagaaaagg tgaataagtt aacaaagaag gaggaatcgc 12840

ctgaacacaa ggaaataaat catttaaatg atcagtggct cgatttgtgc cgtcagtcta 12900

acaacctgtg cttgcaaagg gaagaggatc ttcagagaac aagagattac catgactgta 12960

tgaatgttgt tgaagtgttc ctagaaaaat ttactacaga atgggataac ttggccagat 13020

ctgatgcaga gagtacagct gtccacctgg aagctttgaa aaagttagca ttggcattgc 13080

aggagagaaa gtatgctatt gaagatctga aagatcaaaa gcagaaaatg atagagcatc 13140

tgaatttaga tgacaaggag ttagtcaaag aacagacgag tcatttagag caacgttggt 13200

ttcagcttga ggacctcatt aaaaggaaaa tccaagtgtc agtcaccaac ttggaggagt 13260

taaatgtggt gcagtccaga tttcaggagc taatggagtg ggcagaagag caacaaccca 13320

acatcgccga ggcccttaag cagagccctc ctccagatat ggctcagaac cttctcatgg 13380

atcacctggc catctgcagt gaactggagg ccaagcagat gctcctgaaa tcgcttataa 13440

aggacgcaga cagggtcatg gcagatcttg gtctcaatga gcgacaggtc atccagaagg 13500

ctctctctga tgcacaaagc cacgtgaatt gtctcagtga cttagtgggc cagcgaagaa 13560

agtacttaaa caaagccttg tccgagaaaa cccagtttct catggcagtg ttccaggcca 13620

ccagccaaat tcagcaacat gagcgaaaga taatgttccg tgaacacatc tgtctgttac 13680

cagatgatgt gagcaaacaa gtcaaaacat gtaagagtgc acaagccagc ctcaagactt 13740

accaaaatga agtcactgga ctttgggccc agggtcgcga actaatgaag gaagtcacag 13800

agcaggaaaa gagtgaagtg ctggggaagc ttcaggaatt gcagagtgtc tatgacagtg 13860

ttttacaaaa gtgcagtcac cggttacaag aactagagaa gaatttggtt tctaggaagc 13920

attttaagga agattttgat aaagcttgcc actggctaaa acaagcagat attgttacat 13980

ttcctgaaat caacctaatg aatgagagtt ctgagcttca tacacaactg gctaaatacc 14040

aaaacattct tgaacaatct ccagaatatg aaaatcttct acttacgctg cagagaactg 14100

ggcagaccat attaccatcg ctgaatgaag tcgatcattc ctacctcagt gaaaagctaa 14160

atgctttgcc tcgacaattt aatgtaattg ttgccttggc taaagacaag ttctataaag 14220

tccaggaagc aattcttgct cggaaggaat atgcttcctt gattgagttg acaacccagt 14280

ctctgagtga acttgaagcc caattcttga ggatgagcaa agttcccacc gacctggccg 14340

ttgaggaggc tctttctctg caagatggtt gcagagccat tctggacgag gtggcgggcc 14400

ttggggaggc ggtggatgaa ctgaaccaga aaaaagaagg ttttcgcagc acaggtcagc 14460

cttggcagcc agacaagatg ctgcaccttg tcaccttata tcacaggctg aagcgacaaa 14520

cagaacagag ggttagctta ttagaagaca ccaccagtgc ttaccaagaa cacgagaaga 14580

tgtgccaaca gctggagaga caactgaagt ctgtaaaaga ggagcagtcc aaagtgaatg 14640

aggaaacgct gcctgcagag gagaagctca aaatgtatca ctccctggca ggaagtctcc 14700

aggactcagg gattgtactg aaacgagtaa ccatacatct tgaagatctt gccccacacc 14760

ttgacccctt ggcttatgag aaagccaggc atcagatcca gtcctggcaa ggggagttaa 14820

aactgttgac ttctgccatt ggtgagacgg tgacagaatg tgagagccga atggtgcaga 14880

gtatagactt ccagactgag atgagtcgct ccctggactg gctgaggaga gtgaaggcag 14940

agctcagtgg gccggtgtac ctagacctca acctgcagga catccaagag gaaatcagaa 15000

aaatccaaat tcatcaggaa gaggtccagt ccagcttgag aatcatgaat gcgctgagtc 15060

acaaggaaaa ggagaagttc acaaaggcca aggagctgat ttctgcggat ttagaacaca 15120

gcctcgctga gctctcagag ctggatggag acatccagga agccttacgc accagacagg 15180

ctaccttgac tgaaatatat agccagtgtc aaaggtatta tcaggtattt caagcagcca 15240

atgactggct tgaggatgcc caagaattgt tacagctggc aggcaatggc ctagacgtgg 15300

agagcgcaga ggaaaatctc aaaagccaca tggaattttt cagtacagag gatcagttcc 15360

atagtaacct ggaggagctc cacagcctgg tagccaccct ggacccactc atcaagccaa 15420

ccggcaaaga agacctagaa cagaaagtgg cttctctgga actcaggagc cagaggatga 15480

gccgggactc tggtgcccaa gtggatctct tgcagagatg cacagctcaa tggcacgatt 15540

accagaaagc aagggaagag gttattgaat tgatgaatga tacagaaaag aaattgtctg 15600

agttttcttt gttgaagact tcgtctagtc atgaagcgga agaaaaattg tcagaacaca 15660

aggctttagt gtcagtggtt aactctttcc atgagaaaat tgtggccctt gaggaaaaag 15720

cttcacaact ggagaaaacc ggaaatgatg ccagcaaagc caccctgagc aggtcaatga 15780

ccaccgtctg gcagcgctgg acacgccttc gagctgtggc ccaggaccag gagaagatcc 15840

tggaagatgc agtggatgag tggacgggct ttaacaacaa ggttaaaaag gccactgaaa 15900

tgattgatca gctgcaagat aagttacctg gaagttcagc agagaaagca tcgaaagcag 15960

agctcttaac tcttcttgaa taccacgaca cgttcgttct ggagctggag cagcagcagt 16020

cggccttggg catgctgcgg cagcaaaccc tgagcatgct ccaggatgga gccgccccaa 16080

cccctgggga agagcctccg ctcatgcagg aaatcaccgc catgcaagat cggtgcctga 16140

acatgcagga gaaagtgaag actaatggaa agttggtgaa gcaagagctg aaggaccgag 16200

aaatggtgga gactcagatc aattctgtga aatgttgggt tcaggaaacg aaagaatatt 16260

tagggaatcc aacaatagaa atagatgctc aacttgaaga acttcagatt ctcctaacag 16320

aagccacaaa tcaccgacag aacattgaaa aaatggcaga agaacagaag gagaagtact 16380

taggtcttta taccatatta ccttctgaac tctcccttca gttggctgaa gtggcgttag 16440

atctaaagat ccgagatcag atccaagaca aaataaaaga agttgagcag agcaaggcca 16500

cgagccagga actcagccgg caaattcaga agttagctaa agacctcaca actattctaa 16560

ctaagctgaa agcgaagaca gataatgtag ttcaagctaa aactgaccaa aaggtgctgg 16620

gagaggaatt agatggctgt aattcaaagt taatggaatt agatgcagca gtacagaaat 16680

tcttggaaca gaatggccaa ctgggtaagc cactggccaa gaagatagga aaactgactg 16740

aacttcacca gcagaccatt agacaagctg agaatcggct ctccaagctc aatcaggcag 16800

catcacattt agaagaatac aatgaaatgc ttgaattaat tttgaagtgg attgaaaaag 16860

ctaaagtctt ggctcatgga actattgcat ggaattctgc aagccagctt cgggaacaat 16920

atattttgca tcagaccctg ctagaagaat ccaaagaaat tgacagtgag ctggaagcaa 16980

tgactgagaa attacagtac ctcactagcg tgtactgtac agaaaaaatg tctcagcaag 17040

tggcagaact gggacgggag actgaggagt tgcgacagat gatcaaaatt cgtttgcaga 17100

acctccaaga tgcagctaag gatatgaaaa aatttgaagc agagttgaaa aagttacaag 17160

ctgccttgga gcaagcccag gcaacactga cttctccaga agttggacgt ctcagtctca 17220

aggagcagct ctctcatcgg cagcatttgt tgtctgagat ggagtcactg aagccgaagg 17280

tgcaagcagt gcagctctgc cagagtgccc tccggatccc cgaggatgtg gttgccagct 17340

tacctctctg tcatgctgct ctgcggctgc aggaagaggc cagccggctg cagcacaccg 17400

ccatccagca gtgtaacatc atgcaggcag ctgtggtaca atatgaacaa tatgagcaag 17460

aaatgaaaca tctccagcaa ctgatagaag gagctcacag agagattgag gataaacctg 17520

ttgccaccag taacatacag gagctgcagg ctcagatttc tcggcatgag gagctggcgc 17580

agaaaattaa gggctaccag gagcagatcg cttctttgaa ttccaagtgc aagatgctga 17640

cgatgaaagc caagcacgcc accatgctgc tgacggtgac cgaggtcgag gggctggcgg 17700

aagggacaga ggacctggat ggggagctcc tccccacgcc ttcggcccac ccctctgtgg 17760

tcatgatgac tgcaggtcgc tgtcacactt tgctgtcacc ggtcactgag gagtctgggg 17820

aggagggaac caacagtgag atttcctctc cacctgcctg tcgctcccct tcacctgtgg 17880

ctaatacaga tgcttctgtt aaccaggaca ttgcatatta ccaagccttg tctgctgaga 17940

ggttgcagac agatgctgca aaaattcacc ccagcacatc cgcatcccag gagttctatg 18000

aaccgggatt ggagccatcc gctactgcca aactgggtga tttgcagcgt tcttgggaaa 18060

ccttaaagaa tgtgatcagt gagaagcagc gcacactcta tgaagctttg gaacgccagc 18120

agaagtacca ggactccctc cagtccatct ctacgaagat ggaggccatt gagctgaaac 18180

tcagtgagag cccagagcct ggcaggagtc cagaaagcca gatggctgaa catcaggcat 18240

tgatggatga gattctcatg ctccaggatg aaatcaatga gctccagtcc tctctcgcag 18300

aggagctggt atccgagtct tgtgaggccg accctgcgga gcagctggcc ttgcagtcca 18360

cgctcactgt cttagccgag cgaatgtcca ccatcaggat gaaagcctcg gggaaacggc 18420

agcttttgga ggagaagttg aatgatcagc tggaggaaca aaggcaggaa caggccctgc 18480

agaggtatcg ctgtgaagcc gatgagctgg acagctggct cttgagtacc aaggccactc 18540

tggacactgc gctgagtcca cccaaggagc ccatggacat ggaggcccag cttatggact 18600

gccagaatat gctggtggaa atagagcaga aggtggtggc tttatcagaa ctgtcagtcc 18660

acaatgagaa cctgctgctg gagggcaaag ctcacaccaa ggacgaggcc gagcagctgg 18720

ctggaaagct gagaaggctc aaggggagcc tgctggagct gcagagagcc ctgcatgata 18780

agcagctcaa catgcaggga acagcacagg agaaggagga gagcgatgtt gacctaacag 18840

ccacgcagag ccccggcgtc caggaatggc tggcccaagc tcgcaccaca tggacccagc 18900

agcggcagag cagtctccag caacaaaaag agttagaaca ggaattagcc gagcagaaga 18960

gtctccttcg ctcagtagcc agtcgtggag aggagattct aattcaacat tcggcggcag 19020

agacctctgg tgatgctggc gaaaaacctg atgtgttatc ccaggagttg gggatggaag 19080

gggagaaatc atccgctgaa gaccagatga gaatgaaatg ggaaagccta catcaagaat 19140

ttagtaccaa gcagaaacta ctacagaatg ttctggaaca ggaacaagag caagtgcttt 19200

atagcaggcc aaatcgactc ttgtctggtg tgccactgta caaaggggac gtgccaaccc 19260

aagataaatc tgcagttaca tctttgctgg atggactgaa ccaagccttc gaggaggttt 19320

catcccagag tggaggggca aagaggcaga gtatacactt ggagcagaag ttgtatgatg 19380
gagtctcagc cacctctact tggttggatg acgttgaaga acgtttattt gttgccacag 19440
cacttttacc agaagaaaca gagacttgtc tcttcaacca agagattctt gccaaagaca 19500
ttaaggaaat gtctgaagaa atggataaga acaaaaactt gttttcccaa gcttttccag 19560
agaatggtga taatcgagat gttattgaag atactttggg ttgtcttttg ggcaggttat 19620
ccttgctaga ctcagtagtg aatcaacgat gtcatcagat gaaagaaaga cttcagcaaa 19680
tactaaattt ccagaatgat ctgaaagtgc tgtttacatc actggctgac aacaaataca 19740
tcattctgca aaaactggca aatgtgtttg aacagcccgt agcagaacaa atagaggcaa 19800
tacaacaggc tgaagatgga ctcaaagaat ttgatgcagg aatcattgaa ttaaagaggc 19860
gtggtgacaa gctacaggtc gagcagccgt ccatgcaaga actctccaag ctccaggaca 19920
tgtatgatga gctgatgatg atcattggct cccggaggag tggtctgaat cagaacctta 19980
cactcaagag tcagtatgag agggccctac aagatctggc tgacctgcta gaaactggtc 20040
aggagaagat ggcaggagac cagaaaatca tcgtgtcttc caaagaggaa atccagcaac 20100
tacttgacaa acataaggaa tactttcagg gcctggaatc tcatatgatc ttgactgaaa 20160
cactcttcag aaagataatc agctttgcag tccaaaagga aacccagttc catacagagc 20220
tgatggctca ggcttctgct gtactgaaac gggctcacaa gaggggtgtg gagctggagt 20280
acattctaga gacgtggtcc catctggatg aggaccagca ggagctcagc agacagctgg 20340
aggtggtgga aagcagcatc ccaagcgtgg gtctggtgga ggagaacgag gacaggctta 20400
ttgaccgcat aacactctac cagcatttaa aatctagcct taatgaatac cagcccaaat 20460
tatatcaagt attagatgat gggaaacgac ttctgatatc catcagctgc tcagatctag 20520
aaagccaact aaatcaactt ggagagtgct ggctaagtaa caccaataaa atgtctaagg 20580
aacttcacag actggaaaca atattgaaac actggaccag atatcaaagt gaatctgcag 20640
atctaattca ctggttacaa tctgcaaaag accggctaga attttggact cagcaatctg 20700
tgacagtccc acaagagctg gaaatggtcc gtgatcatct aaatgctttc ctggagtttt 20760
ctaaagaagt ggatgcccaa tcttccctga aatcatctgt tctgagtact ggaaatcagc 20820
tccttcgact aaaaaaggtg gacacagcca cgctgcgctc tgagctgtcg cgcattgata 20880
gccagtggac tgacctgcta accaatatcc cagccgtcca ggagaagctc caccagctcc 20940
agatggataa actgccttcc cgccatgcca tttctgaagt catgagttgg atttctctaa 21000
tggaaaatgt tattcagaag gatgaagata atattaaaaa ttccataggt tacaaggcaa 21060
ttcatgaata ccttcagaaa tataagggtt ttaagataga cattaactgt aaacagctga 21120
cagtggattt tgtgaaccag tccgtgctac aaatcagcag tcaggatgtg gaaagtaagc 21180
gtagtgataa gactgatttt gctgagcaac ttggagcaat gaataaaagt tggcaaattc 21240

tgcaaggtct agtaactgag aagatccagc tgttggaagg cttattggaa tcttggtcag 21300

aatatgaaaa taatgtacaa tgtctgaaaa catggtttga aacccaggaa aagagactaa 21360

aacaacagca tcgaattgga gatcaggctt ctgttcaaaa tgcactgaaa gactgtcagg 21420

atctggaaga tttgattaaa gcaaaagaaa aagaagtaga gaaaattgag cagaatggac 21480

ttgctttgat tcagaacaag aaagaagacg tctctagcat tgtcatgagc acactgcgag 21540

agctcggcca aacctgggca aatttagatc acatggttgg acaattaaag atactgctga 21600

aatcagtgct tgaccaatgg agtagtcaca aagtggcctt tgacaagata aacagttacc 21660

tcatggaggc cagatactct ctttcccgat tccgtctgct gactggctcc ttagaagctg 21720

tgcaagttca ggtggacaat cttcagaatc tccaagatga tctggaaaaa caggaaagga 21780

gcttacagaa atttggctct atcaccaacc aattattaaa agagtgtcac ccacccgtga 21840

cagaaactct taccaataca ctgaaagaag tcaacatgag atggaataac ttgctggaag 21900

agattgctga gcagctacag tccagcaagg ccctacttca gctttggcaa agatacaagg 21960

actactccaa acagtgtgct tcgacagttc agcagcagga ggatcgaacc aatgagctgt 22020

tgaaggcagc cacaaacaag gacattgccg atgatgaggt tgccacatgg attcaagatt 22080

gcaacgacct cctcaaagga ctgggcacag ttaaagattc cctctttttt ctccatgagc 22140

tgggagagca actgaagcaa caagtggatg cttccgcagc atcagctatt caatcggatc 22200

aactctcttt gagtcaacac ttgtgtgccc tggagcaagc tctctgcaaa cagcagactt 22260

cattacaggc tggagttctt gattatgaaa cctttgccaa gagtttagaa gctttggagg 22320

cctggatagt ggaagctgaa gaaatactac aagggcagga ccctagccac tcatctgacc 22380

tctccacaat ccaggaaagg atggaagaac ttaagggaca gatgttaaaa ttcagcagca 22440

tggctccaga tttagaccgt ctaaatgagc ttggatatag gttacccttg aatgataagg 22500

aaatcaaaag aatgcagaat ctgaaccgcc attggtctct gatctcctct cagactacag 22560

aaagattcag caagttgcag tcatttttgc tacaacatca gactttcttg gaaaaatgtg 22620

aaacatggat ggaattccta gttcagacag aacaaaagtt agcagtagag atttcaggaa 22680

attatcagca ccttttggaa cagcagagag cacacgagtt gtttcaagcc gagatgttca 22740

gtcgtcagca gattttgcac tcaatcatta ttgatgggca acgtcttcta gaacaaggtc 22800

aagttgatga cagggatgaa ttcaacctga aattgacact cctcagtaat caatggcagg 22860

gagtgattcg cagggcccag cagaggcggg ggatcattga cagccagatt cgccagtggc 22920

agcgctatag ggagatggca gaaaagcttc gtaaatggtt ggttgaagtg tcctacctcc 22980

ccatgagtgg tctcggaagt gttcctatac cactgcaaca agcaaggacc ctctttgatg 23040

aagtgcagtt caaagaaaaa gtgtttctgc ggcaacaagg cagctacatc ctgactgtgg 23100

aggctggcaa gcaactcctt ctctcggcgg acagtggcgc tgaggccgcc ttgcaggccg 23160
aactcgctga aatccaagag aaatggaaat cagccagcat gcggctggaa gaacagaaga 23220
aaaaactagc cttcttgttg aaagactggg aaaaatgtga gaaaggaata gcagattccc 23280
tggagaaact acgaactttc aaaaagaagc tttcgcagtc tctcccggat caccatgaag 23340
agctccatgc agaacaaatg cgttgcaagg aattagaaaa tgcagttggg agctggacag 23400
atgacttgac ccagttgagc ctgctgaagg acaccctctc tgcctatatc agtgctgatg 23460
atatctccat tcttaatgaa cgcgtagagc ttctgcaaag gcagtgggaa gaactatgcc 23520
accagctctc cttaaggcgg cagcaaatag gtgaaagatt gaatgaatgg gcagtcttca 23580
gtgaaaagaa caaggaactc tgtgagtggt tgactcaaat ggaaagcaaa gtttctcaga 23640
atggagacat tctcattgaa gaaatgatag agaagctcaa gaaggattat caagaggaaa 23700
ttgctattgc tcaagagaac aaaatacagc tccaacaaat gggagaacga cttgctaaag 23760
ccagccatga aagcaaagca tctgagattg aatacaagct gggaaaggtc aacgaccggt 23820
ggcagcatct cctggacctc attgcagcca gggtgaagaa gctgaaggag accctggtag 23880
ccgtgcagca gcttgataag aacatgagca gcctgaggac ctggctcgct cacatcgagt 23940
cagagctggc caagccaata gtctacgatt cctgtaactc ggaagaaata cagagaaagc 24000
ttaatgagca gcaggagctt cagagagaca tagagaagca cagtacaggt gttgcatctg 24060
tcctcaacct gtgtgaagtc ctgctgcacg actgtgacgc ctgtgccact gatgccgagt 24120
gtgactctat acagcaggct acgagaaacc tggaccggcg gtggagaaac atttgtgcta 24180
tgtccatgga aaggaggctg aaaatcgaag agacgtggcg attgtggcag aaatttctgg 24240
atgactattc acgttttgaa gattggctga agtcttcaga aaggacagct gcttttccca 24300
gctcttctgg ggtgatctat acagttgcca aggaagaact aaagaaattt gaggctttcc 24360
agcgacaggt ccacgagtgc ctgacgcagc tggaactgat caacaagcag taccgccgcc 24420
tggccaggga gaaccgcact gattcagcat gtagcctcaa acagatggtt cacgaaggca 24480
accagagatg ggacaacctg caaaagcgtg tcacctccat cttgcgcaga ctcaagcatt 24540
ttattggcca gcgtgaggag tttgagactg cgcgggacag cattctggtc tggctcacag 24600
agatggatct gcagctcact aatattgaac atttttctga gtgtgatgtt caagctaaaa 24660
taaagcaact caaggccttc cagcaggaaa tttcactgaa ccacaataag attgagcaga 24720
taattgccca aggagaacag ctgatagaaa agagtgagcc cttggatgca gcgatcatcg 24780
aggaggaact agatgagctc cgacggtact gccaggaggt cttcgggcgt gtggaaagat 24840
accataagaa actgatccgc ctgcctctcc cagacgatga gcacgacctc tcagacaggg 24900
agctggagct ggaagactct gcagctctgt cggacctgca ctggcacgac cgctctgcag 24960
acagcctgct ttctccacag ccttcctcca atctctccct ctcgctcgct cagcccctcc 25020

ggagcgagcg gtcaggacga gacaccccgg ctagtgtgga ctccatcccc ctggagtggg 25080

atcacgacta tgacctcagt cgggacctgg agtctgcaat gtccagagct ctgccctctg 25140

aggatgaaga aggtcaggat gacaaagatt tctacctccg gggagctgtt ggcttatcag 25200

atgtaatgat ccccgaaagc cctgaggcct atgtaaaact cacagaaaat gcaatcaaaa 25260

atacctccgg ggaccacagt gccctagagt cacagatccg acaactgggc aaagccctgg 25320

atgatagccg ttttcagata cagcaaaccg aaaatatcat tcgcagcaaa actcccacgg 25380

ggccggagct agacaccagc tacaaaggct acatgaaact gctgggcgaa tgcagtagca 25440

gtatagactc cgtgaagaga ctggagcaca aactgaagga ggaagaggag agccttcctg 25500

gctttgttaa cctgcatagt accgaaaccc aaacggctgg tgtgattgac cgatgggagc 25560

ttctccaggc ccaggcattg agcaaggagt tgaggatgaa gcagaacctc cagaagtggc 25620

agcagtttaa ctcagacttg aacagcatct gggcctggct gggggacacg gaggaggagt 25680

tggaacagct ccagcgtctg gaactcagca ctgacatcca gaccatcgag ctccagatca 25740

aaaagctcaa ggagctccag aaagctgtgg accaccgcaa agccatcatc ctctccatca 25800

atctctgcag ccctgagttc acccaggctg acagcaagga gagccgggac ctgcaggatc 25860

gcttgtcgca gatgaatggg cgctgggacc gagtgtgctc tctgctggag gagtggcggg 25920

gcctgctgca ggatgccctg atgcagtgcc agggtttcca tgaaatgagc catggtttgc 25980

ttcttatgct ggagaacatt gacagaagga aaaatgaaat tgtccctatt gattctaacc 26040

ttgatgcaga gatacttcag gaccatcaca aacagcttat gcaaataaag catgagctgt 26100

tggaatccca actcagagta gcctctttgc aagacatgtc ttgccaacta ctggtgaatg 26160

ctgaaggaac agactgttta gaagccaaag aaaaagtcca tgttattgga aatcggctca 26220

aacttctctt gaaggaggtc agtcgtcata tcaaggaact ggagaagtta ttagacgtgt 26280

caagtagtca gcaggatttg tcttcctggt cttctgctga tgaactggac acctcagggt 26340

ctgtgagtcc cacatcagga aggagcaccc caaacagaca gaaaacgcca cgaggcaagt 26400

gtagtctctc acagcctgga ccctctgtca gcagtccaca tagcaggtcc acaaaaggtg 26460

gctccgattc ctccctttct gagccagggc caggtcggtc cggccgcggc ttcctgttca 26520

gagtcctccg agcagctctt ccccttcagc ttctcctgct cctcctcatc gggcttgcct 26580

gccttgtacc aatgtcagag gaagactaca gctgtgccct ctccaacaac tttgcccggt 26640

cattccaccc catgctcaga tacacgaatg gccctcctcc actctgaact aagcagatgc 26700

catctgcaga agtgctggta gcataaggag gatcgggtca taagcaatcc caaactacca 26760

acaagaggac cttgatcttg gcgaaagccc tcggtgtggc agctttagcc ctcctccaga 26820

tcacatgtgt gcaaattatg gcttcagagg tggaagataa acagtgacgg gggaacaaac 26880

agacaacaag aaggtttgga agaaatctgg tttgagactc tgaaccttag cactaaggag 26940

attgagtaag gacctccaaa gttccccgga ctcatgaatt ctgggccctt ggcccattct 27000

gtgcacagcc aaggacttca gtagaccatc tgggcagctt tcccatggtg ctgctccaac 27060

catcagataa atgaccctcc caagcaccat gtcagtgtcg tacaatctac caaccaacca 27120

gtgctgaaga gattttagaa ccttgtaaca tacaattttt aagagcttat atggcagctt 27180

cctttttacc ttgttttcct ttggggcatg atgttttaac ctttgcttta gaagcacaag 27240

ctgtaaatct aaaaggcact tttttttaga ggtataaaga aaaactagat gtaataaata 27300

agatcatgga aggctttatg tgaaaaaagt tgaatgttat agtaaaaaaa aaaagatatt 27360

tatgtatgta cagtttgcta aagccaagtt ttgtttgtat tgatttcttt gcatttatta 27420

tagatattat aaaata 27436

<210> 31
<211> 3894
<212> DNA
<213> Homo sapiens

<400> 31
aaggtaaagc cactagagag aaactgaaag aaaacattct taagataaat tgaattgaca 60

ttttctctct aaaatatgat ttatagacca cagataggaa ttaagagttt cctgataatt 120

ttggcttcat attattttaa aggattatca agaggaaatt gctattgctc aagagaacaa 180

aatacagctc caacaaatgg gagaacgact tgctaaagcc agccatgaaa gcaaagcatc 240

tgagattgaa tacaagctgg gaaaggtcaa cgaccggtgg cagcatctcc tggacctcat 300

tgcagccagg gtgaagaagc tgaaggagac cctggtagcc gtgcagcagc ttgataagaa 360

catgagcagc ctgaggacct ggctcgctca catcgagtca gagctggcca agccaatagt 420

ctacgattcc tgtaactcgg aagaaataca gagaaagctt aatgagcagc aggagcttca 480

gagagacata gagaagcaca gtacaggtgt tgcatctgtc ctcaacctgt gtgaagtcct 540

gctgcacgac tgtgacgcct gtgccactga tgccgagtgt gactctatac agcaggctac 600

gagaaacctg gaccggcggt ggagaaacat ttgtgctatg tccatggaaa ggaggctgaa 660

aatcgaagag acgtggcgat tgtggcagaa atttctggat gactattcac gttttgaaga 720

ttggctgaag tcttcagaaa ggacagctgc ttttcccagc tcttctgggg tgatctatac 780

agttgccaag gaagaactaa agaaatttga ggctttccag cgacaggtcc acgagtgcct 840

gacgcagctg gaactgatca acaagcagta ccgccgcctg gccagggaga accgcactga 900

ttcagcatgt agcctcaaac agatggttca cgaaggcaac cagagatggg acaacctgca 960

aaagcgtgtc acctccatct tgcgcagact caagcatttt attggccagc gtgaggagtt 1020

tgagactgcg cgggacagca ttctggtctg gctcacagag atggatctgc agctcactaa 1080

tattgaacat ttttctgagt gtgatgttca agctaaaata aagcaactca aggccttcca 1140

gcaggaaatt tcactgaacc acaataagat tgagcagata attgcccaag gagaacagct 1200

gatagaaaag agtgagccct tggatgcagc gatcatcgag gaggaactag atgagctccg 1260

acggtactgc caggaggtct tcgggcgtgt ggaaagatac cataagaaac tgatccgcct 1320

gcctctccca gacgatgagc acgacctctc agacagggag ctggagctgg aagactctgc 1380

agctctgtcg gacctgcact ggcacgaccg ctctgcagac agcctgcttt ctccacagcc 1440

ttcctccaat ctctccctct cgctcgctca gcccctccgg agcgagcggt caggacgaga 1500

caccccggct agtgtggact ccatccccct ggagtgggat cacgactatg acctcagtcg 1560

ggacctggag tctgcaatgt ccagagctct gccctctgag gatgaagaag gtcaggatga 1620

caaagatttc tacctccggg gagctgttgg cttatcagat gtaatgatcc ccgaaagccc 1680

tgaggcctat gtaaaactca cagaaaatgc aatcaaaaat acctccgggg accacagtgc 1740

cctagagtca cagatccgac aactgggcaa agccctggat gatagccgtt ttcagataca 1800

gcaaaccgaa aatatcattc gcagcaaaac tcccacgggg ccggagctag acaccagcta 1860

caaaggctac atgaaactgc tgggcgaatg cagtagcagt atagactccg tgaagagact 1920

ggagcacaaa ctgaaggagg aagaggagag ccttcctggc tttgttaacc tgcatagtac 1980

cgaaacccaa acggctggtg tgattgaccg atgggagctt ctccaggccc aggcattgag 2040

caaggagttg aggatgaagc agaacctcca gaagtggcag cagtttaact cagacttgaa 2100

cagcatctgg gcctggctgg gggacacgga ggaggagttg gaacagctcc agcgtctgga 2160

actcagcact gacatccaga ccatcgagct ccagatcaaa aagctcaagg agctccagaa 2220

agctgtggac caccgcaaag ccatcatcct ctccatcaat ctctgcagcc ctgagttcac 2280

ccaggctgac agcaaggaga gccgggacct gcaggatcgc ttgtcgcaga tgaatgggcg 2340

ctgggaccga gtgtgctctc tgctggagga gtggcggggc ctgctgcagg atgccctgat 2400

gcagtgccag ggtttccatg aaatgagcca tggtttgctt cttatgctgg agaacattga 2460

cagaaggaaa aatgaaattg tccctattga ttctaacctt gatgcagaga tacttcagga 2520

ccatcacaaa cagcttatgc aaataaagca tgagctgttg gaatcccaac tcagagtagc 2580

ctctttgcaa gacatgtctt gccaactact ggtgaatgct gaaggaacag actgtttaga 2640

agccaaagaa aaagtccatg ttattggaaa tcggctcaaa cttctcttga aggaggtcag 2700

tcgtcatatc aaggaactgg agaagttatt agacgtgtca agtagtcagc aggatttgtc 2760

ttcctggtct tctgctgatg aactggacac ctcagggtct gtgagtccca catcaggaag 2820

gagcacccca aacagacaga aaacgccacg aggcaagtgt agtctctcac agcctggacc 2880

ctctgtcagc agtccacata gcaggtccac aaaaggtggc tccgattcct ccctttctga 2940

gccagggcca ggtcggtccg gccgcggctt cctgttcaga gtcctccgag cagctcttcc 3000

ccttcagctt ctcctgctcc tcctcatcgg gcttgcctgc cttgtaccaa tgtcagagga 3060

agactacagc tgtgccctct ccaacaactt tgcccggtca ttccacccca tgctcagata 3120

cacgaatggc cctcctccac tctgaactaa gcagatgcca tctgcagaag tgctggtagc 3180

ataaggagga tcgggtcata agcaatccca aactaccaac aagaggacct tgatcttggc 3240

gaaagccctc ggtgtggcag ctttagccct cctccagatc acatgtgtgc aaattatggc 3300

ttcagaggtg gaagataaac agtgacgggg gaacaaacag acaacaagaa ggtttggaag 3360

aaatctggtt tgagactctg aaccttagca ctaaggagat tgagtaagga cctccaaagt 3420

tccccggact catgaattct gggcccttgg cccattctgt gcacagccaa ggacttcagt 3480

agaccatctg ggcagctttc ccatggtgct gctccaacca tcagataaat gaccctccca 3540

agcaccatgt cagtgtcgta caatctacca accaaccagt gctgaagaga ttttagaacc 3600

ttgtaacata caatttttaa gagcttatat ggcagcttcc tttttacctt gttttccttt 3660

ggggcatgat gttttaacct ttgctttaga agcacaagct gtaaatctaa aaggcacttt 3720

tttttagagg tataaagaaa aactagatgt aataaataag atcatggaag gctttatgtg 3780

aaaaaagttg aatgttatag taaaaaaaaa aagatattta tgtatgtaca gtttgctaaa 3840

gccaagtttt gtttgtattg atttctttgc atttattata gatattataa aata 3894

<210> 32
<211> 27745
<212> DNA
<213> Homo sapiens

<400> 32
cccgggcagt ggaaaccgtg ggcaaaagtt agctggcagg acagcgcagc tcctccaggc 60

agcggaggca gcgcgtcccg gctctcaggg acatttcctt cccacctcga cccccgggag 120

gtggtcccgg tataaaggct cgctgagcgg gtgggtcaca gcacagcttt gcagctgcgg 180

agaaacgccc aaggccgtgc atctccagga gggcggctgg gctcccgcag tcctgcagac 240

cgcgcccgat cccggcgaca gggcgggcgg acagccgcgc atccccgggg tcccgccgag 300

cctgggcgca gagagccggg aggaagcgtt cgctcgcttc gccttgctgc tgggaaactg 360

aacgaggccg agagagaagg ttcttgagtt catgtaagag gacagtctta aaacggaaga 420

agaaaaagaa gcagttcagt ctttgggaga gctgcctcct tgttgagtgc tgcaaaggcc 480

tggaattcat ttatgacaga atagatctag aaaagtccaa gcatgttttc tagagtggtg 540

tagccctgtg ctgcctccag tgaagagtct cttggtgttg gcttcgtgct tccggaggga 600

ccatggcaac ctccagaggg gcctcccggt gtcctcggga tatcgccaat gtgatgcaga 660

ggctgcaaga tgagcaagag atagtacaaa aacgaacttt cacaaaatgg atcaactctc 720

atctggccaa gcggaaacct ccaatggtgg tggacgatct ttttgaagac atgaaagatg 780

gtgttaaact gcttgccctt ctggaggtcc tgtctgggca gaaactgcct tgtgaacaag 840

gacgccggat gaagcgaatc catgctgtgg ctaacattgg cacggcactc aagttcctcg 900

aaggaagaaa gattaaatta gtcaacatta actccaccga tatagctgat ggccgaccct 960

caatagttct tggattgatg tggaccatta ttctatattt ccagattgaa gagttgacca 1020

gcaacctgcc ccagctccag tctttgtcca gcagcgcatc ctccgtggac agcatagtta 1080

gctctgagac tcccagccca ccaagtaaac ggaaggtgac caccaagatc caaggaaatg 1140

ctaagaaggc tttattaaag tgggttcagt acacagctgg caagcagact ggaatagaag 1200

taaaagattt tgggaagagt tggagaagcg gggttgcctt tcattcagtt attcatgcca 1260

ttcgaccgga attggtggac ttggagacag tgaaaggcag atccaaccga gaaaatttgg 1320

aggatgcttt cactatcgcc gaaacagaac tggggatccc aagactgcta gatcctgaag 1380

acgttgatgt ggataaacca gatgagaaat ctattatgac ctatgtagcc cagtttctga 1440

aacattatcc tgacatccac aatgcaagca ctgatgggca agaggatgat gaaatacttc 1500

caggtttccc atcttttgca aattctgtac aaaattttaa gagagaagac agagtaattt 1560

ttaaggaaat gaaagtttgg atagaacaat ttgagagaga tttgacaaga gcacagatgg 1620

tggaatcaaa tttacaggat aaatatcagt catttaagca cttcagagtt caatatgaaa 1680

tgaagaggaa acagattgaa catttaatac aaccattaca cagagacggt aaattgtcac 1740

ttgaccaagc attggtaaaa caatcttggg atagagtgac ctccaggctc tttgactggc 1800

atatacagct tgataaatct cttcctgcac ctctgggcac cataggtgcc tggctgtaca 1860

gagcggaggt ggccctgaga gaggaaataa ccgttcaaca ggtccacgag gaaacagcaa 1920

acacgataca acggaaactt gagcaacata aggatctgct tcaaaacacg gatgcccaca 1980

aaagagcatt ccatgaaatc taccggacca ggtctgttaa cgggattcca gtgccacctg 2040

atcaattaga ggacatggcc gagaggtttc attttgtttc ctccacatca gagctacacc 2100

taatgaaaat ggaattttta gaattaaagt accgtctgct ctcactgctg gttcttgcag 2160

agtcaaagct gaagtcttgg atcattaagt acgggaggag agagtcagtg gagcagcttc 2220

tacaaaacta cgtgtctttt atagaaaata gcaagttctt tgaacaatat gaggtgacat 2280

accagatctt gaaacagaca gctgagatgt atgtcaaagc agatggttca gtggaagaag 2340

ctgagaatgt gatgaaattc atgaatgaaa ccaccgctca gtggaggaat ctctcagtag 2400

aagtgaggag tgtgaggagc atgctggaag aagtgatctc taactgggat cgctatggca 2460

atacagtggc tagtctgcaa gcctggctag aggatgctga aaaaatgctc aatcaatcag 2520

aaaatgccaa aaaggatttt tttcgaaatt tacctcattg gattcagcag catactgcca 2580

tgaacgatgc tggcaatttt ctaattgaaa cctgtgatga gatggtttcc cgtgacctga 2640

agcagcaatt actgttgcta aatgggcggt ggagggagtt gtttatggaa gtcaagcaat 2700

atgctcaagc tgatgagatg gacagaatga agaaggaata cacagactgt gttgttaccc 2760

tgtctgcttt tgcaacggaa gcccataaga aactttctga acccttagaa gtctctttta 2820

tgaatgtcaa gctattaatt caagacttgg aggatattga gcagagggtg cctgtgatgg 2880

atgcccaata caagataatt acaaagacag cacacctcat taccaaagaa agcccccaag 2940

aagaaggaaa agaaatgttt gcgaccatgt caaagctcaa agagcagcta accaaggtca 3000

aagaatgtta ctccccactc ctttatgagt ctcagcagct gttgattccg ttggaggaat 3060

tagaaaagca gatgacgtcc ttttatgact cacttgggaa aatcaatgaa attatcacag 3120

ttcttgagcg tgaggcacaa tcgagtgccc tttttaaaca aaaacatcag gaactgttag 3180

cttgtcaaga aaactgtaag aaaaccttga cacttattga gaaaggcagt caaagtgttc 3240

aaaagtttgt gaccttgagc aacgtgttaa agcattttga tcagacgagg ctacaaagac 3300

agattgcaga tattcatgtt gcttttcaga gtatggtaaa gaaaactgga gattggaaga 3360

agcatgtgga aaccaacagt cgcttgatga agaagtttga ggagtctcga gcagagttgg 3420

agaaggtact gcggattgct caggagggcc tggaggaaaa gggggatcca gaggagctcc 3480

tgcggagaca cactgagttt ttcagtcagc tggatcagag ggtgctcaat gctttcctga 3540

aagcttgtga tgaactcacc gacatccttc cagagcagga gcagcagggg ctgcaggaag 3600

ctgttcgaaa gctccacaaa caatggaagg atcttcaagg agaagcccct tatcatttgc 3660

ttcatctgaa gattgatgtg gagaagaata ggttcttagc ctctgtagaa gaatgcagaa 3720

ctgagctgga tcgagagacc aagctgatgc cccaggaagg cagtgaaaag ataattaaag 3780

agcacagggt tttcttcagt gacaaaggtc ctcatcatct ctgtgagaaa aggttacagc 3840

tcatcgagga actctgtgtg aaactcccag tgcgggaccc agtaagggac acacctggaa 3900

cctgtcacgt gactctcaaa gagctcagag ctgccattga cagcacctac aggaagctca 3960

tggaagaccc agacaagtgg aaggactaca ctagcagatt ctctgagttc tcatcttgga 4020

tatctacaaa tgagacacaa ttaaagggga tcaagggtga ggccatcgat actgccaacc 4080

acggagaggt taaacgtgcc gttgaagaga tcagaaatgg tgttaccaaa aggggtgaga 4140

ccctcagctg gctgaaatcc aggctgaaag ttttgacaga agtttcttct gagaatgaag 4200

cccaaaagca gggagatgag ctggcaaaat tatccagctc tttcaaggct cttgtgacgc 4260

tgctgtcaga ggttgaaaag atgctaagca attttgggga ctgtgtccag tacaaagaaa 4320

tagtcaaaaa ttctctcgaa gaattaattt ctggctctaa agaagtccag gaacaagctg 4380

agaagatctt ggatactgaa aatctgtttg aagcacagca gttacttctt catcaccagc 4440

aaaagacaaa gcggatctca gcaaagaaga gagatgtgca gcagcagatc gcgcaggcgc 4500

agcagggaga aggggggctg cctgaccgag gccacgagga gctgcggaag ctggagagca 4560

cactggatgg cctggagcgc agccgggaga ggcaggaacg ccgcatccag gtcacattaa 4620

gaaaatggga gcgatttgaa acaaacaaag aaacagtagt aagatacctt tttcaaacag 4680

gttccagtca tgaacgcttc ttgagtttta gcagtttgga aagtttatct tcagaactgg 4740

aacaaacaaa ggagttttct aaacggacag aaagtattgc agtccaggct gagaaccttg 4800

taaaggaagc ttcagagata ccgcttgggc cccaaaataa gcagctgctt caacagcagg 4860

ccaagtcaat caaagaacaa gtcaaaaaat tagaagacac gcttgaagaa gatattaaaa 4920

ccatggaaat ggtgaaaacc aagtgggatc attttggcag taattttgag actctgtccg 4980

tctggataac tgagaaagaa aaagaactca atgccttgga aacttcgtca tctgccatgg 5040

acatgcaaat cagccaaatt aaggtcacaa ttcaggaaat agaaagtaag ctcagcagca 5100

ttgtaggatt agaagaagaa gcccagtctt ttgctcagtt tgttaccact ggagaatctg 5160

ctcgaattaa agccaagttg acacaaataa gaagatacgg ggaagagctt cgagagcatg 5220

cacagtgtct ggaaggaaca atcctgggac atttatctca gcagcaaaag tttgaagaga 5280

accttagaaa gatccagcaa tctgtgtctg aatttgaaga taaacttgct gttccaatta 5340

aaatatgttc ttcagctaca gaaacataca aagttcttca agaacatatg gatctctgcc 5400

aggccctgga gtcactgagc agcgcgatca ctgccttctc agccagtgcc aggaaggttg 5460

tgaacagaga ttcctgtgtt caggaggctg cggctctaca gcagcaatac gaggacatcc 5520

taaggagggc gaaggagaga cagacggcgc tggagaatct gctggcccac tggcagaggc 5580

tagagaaaga actatcatcc tttttgacct ggttagagcg gggtgaagct aaagccagtt 5640

ccccagaaat ggacatttct gcagacagag tcaaagtgga aggtgaactt cagttaatac 5700

aggcactgca aaatgaagtt gtatcccagg cctcattcta tagcaaactt ttgcaattga 5760

aggaatcatt gttctcagta gcctccaaag atgatgtgaa aatgatgaaa ctacatttgg 5820

agcagttgga tgagagatgg agagatttac cacagatcat taacaaaagg attaattttc 5880

ttcagtctgt ggttgctgaa caccagcaat ttgatgagct gctgctttcc ttttctgtct 5940

ggattaaact gtttctcagt gaattacaaa ctacctctga gattagcata atggaccatc 6000

aagtagccct tactcggcat aaggaccacg cagcagaagt agagagcaaa aagggcgaat 6060

tgcagagtct gcagggtcac ttagcaaagt tgggttctct gggccgtgct gaggacctcc 6120

acctcctgca gggaaaggct gaggactgct tccagctgtt tgaggaggcc agccaggttg 6180

tggagaggcg gcagcttgcc ctgtcccatt tggcagaatt cctccagagc catgcctctc 6240

tgtccggcat tctccgccag ctgaggcaaa cagtggaagc aaccaacagt atgaataaga 6300

acgagtctga tttgatagaa aaggacctca atgatgctct tcaaaatgct aaagcattag 6360

aatctgctgc cgtcagtctg gatggcattc tttccaaagc ccaataccat ctgaaaatcg 6420

ggagctctga gcaaaggact tcctgcagag ccacggctga tcagctctgt ggagaggtag 6480

agaggatcca gaaccttctg ggaaccaagc agagtgaggc agatgctctg gcagtgttga 6540

aaaaagcatt ccaagaccag aaagaggagc ttctgaaaag cattgaggac attgaagaaa 6600

ggactgacaa agagcgattg aaagaaccta cccgccaagc tcttcagcag aggttaagag 6660

tgtttaatca gctagaagat gaattgaatt ctcacgagca tgaactatgt tggttgaaag 6720

acaaagccaa gcaaattgcc cagaaagatg tagcttttgc acctgaagtt gacagggaga 6780

taaaccgctt agaggtcacc tgggatgata ccaaaagact aattcatgaa aatcagggtc 6840

agtgctgtgg acttattgac ttaatgagag aatatcagaa cctgaaatca gctgtatcta 6900

aagtcttaga aaatgccagc agtgtgattg taaccagaac taccataaaa gatcaggagg 6960

atcttaaatg ggctttttcc aagcatgaaa ctgccaagaa caaaatgaat tacaaacaga 7020

aagacttgga taactttacc agcaaaggaa aacacttgtt atctgagctg aagaaaattc 7080

acagtagtga tttcagcttg gtgaaaacag acatggagag caccgtggac aaatggctgg 7140

atgtatcaga gaaacttgaa gaaaacatgg ataggctgag agtaagcctg tccatttggg 7200

atgatgtact gtcaactaga gatgagattg agggatggtc aaacaactgc gttccacaga 7260

tggcagaaaa catcagcaac ctggataacc acctcagagc tgaagaactg cttaaagaat 7320

ttgagtctga agttaaaaac aaagcattga gattggaaga actgcattcc aaagttaatg 7380

atctgaaaga attaactaaa aatctagaaa caccgccaga ccttcagttt atagaagcag 7440

acttaatgca gaaactggag catgccaaag aaataactga agtagcaaaa ggaaccctga 7500

aggatttcac ggctcaaagt acacaagtgg agaagtttat taatgacata acaacatggt 7560

tcacaaaagt ggaagaatcg ttgatgaact gtgcccaaaa tgagacttgt gaagcattga 7620

aaaaagtcaa ggatatacaa aaagaacttc aaagtcaaca aagcaacatc agctctaccc 7680

aagaaaatct caatagcttg tgccgcaagt accactcagc tgagttggag agcctgggcc 7740

gtgcaatgac tggtctgata aagaaacatg aagccgtgag ccagttgtgc tccaaaaccc 7800

aggccagcct gcaggaatct ctggaaaaac acttcagtga gtctatgcag gaattccaag 7860

aatggttttt gggagcaaag gcagcagcaa aagaatcatc agatcgcacc ggtgacagca 7920

aagttctaga agcaaagctc catgatcttc agaacatttt ggactcagtc agtgatgggc 7980

agagcaaact tgatgcagtg actcaagaag gacaaacttt gtatgcacat ttgtctaaac 8040

aaattgtcag tagcattcaa gaacaaatca caaaggccaa tgaagagttt caagcatttc 8100

tgaaacaatg ccttaaagat aagcaggctc ttcaagactg tgcttcagaa cttggaagct 8160

ttgaagatca gcacagaaaa ctgaacttat ggatccatga aatggaagaa aggttcaata 8220

cggaaaactt gggagagagt aaacagcaca ttcctgagaa gaaaaatgaa gttcataaag 8280

ttgaaatgtt tttgggagaa ctgctggctg caagagagtc tcttgataag ctttcccaga 8340

gagggcagct tctgagtgaa gaaggccacg gtgctgggca ggagggccgc ctgtgttccc 8400

agctcctcac aagccaccag aacctactta gaatgaccaa agagaaactc cggagctgcc 8460

aggtggccct tcaggagcac gaagccctgg aggaagcact gcaaagcatg tggttctggg 8520

tgaaggccat tcaggacaga ctggcctgtg cagagagcac tcttgggagc aaagacaccc 8580

tggagaaacg gctgtcacaa atacaggata ttctcctgat gaaaggtgaa ggggaagtta 8640

agttgaatat ggccattggc aagggggaac aggccttgag aagtagcaac aaagaaggtc 8700

agagggtgat tcagactcag ttagagaccc ttaaagaagt gtgggctgac atcatgagct 8760

cctccgtcca cgctcaaagc actttagagt ctgtgattag ccaatggaat gactatgtag 8820

agaggaaaaa ccagttggag cagtggatgg aatcagtgga tcaaaaaata gaacatccct 8880

tacaaccaca gccaggtctg aaagagaagt tcgtcctgct tgaccacctc cagtccatcc 8940

tgtctgaggc agaagatcac acgagagccc ttcaccgtct aattgcgaag tccagggagc 9000

tctacgaaaa gacagaggat gagtctttca aggacacagc tcaagaggag ctgaaaacac 9060

agtttaatga tataatgact gttgccaagg aaaaaatgag gaaagtggaa gagattgtga 9120

aagatcatct aatgtattta gatgcggtcc acgagttcac agattggctc cattcagcaa 9180

aggaagaact tcaccggtgg tcagatatgt ctggagattc atcagccacc cagaaaaagt 9240

tatcaaaaat taaggagctg atagattcca gagagattgg tgcaagccgt ctcagcagag 9300

tggagtcgct ggctcccgaa gtgaaacaga acacaactgc cagtgggtgt gagctcatgc 9360

acacggagat gcaggccctg cgtgccgact ggaagcagtg ggaagacagt gtattccaaa 9420

cgcagagctg tttggagaac ctggtcagcc agatggccct ttcggagcag gaattctcag 9480

gccaagtggc tcaactggag caggccctgg aacagttcag tgcccttctg aaaacctggg 9540

ctcagcagtt aaccctcctg gaaggcaaga acacggatga ggagatagtg gaatgctggc 9600

acaaaggaca agagatactg gatgctttgc aaaaagcaga gcctagaaca gaggatctca 9660

agtctcagct gaatgaactt tgtcgatttt ccagagacct gagtacctac agtggaaaag 9720

tttctggctt gattaaagag tataattgtc tttgtttgca agcatccaag ggctgccaga 9780

ataaagaaca gattttacag caaagatttc gaaaggcctt cagggatttc cagcagtggt 9840

tggttaatgc aaaaatcact accgccaagt gttttgatat acctcaaaat ataagtgaag 9900

tttcaactag tcttcagaaa atacaggagt ttttgtcaga aagtgaaaat ggacagcaca 9960

agctaaacat gatgctgtct aaaggggaac ttctgagtac cctgctgacc aaagagaaag 10020

cgaaagggat ccaggccaaa gttacagctg caaaagaaga ttggaaaaat tttcattcaa 10080

atctccacca aaaagaatct gctctagaga atctaaagat ccaaatgaag gactttgaag 10140

taagtgctga gcctatccag gactggctga gtaaaactga gaagatggtc catgaaagca 10200

gcaatcgcct ctatgatctg ccagcaaaga ggagggagca gcagaagctc cagtctgtcc 10260

ttgaggaaat acactgctac gagcctcagc tcaacaggct gaaagagaaa gctcagcagc 10320

tgtgggaagg acaagctgcc agcaagagct ttaggcacag agtgtcgcag ctgtcttctc 10380

agtatctagc gctaagcaat ttaacaaagg agaaagtgtc aagactggat agaatcgttg 10440

cagaacacaa tcagttctct cttgggatta aagaattaca agactggatg acggatgcga 10500

ttcacatgct ggattcatac tgccacccga catccgacaa agtgtgctgg gacagcagga 10560

cgctcaagct cgaggctcta ttatcagtca aacaggaaaa agagattcag atgaaaatga 10620

tagtgaccag gggagaatct gtccttcaga atacttctcc agaaggcatt cccactattc 10680

agcagcagct gcagagtgtg aaggatatgt gggcatccct tttgtctgca gggattcgtt 10740

gtaaaagcca actcgaagga gctctctcca agtggacaag ttatcaggat ggcgttcgac 10800

agttctccgg ttggatggat agtatggaag ccaacctgaa tgaatcagaa aggcagcatg 10860

cggagctgcg ggataaaaca acgatgctcg gaaaagccaa gttattaaat gaagaagtgc 10920

tgagttacag cagcttgctg gagaccatcg aagtcaaagg ggctggcatg acagaacact 10980

atgtcaccca gctagaactc caggatctac aggaacgata cagagccatc caagagaggg 11040

ccaaggaagc cgtaaccaag tctgaaaaac ttgtccgcct gcaccaagag tatcagagag 11100

acctaaaggc atttgaagtt tggttggggc aagaacaaga aaagctcgac cagtattcag 11160

ttcttgaagg tgatgcccac actcatgaga caacattgcg tgatcttcag gagctacagg 11220

tacactgtgc agaggggcag gccctgttga actcagtgct gcacaccaga gaggatgtga 11280

tcccatcagg tatcccacag gcagaggacc gggctttaga gtctctccgg caagactggc 11340

aggcttacca gcacaggctg tccgagactc gaactcagtt caataacgtg gtgaacaaat 11400

tgaggctaat ggagcaaaag tttcagcaag tagatgaatg gctcaaaaca gcagaggaga 11460

aagttagtcc caggaccaga cgtcagtcta acagggcaac caaggagata caattacatc 11520

agatgaagaa gtggcacgaa gaagtgactg catacagaga tgaagttgag gaagtgggag 11580

ctagagctca ggagatactg gacgagagcc acgtgaacag cagaatgggt tgccaggcca 11640

cccagctgac ttccagatac caggccctgc ttctccaagt gctggaacaa ataaaattcc 11700

tggaggagga gattcagagt ttggaggaat cagaatcatc cctcagttcc tattctgatt 11760

ggtatggctc tactcataaa aacttcaaga atgtggctac caagattgac aaagtagata 11820

cagtaatgat ggggaagaaa ttgaagacgt tggaggtttt gctcaaagac atggagaaag 11880

gtcacagttt gctgaaatca gcccgggaga aaggagagag ggctgttaaa tacttggagg 11940

aaggcgaggc agagaggtta agaaaggaga ttcatgatca catggagcag ttgaaggaac 12000

tgaccagcac tgtccggaaa gaacacatga cgctggaaaa aggtcttcat ttagcaaagg 12060

aattctcaga taaatgcaaa gcactgacac agtggatagc agaataccag gaaattctac 12120

atgttcctga agaacccaaa atggaattat atgagaaaaa agctcagtta tctaaataca 12180

agtcacttca acaaacggtg ctgtcccatg aaccatcagt aaagtcagtg agagagaagg 12240

gtgaagctct tttggaactg gtgcaggacg tcactttaaa ggacaaaata gatcaacttc 12300

aaagtgatta ccaggacctg tgcagcatag gaaaggagca tgtcttcagt ctggaggcga 12360

aagtcaaaga ccatgaagac tacaacagtg agctccaaga ggtcgaaaag tggctgctgc 12420

agatgtctgg cagactggtg gcacctgacc tcctggagac aagcagcctg gagacaatca 12480

cccagcaatt ggcccaccac aaggcaatga tggaagaaat tgctggtttt gaagaccgtt 12540

tgaacaatct tcaaatgaaa ggtgatactt tgattggcca atgtgcagac cacctgcaag 12600

cgaaacttaa acaaaacgtg catgctcatc tgcagggcac aaaggacagc tactcagcga 12660

tctgcagcac agctcagagg atgtaccaga gtttggaaca cgaacttcag aagcacgtca 12720

gccgacaaga caccctgcag cagtgccagg cctggctttc tgcagtccag ccggatttag 12780

agccaagtcc tcaaccacct cttagtaggg cagaagccat taagcaggtc aaacacttca 12840

gagctttgca agagcaggca aggacctacc tagatctcct ttgctccatg tgtgacctgt 12900

caaatgcttc ggtgaaaacc acagcaaaag acattcaaca aacagagcaa acgattgaac 12960

aaaagcttgt ccaggcccag aacttaactc agggctggga agagatcaag cacctgaagt 13020

ctgagctctg gatttacctg caagatgctg atcagcaact gcagaacatg aagaggaggc 13080

actctgagct ggagctgaac attgcacaga acatggtttc acaagttaag gattttgtta 13140

agaaactaca gagcaaacag gcatccgtga acaccataat agaaaaggtg aataagttaa 13200

caaagaagga ggaatcgcct gaacacaagg aaataaatca tttaaatgat cagtggctcg 13260

atttgtgccg tcagtctaac aacctgtgct tgcaaaggga agaggatctt cagagaacaa 13320

gagattacca tgactgtatg aatgttgttg aagtgttcct agaaaaattt actacagaat 13380

gggataactt ggccagatct gatgcagaga gtacagctgt ccacctggaa gctttgaaaa 13440

agttagcatt ggcattgcag gagagaaagt atgctattga agatctgaaa gatcaaaagc 13500

agaaaatgat agagcatctg aatttagatg acaaggagtt agtcaaagaa cagacgagtc 13560

atttagagca acgttggttt cagcttgagg acctcattaa aaggaaaatc caagtgtcag 13620

tcaccaactt ggaggagtta aatgtggtgc agtccagatt tcaggagcta atggagtggg 13680

cagaagagca acaacccaac atcgccgagg cccttaagca gagccctcct ccagatatgg 13740

ctcagaacct tctcatggat cacctggcca tctgcagtga actggaggcc aagcagatgc 13800

tcctgaaatc gcttataaag gacgcagaca gggtcatggc agatcttggt ctcaatgagc 13860

gacaggtcat ccagaaggct ctctctgatg cacaaagcca cgtgaattgt ctcagtgact 13920

tagtgggcca gcgaagaaag tacttaaaca aagccttgtc cgagaaaacc cagtttctca 13980

tggcagtgtt ccaggccacc agccaaattc agcaacatga gcgaaagata atgttccgtg 14040

aacacatctg tctgttacca gatgatgtga gcaaacaagt caaaacatgt aagagtgcac 14100

aagccagcct caagacttac caaaatgaag tcactggact ttgggcccag ggtcgcgaac 14160

taatgaagga agtcacagag caggaaaaga gtgaagtgct ggggaagctt caggaattgc 14220

agagtgtcta tgacagtgtt ttacaaaagt gcagtcaccg gttacaagaa ctagagaaga 14280

atttggtttc taggaagcat tttaaggaag attttgataa agcttgccac tggctaaaac 14340

aagcagatat tgttacattt cctgaaatca acctaatgaa tgagagttct gagcttcata 14400

cacaactggc taaataccaa aacattcttg aacaatctcc agaatatgaa aatcttctac 14460

ttacgctgca gagaactggg cagaccatat taccatcgct gaatgaagtc gatcattcct 14520

acctcagtga aaagctaaat gctttgcctc gacaatttaa tgtaattgtt gccttggcta 14580

aagacaagtt ctataaagtc caggaagcaa ttcttgctcg gaaggaatat gcttccttga 14640

ttgagttgac aacccagtct ctgagtgaac ttgaagccca attcttgagg atgagcaaag 14700

ttcccaccga cctggccgtt gaggaggctc tttctctgca agatggttgc agagccattc 14760

tggacgaggt ggcgggcctt ggggaggcgg tggatgaact gaaccagaaa aaagaaggtt 14820

ttcgcagcac aggtcagcct tggcagccag acaagatgct gcaccttgtc accttatatc 14880

acaggctgaa gcgacaaaca gaacagaggg ttagcttatt agaagacacc accagtgctt 14940

accaagaaca cgagaagatg tgccaacagc tggagagaca actgaagtct gtaaaagagg 15000

agcagtccaa agtgaatgag gaaacgctgc ctgcagagga gaagctcaaa atgtatcact 15060

ccctggcagg aagtctccag gactcaggga ttgtactgaa acgagtaacc atacatcttg 15120

aagatcttgc cccacacctt gaccccttgg cttatgagaa agccaggcat cagatccagt 15180

cctggcaagg ggagttaaaa ctgttgactt ctgccattgg tgagacggtg acagaatgtg 15240

agagccgaat ggtgcagagt atagacttcc agactgagat gagtcgctcc ctggactggc 15300

tgaggagagt gaaggcagag ctcagtgggc cggtgtacct agacctcaac ctgcaggaca 15360

tccaagagga aatcagaaaa atccaaattc atcaggaaga ggtccagtcc agcttgagaa 15420

tcatgaatgc gctgagtcac aaggaaaagg agaagttcac aaaggccaag gagctgattt 15480

ctgcggattt agaacacagc ctcgctgagc tctcagagct ggatggagac atccaggaag 15540

ccttacgcac cagacaggct accttgactg aaatatatag ccagtgtcaa aggtattatc 15600

aggtatttca agcagccaat gactggcttg aggatgccca agaattgtta cagctggcag 15660

gcaatggcct agacgtggag agcgcagagg aaaatctcaa aagccacatg gaatttttca 15720

gtacagagga tcagttccat agtaacctgg aggagctcca cagcctggta gccaccctgg 15780

acccactcat caagccaacc ggcaaagaag acctagaaca gaaagtggct tctctggaac 15840

tcaggagcca gaggatgagc cgggactctg gtgcccaagt ggatctcttg cagagatgca 15900

cagctcaatg gcacgattac cagaaagcaa gggaagaggt tattgaattg atgaatgata 15960

cagaaaagaa attgtctgag ttttctttgt tgaagacttc gtctagtcat gaagcggaag 16020

aaaaattgtc agaacacaag gctttagtgt cagtggttaa ctctttccat gagaaaattg 16080

tggcccttga ggaaaaagct tcacaactgg agaaaaccgg aaatgatgcc agcaaagcca 16140

ccctgagcag gtcaatgacc accgtctggc agcgctggac acgccttcga gctgtggccc 16200

aggaccagga gaagatcctg gaagatgcag tggatgagtg gacgggcttt aacaacaagg 16260

ttaaaaaggc cactgaaatg attgatcagc tgcaagataa gttacctgga agttcagcag 16320

agaaagcatc gaaagcagag ctcttaactc ttcttgaata ccacgacacg ttcgttctgg 16380

agctggagca gcagcagtcg gccttgggca tgctgcggca gcaaaccctg agcatgctcc 16440

aggatggagc cgccccaacc cctggggaag agcctccgct catgcaggaa atcaccgcca 16500

tgcaagatcg gtgcctgaac atgcaggaga aagtgaagac taatggaaag ttggtgaagc 16560

aagagctgaa ggaccgagaa atggtggaga ctcagatcaa ttctgtgaaa tgttgggttc 16620

aggaaacgaa agaatattta gggaatccaa caatagaaat agatgctcaa cttgaagaac 16680

ttcagattct cctaacagaa gccacaaatc accgacagaa cattgaaaaa atggcagaag 16740

aacagaagga gaagtactta ggtctttata ccatattacc ttctgaactc tcccttcagt 16800

tggctgaagt ggcgttagat ctaaagatcc gagatcagat ccaagacaaa ataaaagaag 16860

ttgagcagag caaggccacg agccaggaac tcagccggca aattcagaag ttagctaaag 16920

acctcacaac tattctaact aagctgaaag cgaagacaga taatgtagtt caagctaaaa 16980

ctgaccaaaa ggtgctggga gaggaattag atggctgtaa ttcaaagtta atggaattag 17040

atgcagcagt acagaaattc ttggaacaga atggccaact gggtaagcca ctggccaaga 17100

agataggaaa actgactgaa cttcaccagc agaccattag acaagctgag aatcggctct 17160

ccaagctcaa tcaggcagca tcacatttag aagaatacaa tgaaatgctt gaattaattt 17220

tgaagtggat tgaaaaagct aaagtcttgg ctcatggaac tattgcatgg aattctgcaa 17280

gccagcttcg ggaacaatat attttgcatc agaccctgct agaagaatcc aaagaaattg 17340

acagtgagct ggaagcaatg actgagaaat tacagtacct cactagcgtg tactgtacag 17400

aaaaaatgtc tcagcaagtg gcagaactgg gacgggagac tgaggagttg cgacagatga 17460

tcaaaattcg tttgcagaac ctccaagatg cagctaagga tatgaaaaaa tttgaagcag 17520

agttgaaaaa gttacaagct gccttggagc aagcccaggc aacactgact tctccagaag 17580

ttggacgtct cagtctcaag gagcagctct ctcatcggca gcatttgttg tctgagatgg 17640

agtcactgaa gccgaaggtg caagcagtgc agctctgcca gagtgccctc cggatccccg 17700

aggatgtggt tgccagctta cctctctgtc atgctgctct gcggctgcag gaagaggcca 17760

gccggctgca gcacaccgcc atccagcagt gtaacatcat gcaggcagct gtggtacaat 17820
atgaacaata tgagcaagaa atgaaacatc tccagcaact gatagaagga gctcacagag 17880
agattgagga taaacctgtt gccaccagta acatacagga gctgcaggct cagatttctc 17940
ggcatgagga gctggcgcag aaaattaagg gctaccagga gcagatcgct tctttgaatt 18000
ccaagtgcaa gatgctgacg atgaaagcca agcacgccac catgctgctg acggtgaccg 18060
aggtcgaggg gctggcggaa gggacagagg acctggatgg ggagctcctc cccacgcctt 18120
cggcccaccc ctctgtggtc atgatgactg caggtcgctg tcacactttg ctgtcaccgg 18180
tcactgagga gtctggggag gagggaacca acagtgagat ttcctctcca cctgcctgtc 18240
gctccccttc acctgtggct aatacagatg cttctgttaa ccaggacatt gcatattacc 18300
aagccttgtc tgctgagagg ttgcagacag atgctgcaaa aattcacccc agcacatccg 18360
catcccagga gttctatgaa ccgggattgg agccatccgc tactgccaaa ctgggtgatt 18420
tgcagcgttc ttgggaaacc ttaaagaatg tgatcagtga gaagcagcgc acactctatg 18480
aagctttgga acgccagcag aagtaccagg actccctcca gtccatctct acgaagatgg 18540
aggccattga gctgaaactc agtgagagcc cagagcctgg caggagtcca gaaagccaga 18600
tggctgaaca tcaggcattg atggatgaga ttctcatgct ccaggatgaa atcaatgagc 18660
tccagtcctc tctcgcagag gagctggtat ccgagtcttg tgaggccgac cctgcggagc 18720
agctggcctt gcagtccacg ctcactgtct tagccgagcg aatgtccacc atcaggatga 18780
aagcctcggg gaaacggcag cttttggagg agaagttgaa tgatcagctg gaggaacaaa 18840
ggcaggaaca ggccctgcag aggtatcgct gtgaagccga tgagctggac agctggctct 18900
tgagtaccaa ggccactctg gacactgcgc tgagtccacc caaggagccc atggacatgg 18960
aggcccagct tatggactgc cagaatatgc tggtggaaat agagcagaag gtggtggctt 19020
tatcagaact gtcagtccac aatgagaacc tgctgctgga gggcaaagct cacaccaagg 19080
acgaggccga gcagctggct ggaaagctga gaaggctcaa ggggagcctg ctggagctgc 19140
agagagccct gcatgataag cagctcaaca tgcagggaac agcacaggag aaggaggaga 19200
gcgatgttga cctaacagcc acgcagagcc ccggcgtcca ggaatggctg gcccaagctc 19260
gcaccacatg gacccagcag cggcagagca gtctccagca acaaaaagag ttagaacagg 19320
aattagccga gcagaagagt ctccttcgct cagtagccag tcgtggagag gagattctaa 19380
ttcaacattc ggcggcagag acctctggtg atgctggcga aaaacctgat gtgttatccc 19440
aggagttggg gatggaaggg gagaaatcat ccgctgaaga ccagatgaga atgaaatggg 19500
aaagcctaca tcaagaattt agtaccaagc agaaactact acagaatgtt ctggaacagg 19560
aacaagagca agtgctttat agcaggccaa atcgactctt gtctggtgtg ccactgtaca 19620
aaggggacgt gccaacccaa gataaatctg cagttacatc tttgctggat ggactgaacc 19680

aagccttcga ggaggtttca tcccagagtg gaggggcaaa gaggcagagt atacacttgg 19740

agcagaagtt gtatgatgga gtctcagcca cctctacttg gttggatgac gttgaagaac 19800

gtttatttgt tgccacagca cttttaccag aagaaacaga gacttgtctc ttcaaccaag 19860

agattcttgc caaagacatt aaggaaatgt ctgaagaaat ggataagaac aaaaacttgt 19920

tttcccaagc ttttccagag aatggtgata atcgagatgt tattgaagat actttgggtt 19980

gtcttttggg caggttatcc ttgctagact cagtagtgaa tcaacgatgt catcagatga 20040

aagaaagact tcagcaaata ctaaatttcc agaatgatct gaaagtgctg tttacatcac 20100

tggctgacaa caaatacatc attctgcaaa aactggcaaa tgtgtttgaa cagcccgtag 20160

cagaacaaat agaggcaata caacaggctg aagatggact caaagaattt gatgcaggaa 20220

tcattgaatt aaagaggcgt ggtgacaagc tacaggtcga gcagccgtcc atgcaagaac 20280

tctccaagct ccaggacatg tatgatgagc tgatgatgat cattggctcc cggaggagtg 20340

gtctgaatca gaaccttaca ctcaagagtc agtatgagag ggccctacaa gatctggctg 20400

acctgctaga aactggtcag gagaagatgg caggagacca gaaaatcatc gtgtcttcca 20460

aagaggaaat ccagcaacta cttgacaaac ataaggaata ctttcagggc ctggaatctc 20520

atatgatctt gactgaaaca ctcttcagaa agataatcag ctttgcagtc caaaaggaaa 20580

cccagttcca tacagagctg atggctcagg cttctgctgt actgaaacgg gctcacaaga 20640

ggggtgtgga gctggagtac attctagaga cgtggtccca tctggatgag gaccagcagg 20700

agctcagcag acagctggag gtggtggaaa gcagcatccc aagcgtgggt ctggtggagg 20760

agaacgagga caggcttatt gaccgcataa cactctacca gcatttaaaa tctagcctta 20820

atgaatacca gcccaaatta tatcaagtat tagatgatgg gaaacgactt ctgatatcca 20880

tcagctgctc agatctagaa agccaactaa atcaacttgg agagtgctgg ctaagtaaca 20940

ccaataaaat gtctaaggaa cttcacagac tggaaacaat attgaaacac tggaccagat 21000

atcaaagtga atctgcagat ctaattcact ggttacaatc tgcaaaagac cggctagaat 21060

tttggactca gcaatctgtg acagtcccac aagagctgga aatggtccgt gatcatctaa 21120

atgctttcct ggagttttct aaagaagtgg atgcccaatc ttccctgaaa tcatctgttc 21180

tgagtactgg aaatcagctc cttcgactaa aaaaggtgga cacagccacg ctgcgctctg 21240

agctgtcgcg cattgatagc cagtggactg acctgctaac caatatccca gccgtccagg 21300

agaagctcca ccagctccag atggataaac tgccttcccg ccatgccatt tctgaagtca 21360

tgagttggat ttctctaatg gaaaatgtta ttcagaagga tgaagataat attaaaaatt 21420

ccataggtta caaggcaatt catgaatacc ttcagaaata taagggtttt aagatagaca 21480

ttaactgtaa acagctgaca gtggattttg tgaaccagtc cgtgctacaa atcagcagtc 21540

aggatgtgga aagtaagcgt agtgataaga ctgattttgc tgagcaactt ggagcaatga 21600
ataaaagttg gcaaattctg caaggtctag taactgagaa gatccagctg ttggaaggct 21660
tattggaatc ttggtcagaa tatgaaaata atgtacaatg tctgaaaaca tggtttgaaa 21720
cccaggaaaa gagactaaaa caacagcatc gaattggaga tcaggcttct gttcaaaatg 21780
cactgaaaga ctgtcaggat ctggaagatt tgattaaagc aaaagaaaaa gaagtagaga 21840
aaattgagca gaatggactt gctttgattc agaacaagaa agaagacgtc tctagcattg 21900
tcatgagcac actgcgagag ctcggccaaa cctgggcaaa tttagatcac atggttggac 21960
aattaaagat actgctgaaa tcagtgcttg accaatggag tagtcacaaa gtggcctttg 22020
acaagataaa cagttacctc atggaggcca gatactctct ttcccgattc cgtctgctga 22080
ctggctcctt agaagctgtg caagttcagg tggacaatct tcagaatctc caagatgatc 22140
tggaaaaaca ggaaaggagc ttacagaaat ttggctctat caccaaccaa ttattaaaag 22200
agtgtcaccc acccgtgaca gaaactctta ccaatacact gaaagaagtc aacatgagat 22260
ggaataactt gctggaagag attgctgagc agctacagtc cagcaaggcc ctacttcagc 22320
tttggcaaag atacaaggac tactccaaac agtgtgcttc gacagttcag cagcaggagg 22380
atcgaaccaa tgagctgttg aaggcagcca caaacaagga cattgccgat gatgaggttg 22440
ccacatggat tcaagattgc aacgacctcc tcaaaggact gggcacagtt aaagattccc 22500
tcttttttct ccatgagctg ggagagcaac tgaagcaaca agtggatgct tccgcagcat 22560
cagctattca atcggatcaa ctctctttga gtcaacactt gtgtgccctg gagcaagctc 22620
tctgcaaaca gcagacttca ttacaggctg gagttcttga ttatgaaacc tttgccaaga 22680
gtttagaagc tttggaggcc tggatagtgg aagctgaaga aatactacaa gggcaggacc 22740
ctagccactc atctgacctc tccacaatcc aggaaaggat ggaagaactt aaggacagaa 22800
tgttaaaatt cagcagcatg gctccagatt tagaccgtct aaatgagctt ggatataggt 22860
tacccttgaa tgataaggaa atcaaaagaa tgcagaatct gaaccgccat tggtctctga 22920
tctcctctca gactacagaa agattcagca agttgcagtc atttttgcta caacatcaga 22980
ctttcttgga aaaatgtgaa acatggatgg aattcctagt tcagacagaa caaaagttag 23040
cagtagagat ttcaggaaat tatcagcacc ttttggaaca gcagagagca cacgagttgt 23100
ttcaagccga gatgttcagt cgtcagcaga ttttgcactc aatcattatt gatgggcaac 23160
gtcttctaga acaaggtcaa gttgatgaca gggatgaatt caacctgaaa ttgacactcc 23220
tcagtaatca atggcaggga gtgattcgca gggcccagca gaggcggggg atcattgaca 23280
gccagattcg ccagtggcag cgctataggg agatggcaga aaagcttcgt aaatggttgg 23340
ttgaagtgtc ctacctcccc atgagtggtc tcggaagtgt tcctatacca ctgcaacaag 23400
caaggaccct ctttgatgaa gtgcagttca aagaaaaagt gtttctgcgg caacaaggca 23460

gctacatcct gactgtggag gctggcaagc aactccttct ctcggcggac agtggcgctg 23520

aggccgcctt gcaggccgaa ctcgctgaaa tccaagagaa atggaaatca gccagcatgc 23580

ggctggaaga acagaagaaa aaactagcct tcttgttgaa agactgggaa aaatgtgaga 23640

aaggaatagc agattccctg gagaaactac gaactttcaa aaagaagctt tcgcagtctc 23700

tcccggatca ccatgaagag ctccatgcag aacaaatgcg ttgcaaggaa ttagaaaatg 23760

cagttgggag ctggacagat gacttgaccc agttgagcct gctgaaggac accctctctg 23820

cctatatcag tgctgatgat atctccattc ttaatgaacg cgtagagctt ctgcaaaggc 23880

agtgggaaga actatgccac cagctctcct taaggcggca gcaaataggt gaaagattga 23940

atgaatgggc agtcttcagt gaaaagaaca aggaactctg tgagtggttg actcaaatgg 24000

aaagcaaagt ttctcagaat ggagacattc tcattgaaga aatgatagag aagctcaaga 24060

aggattatca agaggaaatt gctattgctc aagagaacaa aatacagctc caacaaatgg 24120

gagaacgact tgctaaagcc agccatgaaa gcaaagcatc tgagattgaa tacaagctgg 24180

gaaaggtcaa cgaccggtgg cagcatctcc tggacctcat tgcagccagg gtgaagaagc 24240

tgaaggagac cctggtagcc gtgcagcagc ttgataagaa catgagcagc ctgaggacct 24300

ggctcgctca catcgagtca gagctggcca agccaatagt ctacgattcc tgtaactcgg 24360

aagaaataca gagaaagctt aatgagcagc aggagcttca gagagacata gagaagcaca 24420

gtacaggtgt tgcatctgtc ctcaacctgt gtgaagtcct gctgcacgac tgtgacgcct 24480

gtgccactga tgccgagtgt gactctatac agcaggctac gagaaacctg gaccggcggt 24540

ggagaaacat ttgtgctatg tccatggaaa ggaggctgaa aatcgaagag acgtggcgat 24600

tgtggcagaa atttctggat gactattcac gttttgaaga ttggctgaag tcttcagaaa 24660

ggacagctgc ttttcccagc tcttctgggg tgatctatac agttgccaag gaagaactaa 24720

agaaatttga ggctttccag cgacaggtcc acgagtgcct gacgcagctg gaactgatca 24780

acaagcagta ccgccgcctg gccagggaga accgcactga ttcagcatgt agcctcaaac 24840

agatggttca cgaaggcaac cagagatggg acaacctgca aaagcgtgtc acctccatct 24900

tgcgcagact caagcatttt attggccagc gtgaggagtt tgagactgcg cgggacagca 24960

ttctggtctg gctcacagag atggatctgc agctcactaa tattgaacat ttttctgagt 25020

gtgatgttca agctaaaata aagcaactca aggccttcca gcaggaaatt tcactgaacc 25080

acaataagat tgagcagata attgcccaag gagaacagct gatagaaaag agtgagccct 25140

tggatgcagc gatcatcgag gaggaactag atgagctccg acggtactgc caggaggtct 25200

tcgggcgtgt ggaaagatac cataagaaac tgatccgcct gcctctccca gacgatgagc 25260

acgacctctc agacagggag ctggagctgg aagactctgc agctctgtcg gacctgcact 25320

ggcacgaccg ctctgcagac agcctgcttt ctccacagcc ttcctccaat ctctccctct 25380

cgctcgctca gcccctccgg agcgagcggt caggacgaga caccccggct agtgtggact 25440

ccatccccct ggagtgggat cacgactatg acctcagtcg ggacctggag tctgcaatgt 25500

ccagagctct gccctctgag gatgaagaag gtcaggatga caaagatttc tacctccggg 25560

gagctgttgg cttatcaggg gaccacagtg ccctagagtc acagatccga caactgggca 25620

aagccctgga tgatagccgt tttcagatac agcaaaccga aaatatcatt cgcagcaaaa 25680

ctcccacggg gccggagcta gacaccagct acaaaggcta catgaaactg ctgggcgaat 25740

gcagtagcag tatagactcc gtgaagagac tggagcacaa actgaaggag gaagaggaga 25800

gccttcctgg ctttgttaac ctgcatagta ccgaaaccca aacggctggt gtgattgacc 25860

gatgggagct tctccaggcc caggcattga gcaaggagtt gaggatgaag cagaacctcc 25920

agaagtggca gcagtttaac tcagacttga acagcatctg ggcctggctg ggggacacgg 25980

aggaggagtt ggaacagctc cagcgtctgg aactcagcac tgacatccag accatcgagc 26040

tccagatcaa aaagctcaag gagctccaga aagctgtgga ccaccgcaaa gccatcatcc 26100

tctccatcaa tctctgcagc cctgagttca cccaggctga cagcaaggag agccgggacc 26160

tgcaggatcg cttgtcgcag atgaatgggc gctgggaccg agtgtgctct ctgctggagg 26220

agtggcgggg cctgctgcag gatgccctga tgcagtgcca gggtttccat gaaatgagcc 26280

atggtttgct tcttatgctg gagaacattg acagaaggaa aaatgaaatt gtccctattg 26340

attctaacct tgatgcagag atacttcagg accatcacaa acagcttatg caaataaagc 26400

atgagctgtt ggaatcccaa ctcagagtag cctctttgca agacatgtct tgccaactac 26460

tggtgaatgc tgaaggaaca gactgtttag aagccaaaga aaaagtccat gttattggaa 26520

atcggctcaa acttctcttg aaggaggtca gtcgtcatat caaggaactg gagaagttat 26580

tagacgtgtc aagtagtcag caggatttgt cttcctggtc ttctgctgat gaactggaca 26640

cctcagggtc tgtgagtccc acatcaggaa ggagcacccc aaacagacag aaaacgccac 26700

gaggcaagtg tagtctctca cagcctggac cctctgtcag cagtccacat agcaggtcca 26760

caaaaggtgg ctccgattcc tccctttctg agccaggccc aggtcggtcc ggccgcggct 26820

tcctgttcag agtcctccga gcagctcttc cccttcagct tctcctgctc ctcctcatcg 26880

ggcttgcctg ccttgtacca atgtcagagg aagactacag ctgtgccctc tccaacaact 26940

ttgcccggtc attccacccc atgctcagat acacgaatgg ccctcctcca ctctgaacta 27000

agcagatgcc atctgcagaa gtgctggtag cataaggagg atcgggtcat aagcaatccc 27060

aaactaccaa caagaggacc ttgatcttgg cgaaagccct cggtgtggca gctttagccc 27120

tcctccagat cacatgtgtg caaattatgg cttcagaggt ggaagataaa cagtgacggg 27180

ggaacaaaca gacaacaaga aggtttggaa gaaatctggt ttgagactct gaaccttagc 27240

actaaggaga ttgagtaagg acctccaaag ttccccggac tcatgaattc tgggcccttg 27300

gcccattctg tgcacagcca aggacttcag tagaccatct gggcagcttt cccatggtgc 27360

tgctccaacc atcagataaa tgaccctccc aagcaccatg tcagtgtcgt acaatctacc 27420

aaccaaccag tgctgaagag attttagaac cttgtaacat acaattttta agagcttata 27480

tggcagcttc ctttttacct tgttttcctt tggggcatga tgttttaacc tttgctttag 27540

aagcacaagc tgtaaatcta aaaggcactt ttttttagag gtataaagaa aaactagatg 27600

taataaataa gatcatggaa ggctttatgt gaaaaaagtt gaatgttata gtaaaaaaaa 27660

aaagatattt atgtatgtac agtttgctaa agccaagttt tgtttgtatt gatttctttg 27720

catttattat agatattata aaata 27745

<210> 33
<211> 690
<212> DNA
<213> Homo sapiens

<400> 33
ccgggcgctc ctagcggtct cccggcccct gccgccctgc cactatgtcc cgccgctcta 60

tgctgcttgc ctgggctctt cccagcctcc ttcgactcgg agcggctcag gagacagaag 120

acccggcctg ctgcagcccc atagtgcccc ggaacgagtg gaaggccctg gcatcagagt 180

gcgcccagca cctgagcctg cccttacgct atgtggtggt atcgcacacg gcgggcagca 240

gctgcaacac ccccgcctcg tgccagcagc aggcccggaa tgtgcagcac taccacatga 300

agacactggg ctggtgcgac gtgggctaca acttcctgat tggagaagac gggctcgtat 360

acgagggccg tggctggaac ttcacgggtg cccactcagg tcacttatgg aaccccatgt 420

ccattggcat cagcttcatg ggcaactaca tggatcgggt gcccacaccc caggccatcc 480

gggcagccca gggtctactg gcctgcggtg tggctcaggg agccctgagg tccaactatg 540

tgctcaaagg acaccgggat gtgcagcgta cactctctcc aggcaaccag ctctaccacc 600

tcatccagaa ttggccacac taccgctccc cctgaggccc tgctgatccg caccccattc 660

ctcccctccc atggccaaaa accccactgt 690

<210> 34
<211> 2677
<212> DNA
<213> Homo sapiens

<400> 34
catttggccc ggggatggtc acacgcgcgg gggccggaac tgccgtcgcc ggcgcggtcg 60

ttgtcgcatt gctctcggcc gcactcgcgc tgtacgggcc gccactggac gcagttttag 120

aaagagcgtt ttcgctacgt aaagcacatt cgataaagga tatggaaaat actttgcagc 180

tggtgagaaa tatcatacct cctctgtctt ccacaaagca caaagggcaa gatggaagaa 240

taggcgtagt tggaggctgt caggagtaca ctggagcccc atattttgca gcaatctcag 300

ctctcaaagt gggcgcagac ttgtcccacg tgttctgtgc cagtgcggcc gcacctgtga 360

ttaaggccta cagcccggag ctgatcgtcc acccagttct tgacagcccc aatgctgttc 420

atgaggtgga gaagtggctg ccccggctgc atgctcttgt cgtaggacct ggcttgggta 480

gagatgatgc gcttctcaga aatgtccagg gcattttgga agtgtcaaag gccagggaca 540

tccctgttgt catcgacgcg gatggcctgt ggctggtcgc tcagcagccg gccctcatcc 600

atggctaccg gaaggctgtg ctcactccca accacgtgga gttcagcaga ctgtatgacg 660

ctgtgctcag aggccctatg gacagcgatg acagccatgg atctgtgcta agactcagcc 720

aagccctggg caacgtgacg gtggtccaga aaggagagcg cgacatcctc tccaacggcc 780

agcaggtgct tgtgtgcagc caggaaggca gcagccgcag gtgtggaggg caaggggacc 840

tcctgtcggg ctccctgggc gtcctggtac actgggcgct ccttgctgga ccacagaaaa 900

caaatggggg catatcagac ttgaaattga caatttgggg tcctgagatt gaaacaggag 960

tcaaaaccag agcccagggt agctgcggcc cccggaccac gacgcccact tccccacacc 1020

tcctgctgtc cccctctccg caggtccagc cctctcctgg tggccgcgtt tggcgcctgc 1080

tctctcacca ggcagtgcaa ccaccaagcc ttccagaagc acggtcgctc caccaccacc 1140

tccgacatga tcgccgaggt gggggccgcc ttcagcaagc tctttgaaac ctgagcccgc 1200

gcagaccaga agtaaacagg caccttggac gggggagagc gtgtgtgtga tgggaaaatc 1260

cggacccacg cgtgtgctga aggcgtacgg tgcttgccag attttcaact tgagcataaa 1320

ttggttgcca ttgagaattt aagaatctgg aatattgcag cttttggtta aacttaatgc 1380

atggttggag atgttatggc gacactaaac aaagtattcc tgaactttcc ttagctcctt 1440

ggtagtaact gggaagacag aaatgaagaa aatcacatga gaatgaagaa ttctttagca 1500

gctcaacaga gtttctcggc ctgctcccag atcggcgaag tttctacttg ttactctctc 1560

tgccggcgcc cttcgttcct cctctgcttc ccttccctag tctttcctcc ggcagggagc 1620

tgggcagggg tccccgggtg tctccctgag tcccgactgc actgactggg tccatcagag 1680

ggctgcttcg ttctccagct catcttcttt taaagtggtg actagcttgg tggtatctgg 1740

ctgctggtgt ttggcttatt gacatactcc agggtaatca atgatgactt tgtttggaaa 1800

cccttttgga ggcaccatgg gaacagaagg aaacatgagt gacgctgacc cttgagtgtg 1860

tgggtgggga gctctgagac gcctcctgtc ccacgctctc cggtgtccgt gtctacacag 1920

gggtccccat gatacccacc ggccccagca gggcagaccg gaccggggac gggcacggtg 1980

aagggctgca gcctggggtc tgacgtggcc cctagtgctg tctcaggaga aggctctgga 2040

ggacttgagg catgctgggc ctggtgcagt gatggcgcta aggagacccg gggaaagaca 2100

gtatcgtggt cacgtatgct taggaagcag cacagccgtg tccttaggga tgttcgcgtc 2160

cagtaaagac actggtaact gcggtttcag ccaacactct tcatggcagt gtcgacctcg 2220

ggttagcttc tgttgtcttt gtggatggtt ttcctggagc ggcctgacgt tgacgtgttc 2280

tctggtccca tgtcttagcg gggcatggta cggtttcgtg cctgacgcgt gcattagggt 2340

gttctcttat actttcagta gcatctttcc acagcaaggg ccaaaccctc ctggttccct 2400

tcagagtctt tttggcctga tgatgactct tgagtgatac cctgtgatgc agacatgccc 2460

cagatggatt ctactttctt taaaactagg gactttcaag attaaaaaaa agattgtcac 2520

tactaatttg acgcctaact tcagaagctt cactgtctac atgtgaactt ttccagaaaa 2580

actgtgccat ggacattttt cctctgggga attaacatct aaattctggt aactattaaa 2640

agacagatct ggttaattta aaaaaaaaaa aaaaaaa 2677

<210> 35
<211> 2063
<212> DNA
<213> Homo sapiens

<400> 35
catcccacaa tcctctcgtc acgtatttcc ggtttatcta gctcagccgt aagtagtttc 60

tctatcagtc gcgcagctgt gttcgcggac tcaggtggaa ggaatttctt ctcttcgttg 120

acgttgctgg tgttcactgt ttggaattag tcaagtttcg ggaatcaccg tcgctgccat 180

caacatgtcg gtcccaagcg ctctcatgaa gcaaccgccc attcagtcta cggctggggc 240

cgtcccagtt cgcaatgaga aaggtgagat ttcaatggaa aaagtgaagg taaagcgtta 300

tgtgtccgga aaaaggccag actatgcccc tatggagtcc tcagatgagg aggatgaaga 360

atttcagttc attaagaaag ccaaagaaca agaagcagag cctgaggaac aggaggagga 420

ttcatccagt gacccccggc tacggcgttt acagaaccgt attagtgaag atgtggaaga 480

gagattggct cgacatcgaa aaatagtgga acctgaagtg gtaggagaga gtgactcaga 540

agtagaagga gatgcttggc gcatggaacg agaagacagc agtgaagaag aggaggagga 600

aattgatgat gaggaaatag agcggcggcg tggcatgatg cgtcagcgag cacaggagag 660

aaaaaatgaa gagatggaag tcatggaagt ggaagatgag ggtcgttctg gagaggagtc 720

agaatcagag tctgagtatg aagagtacac agacagtgaa gatgagatgg agcctcgcct 780

taagccagtc ttcattcgaa agaaggaccg agtgacagtt caagaacgtg aagccgaagc 840

attgaaacag aaggagctgg agcaggaagc aaaacgcatg gctgaggaaa ggcgcaagta 900

cacactcaag attgtcgaag aggaaaccaa aaaagagctg gaagagaaca agcgatccct 960

ggctgcattg gatgcactca atactgatga tgaaaatgat gaggaggaat atgaggcatg 1020

gaaagttcga gagctaaaaa gaatcaagag ggacagagaa gatcgagaag cgcttgagaa 1080

ggagaaagca gaaattgaac gcatgcgaaa cctgactgag gaagagagga gagctgaact 1140

tcgggcaaac ggcaaagtca ttaccaacaa agctgttaag ggcaaataca agttcttaca 1200

gaagtattat caccggggtg ccttcttcat ggatgaggat gaagaagtat acaagagaga 1260

tttcagcgct cctaccctgg aggatcattt caataaaacc attcttccta aagtcatgca 1320

ggtcaagaac tttggacgct caggtcgcac caaatacact caccttgtgg atcaagatac 1380

cacctccttt gactcagctt ggggccaaga gagtgcccag aacacaaagt tcttcaaaca 1440

aaaggcagct ggggtacgag atgtatttga gcggccatct gccaagaagc ggaaaactac 1500

ctagggtcca actgcttatt cttccaactg tggaacacaa ggggagtctc agcatctggt 1560

ccttgatttg gttttttcat tgtttccttg gcccctgtat ccagatattg gacttactgc 1620

tatacttgtg atactgggta gcccagactt tgaaggtgct ttgtgaggtt tggactcatg 1680

ctgagaaacc cacaggaaag cactgtccag gtaggattag aggcttccca cttaaaacta 1740

tttctgagaa atcttaggtt ttatcactgc tatggtttcc catatttact tgggactgtt 1800

ctgactttct ttttccagcc cttagcttgg gttagaaaag tggacatgta agtgaacaat 1860

gcattacttc taccttaggt ttaggagtaa tatacccgga aatctaagct catggaaaca 1920

tgttttccat ttttgcttgg agtccgtttt tctagttgta catacttttt gatccatata 1980

tgtgtgcatg tcaagaaata aaagaatcac acaacaagga gatttttagc atgatactgg 2040

aaataaaatg tccaagctga caa 2063

<210> 36
<211> 3080
<212> DNA
<213> Homo sapiens

<400> 36
gggtgcctca tattgccaga caagagctca gacctgagga gagtgactag cttctctgtg 60

tcccaggtgg ccaccttcca ctgtggaagc tcatggactc cattgggtct tcagggttgc 120

ggcaggggga agaaaccctg agttgctctg aggagggctt gcccgggccc tcagacagct 180

cagagctggt gcaggagtgc ctgcagcagt tcaaggtgac aagggcacag ctacagcaga 240

tccaagccag cctcttgggt tccatggagc aggcgctgag gggacaggcc agccctgccc 300

ctgcggtccg gatgctgcct acatacgtgg ggtccacccc acatggcact gagcaaggag 360

acttcgtggt gctggagctg ggggccacag gggcctcact gcgtgttttg tgggtgactc 420

taactggcat tgaggggcat agggtggagc ccagaagcca ggagtttgtg atcccccaag 480

aggtgatgct gggtgctggc cagcagctct ttgactttgc tgcccactgc ctgtctgagt 540

tcctggatgc gcagcctgtg aacaaacagg gtctgcagct tggcttcagc ttctctttcc 600

cttgtcacca gacgggcttg gacaggagca ccctcatttc ctggaccaaa ggttttaggt 660

gcagtggtgt ggaaggccag gatgtggtcc agctgctgag agatgccatt cggaggcagg 720

gggcctacaa catcgacgtg gttgctgtgg tgaacgacac agtgggcacc atgatgggct 780

gtgagccggg ggtcaggccg tgtgaggttg ggctagttgt agacacgggc accaacgcgt 840

gttacatgga ggaggcacgg catgtggcag tgctggacga agaccggggc cgcgtctgcg 900

tcagcgtcga gtggggctcc ttcagcgatg atggggcgct gggaccagtg ctgaccacct 960

tcgaccatac cctggaccat gagtccctga atcctggtgc tcagaggttt gagaagatga 1020

tcggaggcct gtacctgggt gagctggtgc ggctggtgct ggctcacttg gcccggtgtg 1080

gggtcctctt tggtggctgc acctcccctg ccctgctgag ccaaggcagc atcctcctgg 1140

aacacgtggc tgagatggag gacccctcta ctggggcagc ccgtgtccat gctatcctgc 1200

aggacttggg cctgagccct ggggcttcgg atgttgagct tgtgcagcac gtctgtgcgg 1260

ccgtgtgcac gcgggctgcc cagctctgtg ctgccgccct ggccgctgtt ctctcctgcc 1320

tccagcacag ccgggagcaa caaacactcc aggttgctgt ggccaccgga ggccgagtgt 1380

gtgagcggca ccccaggttc tgcagcgtcc tgcaggggac agtgatgctc ctggccccgg 1440

aatgcgatgt ctccttaatc ccctctgtgg atggtggtgg ccggggagtg gcgatggtga 1500

ctgccgtggc tgcccgtctg gctgcccacc ggcgcctgct ggaggagacc ctggccccat 1560

tccggttgaa ccatgatcaa ctggctgcgg ttcaggcaca gatgcggaag gccatggcca 1620

aggggctccg aggggaggcc tcctcccttc gcatgctgcc cactttcgtc cgggccaccc 1680

ctgacggcag cgagcgaggg gatttcctgg ccctggacct cgggggcacg aacttccgtg 1740

tcctcctggt acgtgtgacc acaggcgtgc agatcaccag cgagatctac tccattcccg 1800

agactgtggc ccagggttct gggcagcagc tctttgacca catcgtggac tgcatcgtgg 1860

acttccagca gaagcagggc ctgagcgggc agagcctccc actgggtttt accttctcct 1920

tcccatgtag gcagcttggc ctagaccagg gcatcctcct gaactggacc aagggtttca 1980

aggcatcaga ctgcgagggc caagatgtcg tgagtctgtt gcgggaagcc atcactcgca 2040

gacaggcagt ggagctgaat gtggttgcca ttgtcaatga cacggtgggg accatgatgt 2100

cctgtggcta tgaggacccc cgttgcgaga taggcctcat tgtcggaacc ggcaccaatg 2160

cctgctacat ggaggagctc cggaatgtgg cgggcgtgcc tggggactca ggccgcatgt 2220

gcatcaacat ggagtggggc gcctttgggg acgatggctc tctggccatg ctcagcaccc 2280

gctttgatgc aagtgtggac caggcgtcca tcaaccccgg caagcagagg tttgaaaaga 2340

tgatcagcgg catgtacctg ggggagatcg tccgccacat ccttttacat ttaaccagcc 2400

ttggcgttct cttccggggc cagcagatcc agcgccttca gaccagggac atcttcaaga 2460

ccaagttcct ctctgagatc gaaagtgaca gcctggccct gcggcaggtc cgagccatcc 2520

tagaggatct ggggctaccc ctgacctcag atgacgccct gatggtgcta gaggtgtgcc 2580

aggctgtgtc ccagagggct gcccagctct gtggggcggg tgtagctgcc gtggtggaga 2640

agatccggga gaaccggggc ctggaagagc tggcagtgtc tgtgggggtg gatggaacgc 2700

tctacaagct gcacccgcgc ttctccagcc tggtggcggc cacagtgcgg gagctggccc 2760

ctcgctgtgt ggtcacgttc ctgcagtcag aggatgggtc cggcaaaggt gcggccctgg 2820

tcaccgctgt tgcctgccgc cttgcgcagt tgactcgtgt ctgaggaaac ctccaggctg 2880

aggaggtctc cgccgcagcc ttgctggagc cgggtcgggg tctgcctgtt tcccagccag 2940

gcccagccac ccaggactcc tgggacatcc catgtgtgac ccctctgcgg ccatttggcc 3000

ttgctccctg gctttccctg agagaagtag cactcaggtt agcaatatat atatataatt 3060

tatttacaaa aaaaaaaaaa 3080

<210> 37
<211> 2180
<212> DNA
<213> Homo sapiens

<400> 37
   ggcgcactgc tcccctctgg actgcggagg cactgcactg cgcgggacgt gggagggtgc 60
   ctctgcagcc cctcaaggcc gccgcccctg cggggagaac tggagaatgc ggacacctag 120
   atgaggccct ggcacccaag ctgggccccc cggaatctca cgttcccttt actcgccagg 180
   catttcgggg caaaacgaag cggagccccc caaaccccca gacctaacca agttcagaga 240
   ttcgcagaag cacgcccccc accccaaatt tattgtgctc tacccaaaat ggaataggac 300
   taggtttatt tacccattgt gagggtagag aggcgagtct ggaggagcag ggattgggag 360
   aaggggtgga aaaatactct gattcttaaa aatactttgt aacctaaagt ccttaaattg 420
   tggaagaaag gaatactcct cctttccatt gtagtctaga gttaagattt caaatccata 480
   aattagagga cctaaaatta gagggcaatt aactgctcat tcattgggcc cccagtcagc 540
   acgggggtgc tggaagagat cgggaataat agcgcagacc aatgagccta gggagatgct 600
   ttcatcgtct ctccttccct caagtgttct ggaacctatc atttgaatta gccgagtcag 660
   gcaggagggg gcggggaatc cttccgccct tcttaggagg ggctgcattg cagggggaga 720
   gtgaactgac agactcagtc actgaagagg gaaaaggagt gagaagacaa agccgtcaaa 780
   gccccaacag ctttgtattt ctccagcccg gcgcagaccc cggagctccc gaggcactcc 840
   ctccatcttt ggaacacgcc agtaattgat tgataacagg aagctatgag ggaccctgtg 900
   agtagccagt acagttcctt tcttttctgg aggatgccca tcccagaact ggatctgtcg 960
   gagctggaag gcctgggtct gtcagataca gccacctaca aggtcaaaga cagcagcgtt 1020
   ggcaaaatga tcgggcaagc aactgcagca gaccaggaga aaaaccctga aggtgatggc 1080
   ctccttgagt acagcacctt caacttctgg agagctccca ttgccagcat ccactccttc 1140
   gaactggact tgctctaagg ccaagacttc tctctcccat caccttgccc tcattgtctt 1200
   ccctctcaag ccccttcctt tccactcctt tcccatttta atcttgttct ctccctactg 1260
   tgttggtggt gctgatgaat ctgccagagt tgagttctat gtatttattt atctatctgt 1320
   ctactccatt tctctcaaaa gccctcaagt cacaaagtaa atggttcaag caatggagta 1380
   ctgggtcaca gggattcctc ctttcccccc caaatattaa ctccagaaac taggcctgac 1440
   tggggacacc tgagagtagt atagtagtgc aaaatggaag actgattttt gactctatta 1500
   taatcagctt cagagattcc ttaaaccttc ctaatttcct gctccagggc agtaaacaca 1560
   aatatttctt caaggggtga tgaaaacctc ggaagtttta atttgaggtt atctgctacg 1620
   aaacagtatt tctaaaaggc taaagtgata agtctcttgc ttttttttga tcctgctctt 1680
   atattctttt ttttcctcag agaaatcagg agggtagtta gaggtataaa acaggaggaa 1740
   atattatgga aaatgaaaat agggaaaata attgaatcat tttagaagta gctaatttct 1800
   tttctcaaaa gagtgtccct tcttcacacc tactcacttt acaactttgc tcctaactgt 1860
   gggttgaaaa ctctagctaa agaaagttat caaatcttaa catgcattcc tactattatg 1920
   atagttttta aggtttcaat tcaatcttct gaacggcata agtcctattt tagccttacc 1980
   tcctgcattt gcaatacgta atactgatca gtgggcacag ttcttcagct acattgagac 2040
   cctgaaatga acaattatat tctgactcga catcttgtcc ccaatccttc caaaaatatt 2100
   gatggtgatt tgtgctacca tttactcgtt tatttaataa agacattcaa tcccaggaaa 2160
   aaaaaaaaaa aaaaaaaaaa 2180

<210> 38
<211> 2046
<212> DNA
<213> Homo sapiens

<400> 38
gcggcttgtt ttctttcctc cagtctcggg gctgcaggct gagcgcgatg cgcggagacc 60

cccgcggggg cggcggcggc cgtgagcccc gatgaggccc gagcgtcccc ggccgcgcgg 120

cagcgccccc ggcccgatgg agaccccgcc gtgggaccca gcccgcaacg actcgctgcc 180

gcccacgctg accccggccg tgccccccta cgtgaagctt ggcctcaccg tcgtctacac 240

cgtgttctac gcgctgctct tcgtgttcat ctacgtgcag ctctggctgg tgctgcgtta 300

ccgccacaag cggctcagct accagagcgt cttcctcttt ctctgcctct tctgggcctc 360

cctgcggacc gtcctcttct ccttctactt caaagacttc gtggcggcca attcgctcag 420

ccccttcgtc ttctggctgc tctactgctt ccctgtgtgc ctgcagtttt tcaccctcac 480

gctgatgaac ttgtacttca cgcaggtgat tttcaaagcc aagtcaaaat attctccaga 540

attactcaaa taccggttgc ccctctacct ggcctccctc ttcatcagcc ttgttttcct 600

gttggtgaat ttaacctgtg ctgtgctggt aaagacggga aattgggaga ggaaggttat 660

cgtctctgtg cgagtggcca ttaatgacac gctcttcgtg ctgtgtgccg tctctctctc 720

catctgtctc tacaaaatct ctaagatgtc cttagccaac atttacttgg agtccaaggg 780

ctcctccgtg tgtcaagtga ctgccatcgg tgtcaccgtg atactgcttt acacctctcg 840

ggcctgctac aacctgttca tcctgtcatt ttctcagaac aagagcgtcc attcctttga 900

ttatgactgg tacaatgtat cagaccaggc agatttgaag aatcagctgg gagatgctgg 960

atacgtatta tttggagtgg tgttatttgt ttgggaactc ttacctacca ccttagtcgt 1020

ttatttcttc cgagttagaa atcctacaaa ggaccttacc aaccctggaa tggtccccag 1080

ccatggattc agtcccagat cttatttctt tgacaaccct cgaagatatg acagtgatga 1140

tgaccttgcc tggaacattg cccctcaggg acttcaggga ggttttgctc cagattacta 1200

tgattgggga caacaaacta acagcttcct ggcacaagca ggaactttgc aagactcaac 1260

tttggatcct gacaaaccaa gccttgggta gcatcagtta acagttttat ggacgattcc 1320

tcagatgaaa agcttcagaa aagcatagtg acagctgaat ttttagggca cttttcctta 1380

agaaatagaa cttgattttt atttgttaca ggtttccaat ggccccatag gaataagcaa 1440

taatgtagac tgataaaccc ttattttagt actaaagagg gagccttgct atttcagtgg 1500

gtataattta aactttttaa agaaaatctg tacttttata aagatgtatt ttgtataact 1560

taaataataa tgctaaagta tactagggtt tttttttctt gagaatgtta ctgcaatcat 1620

gttgtagttt gcacagactt ttatgcataa ttcactttaa aaatatagaa tatatggtct 1680

aatagttttt taaagctttt ggactaaagt attccacaaa tcttacctct ttaggtcact 1740

gatggtcact ccgattctga gtgccacatt ggtagactcc taaaatacag ttgacaactt 1800

agccaattgc aactccagtg ttgataatta aaatgaaatg gtaaagcagc agactgtaag 1860

gtctttagag attttttttt taaggttcag gccgtaggtt cctcaaggaa tctcttaagt 1920

tttgcccaaa gactggtact tcctttcagt agggcgctaa tgtatacaca ttaatgataa 1980

gttgataaca ttaaaaatgt agctgactta tcctattaaa cctcctctgc tatgttcaca 2040

gaaaaa 2046

<210> 39
<211> 1025
<212> DNA
<213> Homo sapiens

<400> 39
cagctgttac cgcgtcacat gagggaggcc ggcggccact cggcggggga ggggaccgtg 60

gctggagccc ggggcggggc cgcgcggcag gcggggcggg agccgggggg cgcagctaga 120

gagccccgga gccgcggcgg gagaggaacg cgcagccagc cttgggaagc ccaggcccgg 180

cagccatggc ggtggaagga ggaatgaaat gtgtgaagtt cttgctctac gtcctcctgc 240

tggccttttg cgcctgtgca gtgggactga ttgccgtggg tgtcggggca cagcttgtcc 300

tgagtcagac cataatccag ggggctaccc ctggctctct gttgccagtg gtcatcatcg 360

cagtgggtgt cttcctcttc ctggtggctt ttgtgggctg ctgcggggcc tgcaaggaga 420

actattgtct tatgatcacg tttgccatct ttctgtctct tatcatgttg gtggaggtgg 480

ccgcagccat tgctggctat gtgtttagag ataaggtgat gtcagagttt aataacaact 540

tccggcagca gatggagaat tacccgaaaa acaaccacac tgcttcgatc ctggacagga 600

tgcaggcaga ttttaagtgc tgtggggctg ctaactacac agattgggag aaaatccctt 660

ccatgtcgaa gaaccgagtc cccgactcct gctgcattaa tgttactgtg ggctgtggga 720

ttaatttcaa cgagaaggcg atccataagg agggctgtgt ggagaagatt gggggctggc 780

tgaggaaaaa tgtgctggtg gtagctgcag cagcccttgg aattgctttt gttttgggaa 840

ttgtctttgc ctgctgcctc gtgaagagta tcagaagtgg ctacgaggtg atgtaggggt 900

ctggtctcct cagcctcctc atctggggga gtggaatagt atcctccagg tttttcaatt 960

aaacggatta ttttttcaga ccgaaaagag atggtctgag tttgtcttag aaaaaaaaaa 1020

aaaaa 1025

<210> 40
<211> 1031
<212> DNA
<213> Homo sapiens

<400> 40
cagctgttac cgcgtcacat gagggaggcc ggcggccact cggcggggga ggggaccgtg 60

gctggagccc ggggcggggc cgcgcggcag gcggggcggg agccgggggg cgcagctaga 120

gagccccgga gccgcggcgg gagaggaacg cgcagccagc cttgggaagc ccaggcccgg 180

cagccatggc ggtggaagga ggaatgaaat gtgtgaagtt cttgctctac gtcctcctgc 240

tggccttttg cgcctgtgca gtgggactga ttgccgtggg tgtcggggca cagcttgtcc 300

tgagtcagac cataatccag ggggctaccc ctggctctct gttgccagtg gtcatcatcg 360

cagtgggtgt cttcctcttc ctggtggctt ttgtgggctg ctgcggggcc tgcaaggaga 420

actattgtct tatgatcacg tttgccatct ttctgtctct tatcatgttg gtggaggtgg 480

ccgcagccat tgctggctat gtgtttagag ataaggtgat gtcagagttt aataacaact 540

tccggcagca gatggagaat tacccgaaaa acaaccacac tgcttcgatc ctggacagga 600

tgcaggcaga ttttaagtgc tgtggggctg ctaactacac agattgggag aaaatccctt 660

ccatgtcgaa gaaccgagtc cccgactcct gctgcattaa tgttactgtg ggctgtggga 720

ttaatttcaa cgagaaggcg atccataagg agggctgtgt ggagaagatt gggggctggc 780

tgaggaaaaa tgtgctggtg gtagctgcag cagcccttgg aattgctttt gtcgaggttt 840

tgggaattgt ctttgcctgc tgcctcgtga agagtatcag aagtggctac gaggtgatgt 900

aggggtctgg tctcctcagc ctcctcatct gggggagtgg aatagtatcc tccaggtttt 960

tcaattaaac ggattatttt ttcagaccga aaagagatgg tctgagtttg tcttagaaaa 1020

aaaaaaaaaa a 1031

<210> 41
<211> 3300
<212> DNA
<213> Homo sapiens

<400> 41
ccctcgccgc agtcgcgggc accccgctcg gcgtcggtgc ctgagggagg ccgcgatggc 60

ggccgaggcc ctggcggcgg aggccgtggc gtcgcgcctg gagcggcagg aggaggacat 120

ccgctggctg tggtcggagg tcgagcgcct gagggacgag cagctgaacg cgccctacag 180

ctgccaggcg gaggggccgt gcctcacgcg ggaggtggcg cagctccggg ccgagaactg 240

cgacctgcgc caccgcctgt gcagcctgcg gctgtgcctc gccgaggagc ggagccgcca 300

ggccacgctg gagagcgcgg agctagaggc ggcgcaggag gccggcgcac agcctcctcc 360

tagtcaaagc caagacaagg acatgaaaaa gaagaaaatg aaggaaagcg aggctgacag 420

cgaggtgaag catcaaccaa ttttcataaa agaaagattg aagctttttg aaatactgaa 480

gaaagaccat cagctcttac ttgccattta tggaaaaaag ggggatacaa gcaacatcat 540

cacagtaaga gtggctgatg ggcaaacagt gcaaggggaa gtctggaaaa caacgcctta 600

ccaagtggct gctgaaatta gtcaggaact ggctgaaagc acggtaatag ccaaagtcaa 660

tggtgaactg tgggacctgg accgcccatt ggaaggggac tcttctctag agctgcttac 720

atttgataat gaggaagctc aagctgtgta ctggcactcc agtgctcaca ttcttgggga 780

ggccatggag ctttactatg gaggccacct gtgctacggt ccgcccattg aaaatggatt 840

ttattatgac atgttcattg aagacagagc agtgtccagc acagaattgt cagccctgga 900

gaatatatgt aaagccatca taaaagaaaa gcaacctttt gaaagactag aagtcagcaa 960

ggaaatcctc ctggaaatgt ttaagtacaa taaatttaaa tgccgcattc tgaatgagaa 1020

agttaacact gcaactacca ccgtgtacag gtgcggtcca ttaattgacc tttgcaaagg 1080

tccacatgta agacacactg gaaaaattaa aaccatcaaa atttttaaga attcctcaac 1140

atattgggag ggcaatccgg aaatggaaac attgcagagg atctatggaa tatcctttcc 1200

tgataacaag atgatgagag actgggaaaa gttccaagag gaagcaaaga accgagatca 1260

caggaagatc gggaaggaac aagaactttt ctttttccac gatttgagtc ctggaagctg 1320

ttttttcctt cccagaggag ccttcattta taatacgctt acagatttca tacgagagga 1380

atatcacaaa cgggacttca cggaggtgct ctctcccaat atgtacaaca gtaaactctg 1440

ggaagcctca ggccactggc agcattacag cgagaacatg tttacctttg agattgaaaa 1500

ggacactttt gccctcaaac ccatgaattg tccagggcac tgtctaatgt ttgcccatcg 1560

tccacgatct tggagggaaa tgcctattag atttgctgat tttggagttc tgcatagaaa 1620

tgaactgtcg gggactctca gcggcttgac cagagtgagg cgcttccagc aggacgatgc 1680

tcacattttt tgcacagtgg agcagattga agaagaaata aaggggtgtt tgcagttttt 1740

gcaatctgtt tactcaacat ttggcttctc ctttcaatta aacctgtcaa caaggccgga 1800

aaacttccta ggagagattg agatgtggaa tgaggctgag aagcaactgc agaacagctt 1860

gatggacttt ggagaaccgt ggaaaatgaa cccaggagat ggagcatttt atggccctaa 1920

aattgacata aaaatcaagg atgctattgg cagataccat caatgtgcta caattcagct 1980

ggacttccaa ctgcctatta gatttaatct cacatatgtt agtaaggatg gggatgataa 2040

gaagagacct gtgatcattc atcgagccat tttgggatca gtggaaagaa tgatagccat 2100

tctttcagaa aactatggcg gaaaatggcc tttctggcta tctcctcgtc aggtgatggt 2160

catccctgtg gggccaactt gtgaaaaata tgcacttcag gtatccagtg aattttttga 2220

agaaggattt atggctgacg ttgacttgga tcacagttgt acactaaata agaaaatacg 2280

aaatgcacag ctggctcagt ataattttat tttggtggtt ggagaaaagg aaaagataga 2340

taatgctgta aacgtgcgaa caagagacaa caaaattcat ggagagattt tagtaacttc 2400

tgccattgat aaactgaaga atctcaggaa gacacggaca ctcaatgctg aggaggcctt 2460

ttgaagtcct tccctgatat ttgcttctgt gtaactttgt tttgaccctt aaaaatgtat 2520

ttttcttaac atgttagtac ttctacgact ttggagccac tgatgggtcc actcatggcc 2580

tcagctgaga aaggagacga tgaacgtgta gctgacatgc acgaagttta atttactcat 2640

gtccacgggg gacgtttaga gggcacgtgg gaaattttcc agcaatcaat gccttgagaa 2700

acttaaatgg ggaaatatta ttcatcgaga aagtgaaaca aaacactagg aaatgattat 2760

gaaatgttag tgattttcaa aagatgggct tcaaataaaa gtctgcagag ttttttttaa 2820

ataggaggga aaatcttatt ttctaatatg tctcaggtat ttttatgact tctactaaaa 2880

ttcacactga aactttatct tctaaactgg aatcattact taattttaac taaccaacaa 2940

ccacaaaagc agcagctact gctaaatatt ggattactga caaaggaatt cagttttgtg 3000

gaatctggtg tttgcactat aggttaagag ttgccattta aatgtttctt attcataatt 3060

aggttttgtt ccgctttaga aaaaaataaa ttcccaaatg aattgcatca gtgtttccag 3120

cgtttcagct gtgggcttct accaccacta agccgctcac ctagagttgt tacttttttg 3180

atgggatgac attccatgga tttatgtact gaaatggatt tcagatttta aagagaggaa 3240

tgctctacgt gctcagaact gaattaaaat ggcattatgt caatagagaa ttcaaaaaaa 3300

<210> 42
<211> 3468
<212> DNA
<213> Homo sapiens

<400> 42
   ttcagcttgt gggtagcact cgggccgagc catgcaggcg gcgcgcgtgg actacatcgc 60
   tccctggtgg gtcgtgtggc tgcacagcgt cccgcacgtc ggcctgcgcc tgcagcccgt 120
   gaacagcacc ttcagccccg gcgacgagag ttaccaggag tcgctgctgt tcctggggct 180
   ggtggccgcc gtctgcctgg gcctgaacct catcttcctt gtggcttacc tggtctgtgc 240
   atgccactgc cggcgggacg atgcggtgca gaccaagcag caccactcct gctgcatcac 300
   ctggacggcc gtggtggccg ggctcatctg ctgtgctgcg gtgggcgttg gtttctatgg 360
   aaacagcgag accaacgatg gggcgtacca gctgatgtac tccttggacg atgccaacca 420
   caccttctct gggatcgatg ctctggtttc cggaactacc cagaagatga aggtggacct 480
   agagcagcac ctggcccggc tcagtgagat ctttgctgcc cggggcgatt acctgcagac 540
   cctgaagttc atacagcaga tggcgggcag cgttgttgtt cagctctcag gactgcccgt 600
   gtggagggag gtcaccatgg agctgaccaa gctatccgac cagactggct acgtggagta 660
   ctacaggtgg ctctcctacc tcctgctctt tatcctggac ctggtcatct gcctcattgc 720
   ctgcctggga ctggccaagc gctccaagtg tctcctggcc tcgatgctgt gctgtggggc 780
   actgagcctg ctcctcagtt gggcatccct ggccgctgat ggctctgcgg cagtggccac 840
   cagtgacttc tgtgtggctc ctgacacctt catcctgaac gtcacggagg gccagatcag 900
   cacagaggtg actcgctact acctgtattg cagccagagt ggaagcagcc ccttccagca 960
   gaccctgacc accttccagc gcgcacttac caccatgcag atccaggtcg cggggctgct 1020
   gcagtttgcc gtgcccctct tctccactgc agaggaagac ctgcttgcaa tccagctcct 1080
   gctgaactcc tcagagtcca gccttcacca gctgaccgcc atggtggact gccgagggct 1140
   gcacaaggat tatctggacg ctcttgctgg catctgctac gacggcctcc agggcttgct 1200
   gtaccttggc ctcttctcct tcctggccgc cctcgccttc tccaccatga tctgtgcagg 1260
   gccaagggcc tggaagcact tcaccaccag aaacagagac tacgatgaca ttgatgatga 1320
   tgaccccttt aacccccaag cctggcgcat ggcggctcac agtcccccga ggggacagct 1380
   tcacagcttc tgcagctaca gcagtggcct gggaagtcag accagcctgc agcccccggc 1440
   ccagaccatc tccaacgccc ctgtctccga gtacatgaac caagccatgc tctttggtag 1500
   gaacccacgc tacgagaacg tgccactaat cgggagagcc tcccctccgc ctacgtactc 1560
   tcccagcatg agagccacct acctgtctgt ggcggatgag cacctgaggc actacgggaa 1620
   tcagtttcca gcctaacaga ctttcggggg ttcctgcctc ctttttccgt tctggttttt 1680
   aattagtgca aatacaagct gcgtttcttt aatagaaacc aaaggcatct ggagcccgag 1740
   aggcctcctg ctgtggcaga ggagcagctg ggattcccga ccaaagcccc agggggtgca 1800
   gaagactcac cacgcgggcc agcctctctc ttttgccctg ctctccacac cagaaatgcc 1860
   cccaggtgct tggctgcctc agaggtacca tccctgagct ggctgcctgg ccctgctcac 1920
   ccctacgcct cgcccttgcc aggaggggag tggcagtgag gaggggccca ggtcaggcac 1980
   caccatcaag agagctgtgt gttctctctg gtcccacaac gatgactctg cctcttgtca 2040
   gcccagccaa gagcccagac gacccctctg tcctcgttcc ctgtcctcgt tccctgcagg 2100
   taacatgaga agggctgatc aggagatgct ctttaagaag ttcgcacccc tgctgacacc 2160
   agaacagccc aaatcagagt tcccagggcc agacaggctc ttcctgggcc acagagggga 2220
   ggcatcagga aagctctgca gtggggggct ggtggctccg gggctggggg atcacaggct 2280
   ggtgaacccc ggtgggaaca gaggtgaaag cctgccacat tccgcctgtc tccctaaccc 2340
   tccattgcct cgcctctatt ccagaatcaa tgctgcagaa tgtgttagct gcagataggc 2400
   atggtctcag gtatgaacag acactttgaa acgactttag gtctttcttt tctccagtgt 2460
   tttaaacatg ttgattatcc aaagaattga aactcctagc acatccagtt tttacaacag 2520
   atttgcagct cattccttac cctggttagg tcactacttt tgcagatttt gctggcactg 2580
   atctggagat ctgcagatct ggaggagacg ggaaggagtc gattcttaaa taaggatcag 2640
   tgaggcatcc tgtcccaagc tactgtttgg tggggatctg ggttcatctc acccacagag 2700
   ggaggatctt taagaggaga aaaaagccaa gagggaaagc cagagttccc tgttctaggg 2760
   gactagccaa atgcctacat cagctgtccc ctccctgttg tctccaagta agtttgccag 2820
   aaaaggtttt agcaaagtgc tacaactgtg tctttatagg aggataggcc tctgccctgc 2880
   cccaccccca ccacctgtcc ccacccagtg tcccaggcca caggagctta ttggccagga 2940
   gggaataatg tcccccaata ctgcctgttg agggaccaga gttggggtct ttggtgcttc 3000
   caacctcctg ccaacctgga gttcacaaca ccagagcccc acggcctcgc acactgaagc 3060
   aggggcgtgc ggtgactcgg tgcttctgtt ttggaagaac cacctgtcat caaaacatgg 3120
   acagcagggt gttctcagct cccagcgaag cctccacaac agaatggggc cacagggcag 3180
   ccgggactcc ctgtctcacc tacattaacc catgcatact gtatgccata aactcacttt 3240
   ggtatatccg cgtcacatgc agagaggaac tctgcgacgt caaagtgttg cttcttaaag 3300
   tttcattatt ggcaactaga gggttgtttt taatgcatgg aaactaaaca gattcctcgg 3360
   ggagttcctg aaggaaccag gtgggcaaac ctttgcttat atacatgcgg cctcacctgg 3420
   aagagaaata aaccacttgt actaaaaaaa aaaaaaaaaa aaaaaaaa 3468

<210> 43
<211> 962
<212> DNA
<213> Homo sapiens

<400> 43
gtcgtgaggc gggccttcgg gctggctcgc cgtcggctgc cggggggttg gccggggtgt 60

cattggctct gggaagcggc agcagaggca gggaccactc ggggtctggt gtcggcacag 120

ccatggcggg cgcgttggtg cggaaagcgg cggactatgt ccgaagcaag gatttccggg 180

actacctcat gagtacgcac ttctggggcc cagtagccaa ctggggtctt cccattgctg 240

ccatcaatga tatgaaaaag tctccagaga ttatcagtgg gcggatgaca tttgccctct 300

gttgctattc tttgacattc atgagatttg cctacaaggt acagcctcgg aactggcttc 360

tgtttgcatg ccacgcaaca aatgaagtag cccagctcat ccagggaggg cggcttatca 420

aacacgagat gactaaaacg gcatctgcat aacaatggaa aaggaagaac aaggtcttga 480

agggacagca ttgccagctg ctgctgagtc acagatttca ttataaatag cctccctaag 540

gaaaatacac tgaatgctat ttttactaac cattctattt ttatagaaat agctgagagt 600

ttctaaacca actctctgct gccttacaag tattaaatat tttacttctt tccataaaga 660

gtagctcaaa atatgcaatt aatttaataa tttctgatga tggttttatc tgcagtaata 720

tgtatatcat ctattagaat ttacttaatg aaaaactgaa gagaacaaaa tttgtaacca 780

ctagcactta agtactcctg attcttaaca ttgtctttaa tgaccacaag acaaccaaca 840

gctggccacg tacttaaaat tttgtcccca ctgtttaaaa atgttacctg tgtatttcca 900

tgcagtgtat atattgagat gctgtaactt aatggcaata aatgatttaa atatttgtta 960

aa 962

<210> 44
<211> 1424
<212> DNA
<213> Homo sapiens

<400> 44
gggacgtgcg gcccagcgag ttggtcggtc ccggggtcac ccgctacggg aagcaggcct 60

cgccacagac taagaaaaat ggctttgtca gcccaacaga tacccagatg gtttaactca 120

gttaagttga ggagcctcat taatgctgca caactcacaa aacgttttac tagaccagca 180

agaacactgt tacatggctt ttctgctcag cctcagatat cctctgacaa ttgctttctc 240

cagtggggat ttaagactta caggacttcc tccttatgga atagttccca gtctactagc 300

tcaagtagtc aggagaataa ttctgcccaa agcagtctgc ttccttccat gaatgaacag 360

tcacagaaga cacaaaatat atccagcttt gattctgagc tgtttctaga agaactggat 420

gaattgcctc cattgtctcc aatgcagcca atttcagagg aagaggctat tcagattatt 480

gcagaccctc cattgccacc agcttcattc acacttcgag actatgtgga tcattctgag 540

actctgcaga agttggttct tctaggcgtg gatttgtcca agatagaaaa acatccagaa 600

gcagcaaacc tccttctgag actggatttt gaaaaagaca ttaagcaaat gcttctgttt 660

cttaaagatg tgggtataga ggataaccaa ctgggagcat tcctgacaaa aaatcatgca 720

attttctctg aagaccttga aaatctgaag accagggtgg cttatctgca ttcaaaaaat 780

ttcagtaaag cagatgttgc acagatggtc agaaaagcac catttttgct gaacttttca 840

gtggaaagac tggataacag attgggattt tttcagaaag aacttgaact tagtgtgaag 900

aagactagag atctggtagt tcgtctccca aggctgctaa ctggaagtct ggaacccgtg 960

aaagaaaata tgaaggttta tcgtcttgaa cttggtttta aacataacga aattcaacat 1020

atgatcacca gaatcccaaa gatgttaact gcaaataaaa tgaaacttac cgagacgttt 1080

gattttgtgc acaatgtgat gagcattccc caccacatca ttgtcaagtt cccacaggta 1140

tttaatacaa ggctgtttaa ggtcaaagaa agacacttgt ttcttaccta tttaggaaga 1200

gcacagtatg atccagcaaa acctaactac atctctttgg acaaactagt atctattcct 1260

gatgaaatat tttgtgaaga gattgccaaa gcatcagtac aggactttga aaaattctta 1320

aaaacgcttt agatttttat gtatgttaaa atgcagtatt gtaaagtgaa tatatatatg 1380

aataaatgaa tatattttta aatgccaaaa aaaaaaaaaa aaaa 1424

<210> 45
<211> 913
<212> DNA
<213> Homo sapiens

<400> 45
agtgctgtac aaagagacag aggctgttag ctatggctga tctatccaat aaaaaagaga 60

gaggaccgca gacgcctggc tctcatcata tgcaatacaa agtttgatca cctgcctgca 120

aggaatgggg ctcactatga catcgtgggg atgaaaaggc tgcttcaagg cctgggctac 180

actgtggttg acgaaaagaa tctcacagcc agggatatgg agtcagtgct gagggcattt 240

gctgccagac cagagcacaa gtcctctgac agcacgttct tggtactcat gtctcatggc 300

atcctagagg gaatctgcgg aactgcgcat aaaaagaaaa aaccggatgt gctgctttat 360

gacaccatct tccagatatt caacaaccgc aactgcctca gtctaaagga caaacccaag 420

gtcatcattg tccaggcctg cagaggtgaa aaacatgggg aactctgggt cagagactct 480

ccagcatcct tggcactcat ctcttcacag tcatctgaga acctggaggc agattctgtt 540

tgcaagatcc acgaggagaa ggacttcatt gctttctgtt cttcaacacc acataacgtg 600

tcctggagag accgcacaag gggctccatc ttcattacgg aactcatcac atgcttccag 660

aaatattctt gctgctgcca cctaatggaa atatttcgga aggtacagaa atcatttgaa 720

gttccacagg ctaaagccca gatgcccacc atagaacgag caaccttgac aagagatttc 780

tacctctttc ctggcaattg aaaatgaaac cacaggcagc ccagccctcc tctgtcaaca 840

tcaaagagca catttaccag tatagcttgc atagtcaata tttggtattt caataaaagt 900

aaagactgta tct 913

<210> 46
<211> 1796
<212> DNA
<213> Homo sapiens

<400> 46
aatcaaaatt tataaaactt ctattttggt gttcaaagat cgggataaat atgtaagtgg 60

agaattcagg taagttcaga tttttccctc cagttggttt aatttctatt tcctaaaaca 120

ttaaaataat aatggaatga ttgaaataat aaacattttt cttattcaag atttcgtcat 180

ggctattgta aaggaaaccc taggaaaatg gtgaaaactt gggcagaaaa agaaatgagg 240

aacttaatca ggctaaacac agcagagata ccatgtccag aaccaataat gctaagaagt 300

catgttcttg tcatgagttt catcggtaaa gatgacatgc ctgcaccact cttgaaaaat 360

gtccagttat cagaatccaa ggctcgggag ttgtacctgc aggtcattca gtacatgaga 420

agaatgtatc aggatgccag acttgtccat gcagatctca gtgaatttaa catgctgtac 480

cacggtggag gcgtgtatat cattgacgtg tctcagtccg tggagcacga ccacccacat 540

gccttggagt tcttgagaaa ggattgcgcc aacgtcaatg atttctttat gaggcacagt 600

gttgctgtca tgactgtgcg ggagctcttt gaatttgtca cagatccatc cattacacat 660

gagaacatgg atgcttatct ctcaaaggcc atggaaatag catctcaaag gaccaaggaa 720

gaacggtcta gccaagatca tgtggatgaa gaggtgttta agcgagcata tattcctaga 780

accttgaatg aagtgaaaaa ttatgagagg gatatggaca taattatgaa attgaaggaa 840

gaggacatgg ccatgaatgc ccaacaagat aatattctat accagactgt tacaggattg 900

aagaaagatt tgtcaggagt tcagaaggtc cctgcactcc tagaaaatca agtggaggaa 960

aggacttgtt ctgattcaga agatattgga agctctgagt gctctgacac agactctgaa 1020

gagcagggag accatgcccg ccccaagaaa cacaccacgg accctgacat tgataaaaaa 1080

gaaagaaaaa agatggtcaa ggaagcccag agagagaaaa gaaaaaacaa aattcctaaa 1140

catgtgaaaa aaagaaagga gaagacagcc aagacgaaaa aaggcaaata gaatgagaac 1200

catattatgt acagtcattt tcctcagttc cttttctcgc ctgaactctt aagctgcatc 1260

tggaagatgg cttattggtt ttaaccagat tgtcatcgtg gcactgtctg tgaagacgga 1320

ttcaaatgtt ttcatgtaac tatgtaaaaa gctctaagct ctagagtcta gatccagtca 1380

ctgactctgt ctggtgttga cagaggattt atttaagcta ttattttaat aaagaacttt 1440

gtacattttt atttttatat ttttttctct tacaaatatg tttttggaag catgataaat 1500

gtttaaatgt agtcaacatc tgtaactctt acatgagtgt ccagaggcac tcatgggaaa 1560

attggttttg ctttctttgt acacaccaga gacccatctg aggtcatctg attataaggc 1620

catgtttata taaagggaat ttcacccaca gttcagctgg ctgttgattt tcactgcaac 1680

tctgcctttg tgtgtattgg cgatcatttg taatgctctt acacttcgtc tttaatgttc 1740

tttttggagt taggacctct cagttcataa agttttttac aattcaaaaa aaaaaa 1796

<210> 47
<211> 2495
<212> DNA
<213> Homo sapiens

<400> 47
   gggtggtgga tctgtcggtc ccgttttccc gtcgcacgtg gtggccactg ttggcttctg 60
   aatggtttgc aaggcggata tccacgccaa ggcctttgga tcggccgtgg gtacatccgt 120
   ctgagccgtt cctttccatc gcagagcggc ggcctccggc ggcgctctcc agtcatggac 180
   taccggcggc ttctcatgag ccgggtggtc cccgggcaat tcgacgacgc ggactcctct 240
   gacagtgaaa acagagactt gaagacagtc aaagagaagg atgacattct gtttgaagac 300
   cttcaagaca atgtgaatga gaatggtgaa ggtgaaatag aagatgagga ggaggagggt 360
   tatgacgatg atgatgatga ctgggactgg gatgaaggag ttggaaaact cgccaagggt 420
   tatgtctgga atggaggaag caacccacag gcaaatcgac agacctccga cagcagttca 480
   gccaaaatgt ctactccagc agacaaggtc ttacggaaat ttgagaataa aattaattta 540
   gataagctaa atgttactga ttccgtcata aataaagtca ccgaaaagtc tagacaaaag 600
   gaagcagata tgtatcgcat caaagataag gcagacagag caactgtaga acaggtgttg 660
   gatcccagaa caagaatgat tttattcaag atgttgacta gaggaatcat aacagagata 720
   aatggctgca ttagcacagg aaaagaagct aatgtatacc atgctagcac agcaaatgga 780
   gagagcagag caatcaaaat ttataaaact tctattttgg tgttcaaaga tcgggataaa 840
   tatgtaagtg gagaattcag atttcgtcat ggctattgta aaggaaaccc taggaaaatg 900
   gtgaaaactt gggcagaaaa agaaatgagg aacttaatca ggctaaacac agcagagata 960
   ccatgtccag aaccaataat gctaagaagt catgttcttg tcatgagttt catcggtaaa 1020
   gatgacatgc ctgcaccact cttgaaaaat gtccagttat cagaatccaa ggctcgggag 1080
   ttgtacctgc aggtcattca gtacatgaga agaatgtatc aggatgccag acttgtccat 1140
   gcagatctca gtgaatttaa catgctgtac cacggtggag gcgtgtatat cattgacgtg 1200
   tctcagtccg tggagcacga ccacccacat gccttggagt tcttgagaaa ggattgcgcc 1260
   aacgtcaatg atttctttat gaggcacagt gttgctgtca tgactgtgcg ggagctcttt 1320
   gaatttgtca cagatccatc cattacacat gagaacatgg atgcttatct ctcaaaggcc 1380
   atggaaatag catctcaaag gaccaaggaa gaacggtcta gccaagatca tgtggatgaa 1440
   gaggtgttta agcgagcata tattcctaga accttgaatg aagtgaaaaa ttatgagagg 1500
   gatatggaca taattatgaa attgaaggaa gaggacatgg ccatgaatgc ccaacaagat 1560
   aatattctat accagactgt tacaggattg aagaaagatt tgtcaggagt tcagaaggtc 1620
   cctgcactcc tagaaaatca agtggaggaa aggacttgtt ctgattcaga agatattgga 1680
   agctctgagt gctctgacac agactctgaa gagcagggag accatgcccg ccccaagaaa 1740
   cacaccacgg accctgacat tgataaaaaa gaaagaaaaa agatggtcaa ggaagcccag 1800
   agagagaaaa gaaaaaacaa aattcctaaa catgtgaaaa aaagaaagga gaagacagcc 1860
   aagacgaaaa aaggcaaata gaatgagaac catattatgt acagtcattt tcctcagttc 1920
   cttttctcgc ctgaactctt aagctgcatc tggaagatgg cttattggtt ttaaccagat 1980
   tgtcatcgtg gcactgtctg tgaagacgga ttcaaatgtt ttcatgtaac tatgtaaaaa 2040
   gctctaagct ctagagtcta gatccagtca ctgactctgt ctggtgttga cagaggattt 2100
   atttaagcta ttattttaat aaagaacttt gtacattttt atttttatat ttttttctct 2160
   tacaaatatg tttttggaag catgataaat gtttaaatgt agtcaacatc tgtaactctt 2220
   acatgagtgt ccagaggcac tcatgggaaa attggttttg ctttctttgt acacaccaga 2280
   gacccatctg aggtcatctg attataaggc catgtttata taaagggaat ttcacccaca 2340
   gttcagctgg ctgttgattt tcactgcaac tctgcctttg tgtgtattgg cgatcatttg 2400
   taatgctctt acacttcgtc tttaatgttc tttttggagt taggacctct cagttcataa 2460
   agttttttac aattcaaaaa aaaaaaaaaa aaaaa 2495

<210> 48
<211> 2816
<212> DNA
<213> Homo sapiens

<400> 48
cgccgcgggc cgggcgcggg gaggtgtcat gcgccggaac ctgcgcttgg ggccaagctc 60

tggagctgac gcgcaggggc aaggcgcccc gcgtcccgga ctggcggctc cgcgcatgct 120

cctcccaccg gcgtcgcagg cctcgagagg ctccggaagt actgggtgca gcctgatggc 180

gcaggaggta gacacggcac agggcgccga gatgcggcgg ggcgcgggcg cggctcgggg 240

acgcgcttcc tggtgctggg ccctggcgct gctttggctc gcggtggttc cgggctggtc 300

ccgggtctcg ggcatcccct cccggcgcca ctggccggtg ccctacaagc gctttgactt 360

ccgtccaaaa cctgatcctt attgtcaagc taagtatact ttctgtccaa ctggctcacc 420

tatcccagtt atggagggtg atgatgacat tgaagttttt cgattacaag ccccagtatg 480

ggaatttaaa tatggagacc tcctgggaca cttgaaaatt atgcatgatg ccattggatt 540

cagaagtaca ttaactggca agaactacac aatggaatgg tatgaacttt tccaacttgg 600

caactgtaca tttccccatc tccgacctga aatggatgcc cctttctggt gtaatcaagg 660

cgctgcctgc ttttttgagg gaattgatga tgttcactgg aaggaaaatg ggacattagt 720

tcaagtagca actatatcag gaaacatgtt caaccaaatg gcaaagtggg tgaaacagga 780

caatgaaaca ggaatttatt atgagacatg gaatgtaaaa gccagcccag aaaagggggc 840

agagacatgg tttgattcct acgactgttc caaatttgtg ttaaggacct ttaacaagtt 900

ggctgaattt ggagcagagt tcaagaacat agaaaccaac tatacaagaa tatttcttta 960

cagtggagaa cctacttatc tgggaaatga aacatctgtt tttgggccaa caggaaacaa 1020

gactcttggt ttagccataa aaagatttta ttaccccttc aaaccacatt tgccaactaa 1080

agaatttctg ttgagtctct tgcaaatttt tgatgcagtg attgtgcaca aacagttcta 1140

tttgttttat aattttgaat attggttttt acctatgaaa ttccctttta ttaaaataac 1200

atatgaagaa atccctttac ctatcagaaa caaaacactc tctggtttat aaaacacctt 1260

aattctactg ctcttttttc tccaatcacc agcatctgtt tttcaggggg tgattttact 1320

tttgtgaatt ccttagcctt tcttccttgg tgcataaagt taaaatgcac atcagcagaa 1380

ttgctgcata ttaacatctc aggactcttc tcttgtaaag aagctgaaat tcgtactata 1440

ttggccaaag tgagcgagtt aggtgatctt ggtttcaatt tccgagcctt tgttaatatg 1500

gagaattatg gttcatatca gttatgtagg acctttggac ccagggtcct acagatagat 1560

atggtgtgcc cagattttaa aaataccttc aaaaataaaa aatacattca gtgacatttt 1620

catggtggga gctcttcttt ctgatatggc agttacactt tttcacttaa gtgctttagt 1680

ttagactaac tttacaactt ctataacttt tggaaccaag tttagtatag tctgattaca 1740

ttccattcac ctaactttag acattcgttt agacaccata actggagtga ttgtgcttct 1800

agatgtggca aatccagtgt taacacatat ttctggctga gattttggaa ctagctagta 1860

actggcttgt gttctttaag catactaaca tcactaaatc ttaggattta ggattgctgt 1920

aaagatgtaa gttgtgtatg tttggcaggt cacattgaat ggcagtgata atgattaatc 1980

aaagaacaaa tgtcatcctt gatcttgcct aatgtagttt atgtgccaaa ctttccaggg 2040

ttttgtagtc acctagattt taagctgata gcatagtgct tcagcggttc ttctaaccgg 2100

ggtatgcagg aacatggctg cagacacgtt tgggtaaaca ggcaccttct gacttcttca 2160

ttgtttcctg tagttctccc tcttcccaca aagctgtcag cgcagtggaa gaggttgcac 2220

ttctccgaga gaggacaggt ttctgttaag atcaccaagt agctgtgctt taattagagc 2280

cagacaagct ttcaaggtcc tttaagtatt tgatgatcaa ctgaacacgt ttctattcaa 2340

ggagaaaaca ccattcagta agaagatgga gtagatatca gataaaacaa ttcacgttta 2400

atatgtaaat gtaccaatta tgtgattcag tttcaacttt caagtacttc ctgagaggtt 2460

agtacattat tattgagctc tcacagaaag cttaatcata gatataatta acctgatcca 2520

aatgagtaaa gtggacctta gaaaggctaa gtgatctttc tctggctatc cagctagtag 2580

taatgaagtc aggtcttgaa ccccggttct gctgactgaa ttggatgcac tatagtacag 2640

gcttttagca ccgaagtgtg gtcctcagac cagtgcctgc caaccagatg ttactggtct 2700

gtgaggaaat aagtacagat actgacagga agcttttata aacaatttat tggagtgttt 2760

ttgtttctgt tgggtctaat taaaaaaatt ggagcttgta aaaaaaaaaa aaaaaa 2816

<210> 49
<211> 3859
<212> DNA
<213> Homo sapiens

<400> 49
aaagatgact cctgtacggt ccctgccgtc caggaccctg gagtcaagtc agaaatggat 60

gtgtccacag ctgattgtgt gacaaggtct ggatttgtgg gtgttttcat tggagtccag 120

ggaccctggt accttatgtg cttttttgcc ttgtttcttt ccagattgct catttagaaa 180

gtggatgtgt gggataacat gtccaatgtg tccgaggaga gaagaaaaag gcagcagaac 240

attaaggaag gactgcagtt tatacagtca ccgctgtcat atccaggaac acaggagcaa 300

tatgcggtat atttgcgtgc tctcgtgaga aatcttttta atgaaggaaa tgacgtttat 360

cgggaacatg attggaacaa ctcgataagc cagtacacgg aagccttgaa tatagctgat 420

tatgcaaaat ctgaagaaat tttaatcccc aaagaaataa ttgaaaaact atatataaat 480

cgtattgcct gctattctaa tatgggtttc catgataaag ttttggagga ctgcaatata 540

gtcctcagtt taaatgccag taactgcaaa gctctgtatc ggaaatctaa ggctttaagt 600

gatttaggaa gatacaaaaa ggcttacgat gctgtagcaa agtgctcctt agcagtgcct 660

caggatgagc atgtaataaa actaactcaa gaactagctc agaaattggg atttaaaata 720

agaaaagcgt atgtcagagc tgagctctca ttaaaatcag ttcctgggga tggggctacc 780

aaggctttga accattctgt ggaagatatt gagccagatt tattaactcc aaggcaagaa 840

gcagttcctg ttgtctcttt accggcaccc agtttttctc atgaagttgg aagtgagctg 900

gcctcagttc ctgttatgcc cttaacttct attttgccac tacaagtgga agagagcgct 960

ctgccatctg cagtgctggc aaatggagga aagatgccct tcactatgcc agaagctttt 1020

ctagatgatg gagatatggt ccttggagat gaactagatg acctgcttga ttctgcacct 1080

gaaactaatg aaactgttat gccgtcagct ttagtcagag gaccccttca gacagccagt 1140

gtctctccta gcatgccctt ttcggcatcg ctgttaggaa ccttacccat tggtgcgagg 1200

tatgctcctc caccctcctt ctcagaattt tatccacctt tgacttcatc cttagaagat 1260

ttttgttctt ctttaaattc attttcaatg agtgaatcca aacgagatct gtccacctca 1320

acttctagag agggaacacc gcttaacaac agtaattctt cccttttact tatgaatgga 1380

ccaggtagtt tgtttgcttc agagaatttc ctgggaattt caagtcagcc tagaaatgac 1440

tttggaaact tttttggaag tgcagttacc aaaccatctt catcagtgac tccaagacat 1500

cccctcgaag gaacccatga attgagacaa gcttgccaga tctgttttgt aaaatcaggc 1560

cctaagttaa tggatttcac ttaccatgct aacatagatc ataagtgtaa gaaagatatt 1620

ttaatcggta ggataaagaa tgttgaagat aaatcatgga aaaaaatacg tccaagacca 1680

acaaaaacaa attatgaagg accatattat atatgtaaag atgttgctgc tgaggaggaa 1740

tgtagatatt caggccactg cacgtttgct tattgccaag aggagataga tgtgtggaca 1800

ctggagcgga aaggagcatt cagccgggag gctttctttg gcggcaatgg aaagattaac 1860

cttactgtgt tcaaacttct ccaggagcat cttggggaat ttatattcct ttgtgagaaa 1920

tgttttgatc ataagcctag aatgataagt aaaagaaata aagataattc tactgcttgt 1980

tctcacccgg ttacaaagca tgagtttgaa gacaataagt gccttgtcca cattttgcga 2040

gagacaacag taaaatactc caaaatacgt tcttttcatg gtcagtgtca gcttgattta 2100

tgtcgacatg aagttcggta tggctgttta agggaagatg agtgctttta tgcccatagt 2160

cttgtggaac tgaaagtctg gataatgcaa aatgaaacag gtatctcaca tgatgctatt 2220

gctcaagagt ctaaacgata ttggcagaat ttggaagcaa atgtacctgg agcgcaggta 2280

cttggtaatc aaataatgcc tggatttctt aatatgaaga taaagtttgt gtgcgcccag 2340

tgtctgagaa acggtcaagt cattgaacca gacaaaaaca gaaaatattg tagtgcaaaa 2400

gcaaggcatt cgtggaccaa agaccggcgt gcgatgagag tgatgtctat tgaacgtaag 2460

aagtggatga acatccgtcc tctccccaca aagaaacaaa tgcctttaca gtttgatctg 2520

tgcaaccata ttgcttctgg gaaaaaatgt caatatgttg gaaactgttc ctttgctcat 2580

agtcctgagg aaagagaagt ttggacttac atgaaggaga atggatacaa agatatggag 2640

caattttacg aactatggct caagagtcaa aaaaatgaaa aaagtgaaga catagccagt 2700

cagtcaaaca aggaaaatgg aaaacaaatt cacatgccaa cagattatgc tgaagttaca 2760

gtggactttc actgctggat gtgtgggaaa aactgcaaca gtgagaagca gtggcagggc 2820

cacatctcct ccgagaagca caaagagaag gttttccaca ccgaggacga ccagtactgc 2880

tggcagcacc gcttcccaac aggctatttc agtatttgtg ataggtatat gaatggcacc 2940

tgcccagaag gaaacagctg taaatttgca catggaaatg ccgaacttca tgaatgggaa 3000

gaaagaagag atgccctaaa gatgaagctc aacaaagcac gaaaagatca cttaattggc 3060

ccaaatgata atgactttgg aaaatatagt tttttgttta aagatttaaa ctaatatgct 3120

ggcttttatg tatgatacct aatcagagca ttgaccagaa aaattgaaag tgttctgagg 3180

cacatagcag aggagctgca gatttcctgc ttgtattggc gtatatcgtt cctcctgagc 3240

agcaacccac agtaggtagg aaaatgggct gtttcacagg cctggccacg ctctcacgga 3300

accactggca tcagatggtg aagtgactgc tacccggttg ccatctgttg aacagacttt 3360

tggatgaagt gtgttgggga agaggataag gttatatcta ggacaactct ttgagttggt 3420

ccttcatata agaatcgtga cggtaagaga ataaacactt gtactgggat cagaatacat 3480

gatggatgaa attctttaca tgttttagca gaatgaattt gtttaatata ataaagtttg 3540

ctacttatct gtatgtaggt tgctaaaaag gatttcttta actcagattt taagccaaat 3600

aaccatttaa cactagtatt tgttaaatgg ggtatttttc tgtatttgta tgtttcacta 3660

taataaggga attaaggata atgtgcattg agaatatttt gaaaaataat tgactcaaat 3720

tttatttctt ggtcttttgc tgtttaaatg atgattttga aagattaaac ctgtactgtt 3780

ggtattgtgt tagtgtatgg accaatactg cctgtaataa agattttata tatagatgcc 3840

tttcaaaaaa aaaaaaaaa 3859

<210> 50
<211> 7209
<212> DNA
<213> Homo sapiens

<400> 50
gacgtcatca cgcagggccg agtcggcgcg gccacatcct ttaaatatgg tcttttttgg 60

gcgcgcgcga caatgtgagg agtggggtgg agcgtgtgtg gtgtgtggct gcggcctggg 120

caagagccgc cgcggaccat gagctgagta agttctggag ggatcctgcc tcttggagcc 180

ttcgcagcca ggcagctgtg aactgtgagc tagagtgaag cagaaatcta ggaagatgag 240

ctccaagatg gtcataagtg aaccaggact gaattgggat atttccccca aaaatggcct 300

taagacattt ttctctcgag aaaattataa agatcattcc atggctccaa gtttaaaaga 360

actacgtgtt ttatccaaca gacgtatagg agaaaatttg aatgcctcag caagttctgt 420

agaaaatgag ccggcagtta gttcagcaac tcaagcaaag gaaaaagtta aaaccacaat 480

tggaatggtt cttcttccaa aaccaagagt tccttatcct cgtttctctc gtttctcaca 540

gagagagcag aggagttatg tggacttgtt ggttaaatac gcaaagattc ctgcaaattc 600

caaagctgtt ggaataaata aaaatgacta cttgcagtac ttggatatga aaaaacatgt 660

gaacgaagaa gttactgagt tcctaaagtt tttgcagaat tctgcaaaga aatgtgcgca 720

ggattataat atgctttctg atgatgcccg tctcttcaca gagaaaattt taagagcttg 780

cattgaacaa gtgaaaaagt attcagaatt ctatactctc cacgaggtca ccagcttaat 840

gggattcttc ccattcagag tagagatggg attaaagtta gaaaaaactc ttctcgcatt 900

gggcagtgta aaatatgtga aaacagtatt tccctcaatg cctataaagt tgcagctgtc 960

aaaggacgat atagctacca ttgaaacgtc agaacaaaca gctgaagcta tgcattatga 1020

tattagtaaa gatccaaatg cagagaagct tgtttccaga tatcaccctc agatagctct 1080

aactagtcag tcattattta ccttattaaa taatcatgga ccaacgtaca aggaacagtg 1140

ggaaattcca gtgtgtattc aagtaatacc tgttgcaggt tcaaaaccag ttaaagtaat 1200

atatattaat tcaccacttc cccaaaagaa aatgactatg agagagagaa atcaaatctt 1260

tcatgaagtt ccattaaaat ttatgatgtc caaaaacaca tctgttccag tctctgcagt 1320

ctttatggac aaacctgaag agtttatatc tgaaatggac atgtcctgtg aagtcaacga 1380

gtgccgaaaa attgagagtc ttgaaaactt gtatttggat tttgatgatg atgtcacaga 1440

acttgaaact tttggagtaa ccaccaccaa agtatcaaaa tcaccaagtc cagcaagtac 1500

ttccacagta cctaacatga cagatgctcc tacagccccc aaagcaggaa ctacaactgt 1560

ggcaccaagt gcaccagaca tttctgctaa ttctagaagt ttatctcaga ttctgatgga 1620

acaattgcaa aaggagaaac agctggtcac tggtatggat ggtggccctg aggaatgcaa 1680

aaataaagat gatcagggat ttgaatcatg tgaaaaggta tcaaattctg acaagccttt 1740

gatacaagat agtgacttga aaacatctga tgccttacag ttagaaaatt ctcaggaaat 1800

tgaaacttct aataaaaatg atatgactat agatatatta catgctgatg gtgaaagacc 1860

taatgttcta gaaaacctag acaactcaaa ggaaaagact gttggatcag aagcagcaaa 1920

aactgaagat acagttctct gcagcagtga tacagatgag gagtgtttaa tcattgatac 1980

agaatgtaaa aataatagtg atggaaagac agctgttgtg ggttctaact taagttccag 2040

accagctagt ccaaattctt cctcaggaca ggcttctgta ggaaaccaga ctaatactgc 2100

ttgtagtcct gaagagtcat gtgttttaaa aaaacctatc aaacgagtat ataaaaaatt 2160

tgatccagtt ggagagattt taaaaatgca ggatgagctc ttaaagccaa tttccagaaa 2220

agtaccagaa ttgcccttaa tgaatttaga aaattctaaa cagccttctg tttctgagca 2280

attgtctggt ccttcagact cctctagttg gccgaaatct ggatggcctt ctgcatttca 2340

gaagccaaaa ggacgattgc catatgaact tcaggactat gttgaagata catcggaata 2400

cctagctcct caggaaggaa attttgttta taagttattt agcctgcaag acctgttgtt 2460

actcgtacgc tgcagtgtcc agaggataga gacaagacca cgttctaaaa aacggaagaa 2520

aatcagaaga caatttccag tttatgtact accaaaagta gagtatcaag cttgttatgg 2580

agttgaagct ctgactgaaa gtgaactttg tcgcttatgg actgaaagtt tattgcattc 2640

caacagctca ttttatgttg ggcatatcga tgcatttact tcaaaacttt ttctactgga 2700

agaaattacc tcagaagaat taaaagaaaa gctttcagca ctcaagattt ccaatttatt 2760

taacatcctc caacacattc taaagaaact aagtagcttg caggagggtt cctacttgtt 2820

atctcatgca gcagaagatt cttcactcct gatttataag gcctctgatg gaaaagttac 2880

taggacagca tacaatttgt ataaaacaca ttgcggcctt cctggtgtac cttccagtct 2940

ctcagttccc tgggtcccat tagatcccag cctgttatta ccatatcata tccatcatgg 3000

aagaatacct tgtacttttc caccgaaatc actggatacc acaacacaac aaaagattgg 3060

tggaacgaga atgcctacac gcagccacag gaatccagtt tccatggaaa ccaaaagcag 3120

ttgcttgcct gctcagcaag ttgaaactga aggagtggct ccacataaaa gaaaaataac 3180

ttgaggactg taccatggaa aactaaattt aaaaaaacag ttataacagt gtttaattta 3240

gataagtttg agggaaaata atcagtaggc aagaggaaca tttttcctgt agtagctaga 3300

gtgccttgaa aaaatgtgtt ggctatgtga aggaatattt caactaaaat ggaatggtat 3360

gcttttcacc cttgaagttt gaggaggatc ttgatatgtt ttaacattat catggcaggg 3420

aaatatataa agaagaaaaa tatttttaca ttaaaccttt tctaaaaatt gtaaatagaa 3480

aaataatttg gttttttatc aagaacaaca cttatcgtta tgtattgtgt tagttatatt 3540

gccagtctgt tgcgactgac tcaaaaagtt aaatgttgcc actgctgaag atgattatga 3600

gcatcgcaaa ctttgtttct gacccatttt gacagttttt atatactcct ttaaaatgat 3660

gaatgttaca ggttaataaa gttaatacct ttaaaaactt ggtgaaattc cattacagaa 3720

gccaaaaata aaaactccct gcctctgaaa agtcagatta ctgacttctt gtttggcaac 3780

catcagtttg tttaataaaa gaaaaaattt ggtggtataa catgtttgat gacagatgcc 3840

tctatctcta gattcaagct gagtgttgaa atacactgct gaaagcaaag agataggtat 3900

gttttccaga aaaaaagtca gtgtcattgc tccagatgac aaggttaatg tggtaaagca 3960

taagcttttt tttttttttg agatggagtc tcgctctgtt gcccaggctg gagtgcagtg 4020

gcatgatctc agctcaccac aacatccacc tcctggatta aagcgattct cctgcctcat 4080

cctcccgagt agctgggact acaggcacct accaccaggc ttggctaatt tttttttgta 4140

tttttagtag agacagggtg ttaccatgtt ggccgggctg gtctcaaact cctgaccttg 4200

tgatccgcct gccttggcct cccaaagtgc tgggattaca ggggtaagcc accgtgcctg 4260

gccagcataa gctattttta tcctcatcta tactgattga tttaaatttt ccgggcctgc 4320

agatgaattt ggaaaagggc ataatcaact gatgttgctg tagcaaattt ggacttaaga 4380

aactacagac agggccgggc gtggtggctc acacctgtaa ttccagcact tcgcaaggcc 4440

gaggcgggag aatcacttga gtccaggggt tggagaccag tctgggcaac acagacctca 4500

tctctacaaa taaaaaagtt agccgggtgt ggtggggtgt gcctgtagtc ccagctactc 4560

aaaaggctga ggcagaagaa ttgcttgagc ccaagaagtc aaggctacag tgagttgtga 4620

tcgcaccaca gaactcagct tgagtatcag agtgtgaccc tgtctcaaag aaaaaaaaaa 4680

acaggacagg cttgtttaat ctttagaacc cttacactaa aattttcaga aattatgtat 4740

gtcatttgta aaatataagt tttgtaatcc tagataattg cttcacattg aaatttttta 4800

gctattctgt gtaaagaata gctattcttt tggcctaatt tctgagaaat tagaccgaaa 4860

aatctcctca agtattgtaa cagttgaaaa tacttatatt caactaccaa gttttgtttc 4920

attttgattt tttgttataa aacattttta agttatttga gaagtaatta ttagtaaaat 4980

tgtatcttga taaatcattt cctcatcctg ccattccctt ttataaaaca ctgagttttt 5040

ttattcctta agtcacatat cccagaaatg ttttggttgt gaatcatttg ccagcgagcc 5100

aagggagagg cagggattcc cttgaaatgt actcttacac tttttatttt tatttttaat 5160

gtacatttgt gtgcaccaga atggaatgtg ctctttttaa agagctggac agtggttgct 5220

tcagacttct gaataccaga aagacttgcc ttttaatttt taaatattac tttttacatc 5280

ataaaatgtg ttatcttatc catgtatttg aataacatca tatataaagg tagttttttg 5340

cttgtgctta gtgaagcctc cttttttaac atacatttga tatgctttat gttctttttc 5400

ttgattttta gtactgagaa tttataagcc aagaagtaac tgacaatatg tttagcagga 5460

tgtaatccca gctacttggg aagctgaggt gggaggatcg cttgagtcta ggagtttgag 5520

gctgcagtga gctatgatgg tgccactgca ctccatcctg ggtgagagat caagacccta 5580

tctcaaaaaa tgagcaagtt tttttttttt ttttaaatca gacttagggc tagcaaggat 5640

gtgatcaccc tggcccattt atacctcagc agtgaaggtg taatttatac acacctcttt 5700

gaaaacagtt taattcagta agtgagtgca actatgtgcc atgcactcca aagtaggtcc 5760

ccacagttat tctctcatag ccttgttttt tcagatctca ccatatttat ttgtttcttt 5820

gattttcttt tcttatagat tacaagctct gcaaggacag gggcatattt gttttgttca 5880

ccattaagta ataaacacct aatacagtgc ctgtcatgta gttgcttagc agatattagt 5940

tagatgcatg aatgtgtagg tatcaaagat tttttttttc aggagatcac attctagtgg 6000

aagacactat actgctttat gtgataagtg ctttgataaa gtaagcaagt catctaacca 6060

gatcttggta gttaaggttt tctgagatag aacttgagct gagtctttta aaaatagtag 6120

gatttataag ggaaaattaa aggtcttcca caaagaaact aagtgaaact aagcagccta 6180

tgaaaaggtg aaagttggag agattatagg ctatgaacat gggagatgag agagaaaact 6240

gcaggatagg gacagaaaca ctgtgctaag acatttgaac tttgtcctgt gcaatgtggg 6300

aaccagtagt gaaggttaat taactgacat gatcagatat gtatttgaaa agggctcttg 6360

ttggcattag tctagagaat gcgatggagc tgggaagcga aagaagaaat gttgggagcc 6420

tgtgttgtag taactgaaaa atggcaatgg cccagaatta aagcagcaag aataggagtg 6480

ggctcaagtg atgtataagg aaaaaaaaaa aaatgacaac tcagttttgt tgttggtaag 6540

gagagtaaag tatggcttct gggtttctca tttgagatac tgagtaaatg atggtgttaa 6600

cccattgaga aaatagtgtg gttagagtgg gaagaaagtt aatttaaaat gttcattttt 6660

ttttttagtc atttctactt ttggattcta ggtttgtgtg ctatattggt tataaccaat 6720

ttgctgaata ataaattatc ccaaaactta gctacttaaa acaacaaaca ttatctcaca 6780

gtttctgtaa gtcaaaagtc caggtctctc atgaagttgc agtcaggctc ttggctaggg 6840

ctgcagtcat ctgaaggatt agtaagtcgc taagcccaca cttaagctcc acccttgaag 6900

ggagaagttt ggaaagaatt tgtgaacata tttttaaatc acatatgcta tgaacaggtt 6960

taggggaggg agaggaaaga aaactttaac ttttcattag catactgcat ctgtagttcc 7020

taatgggcat gttgtaaaga tgttaagacg ttttctatgc attataggta tatagtagta 7080

ttttgttttg ttctgttttt ttgtttggta tgtagtagtt tttaatcaaa ccttaacgta 7140

tttgatatta agatttattg agtttcttgt tgagatgtag aaataaaggg gatccatttc 7200

atttatcca 7209

<210> 51
<211> 7049
<212> DNA
<213> Homo sapiens

<400> 51
   gacgtcatca cgcagggccg agtcggcgcg gccacatcct ttaaatatgg tctttttggg 60
   gcgcgcgcga caatgtgagg agtggggtgg agcgtgtgtg gtgtgtggct gcggcctggg 120
   caagagccgc cgcggaccat gagctgagta agttctggag ggatcctgcc tcttggagcc 180
   ttcgcagcca ggcagctgtg aactgtgagc tagagtgaag cagaaatcta ggaagatgag 240
   ctccaagatg gtcataagtg aaccaggact gaattgggat atttccccca aaaatggcct 300
   taagacattt ttctctcgag aaaattataa agatcattcc atggctccaa gtttaaaaga 360
   actacgtgtt ttatccaaca gagagagcag aggagttatg tggacttgtt ggttaaatac 420
   gcaaagattc ctgcaaattc caaagctgtt ggaataaata aaaatgacta cttgcagtac 480
   ttggatatga aaaaacatgt gaacgaagaa gttactgagt tcctaaagtt tttgcagaat 540
   tctgcaaaga aatgtgcgca ggattataat atgctttctg atgatgcccg tctcttcaca 600
   gagaaaattt taagagcttg cattgaacaa gtgaaaaagt attcagaatt ctatactctc 660
   cacgaggtca ccagcttaat gggattcttc ccattcagag tagagatggg attaaagtta 720
   gaaaaaactc ttctcgcatt gggcagtgta aaatatgtga aaacagtatt tccctcaatg 780
   cctataaagt tgcagctgtc aaaggacgat atagctacca ttgaaacgtc agaacaaaca 840
   gctgaagcta tgcattatga tattagtaaa gatccaaatg cagagaagct tgtttccaga 900
   tatcaccctc agatagctct aactagtcag tcattattta ccttattaaa taatcatgga 960
   ccaacgtaca aggaacagtg ggaaattcca gtgtgtattc aagtaatacc tgttgcaggt 1020
   tcaaaaccag ttaaagtaat atatattaat tcaccacttc cccaaaagaa aatgactatg 1080
   agagagagaa atcaaatctt tcatgaagtt ccattaaaat ttatgatgtc caaaaacaca 1140
   tctgttccag tctctgcagt ctttatggac aaacctgaag agtttatatc tgaaatggac 1200
   atgtcctgtg aagtcaacga gtgccgaaaa attgagagtc ttgaaaactt gtatttggat 1260
   tttgatgatg atgtcacaga acttgaaact tttggagtaa ccaccaccaa agtatcaaaa 1320
   tcaccaagtc cagcaagtac ttccacagta cctaacatga cagatgctcc tacagccccc 1380
   aaagcaggaa ctacaactgt ggcaccaagt gcaccagaca tttctgctaa ttctagaagt 1440
   ttatctcaga ttctgatgga acaattgcaa aaggagaaac agctggtcac tggtatggat 1500
   ggtggccctg aggaatgcaa aaataaagat gatcagggat ttgaatcatg tgaaaaggta 1560
   tcaaattctg acaagccttt gatacaagat agtgacttga aaacatctga tgccttacag 1620
   ttagaaaatt ctcaggaaat tgaaacttct aataaaaatg atatgactat agatatatta 1680
   catgctgatg gtgaaagacc taatgttcta gaaaacctag acaactcaaa ggaaaagact 1740
   gttggatcag aagcagcaaa aactgaagat acagttctct gcagcagtga tacagatgag 1800
   gagtgtttaa tcattgatac agaatgtaaa aataatagtg atggaaagac agctgttgtg 1860
   ggttctaact taagttccag accagctagt ccaaattctt cctcaggaca ggcttctgta 1920
   ggaaaccaga ctaatactgc ttgtagtcct gaagagtcat gtgttttaaa aaaacctatc 1980
   aaacgagtat ataaaaaatt tgatccagtt ggagagattt taaaaatgca ggatgagctc 2040
   ttaaagccaa tttccagaaa agtaccagaa ttgcccttaa tgaatttaga aaattctaaa 2100
   cagccttctg tttctgagca attgtctggt ccttcagact cctctagttg gccgaaatct 2160
   ggatggcctt ctgcatttca gaagccaaaa ggacgattgc catatgaact tcaggactat 2220
   gttgaagata catcggaata cctagctcct caggaaggaa attttgttta taagttattt 2280
   agcctgcaag acctgttgtt actcgtacgc tgcagtgtcc agaggataga gacaagacca 2340
   cgttctaaaa aacggaagaa aatcagaaga caatttccag tttatgtact accaaaagta 2400
   gagtatcaag cttgttatgg agttgaagct ctgactgaaa gtgaactttg tcgcttatgg 2460
   actgaaagtt tattgcattc caacagctca ttttatgttg ggcatatcga tgcatttact 2520
   tcaaaacttt ttctactgga agaaattacc tcagaagaat taaaagaaaa gctttcagca 2580
   ctcaagattt ccaatttatt taacatcctc caacacattc taaagaaact aagtagcttg 2640
   caggagggtt cctacttgtt atctcatgca gcagaagatt cttcactcct gatttataag 2700
   gcctctgatg gaaaagttac taggacagca tacaatttgt ataaaacaca ttgcggcctt 2760
   cctggtgtac cttccagtct ctcagttccc tgggtcccat tagatcccag cctgttatta 2820
   ccatatcata tccatcatgg aagaatacct tgtacttttc caccgaaatc actggatacc 2880
   acaacacaac aaaagattgg tggaacgaga atgcctacac gcagccacag gaatccagtt 2940
   tccatggaaa ccaaaagcag ttgcttgcct gctcagcaag ttgaaactga aggagtggct 3000
   ccacataaaa gaaaaataac ttgaggactg taccatggaa aactaaattt aaaaaaacag 3060
   ttataacagt gtttaattta gataagtttg agggaaaata atcagtaggc aagaggaaca 3120
   tttttcctgt agtagctaga gtgccttgaa aaaatgtgtt ggctatgtga aggaatattt 3180
   caactaaaat ggaatggtat gcttttcacc cttgaagttt gaggaggatc ttgatatgtt 3240
   ttaacattat catggcaggg aaatatataa agaagaaaaa tatttttaca ttaaaccttt 3300
   tctaaaaatt gtaaatagaa aaataatttg gttttttatc aagaacaaca cttatcgtta 3360
   tgtattgtgt tagttatatt gccagtctgt tgcgactgac tcaaaaagtt aaatgttgcc 3420
   actgctgaag atgattatga gcatcgcaaa ctttgtttct gacccatttt gacagttttt 3480
   atatactcct ttaaaatgat gaatgttaca ggttaataaa gttaatacct ttaaaaactt 3540
   ggtgaaattc cattacagaa gccaaaaata aaaactccct gcctctgaaa agtcagatta 3600
   ctgacttctt gtttggcaac catcagtttg tttaataaaa gaaaaaattt ggtggtataa 3660
   catgtttgat gacagatgcc tctatctcta gattcaagct gagtgttgaa atacactgct 3720
   gaaagcaaag agataggtat gttttccaga aaaaaagtca gtgtcattgc tccagatgac 3780
   aaggttaatg tggtaaagca taagcttttt tttttttttg agatggagtc tcgctctgtt 3840
   gcccaggctg gagtgcagtg gcatgatctc agctcaccac aacatccacc tcctggatta 3900
   aagcgattct cctgcctcat cctcccgagt agctgggact acaggcacct accaccaggc 3960
   ttggctaatt tttttttgta tttttagtag agacagggtg ttaccatgtt ggccgggctg 4020
   gtctcaaact cctgaccttg tgatccgcct gccttggcct cccaaagtgc tgggattaca 4080
   ggggtaagcc accgtgcctg gccagcataa gctattttta tcctcatcta tactgattga 4140
   tttaaatttt ccgggcctgc agatgaattt ggaaaagggc ataatcaact gatgttgctg 4200
   tagcaaattt ggacttaaga aactacagac agggccgggc gtggtggctc acacctgtaa 4260
   ttccagcact tcgcaaggcc gaggcgggag aatcacttga gtccaggggt tggagaccag 4320
   tctgggcaac acagacctca tctctacaaa taaaaaagtt agccgggtgt ggtggggtgt 4380
   gcctgtagtc ccagctactc aaaaggctga ggcagaagaa ttgcttgagc ccaagaagtc 4440
   aaggctacag tgagttgtga tcgcaccaca gaactcagct tgagtatcag agtgtgaccc 4500
   tgtctcaaag aaaaaaaaaa acaggacagg cttgtttaat ctttagaacc cttacactaa 4560
   aattttcaga aattatgtat gtcatttgta aaatataagt tttgtaatcc tagataattg 4620
   cttcacattg aaatttttta gctattctgt gtaaagaata gctattcttt tggcctaatt 4680
   tctgagaaat tagaccgaaa aatctcctca agtattgtaa cagttgaaaa tacttatatt 4740
   caactaccaa gttttgtttc attttgattt tttgttataa aacattttta agttatttga 4800
   gaagtaatta ttagtaaaat tgtatcttga taaatcattt cctcatcctg ccattccctt 4860
   ttataaaaca ctgagttttt ttattcctta agtcacatat cccagaaatg ttttggttgt 4920
   gaatcatttg ccagcgagcc aagggagagg cagggattcc cttgaaatgt actcttacac 4980
   ttttttattt tatttttaat gtacatttgt gtgcaccaga atggaatgtg ctctttttaa 5040
   agagctggac agtggttgct tcagacttct gaataccaga aagacttgcc ttttaatttt 5100
   taaatattac tttttacatc ataaaatgtg ttatcttatc catgtatttg aataacatca 5160
   tatataaagg tagttttttg cttgtgctta gtgaagcctc cttttttaac atacatttga 5220
   tatgctttat gttctttttc ttgattttta gtactgagaa tttataagcc aagaagtaac 5280
   tgacaatatg tttagcagga tgtaatccca gctacttggg aagctgaggt gggaggatcg 5340
   cttgagtcta ggagtttgag gctgcagtga gctatgatgg tgccactgca ctccatcctg 5400
   ggtgagagat caagacccta tctcaaaaaa tgagcaagtt tttttttttt ttttaaatca 5460
   gacttagggc tagcaaggat gtgatcaccc tggcccattt atacctcagc agtgaaggtg 5520
   taatttatac acacctcttt gaaaacagtt taattcagta agtgagtgca actatgtgcc 5580
   atgcactcca aagtaggtcc ccacagttat tctctcatag ccttgttttt tcagatctca 5640
   ccatatttat ttgtttcttt gattttcttt tcttatagat tacaagctct gcaaggacag 5700
   gggcatattt gttttgttca ccattaagta ataaacacct aatacagtgc ctgtcatgta 5760
   gttgcttagc agatattagt tagatgcatg aatgtgtagg tatcaaagat tttttttttc 5820
   aggagatcac attctagtgg aagacactat actgctttat gtgataagtg ctttgataaa 5880
   gtaagcaagt catctaacca gatcttggta gttaaggttt tctgagatag aacttgagct 5940
   gagtctttta aaaatagtag gatttataag ggaaaattaa aggtcttcca caaagaaact 6000
   aagtgaaact aagcagccta tgaaaaggtg aaagttggag agattatagg ctatgaacat 6060
   gggagatgag agagaaaact gcaggatagg gacagaaaca ctgtgctaag acatttgaac 6120
   tttgtcctgt gcaatgtggg aaccagtagt gaaggttaat taactgacat gatcagatat 6180
   gtatttgaaa agggctcttg ttggcattag tctagagaat gcgatggagc tgggaagcga 6240
   aagaagaaat gttgggagcc tgtgttgtag taactgaaaa atggcaatgg cccagaatta 6300
   aagcagcaag aataggagtg ggctcaagtg atgtataagg aaaaaaaaaa aaatgacaac 6360
   tcagttttgt tgttggtaag gagagtaaag tatggcttct gggtttctca tttgagatac 6420
   tgagtaaatg atggtgttaa cccattgaga aaatagtgtg gttagagtgg gaagaaagtt 6480
   aatttaaaat gttcattttt ttttttagtc atttctactt ttggattcta ggtttgtgtg 6540
   ctatattggt tataaccaat ttgctgaata ataaattatc ccaaaactta gctacttaaa 6600
   acaacaaaca ttatctcaca gtttctgtaa gtcaaaagtc caggtctctc atgaagttgc 6660
   agtcaggctc ttggctaggg ctgcagtcat ctgaaggatt agtaagtcgc taagcccaca 6720
   cttaagctcc acccttgaag ggagaagttt ggaaagaatt tgtgaacata tttttaaatc 6780
   acatatgcta tgaacaggtt taggggaggg agaggaaaga aaactttaac ttttcattag 6840
   catactgcat ctgtagttcc taatgggcat gttgtaaaga tgttaagacg ttttctatgc 6900
   attataggta tatagtagta ttttgttttg ttctgttttt ttgtttggta tgtagtagtt 6960
   tttaatcaaa ccttaacgta tttgatatta agatttattg agtttcttgt tgagatgtag 7020
   aaataaaggg gatccatttc atttatcca 7049

<210> 52
<211> 1013
<212> DNA
<213> Homo sapiens

<400> 52
acgctggtgg gcctgttgtg gagtacgctt tggactgaga agcatcgagg ctataggacg 60

cagctgttgc catgacggcc caggggggcc tggtggctaa ccgaggccgg cgcttcaagt 120

gggccattga gctaagcggg cctggaggag gcagcagggg tcgaagtgac cggggcagtg 180

gccagggaga ctcgctctac ccagtcggtt acttggacaa gcaagtgcct gataccagcg 240

tgcaagagac agaccggatc ctggtggaga agcgctgctg ggacatcgcc ttgggtcccc 300

tcaaacagat tcccatgaat ctcttcatca tgtacatggc aggcaatact atctccatct 360

tccctactat gatggtgtgt atgatggcct ggcgacccat tcaggcactt atggccattt 420

cagccacttt caagatgtta gaaagttcaa gccagaagtt tcttcagggt ttggtctatc 480

tcattgggaa cctgatgggt ttggcattgg ctgtttacaa gtgccagtcc atgggactgt 540

tacctacaca tgcatcggat tggttagcct tcattgagcc ccctgagaga atggagttca 600

gtggtggagg actgcttttg tgaacatgag aaagcagcgc ctggtcccta tgtatttggg 660

tcttatttac atccttcttt aagcccagtg gctcctcagc atactcttaa actaatcact 720

tatgttaaaa agaaccaaaa gactcttttc tccatggtgg ggtgacaggt cctagaagga 780

caatgtgcat attacgacaa acacaaagaa actataccat aacccaaggc tgaaaataat 840

gtagaaaact ttatttttgt ttccagtaca gagcaaaaca acaacaaaaa aacataacta 900

tgtaaacaag agaataactg ctgctaaatc aagaactgtt gcagcatctc ctttcaataa 960

attaaatggt tgagaacaat gcataaaaaa aaaaaaaaaa aaaaaaaaaa aaa 1013

<210> 53
<211> 2622
<212> DNA
<213> Homo sapiens

<400> 53

ggtgcggggc ggaagtgggc ggctgcggga cgcgcgcgga gtcgcgcggc gggcgggacc 60

tggccgagct ggagggcgcc ggggagcggg gctcgggcgg tccccgaggc ccggcggagc 120

gggcttctgg ggtgtctgcg gcggcgccgg gggaacgggc tggggatggg gcgcctagcc 180

gggcggtggc cggggcctcg gccatgttcg cggggctgca ggacctgggc gtggccaacg 240

gcgaggacct gaaggagacc ctgaccaact gcacggagcc gctcaaggcc atcgagcagt 300

tccagacaga gaatggtgtg ctgctgccat ctcttcagtc agccctcccc ttcttggacc 360

tgcacgggac gccgcggctg gagttccacc agtcggtatt cgatgagctg cgggacaagc 420

tgctggagcg agtgtcagcc atcgcttcgg aggggaaggc tgaggaaagg tacaagaagc 480

tggaagacct tctggagaag agcttttctc tggtgaagat gccgtccctg cagcccgtgg 540

tgatgtgcgt catgaagcac ctgcccaagg ttccggagaa aaaactgaag ctggttatgg 600

ctgacaagga gctgtatcga gcctgcgccg tggaggtgaa gcggcagatc tggcaagaca 660

accaggccct cttcggggac gaggtttccc cactcctgaa gcagtacatc ctggagaagg 720

agagcgctct cttcagtaca gagctctctg tcctgcacaa ctttttcagt ccttccccca 780

agaccaggcg ccagggcgag gtggtgcagc ggctgacgcg gatggtgggg aagaacgtga 840

agctgtacga catggtgctg cagtttctgc gcacgctctt cctgcgcacg cggaatgtgc 900

actactgcac gctgcgggct gagctgctca tgtccctgca cgacctggac gtgggtgaaa 960

tctgcaccgt ggacccgtgc cacaagttca cctggtgcct ggacgcctgc atccgagagc 1020

ggttcgtgga cagcaagagg gcgcgggagc tgcaggggtt tctcgatggc gtcaagaagg 1080

gccaggagca ggtgctgggg gacctgtcca tgatcctgtg tgaccccttc gccatcaaca 1140

cgctggcact gagcacagtc aggcacctgc aggagctggt cggccaggag acactgccca 1200

gggacagccc cgacctcctg ctgctgctcc ggctgctggc gctgggccag ggagcctggg 1260

acatgatcga cagccaggtc ttcaaggagc ccaagatgga ggtagagctc atcaccaggt 1320

tcctcccgat gctcatgtcc ttcctggtgg atgactacac tttcaatgtg gatcagaaac 1380

ttccggctga ggagaaagcc ccagtctcat atccaaacac acttcccgaa agcttcacta 1440

agtttctgca ggagcagcgc atggcctgcg aggtggggct gtactacgtc ctgcacatca 1500

ccaagcagag gaacaagaac gcgctcctcc gcctgctgcc cgggctggtg gagacctttg 1560

gcgacttggc ctttggcgac atcttcctcc acctgctcac gggcaacctt gcgctgctgg 1620

ccgacgaatt tgcccttgag gacttctgca gcagcctctt cgatggcttc ttcctcaccg 1680

cctctccaag gaaggagaac gtgcaccggc acgcgctgcg gctcctcatt cacctgcacc 1740

ccagggtggc cccgtctaag ctggaggcgt tgcagaaggc cctggagcct acaggccaga 1800

gcggagaggc agtgaaggag ctttactccc agctcggcga gaagctggaa cagctggatc 1860

accggaagcc cagcccggca caggctgcgg agacgccggc cctggagctg cccctcccca 1920

gcgtgcccgc ccctgccccg ctctgagggc cctccagacc tgctcgggtg ctggggccat 1980

gccgagtcgc ggccctgctc agccggaaga ggctcccgga cctggatgta cagggcagtc 2040

tctcttcccg gggctatggc tgggcctgtc ctgccgtcat ggccccctgc ttcctgctcc 2100

ttggagctgg ctcccggacc ttgcccacca tccatgcagt ggctcccagg gcagagcctc 2160

tccttgtact ttggcagcca tagaaagcgt gctcattttc tgttttcctg tgttaggaaa 2220

aaaccacctg ttttccaagg ggagagggcg gggcctgagg gtgggggcgg ggcctcttca 2280

ttggcccagc ttggcgaaag cgaggcacac tgcttactgc cttggggttg tggagatgga 2340

cccgtgacct cgtggaggcc gtgtgggggc agcagcctgg cctgtgccat ggtgggtgtc 2400

ctggggcctg tgcggaggga gccacctcac cctgcagccc agtttgcagg tgtggccttg 2460

tttctccttg cccagcagtg ctgccttcag cggccgtgac ggggccagct ggacacacgg 2520

tgagattttc tcgtatgtaa ataaaaggca ttttggtaaa cgtggaaaaa aaaaaaaaaa 2580

aaaaaaaaaa aaaaaaaaaa aaaaaaaaaa aaaaaaaaaa aa 2622

<210> 54
<211> 2577
<212> DNA
<213> Homo sapiens

<400> 54
tctgcttagt catggtgacc tgcgcgcgct ccgcgcctcc cccacgcgca gcgatggagg 60

cgccggggct cgggcggtgg aggcggagcc ggagcgcggc catggcgggg tccctgagtg 120

ccagaggtgg tggtgttgct tatcttctgg aaccccatgc agccagatcc caggcctagc 180

ggggctgggg cctgctgccg attcctgccc ctgcagtcac agtgccctga gggggcaggg 240

gacgcggtga tgtacgcctc cactgagtgc aaggcggagg tgacgccctc ccagcatggc 300

aaccgcacct tcagctacac cctggaggat cataccaagc aggcctttgg catcatgaac 360

gagctgcggc tcagccagca gctgtgtgac gtcacactgc aggtcaagta ccaggatgca 420

ccggccgccc agttcatggc ccacaaggtg gtgctggcct catccagccc tgtcttcaag 480

gccatgttca ccaacgggct gcgggagcag ggcatggagg tggtgtccat tgagggtatc 540

caccccaagg tcatggagcg cctcattgaa ttcgcctaca cggcctccat ctccatgggc 600

gagaagtgtg tcctccacgt catgaacggt gctgtcatgt accagatcga cagcgttgtc 660

cgtgcctgca gtgacttcct ggtgcagcag ctggacccca gcaatgccat cggcatcgcc 720

aacttcgctg agcagattgg ctgtgtggag ttgcaccagc gtgcccggga gtacatctac 780

atgcattttg gggaggtggc caagcaagag gagttcttca acctgtccca ctgccaactg 840

gtgaccctca tcagccggga cgacctgaac gtgcgctgcg agtccgaggt cttccacgcc 900

tgcatcaact gggtcaagta cgactgcgaa cagcgacggt tctacgtcca ggcgctgctg 960

cgggccgtgc gctgccactc gttgacgccg aacttcctgc agatgcagct gcagaagtgc 1020

gagatcctgc agtccgactc ccgctgcaag gactacctgg tcaagatctt cgaggagctc 1080

accctgcaca agcccacgca ggtgatgccc tgccgggcgc ccaaggtggg ccgcctgatc 1140

tacaccgcgg gcggctactt ccgacagtcg ctcagctacc tggaggctta caaccccagt 1200

gacggcacct ggctccggtt ggcggacctg caggtgccgc ggagcggcct ggccggctgc 1260

gtggtgggcg ggctgttgta cgccgtgggc ggcaggaaca actcgcccga cggcaacacc 1320

gactccagcg ccctggactg ttacaacccc atgaccaatc agtggtcgcc ctgcgccccc 1380

atgagcgtgc cccgtaaccg catcggggtg ggggtcatcg atggccacat ctatgccgtc 1440

ggcggctccc acggctgcat ccaccacaac agtgtggaga ggtatgagcc agagcgggat 1500

gagtggcact tggtggcccc aatgctgaca cgaaggatcg gggtgggcgt ggctgtcctc 1560

aatcgtctcc tttatgccgt ggggggcttt gacgggacaa accgccttaa ttcagctgag 1620

tgttactacc cagagaggaa cgagtggcga atgatcacag caatgaacac catccgaagc 1680

ggggcaggcg tctgcgtcct gcacaactgt atctatgctg ctgggggcta tgatggtcag 1740

gaccagctga acagcgtgga gcgctacgat gtggaaacag agacgtggac tttcgtagcc 1800

cccatgaagc accggcgaag tgccctgggg atcactgtcc accaggggag aatctacgtc 1860

cttggaggct atgatggtca cacgttcctg gacagtgtgg agtgttacga cccagataca 1920

gacacctgga gcgaggtgac ccgaatgaca tcgggccgga gtggggtggg cgtggctgtc 1980

accatggagc cctgccggaa gcagattgac cagcagaact gtacctgttg aggcactttt 2040

gtttcttggg caaaaataca gtccaatggg gagtatcatt gtttttgtac aaaaaccggg 2100

actaaaagaa aagacagcac tgcaaataac ccatcttccg ggaagggagg ccaggatgcc 2160

tcagtgttaa aatgacatct caaaagaagt ccaaagcggg aatcatgtgc ccctcagcgg 2220

agccccggga gtgtccaaga cagcctggct gggaaagggg gtgtggaaag agcaggcttc 2280

caggagagag gcccccaaac cctctggccg ggtaataggc ctgggtccca ctcacccatg 2340

ccggcagctg tcaccatgtg atttattctt ggatacctgg gagggggcca atgggggcct 2400

cagggggagg ccccctctgg aaatgtggtt cccagggatg ggcctgtaca tagaagccac 2460

cggatggcac ttccccaccg gatggacagt tattttgttg ataagtaacc ctgtaatttt 2520

ccaaggaaaa taaagaacag actaactagt gtctttcacc ctgaaaaaaa aaaaaaa 2577

<210> 55
<211> 2606
<212> DNA
<213> Homo sapiens

<400> 55
   ctttccgccc tctccccgcc tccttttcgg gcgtcccgag gccgctcccc aaccgacaac 60
   caagaccccg caggccacgc agccctggag ccgaggcccc ccgacggcgg aggcgcccgc 120
   gggtccccta cagccaaggt ccctgagtgc cagaggtggt ggtgttgctt atcttctgga 180
   accccatgca gccagatccc aggcctagcg gggctggggc ctgctgccga ttcctgcccc 240
   tgcagtcaca gtgccctgag gggcaggggg acgcggtgat gtacgcctcc actgagtgca 300
   aggcggaggt gacgccctcc cagcatggca accgcacctt cagctacacc ctggaggatc 360
   ataccaagca ggcctttggc atcatgaacg agctgcggct cagccagcag ctgtgtgacg 420
   tcacactgca ggtcaagtac caggatgcac cggccgccca gttcatggcc cacaaggtgg 480
   tgctggcctc atccagccct gtcttcaagg ccatgttcac caacgggctg cgggagcagg 540
   gcatggaggt ggtgtccatt gagggtatcc accccaaggt catggagcgc ctcattgaat 600
   tcgcctacac ggcctccatc tccatgggcg agaagtgtgt cctccacgtc atgaacggtg 660
   ctgtcatgta ccagatcgac agcgttgtcc gtgcctgcag tgacttcctg gtgcagcagc 720
   tggaccccag caatgccatc ggcatcgcca acttcgctga gcagattggc tgtgtggagt 780
   tgcaccagcg tgcccgggag tacatctaca tgcattttgg ggaggtggcc aagcaagagg 840
   agttcttcaa cctgtcccac tgccaactgg tgaccctcat cagccgggac gacctgaacg 900
   tgcgctgcga gtccgaggtc ttccacgcct gcatcaactg ggtcaagtac gactgcgaac 960
   agcgacggtt ctacgtccag gcgctgctgc gggccgtgcg ctgccactcg ttgacgccga 1020
   acttcctgca gatgcagctg cagaagtgcg agatcctgca gtccgactcc cgctgcaagg 1080
   actacctggt caagatcttc gaggagctca ccctgcacaa gcccacgcag gtgatgccct 1140
   gccgggcgcc caaggtgggc cgcctgatct acaccgcggg cggctacttc cgacagtcgc 1200
   tcagctacct ggaggcttac aaccccagtg acggcacctg gctccggttg gcggacctgc 1260
   aggtgccgcg gagcggcctg gccggctgcg tggtgggcgg gctgttgtac gccgtgggcg 1320
   gcaggaacaa ctcgcccgac ggcaacaccg actccagcgc cctggactgt tacaacccca 1380
   tgaccaatca gtggtcgccc tgcgccccca tgagcgtgcc ccgtaaccgc atcggggtgg 1440
   gggtcatcga tggccacatc tatgccgtcg gcggctccca cggctgcatc caccacaaca 1500
   gtgtggagag gtatgagcca gagcgggatg agtggcactt ggtggcccca atgctgacac 1560
   gaaggatcgg ggtgggcgtg gctgtcctca atcgtctcct ttatgccgtg gggggctttg 1620
   acgggacaaa ccgccttaat tcagctgagt gttactaccc agagaggaac gagtggcgaa 1680
   tgatcacagc aatgaacacc atccgaagcg gggcaggcgt ctgcgtcctg cacaactgta 1740
   tctatgctgc tgggggctat gatggtcagg accagctgaa cagcgtggag cgctacgatg 1800
   tggaaacaga gacgtggact ttcgtagccc ccatgaagca ccggcgaagt gccctgggga 1860
   tcactgtcca ccaggggaga atctacgtcc ttggaggcta tgatggtcac acgttcctgg 1920
   acagtgtgga gtgttacgac ccagatacag acacctggag cgaggtgacc cgaatgacat 1980
   cgggccggag tggggtgggc gtggctgtca ccatggagcc ctgccggaag cagattgacc 2040
   agcagaactg tacctgttga ggcacttttg tttcttgggc aaaaatacag tccaatgggg 2100
   agtatcattg tttttgtaca aaaaccggga ctaaaagaaa agacagcact gcaaataacc 2160
   catcttccgg gaagggaggc caggatgcct cagtgttaaa atgacatctc aaaagaagtc 2220
   caaagcggga atcatgtgcc cctcagcgga gccccgggag tgtccaagac agcctggctg 2280
   ggaaaggggg tgtggaaaga gcaggcttcc aggagagagg cccccaaacc ctctggccgg 2340
   gtaataggcc tgggtcccac tcacccatgc cggcagctgt caccatgtga tttattcttg 2400
   gatacctggg agggggccaa tgggggcctc agggggaggc cccctctgga aatgtggttc 2460
   ccagggatgg gcctgtacat agaagccacc ggatggcact tccccaccgg atggacagtt 2520
   attttgttga taagtaaccc tgtaattttc caaggaaaat aaagaacaga ctaactagtg 2580
   tctttcaccc tgaaaaaaaa aaaaaa 2606

<210> 56
<211> 2277
<212> DNA
<213> Homo sapiens

<400> 56
gggaaatggc ggccgcgggc ttggtcgctg tggcagcggc tgccgagtac tctggcacgg 60

tagcgtcggg aggtaacctc cctggtgttc actgcggccc aagctccggg gcaggccctg 120

gttttggccc gggctcgtgg agccgctctc tcgatcgagc cctggaggag gcggcggtca 180

ctggggtgct gagcctgagc ggccggaaac tgagggagtt tccccgggga gcggccaacc 240

acgacctgac ggacaccacc cgggcggacc tgtcgcgaaa tcgcctttca gaaattccta 300

tagaagcatg tcactttgtt tctctggaaa atctcaactt gtaccaaaat tgtattcgtt 360

atattccaga ggcaatttta aacctacaag ctctaacatt cttaaatatt agtcggaacc 420

aactgtcaac attgccggta cacttgtgta atttgccatt gaaagtctta attgctagta 480

ataacaaatt ggtgtcactt ccagaagaaa ttggacacct tagacatttg atggaacttg 540

atgtgagctg caatgaaatt caaactatac cttcccaaat tggtaacctg gaggccttga 600

gagaccttaa tgtaagaaga aatcacctag tacatttgcc tgaagagctg gcggagttgc 660

ctttgatacg gttagacttc tcatgcaata aaattaccac aatccctgtt tgttatcgga 720

acctcaggca cctacagacg atcaccctag ataacaatcc actacaatca cctcctgcac 780

agatatgtat aaaaggcaaa gtccacatat ttaaatacct gaacatacaa gcttgtaaga 840

ttgctccaga tctgccggat tatgatagga gaccgttggg ttttggctcc tgccatgaag 900

aactgtactc aagtcgccct tatggagccc ttgattcagg cttcaatagt gtggacagtg 960

gtgataagag atggtcaggg aatgaaccta cagatgaatt ttcagatctg cctcttcgag 1020

tagcagagat tactaaagaa caaagactac gaagagaaag ccagtaccaa gagaaccgcg 1080

gcagtttggt agtaacaaac ggcggagtgg aacatgatct ggatcagatt gactacatag 1140

acagctgcac cgcagaggaa gaggaggccg aggtgagaca gcccaaggga ccagacccag 1200

acagccttag ttcacagttt atggcgtata ttgaacagcg gcgaatctct catgagggtt 1260

caccagtaaa gccagtagcc attagggagt ttcaaaaaac agaagatatg agaagatact 1320

tacatcaaaa cagggttcca gctgagccat cttccctcct gtcactatca gcaagtcaca 1380

atcagctgtc acacacagac ctggaacttc atcagagaag ggagcagtta gtagagcgca 1440

ctcggagaga ggctcagctt gctgccctgc agtatgagga ggagaaaata aggaccaagc 1500

agatccagag agatgctgtc ctggactttg tcaaacaaaa agcatcacaa agtccacaaa 1560

aacagcaccc gctcctagat ggcgtagatg gtgagtgccc cttcccatcc agaaggtctc 1620

agcacactga tgatagtgcc ttgtgcatgt cgctgtcagg gttgaatcaa gtgggctgtg 1680

ctgctaccct gcctcattct tctgccttca cgcctcttaa gagtgatgac agacctaatg 1740

ctctattaag ttcacctgca acagaaacag ttcatcattc ccctgcatat tcttttcctg 1800

ctgctatcca gagaaatcag cctcagcgcc ctgaaagctt ccttttccga gcaggtgtca 1860

gggcagaaac caacaaaggt catgcttcac cccttcctcc atctgctgca cctaccactg 1920

attctacaga ttccataaca ggacagaatt caagacagag agaagaagag ctggaattaa 1980

tagaccaact gcgtaaacat attgagtacc ggttgaaagt gtctctacct tgtgatctcg 2040

gagcagctct aactgacggt gttgttcttt gccatttggc caatcatgtg cgacctcgat 2100

ctgtcccaag cattcatgtt ccctcaccag ctgtagtaag ttgataatcc taaaaagcct 2160

tggctattca tcagacattt gttaagcaca gtggacagac atttcttaag cattgtgcta 2220

gatattaagg atacagatat aaagagtcca gtctctacaa aaaaaaaaaa aaaaaaa 2277

<210> 57
<211> 4431
<212> DNA
<213> Homo sapiens

<400> 57
   caagagcagg cctggagacc cgggagtgcc ttggggagag ggtccccgcc ctgccgggag 60
   cccggccctc ccggtgccgg ggtggacact gttagtgtag tcattgtccc tatggagaaa 120
   ctgaggcatg gagaggctga agtcacacaa gcccgccacg atgactatca tggtggagga 180
   catcatgaag ctgctgtgct ccctttctgg ggagaggaag atgaaggcgg ctgtcaagca 240
   ctctgggaag ggtgccctgg tcacaggggc catggccttc gtcgggggtt tggtgggcgg 300
   cccaccggga ctcgccgttg ggggggctgt cggggggctg ttaggtgcct ggatgacaag 360
   tggacagttt aagccggttc ctcagatcct aatggagctg ccccctgccg agcaacagag 420
   gctctttaac gaagccgcag ccatcatcag gcacctggag tggacggacg ccgtgcagct 480
   gaccgcgctg gtcatgggca gcgaggccct gcagcagcag ctgctggcca tgctggtgaa 540
   ctacgtcacc aaggagctgc gggccgagat ccagtatgat gactaggccg cacctccggg 600
   gaggtggggg gcccctttaa atgactctgt gattctgaag aggtggcttg ggagttggga 660
   gaagcccagc ggatgccccc tggggaatct ccacatcatc agtgtattac tagtaatgtc 720
   ccgctggaga ggccaccgct gtgcagtgtc atgttccaga aattactgat gaagcagcat 780
   gtgttggtgg catgtgcact ggcctgccat gacagccctc tgactggccc cccagtgaag 840
   agtaaaggcc tgcctgccgc aggcttcgga ggcgtctgct gagtcctctc acccgcatgg 900
   gtctggggaa gtgatcacgc tcagccgacg gtctgaccac acttcatcct ccccccgggg 960
   ccttctcatc ttgggagatg actcctcttc agagcacctg ctgcaggact ggatcccacc 1020
   cccctgcagg tcctggggtc tcagggcctt ggagcagccc atgctggaat catgtttacc 1080
   tcctagtgca accgtcccct acccagggac tgtcgaatgg ccccacggag gggacgggcg 1140
   gcctgctgag tgaagccaca aataccgagt ggacttgacc ccggccccca ctaggctgca 1200
   cacctagact cgccctgcca gggcctcgct cttcccatct gaaaagtcct ggtagttctt 1260
   gaggtttact tctcaaatga aatattttta gtaaaaagta caggtatatc tcggagatat 1320
   tgtgggttca gttccagacc acctcggtaa agccaacatc acaataaagc aaggaagcgc 1380
   attgttttag tttcccagtg catctaagtc atgtttactg catattgcag tccactaaat 1440
   gtgcaatagc attatgtcta acaaatgtac aaaccttaat ttaaaaatat ttactgttca 1500
   aaatgctgac acagaaacgc aaagtgagca catgctgttg gaaaatggtg ccaaatagac 1560
   ttgcctgatg ccaggctgct acaaaccttc aatttaaaaa aaaaacacag tattcacaaa 1620
   gcatagtaga atgaggtatg cctgtattgc tctttctgaa gtggtgtgat ataaaccatc 1680
   tctaagaaat gtttctaccc taaagatttc cccagtacag tcagctctcc gtaactgtgg 1740
   tctccacatt tagatccaac cagccttgga taggaaatat ttgaaaaaag aaattgcatt 1800
   ggtactgaac acgtacagac ctttttttct tgccattatt ccctaaacaa tatggtgtag 1860
   catatttaca tagcatttat attgtatttg gtattataag aaatctagag atgatttaaa 1920
   ttatacagga aggtgtgcgt aggttacgtg caaacgctat gccattgccc atcagggact 1980
   tgagcatcct cagatgtcgg tgtctgaggg ttgaggttgc agtcctggaa cccatccccc 2040
   atggatactg aggcatagct gtactgtgtg ttttcacttt gctttcagaa ctacgacttg 2100
   aatgtgatcg attacaataa atgtttttct aaaaagccat tttcccacat attatgtgta 2160
   aaatatctct aatattcaaa gtcttacaaa tggaacaaga gggaaggtac cagatttgtg 2220
   gctttaaaga atgcaagagt aggtcaggtg aggtggctca tgcttgtaat gtcggcactt 2280
   ttggaggcca aagtgggagg atcgcttgag gccaagagtt tgaaactagc ctgggcaata 2340
   tagtgagacc tccatctcta caaaaaatgt tattccttgg tccccaggtc ctggggcagg 2400
   gcagggactt ggggctctcc tctcaggccc tggaggctcg gagggaggca cttctgctga 2460
   ggccctgctt ataggaaagc tcagtatcct ggcacctcca tgtgtgtaag gacgtcaggg 2520
   tgaattataa cccatggtga ttaaggaagg gccgagcctt tctcttgcag agcgattgct 2580
   cttctgctgg cctcgtctag cacaaatcag gtgccctagc acaggccaca ctcctggagt 2640
   taatgaagcc actttaatca tttatatgcc acttaagttg atgggggaca gggcaggggt 2700
   gggaaggagg ggttgtctgt ctgtgaaaac tgagtgtggc ttttcttgtt gaactgatca 2760
   ttcctgctct tcctgcaaat aagtcctgca tacggaccct ggaactaaaa atggaaaatc 2820
   agagcatgcc ccctcccaat tttgtatagc tttagtgggc tctaaagttg cccgttttta 2880
   gtgtgaagga aaaaacgttg atttgcagat atcgtgagaa tgaaacctca acaaagatgt 2940
   ttggttcagt gcttcaaagt tgggggacac tttttccatg ttgaacaaat gccaacttct 3000
   ccggttgctt acagcaaatc cttctggaac aatcggggct gaaattgagt tgcctttgtt 3060
   aggcgattgg gccccattca ttcttactcg tgcatcaggt cctggtctgt gtcaggccca 3120
   ggggacacag gtggtcccag ctcagaggcc cagtgtccac tgcagcccct cccacagcct 3180
   gcccacccct actgcaggga aaaatgccca gggaggagat ggtccaactc ctgatcagtt 3240
   ttgtgtccga tggagcaggc cttgctgagt gaagacactg gaactagctg ggtcctgggg 3300
   tgacttggag gctttgggcc taaaagggca gcctgaacct ggagtcttat ctcccccagg 3360
   agccgaaagc acttttcttg atttccccca ggaaatcaag cgctgcttct cagctcctgt 3420
   ggttttagta tttatatatc tgtatcttct ttgtagaaat ttatttattt ttgaataagt 3480
   aatacctgcc tggtacaaaa tttaaaaggt acgggagggc gcaagctgca agggaaggcc 3540
   tgctcctatg ccgaccccag aggcagccac tgttaccaat ttcatgtgta ttcctttaac 3600
   tctgttttaa agtaagtctc tgaaaactgt tcatttcctt ttgtcagtat ttgttgctga 3660
   aaacctagaa aaacccagaa aatataatga aataaaaact acaaatttca caacccagaa 3720
   aactgctggt aacagatttg gtgtctttcc tgcaattgtt ttaatgctat gtaggtacac 3780
   atatattttt ttcttttttt cttttttttt tttttgagat ggagtctcgc actgtcgccc 3840
   gggctggagt gcagtagcgc gatcttggtt cactgcaagc tctgcctcct gggttcaagc 3900
   cattctcctg cctcagcctc ccgagtagct ttgactacag tcgcccgcca ccatgcccgg 3960
   ctaattttta tttttttatt ttttttttat ttttagtaga gacggggttt caccatgtta 4020
   gccaggatgg tctcaatctc cttacctcgt gatctgccca cctcagcctc ccaaagtgct 4080
   gggattacag gcgtgagcca ctgcgcccgg cctatgtatg tatatttcta acgtacttgc 4140
   cttcttatcc tatatgcccc tttgaacacc tgtatcaatg aatgttaaat aatatttttg 4200
   atgtaattta aaattccttg taaatatttg taatggctgc ataatattcc actgtgccta 4260
   accatgtgcc tggtgtgaat tccatttgtg gatttttact actgtaatgc tgaagtatta 4320
   cagtaatact gtgttaatat tctctttatt gaattctggt atatagtctt tgggattcat 4380
   tcctcaaagt gaaattgctg gatttaaaag taaaaaaaaa aaaaaaaaaa a 4431

<210> 58
<211> 4702
<212> DNA
<213> Homo sapiens

<400> 58
   gtgatcatgc gtgcgcgtgg gagaaaggca gggctgggcc tgcgggagcg cggccttgcg 60
   gttcccagga ctcttctccg ggcgcctcgt ctccttacgc cacccgcaac ccaagccagg 120
   gccccggtga cagcggcggg gtgggccagg accggggagg gggtgcccag cagcgacccc 180
   cggctccccc taaggccggg cgcagctcgg agccaggagc tggcccggcg cgtggcttcc 240
   cggaaggccc ggcgcagccg gaaggtggga cggagggcgg ggccagcgcc ggggccgcca 300
   ccaaggcctg cgcgaccctc cgcggggctg gggagctggg cggggagccc ggggcctgcc 360
   aggcccgggc tgcagccgcg tctgatcgcc gagcgcgccg cgtagacctc cgctccccca 420
   ggcccgccac gatgactatc atggtggagg acatcatgaa gctgctgtgc tccctttctg 480
   gggagaggaa gatgaaggcg gctgtcaagc actctgggaa gggtgccctg gtcacagggg 540
   ccatggcctt cgtcgggggt ttggtgggcg gcccaccggg actcgccgtt gggggggctg 600
   tcggggggct gttaggtgcc tggatgacaa gtggacagtt taagccggtt cctcagatcc 660
   taatggagct gccccctgcc gagcaacaga ggctctttaa cgaagccgca gccatcatca 720
   ggcacctgga gtggacggac gccgtgcagc tgaccgcgct ggtcatgggc agcgaggccc 780
   tgcagcagca gctgctggcc atgctggtga actacgtcac caaggagctg cgggccgaga 840
   tccagtatga tgactaggcc gcacctccgg ggaggtgggg ggccccttta aatgactctg 900
   tgattctgaa gaggtggctt gggagttggg agaagcccag cggatgcccc ctggggaatc 960
   tccacatcat cagtgtatta ctagtaatgt cccgctggag aggccaccgc tgtgcagtgt 1020
   catgttccag aaattactga tgaagcagca tgtgttggtg gcatgtgcac tggcctgcca 1080
   tgacagccct ctgactggcc ccccagtgaa gagtaaaggc ctgcctgccg caggcttcgg 1140
   aggcgtctgc tgagtcctct cacccgcatg ggtctgggga agtgatcacg ctcagccgac 1200
   ggtctgacca cacttcatcc tccccccggg gccttctcat cttgggagat gactcctctt 1260
   cagagcacct gctgcaggac tggatcccac ccccctgcag gtcctggggt ctcagggcct 1320
   tggagcagcc catgctggaa tcatgtttac ctcctagtgc aaccgtcccc tacccaggga 1380
   ctgtcgaatg gccccacgga ggggacgggc ggcctgctga gtgaagccac aaataccgag 1440
   tggacttgac cccggccccc actaggctgc acacctagac tcgccctgcc agggcctcgc 1500
   tcttcccatc tgaaaagtcc tggtagttct tgaggtttac ttctcaaatg aaatattttt 1560
   agtaaaaagt acaggtatat ctcggagata ttgtgggttc agttccagac cacctcggta 1620
   aagccaacat cacaataaag caaggaagcg cattgtttta gtttcccagt gcatctaagt 1680
   catgtttact gcatattgca gtccactaaa tgtgcaatag cattatgtct aacaaatgta 1740
   caaaccttaa tttaaaaata tttactgttc aaaatgctga cacagaaacg caaagtgagc 1800
   acatgctgtt ggaaaatggt gccaaataga cttgcctgat gccaggctgc tacaaacctt 1860
   caatttaaaa aaaaaacaca gtattcacaa agcatagtag aatgaggtat gcctgtattg 1920
   ctctttctga agtggtgtga tataaaccat ctctaagaaa tgtttctacc ctaaagattt 1980
   ccccagtaca gtcagctctc cgtaactgtg gtctccacat ttagatccaa ccagccttgg 2040
   ataggaaata tttgaaaaaa gaaattgcat tggtactgaa cacgtacaga cctttttttc 2100
   ttgccattat tccctaaaca atatggtgta gcatatttac atagcattta tattgtattt 2160
   ggtattataa gaaatctaga gatgatttaa attatacagg aaggtgtgcg taggttacgt 2220
   gcaaacgcta tgccattgcc catcagggac ttgagcatcc tcagatgtcg gtgtctgagg 2280
   gttgaggttg cagtcctgga acccatcccc catggatact gaggcatagc tgtactgtgt 2340
   gttttcactt tgctttcaga actacgactt gaatgtgatc gattacaata aatgtttttc 2400
   taaaaagcca ttttcccaca tattatgtgt aaaatatctc taatattcaa agtcttacaa 2460
   atggaacaag agggaaggta ccagatttgt ggctttaaag aatgcaagag taggtcaggt 2520
   gaggtggctc atgcttgtaa tgtcggcact tttggaggcc aaagtgggag gatcgcttga 2580
   ggccaagagt ttgaaactag cctgggcaat atagtgagac ctccatctct acaaaaaatg 2640
   ttattccttg gtccccaggt cctggggcag ggcagggact tggggctctc ctctcaggcc 2700
   ctggaggctc ggagggaggc acttctgctg aggccctgct tataggaaag ctcagtatcc 2760
   tggcacctcc atgtgtgtaa ggacgtcagg gtgaattata acccatggtg attaaggaag 2820
   ggccgagcct ttctcttgca gagcgattgc tcttctgctg gcctcgtcta gcacaaatca 2880
   ggtgccctag cacaggccac actcctggag ttaatgaagc cactttaatc atttatatgc 2940
   cacttaagtt gatgggggac agggcagggg tgggaaggag gggttgtctg tctgtgaaaa 3000
   ctgagtgtgg cttttcttgt tgaactgatc attcctgctc ttcctgcaaa taagtcctgc 3060
   atacggaccc tggaactaaa aatggaaaat cagagcatgc cccctcccaa ttttgtatag 3120
   ctttagtggg ctctaaagtt gcccgttttt agtgtgaagg aaaaaacgtt gatttgcaga 3180
   tatcgtgaga atgaaacctc aacaaagatg tttggttcag tgcttcaaag ttgggggaca 3240
   ctttttccat gttgaacaaa tgccaacttc tccggttgct tacagcaaat ccttctggaa 3300
   caatcggggc tgaaattgag ttgcctttgt taggcgattg ggccccattc attcttactc 3360
   gtgcatcagg tcctggtctg tgtcaggccc aggggacaca ggtggtccca gctcagaggc 3420
   ccagtgtcca ctgcagcccc tcccacagcc tgcccacccc tactgcaggg aaaaatgccc 3480
   agggaggaga tggtccaact cctgatcagt tttgtgtccg atggagcagg ccttgctgag 3540
   tgaagacact ggaactagct gggtcctggg gtgacttgga ggctttgggc ctaaaagggc 3600
   agcctgaacc tggagtctta tctcccccag gagccgaaag cacttttctt gatttccccc 3660
   aggaaatcaa gcgctgcttc tcagctcctg tggttttagt atttatatat ctgtatcttc 3720
   tttgtagaaa tttatttatt tttgaataag taatacctgc ctggtacaaa atttaaaagg 3780
   tacgggaggg cgcaagctgc aagggaaggc ctgctcctat gccgacccca gaggcagcca 3840
   ctgttaccaa tttcatgtgt attcctttaa ctctgtttta aagtaagtct ctgaaaactg 3900
   ttcatttcct tttgtcagta tttgttgctg aaaacctaga aaaacccaga aaatataatg 3960
   aaataaaaac tacaaatttc acaacccaga aaactgctgg taacagattt ggtgtctttc 4020
   ctgcaattgt tttaatgcta tgtaggtaca catatatttt tttctttttt tctttttttt 4080
   ttttttgaga tggagtctcg cactgtcgcc cgggctggag tgcagtagcg cgatcttggt 4140
   tcactgcaag ctctgcctcc tgggttcaag ccattctcct gcctcagcct cccgagtagc 4200
   tttgactaca gtcgcccgcc accatgcccg gctaattttt atttttttat ttttttttta 4260
   tttttagtag agacggggtt tcaccatgtt agccaggatg gtctcaatct ccttacctcg 4320
   tgatctgccc acctcagcct cccaaagtgc tgggattaca ggcgtgagcc actgcgcccg 4380
   gcctatgtat gtatatttct aacgtacttg ccttcttatc ctatatgccc ctttgaacac 4440
   ctgtatcaat gaatgttaaa taatattttt gatgtaattt aaaattcctt gtaaatattt 4500
   gtaatggctg cataatattc cactgtgcct aaccatgtgc ctggtgtgaa ttccatttgt 4560
   ggatttttac tactgtaatg ctgaagtatt acagtaatac tgtgttaata ttctctttat 4620
   tgaattctgg tatatagtct ttgggattca ttcctcaaag tgaaattgct ggatttaaaa 4680
   gtaaaaaaaa aaaaaaaaaa aa 4702

<210> 59
<211> 1537
<212> DNA
<213> Homo sapiens

<400> 59
cggacccgga agtgcttggc cacagtcgca gccccggcgc cccgaagcgg gaaaaaggct 60

gggtgccgcc gtcccccagc tgcgcaaccc taggaactct cgggttcaca gtctctcttt 120

ttctaggaac ttggctgtgt tgtcctgcct cagagacaaa ttcatctatt gtaggcctag 180

cccctgcctt tgaaaacaag gaaaggttgg tagaacatca acacagcatg gaatttccag 240

ggaggtctca tttcaaaact tcataaagaa caagaaccac ctggacttct gtgagggcga 300

tgattaaact ggcctgagtt tgaatgaaag gataatgtat gctcaacctg tgactaacac 360

caaggaggtc aagtggcaga aggtcttgta tgagcgacag ccctttcctg ataactatgt 420

ggaccggcga ttcctggaag agctccggaa aaacatccat gctcggaaat accaatattg 480

ggctgtggta tttgagtcca gtgtggtgat ccagcagctg tgcagtgttt gtgtttttgt 540

ggttatctgg tggtatatgg atgaggtctt tctggccccc cattggcttt tagggactgg 600

tctggcttct tcactgattg ggtatgtttt gtttgatctc attgatggag gtgaagggcg 660

gaagaagagt gggcagaccc ggtgggctga cctgaagagt gccctagtct tcattacttt 720

cacttatggg ttttcaccag tgctgaagac ccttacagag tctgtcagca ctgacaccat 780

ctatgccatg tcagtcttca tgctgttagg ccatctcatc ttttttgact atggtgccaa 840

tgctgccatt gtatccagca cactatcctt gaacatggcc atctttgctt ctgtatgctt 900

ggcatcacgt cttccccggt ccctgcatgc cttcatcatg gtgacatttg ccattcagat 960

ttttgccctg tggcccatgt tgcagaagaa actaaaggca tgtactcccc ggagctatgt 1020

gggggtcaca ctgctttttg cattttcagc cgtgggaggc ctactgtcca ttagtgctgt 1080

gggagccgta ctctttgccc ttctgctgat gtctatctca tgtctgtgtc cattctacct 1140

cattcgcttg cagcttttta aagaaaacat tcatgggcct tgggatgaag ctgaaatcaa 1200

ggaagacttg tccaggttcc tcagttaaat taggacatcc attacattat taaagcaagc 1260

tgatagatta gcctcctaac tagtatagaa cttaaagaca gagttccatt ctggaagcag 1320

catgtcattg tggtaagaga atagagatca aaaccaaaaa aaatgaacca aaggcttggg 1380

tggtgagggt gcttatcctt tctgttattt tgtagatgaa aaaactttct ggggacctct 1440

tgaattacat gctgtaacat atgaagtgat gtggtttcta ttaaaaaaat aacacatcca 1500

aaaaaaaaaa aaaaaaaaaa aaaaaaaaaa aaaaaaa 1537

<210> 60
<211> 1514
<212> DNA
<213> Homo sapiens

<400> 60
cggacccgga agtgcttggc cacagtcgca gccccggcgc cccgaagcgg gaaaaaggct 60

gggtgccgcc gtcccccagc tgcgcaaccc taggaactct cgggaacttg gctgtgttgt 120

cctgcctcag agacaaattc atctattgta ggcctagccc ctgcctttga aaacaaggaa 180

aggttggtag aacatcaaca cagcatggaa tttccaggga ggtctcattt caaaacttca 240

taaagaacaa gaaccacctg gacttctgtg agggcgatga ttaaactggc ctgagtttga 300

atgaaaggat aatgtatgct caacctgtga ctaacaccaa ggaggtcaag tggcagaagg 360

tcttgtatga gcgacagccc tttcctgata actatgtgga ccggcgattc ctggaagagc 420

tccggaaaaa catccatgct cggaaatacc aatattgggc tgtggtattt gagtccagtg 480

tggtgatcca gcagctgtgc agtgtttgtg tttttgtggt tatctggtgg tatatggatg 540

agggtcttct ggccccccat tggcttttag ggactggtct ggcttcttca ctgattgggt 600

atgttttgtt tgatctcatt gatggaggtg aagggcggaa gaagagtggg cagacccggt 660

gggctgacct gaagagtgcc ctagtcttca ttactttcac ttatgggttt tcaccagtgc 720

tgaagaccct tacagagtct gtcagcactg acaccatcta tgccatgtca gtcttcatgc 780

tgttaggcca tctcatcttt tttgactatg gtgccaatgc tgccattgta tccagcacac 840

tatccttgaa catggccatc tttgcttctg tatgcttggc atcacgtctt ccccggtccc 900

tgcatgcctt catcatggtg acatttgcca ttcagatttt tgccctgtgg cccatgttgc 960

agaagaaact aaaggcatgt actccccgga gctatgtggg ggtcacactg ctttttgcat 1020

tttcagccgt gggaggccta ctgtccatta gtgctgtggg agccgtactc tttgcccttc 1080

tgctgatgtc tatctcatgt ctgtgtccat tctacctcat tcgcttgcag ctttttaaag 1140

aaaacattca tgggccttgg gatgaagctg aaatcaagga agacttgtcc aggttcctca 1200

gttaaattag gacatccatt acattattaa agcaagctga tagattagcc tcctaactag 1260

tatagaactt aaagacagag ttccattctg gaagcagcat gtcattgtgg taagagaata 1320

gagatcaaaa ccaaaaaaaa tgaaccaaag gcttgggtgg tgagggtgct tatcctttct 1380

gttattttgt agatgaaaaa actttctggg gacctcttga attacatgct gtaacatatg 1440

aagtgatgtg gtttctatta aaaaaataac acatccaaaa aaaaaaaaaa aaaaaaaaaa 1500

aaaaaaaaaa aaaa 1514

<210> 61
<211> 2485
<212> DNA
<213> Homo sapiens

<400> 61
tgccccgttg tgaggtgata aagtgttgcg ctccgggacg ccagcgccgc ggctgccgcc 60

tctgctgggg tctaggctgt ttctctcgcg ccaccactgg ccgccggccg cagctccagg 120

tgtcctagcc gcccagcctc gacgccgtcc cgggacccct gtgctctgcg cgaagccctg 180

gccccggggg ccggggcatg ggccaggggc gcggggtgaa gcggcttccc gcggggccgt 240

gactgggcgg gcttcagcca tgaagaccct catagccgcc tactccgggg tcctgcgcgg 300

cgagcgtcag gccgaggctg accggagcca gcgctctcac ggaggacctg cgctgtcgcg 360

cgaggggtct gggagatggg gcactggatc cagcatcctc tccgccctcc aggacctctt 420

ctctgtcacc tggctcaata ggtccaaggt ggaaaagcag ctacaggtca tctcagtgct 480

ccagtgggtc ctgtccttcc ttgtactggg agtggcctgc agtgccatcc tcatgtacat 540
attctgcact gattgctggc tcatcgctgt gctctacttc acttggctgg tgtttgactg 600
gaacacaccc aagaaaggtg gcaggaggtc acagtgggtc cgaaactggg ctgtgtggcg 660
ctactttcga gactactttc ccatccagct ggtgaagaca cacaacctgc tgaccaccag 720
gaactatatc tttggatacc acccccatgg tatcatgggc ctgggtgcct tctgcaactt 780
cagcacagag gccacagaag tgagcaagaa gttcccaggc atacggcctt acctggctac 840
actggcaggc aacttccgaa tgcctgtgtt gagggagtac ctgatgtctg gaggtatctg 900
ccctgtcagc cgggacacca tagactattt gctttcaaag aatgggagtg gcaatgctat 960
catcatcgtg gtcgggggtg cggctgagtc tctgagctcc atgcctggca agaatgcagt 1020
caccctgcgg aaccgcaagg gctttgtgaa actggccctg cgtcatggag ctgacctggt 1080
tcccatctac tcctttggag agaatgaagt gtacaagcag gtgatcttcg aggagggctc 1140
ctggggccga tgggtccaga agaagttcca gaaatacatt ggtttcgccc catgcatctt 1200
ccatggtcga ggcctcttct cctccgacac ctgggggctg gtgccctact ccaagcccat 1260
caccactgtt gtgggagagc ccatcaccat ccccaagctg gagcacccaa cccagcaaga 1320
catcgacctg taccacacca tgtacatgga ggccctggtg aagctcttcg acaagcacaa 1380
gaccaagttc ggcctcccgg agactgaggt cctggaggtg aactgagcca gccttcgggg 1440
ccaattccct ggaggaacca gctgcaaatc acttttttgc tctgtaaatt tggaagtgtc 1500
atgggtgtct gtgggttatt taaaagaaat tataacaatt ttgctaaacc attacaatgt 1560
taggtctttt ttaagaagga aaaagtcagt atttcaagtt ctttcacttc cagcttgccc 1620
tgttctaggt ggtggctaaa tctgggccta atctgggtgg ctcagctaac ctctcttctt 1680
cccttcctga agtgacaaag gaaactcagt cttcttgggg aagaaggatt gccattagtg 1740
acttggacca gttagatgat tcactttttg cccctaggga tgagaggcga aagccacttc 1800
tcatacaagc ccctttattg ccactacccc acgctcgtct agtcctgaaa ctgcaggacc 1860
agtttctctg ccaaggggag gagttggaga gcacagttgc cccgttgtgt gagggcagta 1920
gtaggcatct ggaatgctcc agtttgatct cccttctgcc acccctacct cacccctagt 1980
cactcatatc ggagcctgga ctggcctcca ggatgaggat gggggtggca atgacaccct 2040
gcaggggaaa ggactgcccc ccatgcacca ttgcagggag gatgccgcca ccatgagcta 2100
ggtggagtaa ctggtttttc ttgggtggct gatgacatgg atgcagcaca gactcagcct 2160
tggcctggag cacatgctta ctggtggcct cagtttacct tccccagatc ctagattctg 2220
gatgtgagga agagatccct cttcagaagg ggcctggcct tctgagcagc agattagttc 2280
caaagcaggt ggcccccgaa cccaagcctc acttttctgt gccttcctga gggggttggg 2340

ccggggagga aacccaaccc tctcctgtgt gttctgttat ctcttgatga gatcattgca 2400

ccatgtcaga cttttgtata tgccttgaaa ataaatgaaa gtgagaatcc tcaaaaaaaa 2460

aaaaaaaaaa aaaaaaaaaa aaaaa 2485

<210> 62
<211> 2499
<212> DNA
<213> Homo sapiens

<400> 62
taccacaaat ttactggctt aaaacgacgc aagtctgtag gtcagaagtc tgacacgggt 60

cttaactggt gacccgagtc agatttggga cacaaagaac agaaaccaag ctgtgcaggt 120

ttctgacagg cagtccggtt agggagccct acagcaaccc gccggtcctc tctctcaggc 180

agttgctgcc atggctcatt attccaaccg gttctcctca gcccagtcta tctcagtggc 240

tccattcata gggtgatgtg cccggcggga cactaaccct aaccaagcag agagacggtc 300

atgcccgtca cgacctcggc cctcgccccg gccgaggctt ctcctgcagg tcgcgagaat 360

caggtgcgtc agcggcgtcc gggaacgccg gaagagccag tggagcggct ctgtagtcca 420

aagtaccccg tcgaccccag cacggccgct ccaccgcctc ctactagacc cagtcctagg 480

gactgcgcag tcgcagagct ccgtccgagt accggaagcc taggccgcca gcacttccgg 540

gaagtgactt cgtctccgaa gccgattggt tgttgctttg ctcccgctcg cgtcggtggc 600

gtttttcctg cagcgcgtgc gtgctgcgct actgagcagc gccatggagg actctgaagc 660

actgggcttc gaacacatgg gcctcgatcc ccggctcctt caggctgtca ccgatctggg 720

ctggtcgcga cctacgctga tccaggagaa ggccatccca ctggccctag aagggaagga 780

cctcctggct cgggcccgca cgggctccgg gaagacggcc gcttatgcta ttccgatgct 840

gcagctgttg ctccatagga aggcgacagg tccggtggta gaacaggcag tgagaggcct 900

tgttcttgtt cctaccaagg agctggcacg gcaagcacag tccatgattc agcagctggc 960

tacctactgt gctcgggatg tccgagtggc caatgtctca gctgctgaag actcagtctc 1020

tcagagagct gtgctgatgg agaagccaga tgtggtagta gggaccccat ctcgcatatt 1080

aagccacttg cagcaagaca gcctgaaact tcgtgactcc ctggagcttt tggtggtgga 1140

cgaagctgac cttctttttt cctttggctt tgaagaagag ctcaagagtc tcctctgtca 1200

cttgccccgg atttaccagg cttttctcat gtcagctact tttaacgagg acgtacaagc 1260

actcaaggag ctgatattac ataacccggt tacccttaag ttacaggagt cccagctgcc 1320

tgggccagac cagttacagc agtttcaggt ggtctgtgag actgaggaag acaaattcct 1380

cctgctgtat gccctgctca agctgtcatt gattcggggc aagtctctgc tctttgtcaa 1440

cactctagaa cggagttacc ggctacgcct gttcttggaa cagttcagca tccccacctg 1500

tgtgctcaat ggagagcttc cactgcgctc caggtgccac atcatctcac agttcaacca 1560

aggcttctac gactgtgtca tagcaactga tgctgaagtc ctgggggccc cagtcaaggg 1620

caagcgtcgg ggccgagggc ccaaagggga caaggcctct gatccggaag caggtgtggc 1680

ccggggcata gacttccacc atgtgtctgc tgtgctcaac tttgatcttc cccaaccccc 1740

tgaggcctac atccatcgag ctggcaggac agcacgcgct aacaacccag gcatagtctt 1800

aacctttgtg cttcccacgg agcagttcca cttaggcaag attgaggagc ttctcagtgg 1860

agagaacagg ggccccattc tgctccccta ccagttccgg atggaggaga tcgagggctt 1920

ccgctatcgc tgcagggatg ccatgcgctc agtgactaag caggccattc gggaggcaag 1980

attgaaggag atcaaggaag agcttctgca ttctgagaag cttaagacat actttgaaga 2040

caaccctagg gacctccagc tgctgcggca tgacctacct ttgcaccccg cagtggtgaa 2100

gccccacctg ggccatgttc ctgactacct ggttcctcct gctctccgtg gcctggtgcg 2160

ccctcacaag aagcggaaga agctgtcttc ctcttgtagg aaggccaaga gagcaaagtc 2220

ccagaaccca ctgcgcagct tcaagcacaa aggaaagaaa ttcagaccca cagccaagcc 2280

ctcctgaggt tgttgggcct ctctggagct gagcacattg tggagcacag gcttacaccc 2340

ttcgtggaca ggcgaggctc tggtgcttac tgcacagcct gaacagacag ttctggggcc 2400

ggcagtgctg ggccctttag ctccttggca cttccaagct ggcatcttgc cccttgacaa 2460

cagaataaaa attttagctg ccccaaaaaa aaaaaaaaa 2499

<210> 63
<211> 2865
<212> DNA
<213> Homo sapiens

<400> 63
gtcgccggaa gtggggcggg actctattgt ggcggtgagg aacaggaagc cctgaagggt 60

caaaagaaat acaaaagcaa aggctatttt cttttttttt ttctttcttt cattcattcc 120

ttcctctgtt tctttctttc ttcctttcat ttttttttct tttttaagag cgagcggctc 180

tgcggtggcg gtttggggtg ggcgccgccg aggtgaggtc gtctcgcctc ccgcgcgccg 240

gtagattggt tgtttcatta tggatggagg ggatgatggt aaccttatta tcaaaaagag 300

gtttgtgtct gaggcagaac tagatgaacg gcgcaaaagg aggcaagaag aatgggagaa 360

agttcgaaaa cctgaagatc cagaagaatg tccagaggag gtttatgacc ctcgatctct 420

atatgaaagg ctacaggaac agaaggacag gaagcagcag gagtacgagg aacagttcaa 480

attcaaaaac atggtaagag gcttagatga agatgagacc aacttccttg atgaggtttc 540

tcgacagcag gaactaatag aaaagcaacg aagagaagaa gaactgaaag aactgaagga 600

atacagaaat aacctcaaga aggttggaat ttctcaagag aacaagaagg aagtggaaaa 660

gaaactgact gtgaagccta tagaaaccaa gaacaagttc tcccaggcga agctgttggc 720
aggagctgtg aagcataaga gctcagagag tggcaacagt gtgaaaagac tgaaaccgga 780
ccctgagcca gatgacaaga atcaagagcc ctcatcctgc aagtctctcg gaaacacctc 840
cctgagtggc ccctccatcc actgcccctc tgctgcagta tgtatcggca tcctcccagg 900
cctgggtgcc tactctggga gcagcgactc cgagtccagc tcagacagcg aaggcaccat 960
caatgccacc ggaaagattg tctcctccat cttccgaacc aacaccttcc tcgaggcccc 1020
ctagtttctc cgtccctaca cagggagctc ctccccaagg gtagatcgga ccgttcatgc 1080
tgcctatagg cattatgtcc ctcaaaaaaa aactcctttg cctgcatcct gtgtacaaca 1140
tgacattttt aaccaatcca atctaaaaat gtgccagaat ccacctgtgg cccgaatcgt 1200
gtttggttcc tctttctact ccactgcaga tgaccaaacc tgtcccgctg ccactttcct 1260
cactgatatt gggaggaggg caaggcccag ccgaagttcc actaaaaatg ccccaggaga 1320
ataggcaccg gctggcttgc caaagggttt gggttttatt gctttctgtt ttttcttttc 1380
ccgacagcac aaagaagtaa gggcagttat tggacaggtg ttatttaaac attctattgt 1440
aaatgaatgt gttgtttggt tctactgcat tgtggagcat gcgggggaag agaactgacc 1500
caggtagtga aatggagccc ttccctggaa ctaaccagtc cttgatgttg tgtgactaag 1560
taaagatgat aaaccccatc tgctgggggt gtcacttcac actcggcatg cattgtgaaa 1620
gctttccata cccttggcca ttccctctct cctctctctc caaccccatt tatgcaggaa 1680
gggactgcta acaagaacgc ttccatctca aacctttctt ctgcctggga aattatttta 1740
tgtttgtttt tgaaataaag gatttagttt aagattctaa attttagaga aacaaacgta 1800
ggccttgttt actaatagcc agacatcaga actgcaggta ggtatgttaa tgagatgact 1860
tatttctggc agctcctgga atcctaatat tgtaaatgag tgggacacac ttgcatattg 1920
tgaccattct attgaggccc ttctctgttt aatgcatatt atacttgtgc ttttaactgt 1980
ggaatctatt tctaacctaa aggtgctgcc ctagtacttt tctttgctgc ctctgctgct 2040
ctttttcctt tccaaacagc aactctgagg ccatgagcag ccaaaaacta gaggtactgc 2100
tccacctcgt ctcataaagg gaaacgggct catcccttgg attctggagg agggagaggg 2160
agatggtgtg gaggcctcga ggacagagat agacatgagc tttgacaaca atctgtaggc 2220
tctcctgctt tagaataagc atgtaccatt ctttatccat tccccttatt cctacatcaa 2280
ttgtttttac tttcttgggt gtgagactga gtgagacaca cacaaaatgt gttgacactg 2340
tgatgccggc aggcagagca gctactgact ttgaacatgg gcagagaggc ccctggatct 2400
catccagccc actccttttc cccttccagt acagtgacac tctggtgccc attggcagat 2460
ggcgacttcc ctgcacccat aactgatgct ttgtgaattc ttcctccttt tcagaactac 2520
tctgtgctaa ttgttctgcc agtatgggcg catcagctcc atcctgacaa acaagacatt 2580

taggtaaaac tttgtaggca ccttctgctt ctctgcttca ttgttcctgt gatagtcctg 2640

ttgttattac agcatgtacc caaaacagcc tcacattgtt acaggaggca ggccaggaca 2700

tcaaagtcat catctttatg tggcatgact cttaagaggc cattactgta tctcatggcc 2760

tcttgatgtg gaaagaagtt gacagagggt tgcagggttt aaaaacatcc attaacatga 2820

aagctaataa acctgtcaga gaacaagaaa aaaaaaaaaa aaaaa 2865

<210> 64
<211> 4353
<212> DNA
<213> Homo sapiens

<400> 64
   ttctctcacg aagccccgcc cgcggagagg ttccatattg ggtaaaatct cggctctcgg 60
   agagtcccgg gagctgttct cgcgagagta ctgcgggagg ctcccgtttg ctggctcttg 120
   gaaccgcgac cactggagcc ttagcgggcg cagcagctgg aacgggagta ctgcgacgca 180
   gcccggagtc ggccttgtag gggcgaaggt gcagggagat cgcggcgggc gcagtcttga 240
   gcgccggagc gcgtccctgc ccttagcggg gcttgcccca gtcgcagggg cacatccagc 300
   cgctgcggct gacagcagcc gcgcgcgcgg gagtctgcgg ggtcgcggca gccgcacctg 360
   cgcgggcgac cagcgcaagg tccccgcccg gctgggcggg cagcaagggc cggggagagg 420
   gtgcgggtgc aggcgggggc ccccacaggc caccttcttg cccggcggct gccgctggaa 480
   aatgtctcag gagaggccca cgttctaccg gcaggagctg aacaagacaa tctgggaggt 540
   gcccgagcgt taccagaacc tgtctccagt gggctctggc gcctatggct ctgtgtgtgc 600
   tgcttttgac acaaaaacgg ggttacgtgt ggcagtgaag aagctctcca gaccatttca 660
   gtccatcatt catgcgaaaa gaacctacag agaactgcgg ttacttaaac atatgaaaca 720
   tgaaaatgtg attggtctgt tggacgtttt tacacctgca aggtctctgg aggaattcaa 780
   tgatgtgtat ctggtgaccc atctcatggg ggcagatctg aacaacattg tgaaatgtca 840
   gaagcttaca gatgaccatg ttcagttcct tatctaccaa attctccgag gtctaaagta 900
   tatacattca gctgacataa ttcacaggga cctaaaacct agtaatctag ctgtgaatga 960
   agactgtgag ctgaagattc tggattttgg actggctcgg cacacagatg atgaaatgac 1020
   aggctacgtg gccactaggt ggtacagggc tcctgagatc atgctgaact ggatgcatta 1080
   caaccagaca gttgatattt ggtcagtggg atgcataatg gccgagctgt tgactggaag 1140
   aacattgttt cctggtacag accatattaa ccagcttcag cagattatgc gtctgacagg 1200
   aacacccccc gcttatctca ttaacaggat gccaagccat gaggcaagaa actatattca 1260
   gtctttgact cagatgccga agatgaactt tgcgaatgta tttattggtg ccaatcccct 1320
   ggctgtcgac ttgctggaga agatgcttgt attggactca gataagagaa ttacagcggc 1380
   ccaagccctt gcacatgcct actttgctca gtaccacgat cctgatgatg aaccagtggc 1440
   cgatccttat gatcagtcct ttgaaagcag ggacctcctt atagatgagt ggaaaagcct 1500
   gacctatgat gaagtcatca gctttgtgcc accacccctt gaccaagaag agatggagtc 1560
   ctgagcacct ggtttctgtt ctgttgatcc cacttcactg tgaggggaag gccttttcac 1620
   gggaactctc caaatattat tcaagtgcct cttgttgcag agatttcctc catggtggaa 1680
   gggggtgtgc gtgcgtgtgc gtgcgtgtta gtgtgtgtgc atgtgtgtgt ctgtctttgt 1740
   gggagggtaa gacaatatga acaaactatg atcacagtga ctttacagga ggttgtggat 1800
   gctccagggc agcctccacc ttgctcttct ttctgagagt tggctcaggc agacaagagc 1860
   tgctgtcctt ttaggaatat gttcaatgca aagtaaaaaa atatgaattg tccccaatcc 1920
   cggtcatgct tttgccactt tggcttctcc tgtgacccca ccttgacggt ggggcgtaga 1980
   cttgacaaca tcccacagtg gcacggagag aaggcccata ccttctggtt gcttcagacc 2040
   tgacaccgtc cctcagtgat acgtacagcc aaaaaggacc aactggcttc tgtgcactag 2100
   cctgtgatta acttgcttag tatggttctc agatcttgac agtatatttg aaactgtaaa 2160
   tatgtttgtg ccttaaaagg agagaagaaa gtgtagatag ttaaaagact gcagctgctg 2220
   aagttctgag ccgggcaagt cgagagggct gttggacagc tgcttgtggg cccggagtaa 2280
   tcaggcagcc ttcataggcg gtcatgtgtg catgtgagca catgcgtata tgtgcgtctc 2340
   tctttctccc tcacccccag gtgttgccat ttctctgctt acccttcacc tttggtgcag 2400
   aggtttcttg aatatctgcc ccagtagtca gaagcaggtt cttgatgtca tgtacttcct 2460
   gtgtactctt tatttctagc agagtgagga tgtgttttgc acgtcttgct atttgagcat 2520
   gcacagctgc ttgtcctgct ctcttcagga ggccctggtg tcaggcaggt ttgccagtga 2580
   agacttcttg ggtagtttag atcccatgtc acctcagctg atattatggc aagtgatatc 2640
   acctctcttc agcccctagt gctattctgt gttgaacaca attgatactt caggtgcttt 2700
   tgatgtgaaa atcatgaaaa gaggaacagg tggatgtata gcatttttat tcatgccatc 2760
   tgttttcaac caactatttt tgaggaatta tcatgggaaa agaccagggc ttttcccagg 2820
   aatatcccaa acttcggaaa caagttattc tcttcactcc caataactaa tgctaagaaa 2880
   tgctgaaaat caaagtaaaa aattaaagcc cataaggcca gaaactcctt ttgctgtctt 2940
   tctctaaata tgattacttt aaaataaaaa agtaacaagg tgtcttttcc actcctatgg 3000
   aaaagggtct tcttggcagc ttaacattga cttcttggtt tggggagaaa taaattttgt 3060
   ttcagaattt tgtatattgt aggaatcctt tgagaatgtg attccttttg atggggagaa 3120
   agggcaaatt attttaatat tttgtatttt caactttata aagataaaat atcctcaggg 3180
   gtggagaagt gtcgttttca taacttgctg aatttcaggc attttgttct acatgaggac 3240
   tcatatattt aagccttttg tgtaataaga aagtataaag tcacttccag tgttggctgt 3300
   gtgacagaat cttgtatttg ggccaaggtg tttccatttc tcaatcagtg cagtgataca 3360
   tgtactccag agggacaggg tggaccccct gagtcaactg gagcaagaag gaaggaggca 3420
   gactgatggc gattccctct cacccgggac tctccccctt tcaaggaaag tgaaccttta 3480
   aagtaaaggc ctcatctcct ttattgcagt tcaaatcctc accatccaca gcaagatgaa 3540
   ttttatcagc catgtttggt tgtaaatgct cgtgtgattt cctacagaaa tactgctctg 3600
   aatattttgt aataaaggtc tttgcacatg tgaccacata cgtgttagga ggctgcatgc 3660
   tctggaagcc tggactctaa gctggagctc ttggaagagc tcttcggttt ctgagcataa 3720
   tgctcccatc tcctgatttc tctgaacaga aaacaaaaga gagaatgagg gaaattgcta 3780
   ttttatttgt attcatgaac ttggctgtaa tcagttatgc cgtataggat gtcagacaat 3840
   accactggtt aaaataaagc ctatttttca aatttagtga gtttctcaag tttattatat 3900
   ttttctcttg tttttattta atgcacaata tggcattata tcaatatcct ttaaactgtg 3960
   acctggcata cttgtctgac agatcttaat actactccta acatttagaa aatgttgata 4020
   aagcttctta gttgtacatt ttttggtgaa gagtatccag gtctttgctg tggatgggta 4080
   aagcaaagag caaatgaacg aagtattaag cattggggcc tgtcttatct acactcgagt 4140
   gtaagagtgg ccgaaatgac agggctcagc agactgtggc ctgaggccca aatctggccc 4200
   accacctgtt tggtgtagcc tgctaagaat ggcttttaca tttttaaatg gttgggaaag 4260
   aaaaaaaaag aagtagtaga ttttgtagca tgtgatgtaa gtaatgtaaa acttaaattc 4320
   cagtatccat aaataaagtt ttatgagaac aga 4353

<210> 65
<211> 4353
<212> DNA
<213> Homo sapiens

<400> 65
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattga tcagttgaag ctcattttaa gactcgttgg 1200

aaccccaggg gctgagcttt tgaagaaaat ctcctcagag tctgcaagaa actatattca 1260

gtctttgact cagatgccga agatgaactt tgcgaatgta tttattggtg ccaatcccct 1320

ggctgtcgac ttgctggaga agatgcttgt attggactca gataagagaa ttacagcggc 1380

ccaagccctt gcacatgcct actttgctca gtaccacgat cctgatgatg aaccagtggc 1440

cgatccttat gatcagtcct ttgaaagcag ggacctcctt atagatgagt ggaaaagcct 1500

gacctatgat gaagtcatca gctttgtgcc accacccctt gaccaagaag agatggagtc 1560

ctgagcacct ggtttctgtt ctgttgatcc cacttcactg tgaggggaag gccttttcac 1620

gggaactctc caaatattat tcaagtgcct cttgttgcag agatttcctc catggtggaa 1680

gggggtgtgc gtgcgtgtgc gtgcgtgtta gtgtgtgtgc atgtgtgtgt ctgtctttgt 1740

gggagggtaa gacaatatga acaaactatg atcacagtga ctttacagga ggttgtggat 1800

gctccagggc agcctccacc ttgctcttct ttctgagagt tggctcaggc agacaagagc 1860

tgctgtcctt ttaggaatat gttcaatgca aagtaaaaaa atatgaattg tccccaatcc 1920

cggtcatgct tttgccactt tggcttctcc tgtgacccca ccttgacggt ggggcgtaga 1980

cttgacaaca tcccacagtg gcacggagag aaggcccata ccttctggtt gcttcagacc 2040

tgacaccgtc cctcagtgat acgtacagcc aaaaaggacc aactggcttc tgtgcactag 2100

cctgtgatta acttgcttag tatggttctc agatcttgac agtatatttg aaactgtaaa 2160

tatgtttgtg ccttaaaagg agagaagaaa gtgtagatag ttaaaagact gcagctgctg 2220

aagttctgag ccgggcaagt cgagagggct gttggacagc tgcttgtggg cccggagtaa 2280

tcaggcagcc ttcataggcg gtcatgtgtg catgtgagca catgcgtata tgtgcgtctc 2340

tctttctccc tcacccccag gtgttgccat ttctctgctt acccttcacc tttggtgcag 2400

aggtttcttg aatatctgcc ccagtagtca gaagcaggtt cttgatgtca tgtacttcct 2460

gtgtactctt tatttctagc agagtgagga tgtgttttgc acgtcttgct atttgagcat 2520

gcacagctgc ttgtcctgct ctcttcagga ggccctggtg tcaggcaggt ttgccagtga 2580

agacttcttg ggtagtttag atcccatgtc acctcagctg atattatggc aagtgatatc 2640

acctctcttc agcccctagt gctattctgt gttgaacaca attgatactt caggtgcttt 2700

tgatgtgaaa atcatgaaaa gaggaacagg tggatgtata gcatttttat tcatgccatc 2760

tgttttcaac caactatttt tgaggaatta tcatgggaaa agaccagggc ttttcccagg 2820

aatatcccaa acttcggaaa caagttattc tcttcactcc caataactaa tgctaagaaa 2880

tgctgaaaat caaagtaaaa aattaaagcc cataaggcca gaaactcctt ttgctgtctt 2940

tctctaaata tgattacttt aaaataaaaa agtaacaagg tgtcttttcc actcctatgg 3000

aaaagggtct tcttggcagc ttaacattga cttcttggtt tggggagaaa taaattttgt 3060

ttcagaattt tgtatattgt aggaatcctt tgagaatgtg attccttttg atggggagaa 3120

agggcaaatt attttaatat tttgtatttt caactttata aagataaaat atcctcaggg 3180

gtggagaagt gtcgttttca taacttgctg aatttcaggc attttgttct acatgaggac 3240

tcatatattt aagccttttg tgtaataaga aagtataaag tcacttccag tgttggctgt 3300

gtgacagaat cttgtatttg ggccaaggtg tttccatttc tcaatcagtg cagtgataca 3360

tgtactccag agggacaggg tggaccccct gagtcaactg gagcaagaag gaaggaggca 3420

gactgatggc gattccctct cacccgggac tctccccctt tcaaggaaag tgaaccttta 3480

aagtaaaggc ctcatctcct ttattgcagt tcaaatcctc accatccaca gcaagatgaa 3540

ttttatcagc catgtttggt tgtaaatgct cgtgtgattt cctacagaaa tactgctctg 3600

aatattttgt aataaaggtc tttgcacatg tgaccacata cgtgttagga ggctgcatgc 3660

tctggaagcc tggactctaa gctggagctc ttggaagagc tcttcggttt ctgagcataa 3720

tgctcccatc tcctgatttc tctgaacaga aaacaaaaga gagaatgagg gaaattgcta 3780

ttttatttgt attcatgaac ttggctgtaa tcagttatgc cgtataggat gtcagacaat 3840

accactggtt aaaataaagc ctatttttca aatttagtga gtttctcaag tttattatat 3900

ttttctcttg tttttattta atgcacaata tggcattata tcaatatcct ttaaactgtg 3960

acctggcata cttgtctgac agatcttaat actactccta acatttagaa aatgttgata 4020

aagcttctta gttgtacatt ttttggtgaa gagtatccag gtctttgctg tggatgggta 4080

aagcaaagag caaatgaacg aagtattaag cattggggcc tgtcttatct acactcgagt 4140

gtaagagtgg ccgaaatgac agggctcagc agactgtggc ctgagggcca aatctggccc 4200

accacctgtt tggtgtagcc tgctaagaat ggcttttaca tttttaaatg gttgggaaag 4260

aaaaaaaaag aagtagtaga ttttgtagca tgtgatgtaa gtaatgtaaa acttaaattc 4320

cagtatccat aaataaagtt ttatgagaac aga 4353

<210> 66
<211> 1431
<212> DNA
<213> Homo sapiens

<400> 66
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattga tcagttgaag ctcattttaa gactcgttgg 1200

aaccccaggg gctgagcttt tgaagaaaat ctcctcagag tctgcaagaa actatattca 1260

gtctttgact cagatgccga agatgaactt tgcgaatgta tttattggtg ccaatcccct 1320

gggtaagttg accatatatc ctcacctcat ggatattgaa ttggttatga tataaattgg 1380

ggatttgaag aagagtttct ccttttgacc aaataaagta ccattagttg a 1431

<210> 67
<211> 4274
<212> DNA
<213> Homo sapiens

<400> 67
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattga tcagttgaag ctcattttaa gactcgttgg 1200

aaccccaggg gctgagcttt tgaagaaaat ctcctcagag tctctgtcga cttgctggag 1260

aagatgcttg tattggactc agataagaga attacagcgg cccaagccct tgcacatgcc 1320

tactttgctc agtaccacga tcctgatgat gaaccagtgg ccgatcctta tgatcagtcc 1380

tttgaaagca gggacctcct tatagatgag tggaaaagcc tgacctatga tgaagtcatc 1440

agctttgtgc caccacccct tgaccaagaa gagatggagt cctgagcacc tggtttctgt 1500

tctgttgatc ccacttcact gtgaggggaa ggccttttca cgggaactct ccaaatatta 1560

ttcaagtgcc tcttgttgca gagatttcct ccatggtgga agggggtgtg cgtgcgtgtg 1620

cgtgcgtgtt agtgtgtgtg catgtgtgtg tctgtctttg tgggagggta agacaatatg 1680

aacaaactat gatcacagtg actttacagg aggttgtgga tgctccaggg cagcctccac 1740

cttgctcttc tttctgagag ttggctcagg cagacaagag ctgctgtcct tttaggaata 1800

tgttcaatgc aaagtaaaaa aatatgaatt gtccccaatc ccggtcatgc ttttgccact 1860

ttggcttctc ctgtgacccc accttgacgg tggggcgtag acttgacaac atcccacagt 1920
ggcacggaga gaaggcccat accttctggt tgcttcagac ctgacaccgt ccctcagtga 1980
tacgtacagc caaaaaggac caactggctt ctgtgcacta gcctgtgatt aacttgctta 2040
gtatggttct cagatcttga cagtatattt gaaactgtaa atatgtttgt gccttaaaag 2100
gagagaagaa agtgtagata gttaaaagac tgcagctgct gaagttctga gccgggcaag 2160
tcgagagggc tgttggacag ctgcttgtgg gcccggagta atcaggcagc cttcataggc 2220
ggtcatgtgt gcatgtgagc acatgcgtat atgtgcgtct ctctttctcc ctcaccccca 2280
ggtgttgcca tttctctgct tacccttcac ctttggtgca gaggtttctt gaatatctgc 2340
cccagtagtc agaagcaggt tcttgatgtc atgtacttcc tgtgtactct ttatttctag 2400
cagagtgagg atgtgttttg cacgtcttgc tatttgagca tgcacagctg cttgtcctgc 2460
tctcttcagg aggccctggt gtcaggcagg tttgccagtg aagacttctt gggtagttta 2520
gatcccatgt cacctcagct gatattatgg caagtgatat cacctctctt cagcccctag 2580
tgctattctg tgttgaacac aattgatact tcaggtgctt ttgatgtgaa aatcatgaaa 2640
agaggaacag gtggatgtat agcattttta ttcatgccat ctgttttcaa ccaactattt 2700
ttgaggaatt atcatgggaa aagaccaggg cttttcccag gaatatccca aacttcggaa 2760
acaagttatt ctcttcactc ccaataacta atgctaagaa atgctgaaaa tcaaagtaaa 2820
aaattaaagc ccataaggcc agaaactcct tttgctgtct ttctctaaat atgattactt 2880
taaaataaaa aagtaacaag gtgtcttttc cactcctatg gaaaagggtc ttcttggcag 2940
cttaacattg acttcttggt ttggggagaa ataaattttg tttcagaatt ttgtatattg 3000
taggaatcct ttgagaatgt gattcctttt gatggggaga aagggcaaat tattttaata 3060
ttttgtattt tcaactttat aaagataaaa tatcctcagg ggtggagaag tgtcgttttc 3120
ataacttgct gaatttcagg cattttgttc tacatgagga ctcatatatt taagcctttt 3180
gtgtaataag aaagtataaa gtcacttcca gtgttggctg tgtgacagaa tcttgtattt 3240
gggccaaggt gtttccattt ctcaatcagt gcagtgatac atgtactcca gagggacagg 3300
gtggaccccc tgagtcaact ggagcaagaa ggaaggaggc agactgatgg cgattccctc 3360
tcacccggga ctctccccct ttcaaggaaa gtgaaccttt aaagtaaagg cctcatctcc 3420
tttattgcag ttcaaatcct caccatccac agcaagatga attttatcag ccatgtttgg 3480
ttgtaaatgc tcgtgtgatt tcctacagaa atactgctct gaatattttg taataaaggt 3540
ctttgcacat gtgaccacat acgtgttagg aggctgcatg ctctggaagc ctggactcta 3600
agctggagct cttggaagag ctcttcggtt tctgagcata atgctcccat ctcctgattt 3660
ctctgaacag aaaacaaaag agagaatgag ggaaattgct attttatttg tattcatgaa 3720
cttggctgta atcagttatg ccgtatagga tgtcagacaa taccactggt taaaataaag 3780

cctatttttc aaatttagtg agtttctcaa gtttattata tttttctctt gtttttattt 3840

aatgcacaat atggcattat atcaatatcc tttaaactgt gacctggcat acttgtctga 3900

cagatcttaa tactactcct aacatttaga aaatgttgat aaagcttctt agttgtacat 3960

tttttggtga agagtatcca ggtctttgct gtggatgggt aaagcaaaga gcaaatgaac 4020

gaagtattaa gcattggggc ctgtcttatc tacactcgag tgtaagagtg gccgaaatga 4080

cagggctcag cagactgtgg cctgagggcc aaatctggcc caccacctgt ttggtgtagc 4140

ctgctaagaa tggcttttac atttttaaat ggttgggaaa gaaaaaaaaa gaagtagtag 4200

attttgtagc atgtgatgta agtaatgtaa aacttaaatt ccagtatcca taaataaagt 4260

tttatgagaa caga 4274

<210> 68
<211> 5386
<212> DNA
<213> Homo sapiens

<400> 68
cgccttgccc aaggcctcag caaccgacgt tcgaaagcca ggagaaaagg cgaatgataa 60

agggcgctcc acgcatgcgt taagaagccg ccccaactcc cccgcggcgt tctttcttgg 120

aacaaaacta gcgcggagcc acggaactcc gcagtttgcg tagacttgaa tttcctattc 180

ctcggacgat ccatgtggaa tccgaaaaat agaaatgaag gtacatatgc acacaaaatt 240

ttgcctcatt tgtttgctga catttatttt tcatcattgc aaccattgcc atgaagaaca 300

tgaccatggc cctgaagcgc ttcacagaca gcatcgtgga atgacagaat tggagccaag 360

caaattttca aagcaagctg ctgaaaatga aaaaaaatac tatattgaaa aactttttga 420

gcgttatggt gaaaatggaa gattatcctt ttttggtttg gagaaacttt taacaaactt 480

gggccttgga gagagaaaag tagttgagat taatcatgag gatcttggcc acgatcatgt 540

ttctcattta gatattttgg cagttcaaga gggaaagcat tttcactcac ataaccacca 600

gcattcccat aatcatttaa attcagaaaa tcaaactgtg accagtgtat ccacaaaaag 660

aaaccataaa tgtgatccag agaaagagac agttgaagtg tctgtaaaat ctgatgataa 720

acatatgcat gaccataatc accgcctacg tcatcaccat cgtttgcatc atcatcttga 780

tcataacaac actcaccatt ttcataatga ttccattact cccagtgagc gtggggagcc 840

tagcaatgaa ccttcaacag agaccaataa aacccaggaa caatctgatg ttaaactacc 900

gaaaggaaag aggaagaaaa aagggaggaa aagtaatgaa aattctgagg ttattacacc 960

aggttttccc cctaaccatg atcagggtga acagtatgag cataatcggg tccacaaacc 1020

tgatcgtgta cataacccag gtcattctca tgtacatctt ccagaacgta atggtcatga 1080

tcctggtcgt ggacaccaag atcttgatcc tgataatgaa ggtgaacttc gacatactag 1140

aaagagagaa gcaccacatg ttaaaaataa tgcaataatt tctttgagaa aagatctaaa 1200

tgaagatgac catcatcatg aatgtttgaa cgtcactcag ttattaaaat actatggtca 1260

tggtgccaac tctcccatct caactgattt atttacatac ctttgccctg cattgttata 1320

tcaaatcgac agcagacttt gtattgagca ttttgacaaa cttttagttg aagatataaa 1380

taaggataaa aacctggttc ctgaagatga ggcaaatata ggggcatcag cctggatttg 1440

tggtatcatt tctatcactg tcattagcct gctttccttg ctaggcgtga tcttggttcc 1500

tatcattaac caaggatgct tcaaattcct tcttacattc cttgttgcat tagctgtagg 1560

aacaatgagt ggagacgccc ttcttcatct actgccccat tctcagggtg gacatgatca 1620

cagtcaccaa catgcacatg ggcatggaca ttctcatgga catgaatcta acaagttttt 1680

ggaagaatat gatgctgtat tgaaaggact tgttgctcta ggaggcattt acttgctatt 1740

tatcattgaa cactgcatta gaatgtttaa gcactacaaa caacaaagag gaaaacagaa 1800

atggtttatg aaacagaaca cagaagaatc aactattgga agaaagcttt cagatcacaa 1860

gttaaacaat acaccagatt ctgactggct tcaactcaag cctcttgccg gaactgatga 1920

ctcggttgtt tctgaagatc gacttaatga aactgaactg acagatttag aaggccaaca 1980

agaatcccct cctaaaaatt acctttgtat agaagaggag aaaatcatag accattctca 2040

cagtgatgga ttacatacca ttcatgagca tgatctccat gctgctgcac ataaccacca 2100

cggcgagaac aaaactgtgc tgaggaagca taatcaccag tggcaccaca agcattctca 2160

tcattcccat ggcccctgtc attctggatc cgatctgaaa gaaacaggaa tagctaatat 2220

agcctggatg gtgatcatgg gggatggcat ccacaacttc agtgatgggc tcgcaattgg 2280

tgcagctttc agtgctggat tgacaggagg aatcagtact tctatagccg tcttctgtca 2340

tgaactgcca catgaattag gagattttgc agttcttctt aaagcaggca tgactgtaaa 2400

gcaagcaatt gtatacaacc tcctctctgc catgatggct tacataggca tgctcatagg 2460

cacagctgtt ggtcagtatg ccaataacat cacactttgg atctttgcag tcactgcagg 2520

catgttcctc tatgtagcct tggtggatat gcttccagaa atgttgcatg gtgatggtga 2580

caatgaagaa catggctttt gtcctgtggg gcaattcatc cttcagaatt taggattgct 2640

ctttggattt gccattatgc tggtgattgc cctctatgaa gataaaattg tgtttgacat 2700

ccagttttga cctttcccag taatcactgt tgattacgag aatgttacca tgcagctttg 2760

catctgttcc ttgtactgta tgcacattgc tcaaaggaaa gtcagtggct tgcactactt 2820

acaagtttca tagatttgag cctaaccaca agaggctggt gcttagtact gttttccctg 2880

cacgtagggg tcttttaaaa atataaagct tgtgataaag agaggagaat atgggactcc 2940

atgaaccagt gttgatatgt ttgattaaga cttttcacaa aataatcata taaaacacta 3000

gtctctttat tagtagaaac ttctgtggct atgcagaaat agagatcgaa ccaaaaaaaa 3060

tcatttaaac tttaaaaata ttttaaatgg actttgggga gacatttttt gtgtgtttta 3120

agaatgaatt gtagtgctct ttaattcagc tacatatatt catgtggtga tagggatcaa 3180

cttgacacaa ctttgaaact gcataaagta gacataggaa ctagaggaaa gctcaggctg 3240

cattagagta tgaatttagc attgggaaaa gcccttattc ttgaatctag agttactatt 3300

tttgtatata tttgcatagt gtttaaacct gcagcctaaa ctactgaaat ttgtgattgt 3360

atgtttgtgt gagcttcagt ttaatgaaag attcataatg gttctttgta ttattataat 3420

acttggtgtt ggggtgttct ttctgttttg ttttttactt taattttgtt ttgatttttt 3480

tttttttttt ttggcggggg taggtgaggg tttggagcat gtggtctttt taaaaaattg 3540

taaccctcta gaaaatatca aagaaatgaa ccagacgtgg tttaaatagt tgattttcct 3600

attttaacag taccaactag ttaattggga aatgtaagtt ctgaatgttc acattgcttt 3660

accagtttgg cactggaacc aagagcacat gtcgtggctg gctacaaggt tgtaaagcag 3720

aaaatcgaag tttaccatgt ctgtaatgtg tacatgaagt gtcaatttag aacagttact 3780

aggataaact ccattattgc catggctgtc atggtaccca agtgacttgg aagatgcatt 3840

taaattactc agctgaaatc acttgatcat cttgtgccaa gatatgctgt tggtgcctga 3900

tagggattag tcttttaggt gccctgttct cctaccataa ttgtgaatga tttgtgagaa 3960

gtgcaagcca tgtttatcct gaatttttac ttaataattt gtattactag tcatatgcat 4020

gtagctttct gtttacatcc tatgccacat ggtcttcatt tatgccaggt aaactgtatt 4080

tgaactatgt gcagctagct ttgttttaat ctgcttggca accagtgtag ctgctgtaac 4140

aatctatctt attgttcaaa tatataagag ccaaactctt ttccattcca tctaaaatgt 4200

tttcatttag tactcttctt tcctcctact ctatgaactt caaaacaaaa acaaaacttt 4260

gagagcagca catgcatcca ggtatttata gattattgcc agtgtctttt ctgtatgcta 4320

taagcaaggg agcttaggtg ttatttcttt aatttatgct tgaatctgaa aaattatttc 4380

tgacttactc catggcctcc ttataataag tagaagtttt atatataatt aattttcagc 4440

attgggcact gaattaggac agtcctcatc tcattgcttg gcccttcaag caacctagct 4500

aaaaggtgct gatattttat ttagtactgc caacttcaag tgatttagat atctatctat 4560

ctagatttct gaaccaagat atatttatag ttcacttttg ggtttttata cccacggtag 4620

gattctgcat tccagcatta aatctgcttc attttagaac ctttataaaa gcaatagctg 4680

gaatatactc ccagttttaa aataaatgcc tgattgattt aaagcaagta ggttatgctg 4740

aagtatataa agaagtttta tattctctca aaaatggtat tatctttctt tatttgctag 4800

attcttacaa atcttttaag agggctgtaa cagttgctgc tagtattagg gttccacatc 4860

attctaatgt atagtttcaa gtcttaatag acaatctgaa ttccactaca tttcttttgg 4920

ctccaacatt ccttttagct tgaccagtct aatttaaaat gtgtttgttg gaggtcatta 4980

acgttacttg tacaatgctg tcactgtgtg acatccatat gaattttggt atatatcaat 5040

caatcaatca atcacattgc attcaatcaa tcagctgtga ttgattgatt atgcttagaa 5100

atactatagt aactagatgc agtgtgaatt ttttccatta acaaacaaac aagtcagtgg 5160

cttaaatgtg attatggtcc tgcaaggtga ttcttgctaa aatatctaaa cttttgtttt 5220

gttttaactg aatcattttt taacttaaaa agctggaaaa tatcaaatgc tgtttttttt 5280

ttttcattgt caacagtggt gtgtcatttt atgtatgttc ctaatgctta tggaactcct 5340

ccaaaataaa gttactcaaa gagagcaaat aaaaaaaaaa aaaaaa 5386

<210> 69
<211> 5337
<212> DNA
<213> Homo sapiens

<400> 69
gagtggacct tgtacgccgc aagcgtagca gggtgtcaga cgcgccggtt tctgcgacgc 60

agttagcgca gtctgctttg gtgaatacac gatttggtgc agccggggtt tggtaccgag 120

cggagaggag atgcacacgg cactcgagtg tgaggaaaaa tagaaatgaa ggtacatatg 180

cacacaaaat tttgcctcat ttgtttgctg acatttattt ttcatcattg caaccattgc 240

catgaagaac atgaccatgg ccctgaagcg cttcacagac agcatcgtgg aatgacagaa 300

ttggagccaa gcaaattttc aaagcaagct gctgaaaatg aaaaaaaata ctatattgaa 360

aaactttttg agcgttatgg tgaaaatgga agattatcct tttttggttt ggagaaactt 420

ttaacaaact tgggccttgg agagagaaaa gtagttgaga ttaatcatga ggatcttggc 480

cacgatcatg tttctcattt agatattttg gcagttcaag agggaaagca ttttcactca 540

cataaccacc agcattccca taatcattta aattcagaaa atcaaactgt gaccagtgta 600

tccacaaaaa gaaaccataa atgtgatcca gagaaagaga cagttgaagt gtctgtaaaa 660

tctgatgata aacatatgca tgaccataat caccgcctac gtcatcacca tcgtttgcat 720

catcatcttg atcataacaa cactcaccat tttcataatg attccattac tcccagtgag 780

cgtggggagc ctagcaatga accttcaaca gagaccaata aaacccagga acaatctgat 840

gttaaactac cgaaaggaaa gaggaagaaa aaagggagga aaagtaatga aaattctgag 900

gttattacac caggttttcc ccctaaccat gatcagggtg aacagtatga gcataatcgg 960

gtccacaaac ctgatcgtgt acataaccca ggtcattctc atgtacatct tccagaacgt 1020

aatggtcatg atcctggtcg tggacaccaa gatcttgatc ctgataatga aggtgaactt 1080

cgacatacta gaaagagaga agcaccacat gttaaaaata atgcaataat ttctttgaga 1140

aaagatctaa atgaagatga ccatcatcat gaatgtttga acgtcactca gttattaaaa 1200

tactatggtc atggtgccaa ctctcccatc tcaactgatt tatttacata cctttgccct 1260

gcattgttat atcaaatcga cagcagactt tgtattgagc attttgacaa acttttagtt 1320

gaagatataa ataaggataa aaacctggtt cctgaagatg aggcaaatat aggggcatca 1380

gcctggattt gtggtatcat ttctatcact gtcattagcc tgctttcctt gctaggcgtg 1440

atcttggttc ctatcattaa ccaaggatgc ttcaaattcc ttcttacatt ccttgttgca 1500

ttagctgtag gaacaatgag tggagacgcc cttcttcatc tactgcccca ttctcagggt 1560

ggacatgatc acagtcacca acatgcacat gggcatggac attctcatgg acatgaatct 1620

aacaagtttt tggaagaata tgatgctgta ttgaaaggac ttgttgctct aggaggcatt 1680

tacttgctat ttatcattga acactgcatt agaatgttta agcactacaa acaacaaaga 1740

ggaaaacaga aatggtttat gaaacagaac acagaagaat caactattgg aagaaagctt 1800

tcagatcaca agttaaacaa tacaccagat tctgactggc ttcaactcaa gcctcttgcc 1860

ggaactgatg actcggttgt ttctgaagat cgacttaatg aaactgaact gacagattta 1920

gaaggccaac aagaatcccc tcctaaaaat tacctttgta tagaagagga gaaaatcata 1980

gaccattctc acagtgatgg attacatacc attcatgagc atgatctcca tgctgctgca 2040

cataaccacc acggcgagaa caaaactgtg ctgaggaagc ataatcacca gtggcaccac 2100

aagcattctc atcattccca tggcccctgt cattctggat ccgatctgaa agaaacagga 2160

atagctaata tagcctggat ggtgatcatg ggggatggca tccacaactt cagtgatggg 2220

ctcgcaattg gtgcagcttt cagtgctgga ttgacaggag gaatcagtac ttctatagcc 2280

gtcttctgtc atgaactgcc acatgaatta ggagattttg cagttcttct taaagcaggc 2340

atgactgtaa agcaagcaat tgtatacaac ctcctctctg ccatgatggc ttacataggc 2400

atgctcatag gcacagctgt tggtcagtat gccaataaca tcacactttg gatctttgca 2460

gtcactgcag gcatgttcct ctatgtagcc ttggtggata tgcttccaga aatgttgcat 2520

ggtgatggtg acaatgaaga acatggcttt tgtcctgtgg ggcaattcat ccttcagaat 2580

ttaggattgc tctttggatt tgccattatg ctggtgattg ccctctatga agataaaatt 2640

gtgtttgaca tccagttttg acctttccca gtaatcactg ttgattacga gaatgttacc 2700

atgcagcttt gcatctgttc cttgtactgt atgcacattg ctcaaaggaa agtcagtggc 2760

ttgcactact tacaagtttc atagatttga gcctaaccac aagaggctgg tgcttagtac 2820

tgttttccct gcacgtaggg gtcttttaaa aatataaagc ttgtgataaa gagaggagaa 2880

tatgggactc catgaaccag tgttgatatg tttgattaag acttttcaca aaataatcat 2940

ataaaacact agtctcttta ttagtagaaa cttctgtggc tatgcagaaa tagagatcga 3000

accaaaaaaa atcatttaaa ctttaaaaat attttaaatg gactttgggg agacattttt 3060

tgtgtgtttt aagaatgaat tgtagtgctc tttaattcag ctacatatat tcatgtggtg 3120

atagggatca acttgacaca actttgaaac tgcataaagt agacatagga actagaggaa 3180

agctcaggct gcattagagt atgaatttag cattgggaaa agcccttatt cttgaatcta 3240

gagttactat ttttgtatat atttgcatag tgtttaaacc tgcagcctaa actactgaaa 3300

tttgtgattg tatgtttgtg tgagcttcag tttaatgaaa gattcataat ggttctttgt 3360

attattataa tacttggtgt tggggtgttc tttctgtttt gtttttactt ttaattttgt 3420

tttgattttt tttttttttt tttggcgggg gtaggtgagg gtttggagca tgtggtcttt 3480

ttaaaaaatt gtaaccctct agaaaatatc aaagaaatga accagacgtg gtttaaatag 3540

ttgattttcc tattttaaca gtaccaacta gttaattggg aaatgtaagt tctgaatgtt 3600

cacattgctt taccagtttg gcactggaac caagagcaca tgtcgtggct ggctacaagg 3660

ttgtaaagca gaaaatcgaa gtttaccatg tctgtaatgt gtacatgaag tgtcaattta 3720

gaacagttac taggataaac tccattattg ccatggctgt catggtaccc aagtgacttg 3780

gaagatgcat ttaaattact cagctgaaat cacttgatca tcttgtgcca agatatgctg 3840

ttggtgcctg atagggatta gtcttttagg tgccctgttc tcctaccata attgtgaatg 3900

atttgtgaga agtgcaagcc atgtttatcc tgaattttta cttaataatt tgtattacta 3960

gtcatatgca tgtagctttc tgtttacatc ctatgccaca tggtcttcat ttatgccagg 4020

taaactgtat ttgaactatg tgcagctagc tttgttttaa tctgcttggc aaccagtgta 4080

gctgctgtaa caatctatct tattgttcaa atatataaga gccaaactct tttccattcc 4140

atctaaaatg ttttcattta gtactcttct ttcctcctac tctatgaact tcaaaacaaa 4200

aacaaaactt tgagagcagc acatgcatcc aggtatttat agattattgc cagtgtcttt 4260

tctgtatgct ataagcaagg gagcttaggt gttatttctt taatttatgc ttgaatctga 4320

aaaattattt ctgacttact ccatggcctc cttataataa gtagaagttt tatatataat 4380

taattttcag cattgggcac tgaattagga cagtcctcat ctcattgctt ggcccttcaa 4440

gcaacctagc taaaaggtgc tgatatttta tttagtactg ccaacttcaa gtgatttaga 4500

tatctatcta tctagatttc tgaaccaaga tatatttata gttcactttt gggtttttat 4560

acccacggta ggattctgca ttccagcatt aaatctgctt cattttagaa cctttataaa 4620

agcaatagct ggaatatact cccagtttta aaataaatgc ctgattgatt taaagcaagt 4680

aggttatgct gaagtatata aagaagtttt atattctctc aaaaatggta ttatctttct 4740

ttatttgcta gattcttaca aatcttttaa gagggctgta acagttgctg ctagtattag 4800

ggttccacat cattctaatg tatagtttca agtcttaata gacaatctga attccactac 4860

atttcttttg gctccaacat tccttttagc ttgaccagtc taatttaaaa tgtgtttgtt 4920

ggaggtcatt aacgttactt gtacaatgct gtcactgtgt gacatccata tgaattttgg 4980

tatatatcaa tcaatcaatc aatcacattg cattcaatca atcagctgtg attgattgat 5040

tatgcttaga aatactatag taactagatg cagtgtgaat tttttccatt aacaaacaaa 5100

caagtcagtg gcttaaatgt gattatggtc ctgcaaggtg attcttgcta aaatatctaa 5160

acttttgttt tgttttaact gaatcatttt ttaacttaaa aagctggaaa atatcaaatg 5220

ctgttttttt tttttcattg tcaacagtgg tgtgtcattt tatgtatgtt cctaatgctt 5280

atggaactcc tccaaaataa agttactcaa agagagcaaa taaaaaaaaa aaaaaaa 5337

<210> 70
<211> 2644
<212> DNA
<213> Homo sapiens

<400> 70
gcgctcgcca cgcccatgcc tccgtcgctg cgcggcccac cccggatgtc agccccccgc 60

gccgaccaga atccgtgaac atggcgaacg aggttatcaa gtgcaaggct gcagttgctt 120

gggaggctgg aaagcctctc tccatagagg agatagaggt ggcaccccca aaggctcatg 180

aagttcgaat caagatcatt gccactgcgg tttgccacac cgatgcctat accctgagtg 240

gagctgatcc tgagggttgt tttccagtga tcttgggaca tgaaggtgct ggaattgtgg 300

aaagtgttgg tgagggagtt actaagctga aggcgggtga cactgtcatc ccactttaca 360

tcccacagtg tggagaatgc aaattttgtc taaatcctaa aactaacctt tgccagaaga 420

taagagtcac tcaagggaaa ggattaatgc cagatggtac cagcagattt acttgcaaag 480

gaaagacaat tttgcattac atgggaacca gcacattttc tgaatacaca gttgtggctg 540

atatctctgt tgctaaaata gatcctttag cacctttgga taaagtctgc cttctaggtt 600

gtggcatttc aaccggttat ggtgctgctg tgaacactgc caagttggag cctggctctg 660

tttgtgccgt ctttggtctg ggaggagtcg gattggcagt tatcatgggc tgtaaagtgg 720

ctggtgcttc ccggatcatt ggtgtggaca tcaataaaga taaatttgca agggccaaag 780

agtttggagc cactgaatgt attaaccctc aggattttag taaacccatc caggaagtgc 840

tcattgagat gaccgatgga ggagtggact attcctttga atgtattggt aatgtgaagg 900

tcatgagagc agcacttgag gcatgtcaca agggctgggg cgtcagcgtc gtggttggag 960

tagctgcttc aggtgaagaa attgccactc gtccattcca gctggtaaca ggtcgcacat 1020

ggaaaggcac tgcctttgga ggatggaaga gtgtagaaag tgtcccaaag ttggtgtctg 1080

aatatatgtc caaaaagata aaagttgatg aatttgtgac tcacaatctg tcttttgatg 1140

aaatcaacaa agcctttgaa ctgatgcatt ctggaaagag cattcgaact gttgtaaaga 1200

tttaattcaa aagagaaaaa taatgtccat cctgtcgtga tgtgatagga gcagcttaac 1260

aggcagggag aagcgcctcc aacctcacag cctcgtagag cttcacagct actccagaaa 1320

atagggttat gtgtgtcatt catgaatctc tataatcaag gacaaggata attcagtcat 1380

gaacctgttt tctggatgct cctccacata aataattgct agtttattaa ggaatatttt 1440

aacataataa aagtaatttc tacatttgtg tggaaattgt cttgttttat gctgtcatca 1500

ttgtcacggt ttgtctgccc attatcttca ttctgcaagg gaaagggaaa ggaagcaggg 1560

cagtggtggg tgtctgaaac ctcagaaaca taacgttgaa cttttaaggg tctcagtccc 1620

cgttgattaa agaacagatc ctagccatca gtgacaaagt taatcaggac ccaagtctgc 1680

ttctgtgata ttatcttgaa gggaggtact gtgccttgtt catacctgta ccccaaattc 1740

ctaggatggc atctgccctt cagggggcac taaaatgtat tattgaaaca gcattctggg 1800

cttaaatagg tgtatgtatg tgttggttgt gactgtacta tttctagtat agtgaactac 1860

atactgaata tccaagttct cagcacctac ttttgtcaaa tcttaacatt ttgccacttc 1920

gagatcacat tgccattcct cccctccaga ggtaacaatt atccacaatt tgatgtttat 1980

cattcctgtg ttgttgtact ttcactgtgt ataacctaaa ccatctactc tttagtactg 2040

ttttatatat ttttaagcct catacttgct cattctacag cttttttcac tcattattgt 2100

ataattatat ctgaagctct cgttcattaa ttttagtcct gtgtagcaga attcaattac 2160

gggaactacc ataatttatc tgttctccag ttgaaggcat gaagttgttg ccagtttctg 2220

tattataaca ctgtagtgga acattcttct gcattgggct cactgcgtgt tacctaagac 2280

gtatcacaga ataaacacat ttagccttat agacattgcc aaattgctct tcaaagtaaa 2340

tgtgagtttt tgtgaattac atgagtatgg aatggtgttt tattatgact ttagtttgca 2400

ttttcctcaa ttctcgttaa atccttcatt ctaatggaca ttttattgtg aagaacctgt 2460

tcatatcctg tgctcaactt tgtattgaat tatttttctc tgaataattt ttaggagttc 2520

ttttattcta gacatcaatc atttgtcagt tttatatgtt gcaaatatct tctagtctat 2580

cttgtgactt ttctttttac tttatggtat tttgttgaat aaagttttaa tgtagtcaca 2640

taaa 2644

<210> 71
<211> 7966
<212> DNA
<213> Homo sapiens

<400> 71
   ggggccgcgc cgggtagcgt ttctttttag tgcctgaggc agctctggct cggagagcct 60
   tttgctagcc ccacggggac ctctgtgcac ggatggaccc gcccggacct ggcgggaagc 120
   ggcctggcag gcggcggccc cggcggcatc agcagagaca ggacggggcc gacgccgcgg 180
   gcccctgagg cgtgcgtgcc caccgggccc ggcggcggca ccatgatgcc gggcgagacc 240
   cattcggcgg cgcccgggac ggcggcggac ctgtcgcgat gtcagggctg cgcctctctg 300
   cagcagaatt tgaatgaata tgttgaagca ttaattacct tgaaacaaaa aattatcaat 360
   acagataatt tgttaacaga atatcagaag aaatgtgatg agctgcagtt tgcaagaaga 420
   gagaatagta atctgcatca ccaagtggaa gagatgcttc aaaaaatttc tcctctacag 480
   aaatgtcagg aagaactggg atctttaaaa gcagagctag aagagaaaaa gagttcttta 540
   aagttgtatc aggatactca tcaggaatat gctcgtgtaa aggaagaatg cttgaagagt 600
   gatgctcaga agaagaaact agaagctaag gtgaagaagc tgcaagaggc tgctgtcaag 660
   caaactcagg acttcaagca actgagaaat gaaaagaaaa tacttgaaaa ggaatttaag 720
   aagacacagg aaaggcttga cgaattttct aaacagaaaa atgaaaagga gttgagacat 780
   attggaacac aaatttcaag tgattcatat ggaagcatag ataaaagaaa agtgaaactg 840
   cttctgaagg aactctggct ctgtgtaaac acaacacaca gactacctgg tgaaggcagc 900
   aggtgtgtcc cagaaaaacc tgccaaagca atcaccagct ccagagtgcc tggggaagat 960
   ggtacgctac ctccaacaca gggcagccct ctcaggacct caaatgtgca gacatgcctc 1020
   acaaaactgt ccatggagat aaaggaggac tttttatgtc aaaatgtgga aaaacagagc 1080
   tccagtggaa caaattgtag ttctgaccat gtttttaatg agaatggaaa tcttgaggtt 1140
   ttagtacaaa gtcatcgtga cggtggtagt actgaatttg ttgatcatga tcattttttt 1200
   gatgaagatc ttcaagctgc aattgacttc ttcaaacttc cccctcctct tctgtcacca 1260
   gtgccctcgc cccctccgat gtcatcacct cacccgggtt ccttaccgtc ttcatttgca 1320
   cctgaaacct actttggaga atacacagat tccagcgata atgactcagt ccagcttaga 1380
   aattctgctg agtgtgtttc agaagatgat acaactgaat cacagaatta ttttggctca 1440
   ttgagaaaaa ataaaggaag tggcacatgg gaggaaaagc ccaaatcaca tgaagctatc 1500
   caagctctga atacatggga agtaaataaa gtgacaactt ctggactcga gactttcaca 1560
   gcaacactga gagaatcttc tgccacacac tccttagttg gtgaaaaaca ctggaccaca 1620
   gcatctcgat ccatgagtga tagaaaaaga gacattttac atgagacaaa aacacaaatg 1680
   gaggttaggg agatggataa gtcagtacaa actgagaaga ccattcataa actcactcga 1740
   ggtctatgca ttgagagatt gtctgccagc cctgcacaag agaaggaagc tgcccctggg 1800
   aagtctgagt tgtgttcttc tccccttggc aaaaggccat taaatgaact catggaatct 1860
   gaaggaaaaa ccgtattgtc taaaatgatg ggatcgccca aatcagagtt tactaagtgg 1920
   acacgaatta atgaaatcac ttctgaacca gaccgtatca cagtttctgg ccattttcac 1980
   agactatcta gagaattgga aaaggaaaaa gaagatactc aagggttcac tttaggagaa 2040
   tcacctgaat cagaagatga tgactcaggt gatggaatgg atgtagcagg gcttgacatt 2100
   gaaaccagtt tttcttcctc ttctaccttg gtagcattgt ctgttggcag taatccccag 2160
   tcttcttctg ggttagactg tggtaatgat acagatatta ctactaaagt attctctact 2220
   gaaccgcatc attcagaaca taaattgcaa actaaaactt taaacacatt acatctgcag 2280
   tctgagccac cggagtgttc tataggagga aacaacttgg agaatagctt gtgtgccttg 2340
   agccctgaat tgggagcatc taattttaat gatcagaaga gcagtgggat agaatataca 2400
   aaagtagtaa aaggcttgac caaaatacat tcacttcctc ggtcagtatt tatgaaagct 2460
   acaaaagatg ggcaatgtga aagtcaagat ccaagaattg agctcacact aaataagcca 2520
   gatttcacat cattaatagg ttctcaggct gccttgatca agagtggttt gggttttgtt 2580
   aaaagtactt catggcacca tagtgattta ttaaggaaag gtggcgaaga aagtctgaga 2640
   gccaaatcag aacatgaaca gaagactagc catcagttac aaaaggcaat gccattccta 2700
   caaaatagag gaccaacacc caagcctgat cttcttagag aaaataacaa tcctgtagaa 2760
   ttcaagacca ctgcatcggt gttgcctaat caagtatcag ttatcacaaa acagacaaga 2820
   cctgaaaagg ttcagagtgc caaattggaa cacttgaggc cacatagggt tgagcctacc 2880
   ttagtaacag aaaatagtgg caacaaaacc ggtatgtcaa ctgtagcaaa atgtgatggg 2940
   gaaagagatg atacaacaca aaacatcacg gaggtggctg ctgtgaaaag catttcacca 3000
   gaagtttctg cctctaggag aaaattagat tttaattctc caggtggttc ttcaccagta 3060
   gaaaattctg attgttccac aaatagcaga ttatctttct ctcctgaaaa tatcctcatc 3120
   caaaaccaag acattgtgag agaagctgca gtgcagggag atgggcagaa gcaaaggcag 3180
   cctcaggcca cagatctgga ctccagtggg acacatggca gtgagatgct tccagccaca 3240
   gaagtgactg tgtcaggagg gttttctgtt gaagaaacca gctgtggaga cacaggagga 3300
   tctggtggtg aggccctggc tgttgcaaat gattctacca gcacaccaca aaatgctaat 3360
   ggactttgga aattgaaatc tacaactccc ggtggtgctt tgcctgagtg ttttggcacc 3420
   acagacacta ctttttcttc agcattttgc agaaaacatg gagagacaca ggatacctcc 3480
   caaagtagcc tgcctggtac cttacattgt tacacaggca ttcgagaggg gggagacgac 3540
   actgaggtag agagtgaggc atttagctgc agtgagggga gcgaacagca agatgctcct 3600
   gatgactcac agaaaaattt aggagacaca gatgctgctg tagccgaggt gagaccttcc 3660
   ttagaggtag gttatttgac gtcagctctg caagatttta acataagtac tttttctgag 3720
   ctggatagac tttccacatc agaggttgtg atgtttcttg agagctgtca gttaggggat 3780
   tatagttcag gggactctgt ttctgaatgt tctagtaaag gaaccctaag taaagaaatg 3840
   aacaaagaat taaaggcaag tgaaatagga gaaaaataca gaaagcaacc ctgtgaggaa 3900
   gaaacacttg gaacctgtga agagtggatt gaatcagagg aagatgatta ttcgttaaaa 3960
   aatacaagtc agctcactca gtgttctttg gaaactctgt ctgaggttct gaccaagatt 4020
   aggcaagaac ttcaaacaaa ttctgaagat tgcaatggta aagatactgg cagtttattg 4080
   ctcttaaatg taaataacaa catgaccact gagaatttaa aagagaaaag tccatttcgg 4140
   gaaacgactg gctcctcatc acatgcttca gaaccaaccc cacaagcagc tgccttggac 4200
   actgagggca gctctcccat cagcggtatg cctcagaatg aaaaccctca gagcagacca 4260
   gaggcccgtt cagatgcagg caggcaaacc gatggtgggg aagaagacct gccagaacct 4320
   gtggagccat cagccttgtg ctctgactct gtgatggagc catccataga gcaaagttct 4380
   aactgcgagg ccgaaacaac atttcagtgt cagatagcaa cagtgacctc agaagttata 4440
   aacgtactta taaataagga tcagaatcta gtcattgaaa agggggacaa ctggacaatc 4500
   atcagtggtg tagctgtctt gccacatgtg gaccaggtca cactgtgtga cattcctgga 4560
   gacatcccta tttctcagga tcaaggagag ctggaagctg gttgcatccc agtgacttct 4620
   gctgagaagt ccccagaggc cagtcacact ggccctgcat ttcaggaggc tccatgtggc 4680
   aataatcttt catgtcccca agaggatgtt tcaagcagtg gtcagagcac caactttgat 4740
   aagagtcgtt tgcgaaatag acccgttaag cctagtatat ggattagttc tcaaatctat 4800
   gatcaaaact tcgagactca gattgttgcg tctgatcaca catattataa ctcaaaacta 4860
   gagccatctg gcaaaaataa gaatcgatca aagatttcaa acaaagatca gtcaaacaaa 4920
   ccagtaaaaa cttcagcgtc gagcagagtt gaaactcatc agagtgaagt tgctcagtca 4980
   ttttcagggg aaaaagctaa tacaaaaact caaagaagcc aaactcagac cattttagca 5040
   aatgctgata catccactcc tacagattgt tctcctgaca cactgagtaa aatacggcaa 5100
   gaggtggggc ctcctttgcc gcctctgctt gctcctctga tagctacacc tccaaggact 5160
   tcacagccac tgtctccact gatatcgagt tctagtcctt cctcaccagc ctctcctgtt 5220
   ggccaggttt ctcccttccg tgaaacccca gtgcctcctg ccatgtctcc atggccagag 5280
   gaccccagac gtgcctctcc tccagatcct tctccatctc catctgcagc ttcagccagt 5340
   gagagggtag tgccgtctcc tctgcagttc tgtgcggcca cgccgaagca cgcacttcct 5400
   gtgcctggcc gactcccacc ctgtgcatct ggccacgctg ctgtgggagg gcctcaggag 5460
   aattctgtga aaatccttga caccatgtat ccagagttat ctgccagggc ccggaccctc 5520
   aacatcctca aagggaatat tcaactcaca cgaggtccgc ctgctgactg taagaattta 5580
   ccgggacctg ccagtgctat gataggattc aaaacgatca cttcagcagc aactgctttt 5640
   gtcaaaactg ggagcagctc tggtggtgac tgtaaccaag acaagtcaag agatttgggg 5700
   actcagcagg attcaagcgg gaaaagaaca ctgtcaacgt ctacactgag aagtgctaaa 5760
   agactgcgcc tggacactgg gtccccagaa ccagaaacca ggggagtcac tgcagaagga 5820
   atccacaaaa acctcccagg gaacctccct ccagctgaag ttgcaacaac aaatgaggaa 5880
   agaagttgtt ctagtccagc cgtcagtgca gtttcacagt tgcctttaag cccaaaagaa 5940
   actgtggagt cccatgataa agccatagct aatgccctga agaaaattgc agagttttct 6000
   tttgatctgt tacctgtcat tcgtagtcat gtgtatgtgg gaaatatctc caaaaagccc 6060
   gtaatgagag atcaagagaa ggaagttgtt tatgaattta gcacaacaaa aaagcattta 6120
   gcagagtgct tgcttcactc tattctctca gaactaaaaa ttcagaagat atctatggac 6180
   cacaattaca ttcacgccct ctgcagggtg tatgtgggta tttgtcggca actcggagac 6240
   ttggaaagag ctcgtttgtt ttgctacagc ctacttaaag aagatttccc ggagtctgaa 6300
   aaattaactt tgtttattgc aaacatgtgg catgatatat ttctctctca atcggtgatt 6360
   aataaagcaa tgcagttagt tgccaggcaa cgtgctaaag gagaggttct gaactgcttg 6420
   agagcttttc ttaattggga aaagaatgcc ccggtagatg ttggcttcat ggtttctaag 6480
   ctgcttttga ccatacagtt atgtccaaaa acagaatttc aacctagtga aaaatttggt 6540
   gaagacctaa gtgataacac ttgggaatac atatttgcca ttgatctgct ctgctgccat 6600
   cagaaatgga tctggacgca tgataacatc ataagtaagg agctgtggcc tgtgatggat 6660
   aaatggataa aatacagaaa aggacatgca aacattgcgt atactcctga tattattata 6720
   gcctcaatac tgaggctgat tggtcgttta ggccaattgg gtttgaaaga aggatttcca 6780
   tctgctgtga aaaatattag ttcggttatt ggtatgttta tacagcatgc tcacgatgaa 6840
   gatataccat ggggtataca gttagcagcc gtgtatgctc tttgtgactt gagtcccagc 6900
   aatccagcag aaatttccaa gatcctggaa gcttggcgga gagaggcctc caaaagcgtt 6960
   ccgtctgcga ttgtcagctg cctagaggaa gtcagtgccc tgagcacaga ggagcttggc 7020
   tgacctggga tgccactgag gcttgagaag tgccttgaca cattttgaac acaaatagtt 7080
   tgatcagctt tcagaataca aagggaggtt tcaaaacaaa aagacataaa atagataaaa 7140
   cagaccagag gctctcctta ttgtttggca aggagacagg agaaacaagc agtcgcatag 7200
   tcgtttttcc ctaaatctaa tacgtttcac ttaaggctgt tgtgatctgt gacatatggg 7260
   ttatattatt gtgtctttgt caaacaacaa aaacttgaac ttagcccttt ttttgctgca 7320
   gaaagtgtcc ttttagtggc ttcttaaaat tgagtggcat tttataatga acttaccaat 7380
   ataaaaacat gatttggttc ctgagctgtt gttgttggac ttgtgttcca atgagtgact 7440
   aggaaaaaat aaattggcaa aaacctagag ttttctgcta tctttgctgg aaatgagttg 7500
   caaaagtttt tctcaagatg tagtgcgtaa ttgatcagag caaaacatgc agagccctta 7560
   gcagaaaccc actttaatgc attttcttca tatccctaaa gttccttaaa aatatgtgac 7620
   aatgcatcag gaagaggaga actgaagagt agaagttccc ttgcagattt ttttatcagt 7680
   gacatgtaat gagcaattca cagatgagcg caggcagagc tctgtgtgcc gtgtacatat 7740
   ggaccgtgct atgatgtgtc tcacattgga tgatattcca ctttgggaat tttagtattt 7800
   gtatatagaa aatgggttta ataactcacc atggttttga tttgtcttat attcgttatt 7860
   tcttaaaact cttgtatgtg tttttataat aaaaaataaa agtaagccat ggatattaaa 7920
   aaaaaaaaaa aaaaaaaaaa aaaaagaaag aaaaaaaaaa aaaaaa 7966

<210> 72
<211> 1523
<212> DNA
<213> Homo sapiens

<400> 72
tggctgagtt cttcctttga caagatggcg gcaggaggca gtggcgttgg tgggaagcgc 60

agctcgaaaa gcgatgccga ttctggtttc ctggggctgc ggcccacttc ggtggaccca 120

gcgctgaggc ggcggcggcg aggcccaaga aataagaagc ggggctggcg gcggcttgct 180

caggagccgc tggggctgga ggttgaccag ttcctggaag acgtgcggct acaggagcgc 240

acgagcggtg gcttgttgtc agaggcccca aatgaaaaac tcttcttcgt ggacactggc 300

tccaaggaaa aagggctgac aaagaagaga accaaagtcc agaagaagtc actgcttctc 360

aagaaacccc ttcgggttga cctcatcctc gagaacacat ccaaagtccc tgcccccaaa 420

gacgtcctcg cccaccaggt ccccaacgcc aagaagctca ggcggaagga gcagctatgg 480

gagaagctgg ccaagcaggg cgagctgccc cgggaggtgc gcagggccca ggcccggctc 540

ctcaaccctt ctgcaacaag ggccaagccc gggccccagg acaccgtaga gcggcccttc 600

tacgacctct gggcctcaga caaccccctg gacaggccgt tggttggcca ggatgagttt 660

ttcctggagc agaccaagaa gaaaggagtg aagcggccag cacgcctgca caccaagccg 720

tcccaggcac ccgccgtgga ggtggcgcct gccggagctt cctacaatcc atcctttgaa 780

gaccaccaga ccctgctctc agcggcccac gaggtggagt tgcagcggca gaaggaggcg 840

gagaagctgg agcggcagct ggccctgccc gccacggagc aggccgccac ccaggagtcc 900

acattccagg agctgtgcga ggggctgctg gaggagtcgg atggtgaggg ggagccaggc 960

cagggcgagg ggccggaggc tggggatgcc gaggtctgtc ccacgcccgc ccgcctggcc 1020

accacagaga agaagacgga gcagcagcgg cggcgggaga aggctgtgca caggctgcgg 1080

gtacagcagg ccgcgttgcg ggccgcccgg ctccggcacc aggagctgtt ccggctgcgc 1140

gggatcaagg cccaggtggc cctgaggctg gcggagctgg cgcggcggca gaggcggcgg 1200

caggcgcggc gggaggctga ggctgacaag ccccgaaggc tggggcggct caagtaccag 1260

gcacctgaca tcgacgtgca gctgagctcg gagctgacag actcgctcag gaccctgaag 1320

cccgagggca acatccttcg agaccggttc aagagcttcc agaggaggaa tatgatcgag 1380

cctcgagaga gagccaagtt caaacgcaag tacaaggtga agctggtgga gaagcgggcg 1440

ttccgtgaga tccagttgta gctgccatca gatgccggag actcgccctt caataaaaaa 1500

tctcttctag ctgatcagtg gga 1523

<210>  73
<211>  2611
<212>  DNA
<213>  Homo sapiens

<400>  73
gcaggcgcag agtccccggg ccaagatggc tgcgcggtgc tccacacgct ggttgctggt      60

ggttgtgggg accccgcggc tgccggctat atcgggtaga ggggcccggc cgcccaggga     120

gggcgtggtg ggggcatggc tgagccgcaa gctgagcgtc cccgcctttg cgtcttccct     180

gacctcttgc ggcccccgag cgctgctgac attgagacct ggtgtcagcc ttacaggaac     240

aaaacataac cctttcattt gtactgcctc cttccacacg agtgcccctt tggccaaaga     300

agattattat cagatattag gagtgcctcg aaatgccagc cagaaagaga tcaagaaagc     360

ctattatcag cttgccaaga agtatcaccc tgacacaaat aaggatgatc ccaaagccaa     420

ggagaagttc tcccagctgg cagaagccta tgaggttttg agtgatgagg tgaagaggaa     480

gcagtacgat gcctacggct ctgcaggctt cgatcctggg gccagcggct cccagcatag     540

ctactggaag ggaggcccca ctgtggaccc cgaggagctg ttcaggaaga tctttggcga     600

gttctcatcc tcttcatttg gagatttcca gaccgtgttt gatcagcctc aggaatactt     660

catggagttg acattcaatc aagctgcaaa gggggtcaac aaggagttca ccgtgaacat     720

catggacacg tgtgagcgct gcaacggcaa ggggaacgag cccggcacca aggtgcagca     780

ttgccactac tgtggcggct ccggcatgga aaccatcaac acaggccctt ttgtgatgcg     840

ttccacgtgt aggagatgtg gtggccgcgg ctccatcatc atatcgccct gtgtggtctg     900

caggggagca ggacaagcca agcagaaaaa gcgagtgatg atccctgtgc ctgcaggagt     960

cgaggatggc cagaccgtga ggatgcctgt gggaaaaagg gaaattttca ttacgttcag    1020

ggtgcagaaa agccctgtgt tccggaggga cggcgcagac atccactccg acctctttat    1080

ttctatagct caggctcttc ttgggggtac agccagagcc cagggcctgt acgagacgat    1140

caacgtgacg atcccccctg ggactcagac agaccagaag attcggatgg gtgggaaagg    1200

catcccccgg attaacagct acggctacgg agaccactac atccacatca agatacgagt    1260

tccaaagagg ctaacgagcc ggcagcagag cctgatcctg agctacgccg aggacgagac    1320

agatgtggag gggacggtga acggcgtcac cctcaccagc tctggaaaaa gatccactgg    1380

aaactaggcc gggaagcagc agcccctcca agggccaggg cacctgggag acgggaggat    1440

tccagaacag cagcactgag ctcccacccg cagagcctct ggacggcctt ggcaacagca    1500

aaatcatggg acaacacctc tctccacgga aaggtcacag tggacagccc gggcagtagg    1560

atgcagcccc agaggctggt ggcagtttcc tgtccattgg taggtgacgg cccctggctc    1620

aggcagaggg agatggttag actcttgcag ggctaaaact ctaatttgga attgaatatt    1680

gtggatatct tagttaaagg ccatgcttac agcttagaaa tgaagcctta agctgcatca 1740

agttacgaag tgattaattt ccttctcagc aaacctccgg gaggttccag aatgagttct 1800

tcctgacagg ttgtcttcac tgggagcgtg gggcccccag gccccaccag caccgtcctc 1860

ccctaatgag gggccctgcc gaggcatcag ctgctctgct cagttagttt ttattcccgg 1920

ggtaccaagc agctgcacag tcggtgcctg ggaggcacgt agaggcccag agagtccctg 1980

ggggttctgc tctgaccgtg tgggtggtga tccttgtcag gatgtacagt ccttgctccc 2040

accccatccg ggatggccgc ctgtccctga ctattgagtc ctgttgttgt aagccaggca 2100

tggagggctc ctgcccttct gctgagccac agcccattgc agcactgtgc tggccagact 2160

tcagctgcct tgggaactga agccctgcca ctgttgctag tcaggggctt ggttctccca 2220

cttacactgt tgacatctat tttctgaagt gtgtttaaat tattcagtgc taatcattgt 2280

tttttccttt gtaaatgttg attcagaaaa ggaaagcaca ggctaagcag ttgaaggttc 2340

cccaccattc agtgagagca gaacccccat tccccagcct ctgctggtag catgtcgcag 2400

tttccatgtg tttcaggatc ttcgggctgt cgttagacag gttaatgaag aacacttctc 2460

aacagtttcc tttttgtttt cctttataat tcactaaaat aaagcatcta ttagtgtctg 2520

atttaggaat gtaaaatgat tctgtattaa tgtaaataag attatctatt gcaaaaagat 2580

atttcaaacc taaaaaaaaa aaaaaaaaaa a 2611

<210> 74
<211> 2728
<212> DNA
<213> Homo sapiens

<400> 74
   gcaggcgcag agtccccggg ccaagatggc tgcgcggtgc tccacacgct ggttgctggt 60
   ggttgtgggg accccgcggc tgccggctat atcgggtaga ggggcccggc cgcccaggga 120
   gggcgtggtg ggggcatggc tgagccgcaa gctgagcgtc cccgcctttg cgtcttccct 180
   gacctcttgc ggcccccgag cgctgctgac attgagacct ggtgtcagcc ttacaggaac 240
   aaaacataac cctttcattt gtactgcctc cttccacacg agtgcccctt tggccaaaga 300
   agattattat cagatattag gagtgcctcg aaatgccagc cagaaagaga tcaagaaagc 360
   ctattatcag cttgccaaga agtatcaccc tgacacaaat aaggatgatc ccaaagccaa 420
   ggagaagttc tcccagctgg cagaagccta tgaggttttg agtgatgagg tgaagaggaa 480
   gcagtacgat gcctacggct ctgcaggctt cgatcctggg gccagcggct cccagcatag 540
   ctactggaag ggaggcccca ctgtggaccc cgaggagctg ttcaggaaga tctttggcga 600
   gttctcatcc tcttcatttg gagatttcca gaccgtgttt gatcagcctc aggaatactt 660
   catggagttg acattcaatc aagctgcaaa gggggtcaac aaggagttca ccgtgaacat 720
   catggacacg tgtgagcgct gcaacggcaa ggggaacgag cccggcacca aggtgcagca 780
   ttgccactac tgtggcggct ccggcatgga aaccatcaac acaggccctt ttgtgatgcg 840
   ttccacgtgt aggagatgtg gtggccgcgg ctccatcatc atatcgccct gtgtggtctg 900
   caggggagca ggacaagcca agcagaaaaa gcgagtgatg atccctgtgc ctgcaggagt 960
   cgaggatggc cagaccgtga ggatgcctgt gggaaaaagg gaaattttca ttacgttcag 1020
   ggtgcagaaa agccctgtgt tccggaggga cggcgcagac atccactccg acctctttat 1080
   ttctatagct caggctcttc ttgggggtac agccagagcc cagggcctgt acgagacgat 1140
   caacgtgacg atcccccctg ggactcagac agaccagaag attcggatgg gtgggaaagg 1200
   catcccccgg attaacagct acggctacgg agaccactac atccacatca agatacgagt 1260
   tccaaagagg ctaacgagcc ggcagcagag cctgatcctg agctacgccg aggacgagac 1320
   agatgtggag gggacggtga acggcgtcac cctcaccagc tctggtggca gcaccatgga 1380
   tagctccgca ggaagcaagg ctaggcgtga ggctggggag gacgaggagg gattcctttc 1440
   caaacttaag aaaatgttta cctcatgata tcccagccga ggaaaaagat ccactggaaa 1500
   ctaggccggg aagcagcagc ccctccaagg gccagggcac ctgggagacg ggaggattcc 1560
   agaacagcag cactgagctc ccacccgcag agcctctgga cggccttggc aacagcaaaa 1620
   tcatggacaa acacctctct ccacggaaag gtcacagtgg acagcccggg cagtaggatg 1680
   cagccccaga ggctggtggc agtttcctgt ccattggtag gtgacggccc ctggctcagg 1740
   cagagggaga tggttagact cttgcagggc taaaactcta atttggaatt gaatattgtg 1800
   gatatcttag ttaaaggcca tgcttacagc ttagaaatga agccttaagc tgcatcaagt 1860
   tacgaagtga ttaatttcct tctcagcaaa cctccgggag gttccagaat gagttcttcc 1920
   tgacaggttg tcttcactgg gagcgtgggg cccccaggcc ccaccagcac cgtcctcccc 1980
   taatgagggg ccctgccgag gcatcagctg ctctgctcag ttagttttta ttcccggggt 2040
   accaagcagc tgcacagtcg gtgcctggga ggcacgtaga ggcccagaga gtccctgggg 2100
   gttctgctct gaccgtgtgg gtggtgatcc ttgtcaggat gtacagtcct tgctcccacc 2160
   ccatccggga tggccgcctg tccctgacta ttgagtcctg ttgttgtaag ccaggcatgg 2220
   agggctcctg cccttctgct gagccacagc ccattgcagc actgtgctgg ccagacttca 2280
   gctgccttgg gaactgaagc cctgccactg ttgctagtca ggggcttggt tctcccactt 2340
   acactgttga catctatttt ctgaagtgtg tttaaattat tcagtgctaa tcattgtttt 2400
   ttcctttgta aatgttgatt cagaaaagga aagcacaggc taagcagttg aaggttcccc 2460
   accattcagt gagagcagaa cccccattcc ccagcctctg ctggtagcat gtcgcagttt 2520
   ccatgtgttt caggatcttc gggctgtcgt tagacaggtt aatgaagaac acttctcaac 2580
   agtttccttt ttgttttcct ttataattca ctaaaataaa gcatctatta gtgtctgatt 2640
   taggaatgta aaatgattct gtattaatgt aaataagatt atctattgca aaaagatatt 2700
   tcaaacctaa aaaaaaaaaa aaaaaaaa 2728

<210> 75
<211> 3818
<212> DNA
<213> Homo sapiens

<400> 75
tgtggtgcga agccacctcc cccgccgaat cgcgcatctg cgcagttgct gttattgtga 60

cttgtcgggc cacggccccg gatgttgtgg ctgccgcggg gagatggctg aggccgaagg 120

ggttcccacg accccaggcc cggcttcggg gtcgactttc aggggccgcc gagatgtgtc 180

aggctcctgg gagcgggacc agcaggttga ggcggcgcag cgggccctgg tggaggtgct 240

ggggccttac gagcctctgc tgagtcgggt gcaggcagcc ctggtgtggg agcggccagc 300

taggagcgct ctgtggtgcc tggggctgaa cgcggctttc tggttttttg ccctgacatc 360

tcttcgtctt gtgtttttac ttgcatttgg cttgatgatc attgtgtgta ttgatcaatg 420

gaagaacaaa atctggcctg aaataaaagt gccaagaccc gacgcattag acaatgagag 480

ctggggcttt gtgcaccctc ggttgctcag cgtgcccgag ctctgccacc atgtagctga 540

agtctgggtt agtgggacca ttttcataag gaatgttttg cttttcaaaa agcaaaaccc 600

aggcaagttc tgcttgctga gctgtgggat actgaccttt ttggctgtct tgggccgcta 660

cgtccctggg cttctgctgt cctacttgat gcttgtcact gtcatgatgt ggccccttgc 720

tgtgtaccac cgactgtggg atcgagcata tgtgcggctg aagccagctc tgcagcggct 780

agacttcagt gtccgtggct acatgatgtc caagcagaga gagagacaat tacgccgcag 840

agctctccac ccagaacgag ccatggacaa ccacagtgac agcgaagagg agcttgctgc 900

cttctgtcct cagctggacg attctactgt tgccagggaa ttggccatca cagactctga 960

gcactcagac gctgaagtct cctgtacaga caatggcaca ttcaatcttt caaggggcca 1020

aacacctcta acggaaggct ctgaagacct agatggtcac agtgatccag aggaatcctt 1080

tgccagagac cttccagact tcccttccat taatatggat cctgctggcc tggatgatga 1140

ggacgacact agcattggca tgcccagctt gatgtaccgt tctccgccag gggctgagga 1200

gccccaggcc ccacctgcca gccgggacga ggctgcgctg ccggagctcc tgcttggtgc 1260

tcttcctgta ggatccaacc tcaccagcaa ccttgccagc ctggtctccc agggtatgat 1320

tcagctggcc ttgtcagggg cctcccaacc aggcccttct ggagcacctg cccagagagc 1380

aacgagaggc ttcctccggt cccccagttc agacctggac actgatgctg agggggatga 1440

ctttgaactt ctggaccagt cggagctgag tcagctggac cctgccagtt ctaggagcca 1500

ctgaggcaga gactcctttt gggagtcact gtggtttagg tttttttctc cccatcccac 1560

ttaaggtgat ggggcaaggg aagaactcag ctcccctccc ctgaattata tttgtatgct 1620

gggtggcctg gctgatgctc agaggcctcc ttagagagga cactcactcc cctcccacca 1680

gctggatgcc catttctgag ctcagtcact gaagtgagag tgtgctcccc caagggaggc 1740

ttctctccat caggatggta ctttggggga acaaaatagt cagggatatt ggttcccctt 1800

tgaggaggtg ctgctgtttg cttttaggta tgagtgctca ggggccctca ctgaaagagc 1860

ccatgcctgc cttcctcctt tcatcgcctc tctagagccc ccaaagtcag gcagcagctg 1920

gagtagttac attgtcatca tctttttttt tgagacagtt tcgctctgtt gcccaggctg 1980

gagtgcagtg gtgtgatctt ggctttctgc aacgtctgcc ttccaggttg aagaggttct 2040

cctgcctcag cctccttagt agtgggatta caggtgcccg ctactatgcc cggctaattt 2100

ttcttttggt atttttagta gaaatggggt ttcaccatgt tggccaggct ggtctcaaac 2160

tcctgacctc aagtgagctg actgccttgg cctcccagag tgctgggatt agtcgtcatc 2220

ttttgttaaa ccaggatttg atttttttct tttcttttct tttcttttct tttttttttt 2280

ttttgagaca gagtctctct ctgttgccca ggctggagtg cagtggcaca atctcggctc 2340

actgcagcct ccgcctgccg ggtcaagcga ttctcctacc tcagcctcct cagtagctga 2400

gattacaggc atgcaccacc atgcccggct aatttttttg tgtttttagt agagatgggg 2460

tttcaccgtg ctggccaggc tggtctagaa ctcctgactg caaatgatca gcccgcctca 2520

gccacccaaa gtgttgggat tacaggtgtg agccactgtg cccagcgtga tttttttttt 2580

tttttaaagc aaacttgtcc tttggttttg cagaacaggc ctgctccctc tcatctagcc 2640

catcatttct tggggcctga accccagtgg tccaaagtat tgcttgtgaa atttaaaaaa 2700

tgtgaatatg atgtggggat gggcctcttc tacattacct tggcccaggg ggatcagctg 2760

gctgggagga ttagtgagca cctctgtatt ttgaggtctg agtcttctgg agctgtgtag 2820

ttaatcttcg gtttctgata acccctgggt ccatctggcc atcagcctca gcagtgagca 2880

aagcaatacc atactcattt ctatgttcct gttccttcct ctgctcctcc tttggagaag 2940

caataattca tgggggatga tacagtagca ctttacaaat ggctccatgt cattcatccc 3000

aggggccata atctcttgca ccacctattc ttacttcctg ttcagctcct ttacagcttt 3060

tattttcaac tgcttcccaa cttggtgggg cctcctttaa ggatgagcca atagtaagaa 3120

tgtggctgta atcagcagag acccctctga ggggtatctg ttctgcagcc cctagtgaaa 3180

tcatgtgatg tgagacagaa acctaaacat ggtacttgat tctaaacctg tgccagtcta 3240

tagcctctgc ctccccaagc agagctcaag ccaaacgctt ctgtcctctt tccttctgca 3300

ttaacccttt gctgatcctc aggggccact cccccaacac ccctgtactt gggtgaggga 3360

tgttggacag agcctgtttt catgtactgc aggtgggggt gtgctgacat gtttgctctt 3420

ggttgatgga gaaggtacag aggccaggga gtgaaaatgg ttgacagaag agggaagagt 3480

taggtgtctc atagtcactc atagtggggt ggtcaggggt aatggcatct ccccacttta 3540

ggcttctcaa acagactttt gacacctctc aagttcagag ctctgatgtg gaaagacagg 3600

aggtgtgggg aaggaggggg atttcgtgtg tttgcatgag tgtgcgcttc aggccttggg 3660

agttggcaag agggagggaa ggaaggagag caaaatcttc ggaaggtgtt tcttgtacct 3720

gagggatcct gccctgaatc tccatagtct ccactgtgaa ctgaggaggg gaggggtgtg 3780

ctggggaata aatcttgtat gagaacaatc tttaaaaa 3818

<210> 76
<211> 20
<212> DNA
<213> Artificial Sequence

<220>
<223> Oligonucleotide

<400> 76
ctcctgcctg catgttctct 20

<210> 77
<211> 16
<212> DNA
<213> Artificial sequence

<220>
<223> Oligonucleotide

<400> 77
tgttgttgga gcctgg 16

<210> 78
<211> 19
<212> DNA
<213> Artificial sequence

<220>
<223> Oligonucleotide

<400> 78
acagaggggt ccactctcc 19

<210> 79
<211> 73
<212> DNA
<213> Artificial sequence

<220>
<223> Oligonucleotide

<400> 79
ctcctgcctg catgttctct ctgttgttgg agcctggagc cttgctctct gggcacagag 60

gggtccactc tcc                                                          73


<210>  80
<211>  22
<212>  DNA
<213>  Artificial sequence

<220>
<223>  Oligonucleotide

<400>  80
gaaacgatgg gacctcagtc tt                                                22


<210>  81
<211>  17
<212>  DNA
<213>  Artificial sequence

<220>
<223>  Oligonucleotide

<400>  81
cttcagcaga ggacttg                                                      17


<210>  82
<211>  25
<212>  DNA
<213>  Artificial sequence

<220>
<223>  Oligonucleotide

<400>  82
tattttgtat ttggcaactg tgggc                                             25


<210>  83
<211>  65
<212>  DNA
<213>  Artificial sequence

<220>
<223>  Oligonucleotide

<400>  83
gaaacgatgg gacctcagtc ttcttcagca gaggacttga tattttgtat ttggcaactg       60

tgggc                                                                   65


<210>  84
<211>  20
<212>  DNA
<213>  Artificial sequence

<220>
<223>  Oligonucleotide

<400>  84
ccccttccta ctcccagaca                                                   20

<210>  85
<211>  16
<212>  DNA
<213>  Artificial sequence

<220>
<223>  Oligonucleotide

<400>  85
caccctcgct tcagcc                                                       16


<210>  86
<211>  23
<212>  DNA
<213>  Artificial sequence

<220>
<223>  Oligonucleotide

<400>  86
cagtttcctc atctgtccag tgg                                               23


<210>  87
<211>  62
<212>  DNA
<213>  Artificial sequence

<220>
<223>  Oligonucleotide

<400>  87
ccccttccta ctcccagaca cccaccctcg cttcagccac agtttcctca tctgtccagt      60

gg                                                                      62


<210>  88
<211>  17
<212>  DNA
<213>  Artificial sequence

<220>
<223>  Oligonucleotide

<400>  88
gccttccctg cctgtgg                                                      17


<210>  89
<211>  20
<212>  DNA
<213>  Artificial sequence

<220>
<223>  Oligonucleotide

<400>  89
ccacttttaa tgggtcctcc                                                   20

<210>  90
<211>  17
<212>  DNA
<213>  Artificial sequence

<220>
<223>  Oligonucleotide

<400>  90
caacggggtc agggagg                                                      17


<210>  91
<211>  68
<212>  DNA
<213>  Artificial sequence

<220>
<223>  Oligonucleotide

<400>  91
gccttccctg cctgtggggg tcatgctgcc acttttaatg ggtcctccac ccaacggggt      60

cagggagg                                                                68


<210>  92
<211>  25
<212>  DNA
<213>  Artificial sequence

<220>
<223>  Oligonucleotide

<400>  92
acgtctggtt caaagagttg gatat                                             25


<210>  93
<211>  21
<212>  DNA
<213>  Artificial sequence

<220>
<223>  Oligonucleotide

<400>  93
taacttccag gagttcctca t                                                 21


<210>  94
<211>  20
<212>  DNA
<213>  Artificial sequence

<220>
<223>  Oligonucleotide

<400>  94
gtgataaaga tgggcgtggc                                                   20

<210>  95
<211>  88
<212>  DNA
<213>  Artificial sequence

<220>
<223>  Oligonucleotide

<400>  95
acgtctggtt caaagagttg gatatcaaca ctgatggtgc agttaacttc caggagttcc      60

tcattctggt gataaagatg ggcgtggc                                          88


<210>  96
<211>  17
<212>  DNA
<213>  Artificial sequence

<220>
<223>  Oligonucleotide

<400>  96
ggccaccctg cctctac                                                      17


<210>  97
<211>  19
<212>  DNA
<213>  Artificial sequence

<220>
<223>  Oligonucleotide

<400>  97
tgtcaaactg tcttggctg                                                    19


<210>  98
<211>  15
<212>  DNA
<213>  Artificial sequence

<220>
<223>  Oligonucleotide

<400>  98
ggctaggggc tgggg                                                        15


<210>  99
<211>  79
<212>  DNA
<213>  Artificial sequence

<220>
<223>  Oligonucleotide

<400>  99
ggccaccctg cctctaccca accagggccc cggggcctgt tatgtcaaac tgtcttggct      60

gtggggctag gggctgggg                                                    79

<210>  100
<211>  29
<212>  DNA
<213>  Artificial sequence

<220>
<223>  Oligonucleotide

<400>  100
tgcagaactt tcaggatgac tattaattc                                          29


<210>  101
<211>  18
<212>  DNA
<213>  Artificial sequence

<220>
<223>  Oligonucleotide

<400>  101
ttgagtggtc caagcctg                                                      18


<210>  102
<211>  21
<212>  DNA
<213>  Artificial sequence

<220>
<223>  Oligonucleotide

<400>  102
tgttttgaac ccacagcagt g                                                  21


<210>  103
<211>  85
<212>  DNA
<213>  Artificial sequence

<220>
<223>  Oligonucleotide

<400>  103
tgcagaactt tcaggatgac tattaattcc tctcagatgt catttttgag tggtccaagc        60

ctgctgtttt gaacccacag cagtg                                              85


<210>  104
<211>  25
<212>  DNA
<213>  Artificial sequence

<220>
<223>  Oligonucleotide

<400>  104
taccttctac aggtcccctc tgagc                                              25

<210>  105
<211>  25
<212>  DNA
<213>  Artificial sequence

<220>
<223>  Oligonucleotide

<400>  105
tgtttcctgg tacagaccat attaa                                              25


<210>  106
<211>  25
<212>  DNA
<213>  Artificial sequence

<220>
<223>  Oligonucleotide

<400>  106
gaacaccggg ccatggctga agaag                                              25

## Description

### INTRODUCTION

Cytokine targeting drugs such as TNFα-blocking agents (herein after referred to as "TBA") are increasingly used in the treatment of various inflammatory diseases. The first indications in which such TBA were approved are rheumatoid arthritis and Crohn's disease. Rheumatoid arthritis (RA) is a chronic, progressive, debilitating auto-immune disease of largely unknown etiology that affects approximately 1% of the population (1). RA is characterized by chronic inflammation of the synovium, which ultimately leads to joint damage, pain and disability (2). The clinical spectrum of RA is heterogeneous, ranging from mild to severe, with variability in secondary organ system involvement. Disease heterogeneity is further illustrated by the current variation in treatment response rates. First line treatment is usually initiated with so called disease-modifying anti-rheumatic drugs (DMARDs), such as methotrexate (MTX). Approximately 30% of patients display a suboptimal response or intolerance to traditional DMARDs (3). In these patients, second line treatment is initiated with "biologics", agents that block molecules or cells thought to be instrumental to disease progression, such as tumor necrosis factor-α (TNFα) and interleukin-1 (IL-1) or B and T-cells. There are indeed nine biologic agents currently available, each with overlapping or unique mechanisms of action (4). The response rates to such treatments vary widely, with a great number of patients remaining refractory to treatment or demonstrating only partial improvement (5). The incomplete understanding of drug mechanisms of action together with disease heterogeneity means that there are no methods of identifying patient suitability for the various biologics prior to the initiation of the treatment. Establishing a rational basis on which to select patients for specific biologics would help patients to be treated more efficiently; those that would be likely to respond would initiate the biologic in question whereas those unlikely to respond could be provided with another treatment.

In absence of reliable literature on efficacy and safety of biologics and given the percentage of patients that do not respond or experience severe adverse effects, the destructive nature of RA, and the societal costs of inefficacious biological treatments, there is a strong need to make predictions on success before starting the therapy. A clinically or radiographic-based test will most probably assess conditions too late for protecting joints from irreversible destruction. Ideally, a molecular biomarker signature as a predictor for therapy responsiveness should be obtained prior to the start of therapy in a readily available bio-sample, such as peripheral blood. Given the systemic nature of RA and the communication between the systemic and organ-specific compartments, the peripheral blood may not directly have implications for the understanding of disease pathogenesis, but it is especially suitable to analyze gene expression profiles that provide a framework to select clinically relevant biomarkers. Furthermore, blood-based tests remain less invasive for the patients than synovial tissue-based tests.

Ultimately, this may lead to a personalized form of medicine, whereby the best suited therapy will be applied to an individual patient.

The same is true for other inflammatory diseases for which TBA have been approved or for which preliminary results indicate that TBA might be a useful treatment. For the moment, TBA have been approved, in addition to the treatment of RA or Crohn's disease, for the treatment of ankylosing spondylitis, psoriatic arthritis, plaque psoriasis, and ulcerative colitis (see notably FDA labels of infliximab and etanercept). In addition, preliminary results suggest that TBA may be useful in the treatment of several other inflammatory diseases, such as vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE) (31 and 32).
In all cases, only a proportion (although sometimes a high proportion) of patients treated with TBA display a clinical response to the treatment (see notably FDA labels of infliximab (Remicade®) and etanercept (Enbrel®)). For all diseases in which TBA may be useful, it would thus be very helpful to be able to predict the capacity of a subject to respond or not to TBA treatment.

A very powerful way to gain insight into the molecular signatures underlying pathophysiological processes has arisen from DNA microarray technology, which allows the identification of the fraction of genes that are differentially expressed in blood and tissue samples among patients with clinically defined disease. These differentially expressed genes may provide insight into biological pathways contributing to disease and represent classifiers for early diagnosis, prognosis, and response prediction.

Several pitfalls were experienced using this multistage and relatively expensive technology, which highly depends on perfectly standardized conditions. Factors that might influence the sensitivity and reproducibility range from sample differences, variation in amount and quality of starting RNA material, amplification and labeling strategies and dyes, to probe sequence and hybridization conditions. In addition, the lack of standardized approaches for normalization and usage of data analysis algorithms could influence the outcome. Furthermore, most microarray studies are not prospectively planned and often do not have detailed protocols, but rather tend to make use of existing samples. Therefore, verification of results is an essential step in microarrays studies and quality criteria have to be set.

Several groups have explored the possibility of identifying molecular traits (single nucleotide polymorphisms, gene expression etc.) capable of classifying patients according to their response to treatment based on retrospective analyses of biological samples (synovium or peripheral blood) collected at treatment baseline. In particular, much interest has been paid to the TNFα-blocking agent Infliximab, with the first report several years ago by T Lequerré and colleagues of gene expression-based prediction of response to therapy (6). Since then, several other groups have similarly reported on large-scale gene expression analyses of peripheral blood as a means to predict response to Infliximab (7) (8) (9). All of these studies reported on differentially expressed genes and combinations thereof for the prediction of response to therapy.

These studies provided important proof of concept for the prediction of response to Infliximab at baseline of therapy. Nevertheless, as with all studies of this kind, the use of microarray technology, measuring thousands of genes simultaneously in relatively small cohorts of patients, runs the risk of over-fitting data, leading to false positive results. Moreover, the mono-centric nature of these studies may limit the relevance of the genes identified to a wider and more demographically varied population.

The present invention overcomes these drawbacks by combining information from multiple existing studies. This approach can increase the reliability and generalizability of results. Quantitative approaches in which individual studies addressing a set of related research hypothesis are statistically integrated and analyzed to determine the effectiveness of interventions (meta-analysis) showed the broad utility of applying meta-analytic approaches to genome-wide data for the purpose of biological discovery. Meta-analysis were already used to identify genes differentially expressed between two groups, to compare results obtained on different microarray platforms (cross-platform classification), to identify overlaps between samples from heterologous datasets, to identify co-expressed genes or to reconstruct gene networks.

Meta-analyses of multiple gene expression microarray datasets provide discriminative gene expression signatures that are identified and validated on a large number of microarray samples, generated by different laboratories and microarray technologies. Predictive models generated by this approach are better validated than those generated on a single data set, while showing high predictive power and improved generalization performance.

In the present invention, the meta-analysis was performed according to the stepwise approach in conducting meta-analysis on microarray datasets (1-identify suitable microarray studies; 2-extract data from studies (this step also involved getting additional information from the authors of selected studies); 3-prepare the individual datasets; 4-annotate the individual datasets; 5-resolve the many-to-many relationship between probes and genes; 6-combine the study-specific estimates; 7-analyze, present, and interpret results) described in Ramasamy *et al.* (10).

Sixty one genes differentially expressed between future responders and non responders to Infliximab therapy have thus been identified. Furthermore, two individual genes have been found to be highly correlated to the Infliximab responsive or non-responsive phenotype of tested subjects, and several combinations of a minimum number of genes are proposed as being predictive of the primary (week 14 and week 22) response to anti-TNF treatment in RA patients. These combinations comprise genes that are known to be involved in inflammatory or immune processes rather than in the metabolism pathway of Infliximab, which clearly gives a rational for their general usefulness for predicting TNFα-blocking agents (TBA) responsive or non-responsive phenotype of subjects suffering from other inflammatory diseases, notably those for which TBA have been approved or have been shown to be useful in preliminary studies.

### DETAILED DESCRIPTION OF THE INVENTION

The invention thus relates to a method for the in vitro diagnosis or prognosis of a cytokine targeting drug (hereafter referred to as CyTD, such as aTNFα-blocking agent, hereafter referred to as TBA) responding or non-responding phenotype, comprising:
(a) determining from a biological sample of a subject suffering from an inflammatory disease an expression profile comprising or consisting of:
   (i) the gene MAPK14; or the genes MAPK14 and S100A9; or the genes MAPK14 and GNLY; or the 61 genes of Table 2, or
   (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (iii) Equivalent Expression Profile of anyone of the expression profiles of (i) and (ii),
   and optionally one or more housekeeping gene(s),
(b) comparing the obtained expression profile with at least one reference expression profile, and
(c) determining the CyTD responding or non-responding phenotype from said comparison.

The invention also relates to a method for designing a CyTD treatment for a subject suffering from an inflammatory disease, said method comprising:
(a) determining from a biological sample of said subject an expression profile comprising or consisting of:
   (i) the gene MAPK14; or the genes MAPK14 and S100A9; or the genes MAPK14 and GNLY; or the 61 genes of Table 2, or
   (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (iii) Equivalent Expression Profile of anyone of the expression profiles of (i) and (ii),
   and optionally one or more housekeeping gene(s),
(b) comparing the obtained expression profile with at least one reference expression profile,
(c) determining the CyTD responding or non-responding phenotype from said comparison, and
(d) designing the dose of CyTD treatment according to said identified CyTD responding or non-responding phenotype.

The invention is also drawn to a method of treatment of a subject suffering from an inflammatory disease with a CyTD, comprising:
(a) determining from a biological sample of the said subject the presence of a CyTD responding or non-responding phenotype using a method according to the invention, and
(b) adapting the CyTD treatment in function of the result of step (a).

Said adaptation of the CyTD treatment may consist in:
- a reduction or suppression of the said CyTD treatment if the subject has been diagnosed as CyTD non-responding, or
- the continuation of the said CyTD treatment if the subject has been diagnosed as CyTD responding.

The invention also refers to a new use of a CyTD in the treatment of an inflammatory disease, comprising the steps of:
(a) determining from a biological sample of a subject suffering from an inflammatory disease the presence of a CyTD responding or non-responding phenotype using a method according to the invention, and
(b) determining the dose of CyTD to administer with respect to the result of step (a).

Optionally; the dose of CyTD determined in step (b) is administered to the subject.

The invention thus relates to a CyTD, for use in treating an inflammatory disease, wherein the CyTD is administered to a subject suffering from said inflammatory disease who has been diagnosed and/or prognosed as responsive using a method according to the invention.

The invention also relates to the use of a CyTD for preparing a drug for the treatment of an inflammatory disease in subjects suffering from said inflammatory disease who have been diagnosed and/or prognosed as responsive using a method according to the invention.

In all the present description, an "inflammatory disease" refers to a disease involving uncontrolled inflammation processes leading to body damages, and includes any disease generally considered as inflammatory diseases by those skilled in the art. Advantageously, said inflammatory disease is known to involve, at least in some cases, a pathogenic inflammatory cytokine (IL-1, IL-6, IL15, IL-17, IL-18, IL-23 or TNF-α) secretion. The methods according to the invention then permit to diagnose the presence of such a pathogenic inflammatory cytokine secretion in a tested subject, to predict his/her capacity to respond to a CyTD treatment, and thus to adapt his/her treatment in view of his CyTD responding/non-responding phenotype. Even more advantageously, said inflammatory disease is known to involve, at least in some cases, a pathogenic TNF-α secretion. The methods according to the invention then permit to diagnose the presence of such a pathogenic TNF-α secretion in a tested subject, to predict his/her capacity to respond to a TBA treatment, and thus to adapt his/her treatment in view of his TBA responding/non-responding phenotype. Such inflammatory diseases may be of autoimmune or non-autoimmune origin. Non limiting examples of inflammatory diseases for which the methods and kits according to the invention are useful, in particular for determining a TBA responding or non-responding phenotype, include rheumatoid arthritis (RA), Crohn's disease, ankylosing spondylitis, psoriatic arthritis, plaque psoriasis, ulcerative colitis, vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE). An advantageous group of diseases for which the methods of the invention are useful are those in the treatment of which TBA have been approved: rheumatoid arthritis (RA), Crohn's disease, ankylosing spondylitis, psoriatic arthritis, plaque psoriasis, ulcerative colitis. The methods according to the invention are particularly useful for RA-suffering patients for determining a TBA responding or non-responding phenotype.

For rheumatoid arthritis (RA), since first line treatment is usually initiated with so called disease-modifying anti-rheumatic drugs (DMARDs), the invention also refers to a method of treatment of an RA-suffering subject, comprising the steps of:
(a) administering a therapeutic dose of a DMARD to the said subject suffering from RA,
(b) determining from a biological sample of the said RA-suffering subject the presence of a CyTD responding or non-responding phenotype using a method according to the invention, and
(c) determining the dose of CyTD to administer with respect to the result of step (b).

Thus the invention also refers to a combination of a DMARD and a CyTD for the treatment of RA, comprising the steps of:
(a) administering a therapeutic dose of a DMARD to a subject suffering from RA,
(b) determining from a biological sample of the said RA-suffering subject the presence of a CyTD responding or non-responding phenotype using a method according to the invention, and
(c) determining the dose of CyTD to administer with respect to the result of step (b).

Optionnally; the dose of CyTD determined in step (c) is administered to the subject.

In a preferred embodiment, the DMARD is methotrexate (MTX).

By "cytokine targeting drug" or "CyTD", it is meant any molecule neutralizing a cytokine signalling, notably by binding to and neutralizing the cytokine or its receptor. Such a binding and neutralizing molecule may notably be an antibody or a fragment thereof specific for said cytokine or cytokine receptor, cytokine receptor antagonists, or any other molecule, such as a recombinant protein, binding to and neutralizing said cytokine or cytokine receptor. Said CyTD preferably targets an inflammatory cytokine such as IL-1, IL-6, IL-15, IL-17, IL-18, IL-23 or TNF-α or a receptor of such inflammatory cytokines. Molecules targeting IL-1 signalling include monoclonal antibodies to IL-1, such as Canakinumab (commercial name Ilaris®), a human anti-IL-1β monoclonal antibody; antagonists of IL-1 receptor such as anakinra (commercial name Kineret®), and a fusion protein between lgG1 Fc portion and ligand-binding domains of human IL-1 RI and IL-1AcP such as Rilonacept (nom commercial Arcalyst™) Molecules targeting II-6 signalling notably include Tocilizumab, an anti-IL-6R monoclonal antibody. Molecules targeting 11-15 signalling notably include HuMax-IL-15 (AMG 714), an anti-IL-15 monoclonal antibody. Molecules targeting II-17 signalling notably include AIN457, an anti-IL-17A monoclonal antibody. In all the present description, a preferred embodiment of a CyTD is a "TNFα-blocking agent" or "TBA".

By "TNFα-blocking agent" or "TBA", it is herein meant a biological agent which is capable of neutralizing the effects of TNFα. Said agent is a preferentially a protein such as a soluble TNFα receptor, e.g. Pegsunercept, or an antibody. In a further preferred embodiment, the said agent is a monoclonal antibody. In an even further preferred embodiment, the said agent is selected in the group consisting of Etanercept (Enbrel®), Infliximab (Remicade®), Adalimumab (Humira®), and Certolizumab pegol (Cimzia®). In an even more preferred embodiment, the said agent is Infliximab.

In a particularly preferred embodiment of any method according to the present invention, the inflammatory disease is rheumatoid arthritis and the CyTD is Infliximab, a particular TBA.

According to the present invention, a "CyTD responding phenotype" is defined as a response state of a subject to the administration of a CyTD. A "response state" means that the said subject (referred to as a CyTD responding subject or a responding subject or a responsive subject: for the purpose of this application, these terms are similar) responds to the treatment, i.e. that the treatment is efficacious in the said subject. The definition of response is an improvement in clinical symptoms. The quantification of such response is made according to ACR20, ACR50, ACR70 criteria (11) and/or EULAR criteria at weeks 14 or 22 or change in DAS28 >1.2. Even more preferred is EULAR response criteria at 14 weeks. These criteria (31) have been established by organizations regrouping the professionals in the field (ACR: American College of Rheumatology; EULAR: European League Against Rheumatism). These criteria are thus well known to the skilled person in the art and need not be detailed here.

In contrast, a "CyTD non-responding phenotype" refers to the absence in said subject (referred to herein as a CyTD non-responding subject or a non responding subject or a non-responsive subject: these terms should be construed in the context of this application as having the same meaning) of a state of response, meaning that said subject remains refractory to the treatment.
In a preferred embodiment of any of the above-described *in vitro* methods of diagnosis/prognosis according to the invention, the said subject is an RA-suffering subject. An "RA-suffering subject" is a subject fulfilling the American College of Rheumatology (ACR) criteria for RA (11). In one further embodiment, the said subject is not treated with a CyTD; in another further embodiment, the said subject is treated with a CyTD.

It will easily be conceived that when the said subject is not treated with a CyTD, the methods of the invention permit a prognosis of the responsiveness/non responsiveness of the said subject. Thus, in this embodiment, the method of the invention allows the person skilled in the art to prognose (i.e. to identify) the subjects susceptible of responding to the CyTD treatment. This is important because of the destructive nature of RA and the societal costs of inefficacious biological treatments. Moreover, since this embodiment of the invention allows for identification of non responsive subjects before any treatment is initiated, the risks for one treated subject to encounter severe adverse effects are greatly diminished.

When the subject according to the invention is treated with a CyTD, the methods of the invention are useful for diagnosing if a subject responds to the said CyTD, and whether the said subject would thus benefit from a continuation of the said treatment. Moreover, they are useful for diagnosing subjects who are not responding to the treatment, i.e. who are refractory to the CyTD, and should thus swiftly shifted to another therapy. In regard of the debilitating nature of RA, this achievement is crucial. In particular, the methods of the invention allow for a diagnosis at week 14 or 22 after the beginning of the CyTD treatment.

In the present description, what is described for CyTD also particularly applies to TBA, which is a preferred embodiment of a CyTD in any method or kit according to the invention.

A "biological sample" may be any sample that may be taken from a subject, such as a serum sample, a plasma sample, an urine sample, a blood sample, a lymph sample, or a biopsy. Such a sample must allow for the determination of an expression profile comprising or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).
Preferred biological samples for the determination of an expression profile include samples such as a blood sample, a plasma sample, a lymph sample, or a biopsy. Preferably, the biological sample is a blood sample. Indeed, such a blood sample may be obtained by a completely harmless blood collection from the patient and thus allows for a non-invasive diagnosis of a CyTD responding or non-responding phenotype.

By "expression profile" is meant the expression levels of a group of genes comprising or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s), since these expression profiles have been demonstrated to be particularly relevant for assessing the responding/non responding phenotype of a subject. In a most preferred embodiment, the expression profile for diagnosing if the subject is responding at week 14 or week 22 after the beginning of the CyTD treatment comprises or preferably consists of the genes IL2RB, S100A9, and CASP5, or of the gene S100A9 or Equivalent Expression Profile thereof, and optionally one or more housekeeping gene(s). In another most preferred embodiment, the expression profile for diagnosing responsiveness at week 14 or week 22 after the beginning of the CyTD treatment (in particular a TBA treatment) comprises or preferably consists of the genes MAPK14 and S100A9, or Equivalent Expression Profile thereof, and optionally one or more housekeeping gene(s). In yet another most preferred embodiment, the expression profile for diagnosing responsiveness at week 14 or week 22 after the beginning of the CyTD treatment (in particular a TBA treatment) comprises or preferably consists of the gene S100A9, or Equivalent Expression Profile thereof, and optionally one or more housekeeping gene(s). In still another most preferred embodiment, the expression profile for diagnosing responsiveness at week 14 or week 22 after the beginning of the CyTD treatment (in particular a TBA treatment) comprises or preferably consists of the genes MAPK14 and GNLY, or Equivalent Expression Profile thereof, and optionally one or more housekeeping gene(s).

The determination of the presence of a CyTD responding or non-responding phenotype is carried out thanks to the comparison of the obtained expression profile with at least one reference expression profile in step (b).

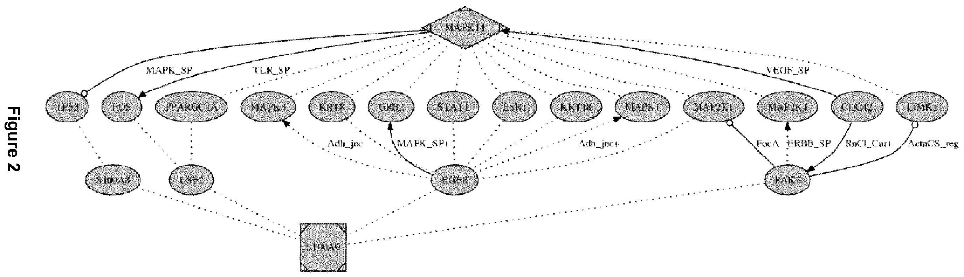

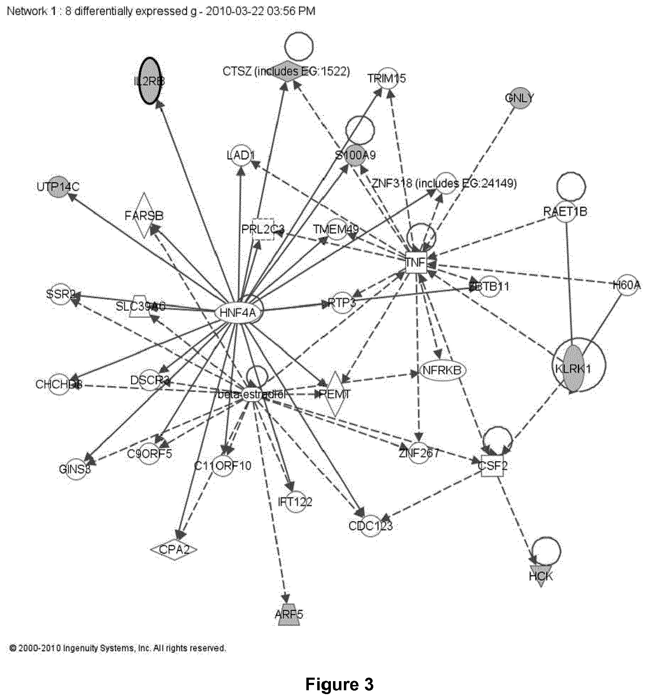

The term "Equivalent Expression Profile" herein refers to expression profiles of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
wherein the addition, deletion or substitution of some of the genes does not change significantly the reliability of the test and is considered as an "acceptable expression profile". As an example, the addition or substitution of some of the genes of the sets described in the present invention by other genes belonging to the same metabolic pathway should also be considered as an equivalent expression profile. For example S100A8 is equivalent to S100A9, and any above mentioned (i) or (ii) expression profile in which S100A9 is replaced by S100A8 (i.e. expression profiles comprising or consisting of the genes MAPK14 and S100A8; 8 or the 61 genes of Table 2 in which S100A9 is replaced by S100A8; or the gene S100A8; or the genes S100A8, IL2RB, and CASP5; or the genes S100A8, IL2RB, KLRK1, HCK, and GNLY; or the genes S100A8, IL2RB, KLRK1, HCK, GNLY, CTSZ, ARF5, and UTP14C) should be considered as Equivalent Expression Profiles of above mentioned (i) and (ii) expression profiles and thus as included in the scope of the present invention. More generally are considered as Equivalent Expression Profiles, any expression profile wherein some of the genes are replaced by genes belonging to the same biological network, such as described in figures 2 and 3 below.

The term "Acceptable Expression Profile" herein refers to an expression profile which is capable of correctly classifying at least 60% of the analyzed samples, preferably 65%, and more preferably 70%, has a sensitivity and specificity of at least 60% preferably 65%, and more preferably 70.The sensitivity value is defined as the ratio of the number of patients actually clinically responding to the CyTD treatment and classified as responding using the test according to the invention amongst all patients treated with the CyTD. Specificity measures the proportion of patients actually clinically not responding to the CyTD treatment which are correctly identified using the test according to the invention amongst all patients treated with the CyTD.

By "Best Expression Profile" is meant an expression profile which is able to correctly classify at least 80% of the analyzed samples, has either a sensitivity or a sensitivity of at least 80%.

Although the lists of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
have been determined as the Best Expression Profiles to assess responsiveness/non responsiveness, an Equivalent Expression Profile such as defined above, still permits to assess responsiveness, with an acceptable reliability. In particular embodiments, sublists of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C,
still permit to assess responsiveness with a good reliability and should be considered as Acceptable Expression Profiles.

While the expression profile used for determining the CyTD (notably TBA) responsive or non-responsive phenotype may comprise and not only consist of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s), it is preferred that the expression profiles consists or essentially consists of one of the above described (i), (ii) or (iii) expression profiles, optionally with one or more housekeeping gene(s), meaning that no more than 50, 40, 30, 25, 20, preferably no more than 15, preferably no more than 10, preferably no more than 9, 8, 7, 6, 5, 4, 3, 2, or 1 genes that are not a gene belonging one the above described (i), (ii) or (iii) expression profiles or a housekeeping gene are included in the expression profile.

By "housekeeping genes", it is meant genes that are constitutively expressed at a relatively constant level across many or all known conditions, because they code for proteins that are constantly required by the cell, hence, they are essential to a cell and always present under any conditions. It is assumed that their expression is unaffected by experimental conditions. The proteins they code are generally involved in the basic functions necessary for the sustenance or maintenance of the cell. Non-limitating examples of housekeeping genes that may be used in methods of the invention include:
- HPRT1 (hypoxanthine phosphoribosyltransferase 1),
- UBC (ubiquitin C),
- YWHAZ (tyrosine 3-monooxygenase/tryptophan 5-monooxygenase activation protein, zeta polypeptide),
- B2M (beta-2-microglobulin),
- GAPDH (glyceraldehyde-3-phosphate dehydrogenase),
- FPGS (folylpolyglutamate synthase),
- DECR1 (2,4-dienoyl CoA reductase 1, mitochondrial),
- PPIB (peptidylprolyl isomerase B (cyclophilin B)),
- ACTB (actin β),
- PSMB2 (proteasome (prosome, macropain) subunit, beta type, 2),
- GPS1 (G protein pathway suppressor 1),
- CANX (calnexin),
- NACA (nascent polypeptide-associated complex alpha subunit),
- TAX1 BP1 (Tax1 (human T-cell leukemia virus type I) binding protein 1), and
- PSMD2 (proteasome (prosome, macropain) 26S subunit, non-ATPase, 2).

Preferably, the number of housekeeping genes used for normalizaztion in methods according to the invention is comprised between one and five with a preference for three.

The determination of the presence of a responsive or non responsive phenotype is carried out thanks to the obtained expression profile with at least one reference profile in step (b).

A "reference expression profile" is a predetermined expression profile, obtained from a biological sample from a subject with a known particular response state. In order to have this comparison meaningful and robust, the "reference expression profile" is determined by the training of an algorithm resulting from the following steps:
- the collection of the expression profiles of biological samples obtained from responsive and non-responsive patients,
- the training of an algorithmon the expression profiles of the aforementioned patients for classifying said expression profiles as responsive and non-responsive phenotypes.

The "comparison of an obtained expression profile with a reference expression profile" can be understood as the application of the adjusted algorithm on the obtained expression profile.

In particular embodiments, the reference expression profile used for comparison with the test sample in step (b) may have been obtained from biological samples from CyTD responsive subjects ("CyTD responsive reference expression profile" or "responsive reference expression profile"; as used herein these expressions are synonymous), and/or from biological samples from CyTD non-responsive subjecst ("CyTD non-responsive reference expression profile" or "non-responsive reference expression profile" ; as used herein these expressions have the same meaning).

Preferably, at least one reference expression profile is a CyTD responsive reference expression profile. Alternatively, at least one reference expression profile may be a CyTD non-responsive reference expression profile. More preferably, the determination of the presence or absence of a CyTD responsive phenotype is carried out by comparison with at least one responder and at least one non-responder reference expression profiles. The diagnosis or prognostic may thus be performed using one responsive reference expression profile and one non-responsive reference expression profile. Advantageously, to get a stronger diagnosis or prognostic, said diagnosis or prognostic is carried out using several responsive reference expression profiles and several non-responsive reference expression profiles.

The comparison of a tested subject expression profile with said reference expression profiles, which permits prediction of the tested subject's clinical response based on his/her expression profile, can be done by those skilled in the art using statistical models or machine learning technologies. The PLS (Partial Least Square) regression is particularly relevant to give prediction in the case of small reference samples. The comparison may also be performed using Support Vector Machines (SVM), logistic regression, Linear Discriminant Analysis, Random Forests, k-NN (Nearest Neighbour) or PAM (Predictive Analysis of Microarrays) statistical methods.

The expression profile may be determined by any technology known by a man skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of protein produced by each of the genes.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a preferred embodiment, the expression profile is determined using quantitative PCR. Quantitative, or real-time, PCR is a well known and easily available technology for those skilled in the art and does not need a precise description.

In a particular embodiment, which should not be considered as limiting the scope of the invention, the determination of the expression profile using quantitative PCR may be performed as follows. Briefly, the real-time PCR reactions are carried out using the TaqMan Universal PCR Master Mix (Applied Biosystems). 6 µL cDNA is added to a 9 µL PCR mixture containing 7.5 µL TaqMan Universal PCR Master Mix, 0.75 µL of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI 7900HT Fast Real-Time PCR System (Applied Biosystems). The number of template transcript molecules in a sample is determined by recording the amplification cycle in the exponential phase (cycle threshold or C_{T}), at which time the fluorescence signal can be detected above background fluorescence. Thus, the starting number of template transcript molecules is inversely related to C_{T}. The level of expression of a gene is measured using the "ΔΔCT method", briefly a gene is normalized by the value of one or a group of reference/housekeeping genes and/or by a reference sample such as a pooled sample or a commercially available reference such as the qPCR Human Universal Reference cDNA, random primed ; Ozyme ; ref 639654.

In another preferred embodiment, the expression profile is determined by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In a preferred embodiment, the nucleic microarray is an oligonucleotide microarray comprising or consisting of oligonucleotides specific for:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).
Preferably, the oligonucleotides are about 50 bases in length. It is acknowledged that the nucleic acid microarray or oligonucleotide microarray of the invention encompasses the microarrays specific for an Equivalent Expression Profile as defined above.

Suitable microarray oligonucleotides specific for any gene of Table 2 may be designed, based on the genomic sequence of each gene (see Table 2 Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

In another embodiment, the expression profile is determined by the use of a protein microarray.

In a particular embodiment of a method according to the invention, said method may further comprise determining at least one additional parameter useful for the diagnosis. Such "parameters useful for the diagnosis" are parameters that cannot be used alone for a diagnosis but that have been described as displaying significantly different values between responsive subjects and subjects who are clearly refractory and may thus also be used to refine and/or confirm the diagnosis according to the above described method according to the invention. They may notably include relevant clinical parameters depending on the inflammatory disease. For rheumatoid arthritis (RA), such clinical parameters include an assessment of the subject's pain, duration of morning stiffness, the number of swollen joints, the number of painful joints etc. Preferably, the parameters useful or diagnosis are determined from a non invasive biological sample of the subject. In particular, for RA, they may be selected from standard biological parameters specific for RA. According to the invention, "standard biological parameters specific for RA" are biological parameters usually used by clinicians to monitor the efficacy of a treatment of RA. These standard biological parameters specific for RA or autoimmune diseases usually comprise serum or plasma concentrations of particular proteins which are well known of those skilled in the art. The said standard biological parameters specific for RA can be determined by tests which include the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test. These tests are well known to the person skilled in the art and not be detailed here. They may be used on their own or in combination.

Such additional parameters may be used to confirm the diagnosis obtained using the expression profile comprising or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

The invention further concerns a kit for the *in vitro* diagnosis of a CyTD responsive or non responsive phenotype, comprising at least one reagent for the determination of an expression profile comprising, or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

By "a reagent for the determination of an expression profile" is meant a reagent which specifically allows for the determination of said expression profile, i.e. a reagent specifically intended for the specific determination of the expression level of the genes comprised in the expression profile. This definition excludes generic reagents useful for the determination of the expression level of any gene, such as Taq polymerase or an amplification buffer, although such reagents may also be included in a kit according to the invention.

In a preferred embodiment of a kit according to the invention, said kit is dedicated to the *in vitro* diagnosis of a CyTD responsive or non responsive phenotype based on expression profiles comprising or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

By "dedicated", it is meant that reagents for the determination of an expression profile in the kit of the invention essentially consist of reagents for determining the expression level of the above (i), (ii) or (iii) expression profiles, optionally with one or more housekeeping gene(s), and thus comprise a minimum of reagents for determining the expression of other genes than those mentioned in above described (i), (ii) or (iii) expression profiles and housekeeping genes. For instance, a dedicated kit of the invention preferably comprises no more than 50, 40, 30, 25, 20, preferably no more than 15, preferably no more than 10, preferably no more than 9, 8, 7, 6, 5, 4, 3, 2, or 1 reagent(s) for determining the expression level of a gene that does not belong to one of the above described (i), (ii) or (iii) expression profiles and that is not a housekeeping gene.

Such a kit for the in vitro diagnosis of a CyTD responsive or non responsive phenotype may further comprise instructions for determination of the presence or absence of a responsive phenotype.

Such a kit for the *in vitro* diagnosis of a responsive phenotype may also further comprise at least one reagent for the determining of at least one additional parameter useful for the diagnosis such as standard biological parameters. In particular, the said reagent is useful for performing of any of the following tests: the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test.

In any kit for the *in vitro* diagnosis of a responsive phenotype according to the invention, the reagent(s) for the determination of an expression profile comprising, or consisting of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s), preferably include specific amplification primers and/or probes for the specific quantitative amplification of transcripts of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s), and/or
a nucleic microarray for the detection of:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).
The determination of the expression profile may thus be performed using quantitative PCR and/or a nucleic microarray, preferably an oligonucleotide microarray, and/or protein microarrays.

In addition, the instructions for the determination of the presence or absence of a CyTD (notably TBA) phenotype preferably include at least one reference expression profile, or at least one reference sample for obtaining a reference expression profile. In a preferred embodiment, at least one reference expression profile is a responsive expression profile. Alternatively, at least one reference expression profile may be a non responsive expression profile. More preferably, the determination of the level of responsiveness is carried out by comparison with both responsive and non-responsive expression profiles as described above.

The invention is also directed to a nucleic acid microarray comprising or consisting of nucleic acids specific for:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).
Said nucleic acid microarray may comprise additional nucleic acids specific for additional genes and optionally one or more housekeeping gene(s), but preferably consists of a maximum of 500, 400, 300, 200 preferably 100, 90, 80, 70 more preferably 60, 50, 45, 40, 35, 30, 25, 20, 15, 10, or even less (for instance 9, 8, 7, 6, 5, 4, 3, 2 or 1) distinct nucleic acids.

In a preferred embodiment, said nucleic acid microarray comprises no more than 50, 40, 30, 25, 20, preferably no more than 15, preferably no more than 10, preferably no more than 9, 8, 7, 6, 5, 4, 3, 2, or 1 distinct nucleic acids specific for a gene that does not belong to one of the above described (i), (ii) or (iii) expression profiles and that is not a housekeeping gene.

Advantageously, said microarray consists of nucleic acids specific for:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

In a preferred embodiment, said nucleic acid microarray is an oligonucleotide microarray comprising or consisting of oligonucleotides specific for:
(i) the gene MAPK14; or the genes MAPK14 and S100A9; or the genes MAPK14 and GNLY; or the 61 genes of Table 2, or
(ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
(iii) Equivalent Expression Profile of anyone of the expression profiles of (i) and (ii),
and optionally one or more housekeeping gene(s).

The present invention also relates to the following embodiments:
1. A method for the *in vitro* diagnosis or prognosis of a cytokine targeting drug (CyTD) responding or non-responding phenotype, comprising:
   (b) determining from a biological sample of a subject suffering from an inflammatory disease an expression profile comprising or consisting of:
      (i)the gene MAPK14; or the genes MAPK14 and S100A9; or the genes MAPK14 and GNLY; or the 61 genes of Table 2, or
      (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
      (iii) Equivalent Expression Profile of anyone of the expression profiles of (i) and (ii),
      and optionally one or more housekeeping gene(s)
   (c) comparing the obtained expression profile with at least one reference expression profile, and
   (d) determining the responding or non-responding phenotype from said comparison.
2. The method of embodiment 1, wherein the obtained expression profile is compared to at least one reference responding and/or non-responding expression profile in step (b).
3. The method of embodiment 2, wherein the obtained expression profile is compared in step (b) to at least one reference responding expression profile and at least one reference non-responding expression profile.
4. The method of anyone of embodiments 1 to 3, wherein the expression profile is determined by measuring the amount of nucleic acid transcripts of said gene(s).
5. The method of embodiment 4, wherein the expression profile is determined using quantitative PCR or an oligonucleotide microarray.
6. The method of anyone of embodiments 1 to 3, wherein the expression profile is determined using a genomic microarray or a proteic microarray.
7. The method according to anyone of embodiments 1 to 6, wherein said biological sample is a blood sample.
8. The method according to anyone of embodiments 1 to 7, wherein said CyTD is a TNF-a blocking agent (TBA).
9. The method according to anyone of embodiments 1 to 8, wherein said inflammatory disease is selected from the group consisting of rheumatoid arthritis (RA), Crohn's disease, ankylosing spondylitis, psoriatic arthritis, plaque psoriasis, ulcerative colitis, vasculitis; Wegener's granulomatosis; sarcoidosis; adult-onset Still's disease, polymyositis/dermatomyositis, and systemic lupus erythematosus (SLE).
10. The method of embodiment 9, wherein said inflammatory disease is rheumatoid arthritis.
11. The method according to embodiment 10, further comprising determining at least one additional parameter, said additional parameter being determined by a test selected from the Antinuclear Antibody test (ANA test), C-Reactive Protein test (CRP test), Cyclic Citrullinated Peptide Antibody test (CCP test), and the Rheumatoid Factor test.
12. A kit for the *in vitro* diagnosis of a CyTD responding or non responding phenotype, comprising at least one reagent for the determination of an expression profile comprising or consisting of:
   (i) the gene MAPK14; or the genes MAPK14 and S100A9; or the genes MAPK14 and GNLY; or the 61 genes of Table 2, or
   (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (iii) Equivalent Expression Profile of anyone of the expression profiles of (i) and (ii),
   and optionally one or more housekeeping gene(s).
13. The kit of embodiment 12, further comprising at least one reagent for determining at least one additional parameter, said reagent selected from the Antinuclear Antibody test (ANA test), C-Reactive Protein test (CRP test), Cyclic Citrullinated Peptide Antibody test (CCP test), and the Rheumatoid Factor test.
14. A nucleic acid microarray comprising nucleic acids specific for:
   (i) the gene MAPK14; or the genes MAPK14 and S100A9; or the genes MAPK14 and GNLY; or the 61 genes of Table 2, or
   (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
   (iii) Equivalent Expression Profile of anyone of the expression profiles of (i) and (ii),
   and optionally one or more housekeeping gene(s).
15. The nucleic acid microarray according to embodiment 14, which is an oligonucleotide microarray.
16. A method for designing a CyTD treatment for a subject suffering from an inflammatory disease, said method comprising:
   (a) determining from a biological sample of said subject an expression profile comprising or consisting of:
      (i) the gene MAPK14; or the genes MAPK14 and S100A9; or the genes MAPK14 and GNLY; or the 61 genes of Table 2, or
      (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
      (iii) Equivalent Expression Profile of anyone of the expression profiles of (i) and (ii),
      and optionally one or more housekeeping gene(s)
   (b) comparing the obtained expression profile with at least one reference expression profile,
   (c) determining the responding or non-responding phenotype of said subject from said comparison, and
   (d) designing a dose of CyTD treatment according to the said identified responding or non-responding phenotype.
17. A method for adapting the CyTD treatment of a subject suffering from an inflammatory disease, said method comprising:
   (a) determining from a biological sample of said subject an expression profile comprising or consisting of:
      (i) the gene MAPK14; or the genes MAPK14 and S100A9; or the genes MAPK14 and GNLY; or the 61 genes of Table 2, or
      (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
      (iii) Equivalent Expression Profile of anyone of the expression profiles of (i) and (ii),
      and optionally one or more housekeeping gene(s),
   (b) comparing the obtained expression profile with at least one reference expression profile,
   (c) determining the responding or non-responding phenotype of said subject from said comparison, and
   (d) adapting the CyTD treatment.
18. A method of treatment of an RA-suffering subject, comprising the steps of:
   (a) administering a therapeutic dose of a disease-modifying anti-rheumatic drug (DMARD) to the said subject suffering from RA,
   (b) determining from a biological sample of a RA-suffering subject an expression profile comprising or consisting of:
      (i) the gene MAPK14; or the genes MAPK14 and S100A9; or the genes MAPK14 and GNLY; or the 61 genes of Table 2, or
      (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
      (iii) Equivalent Expression Profile of anyone of the expression profiles of (i) and (ii),
      and optionally one or more housekeeping gene(s)
   (c) comparing the obtained expression profile with at least one reference expression profile,
   (d) determining the responding or non-responding phenotype of said RA-suffering subject from said comparison,
   (e) determining a dose of CyTD, and
   (f) administering the said dose of CyTD.
19. The method of embodiment 18, wherein the DMARD is methotrexate.
20. A method of treatment of a subject suffering from an inflammatory disease, comprising the steps of:
   (a) determining from a biological sample of said subject an expression profile comprising or consisting of:
      (i) the gene MAPK14; or the genes MAPK14 and S100A9; or the genes MAPK14 and GNLY; or the 61 genes of Table 2, or
      (ii) the gene S100A9; or the genes S100A9, IL2RB, and CASP5; or the genes S100A9, IL2RB, KLRK1, HCK, and GNLY; or the genes S100A9, IL2RB, KLRK1, HCK, GNLY, CTSZ, ARF5, and UTP14C, or
      (iii) Equivalent Expression Profile of anyone of the expression profiles of (i) and (ii),
      and optionally one or more housekeeping gene(s),
   (b) comparing the obtained expression profile with at least one reference expression profile,
   (c) determining the responding or non-responding phenotype of said subject from said comparison,
   (d) determining a dose of CyTD, and
   (e) administering the said dose of CyTD.
   (f)
21. The method of anyone of embodiments 16 to 20, wherein the expression profile of step (a) is determined at 14 or 22 weeks after the beginning of the CyTD treatment.
22. The method of embodiment 21, wherein the expression profile of step (a) consists of the genes IL2RB, S100A9, and CASP5, or Equivalent Expression Profiles thereof.
23. The method of embodiment 21, wherein the expression profile of step (a) consists of the genes MAPK14 and S100A9, or Equivalent Expression Profiles thereof.
24. The method of embodiment 21, wherein the expression profile of step (a) consists of the gene S100A9, or Equivalent Expression Profiles thereof.
25. The method of embodiment 21, wherein the expression profile of step (a) consists of the genes MAPK14 and GNLY, or Equivalent Expression Profiles thereof.
26. The method of anyone of embodiments 16 to 25, wherein said inflammatory disease is selected from the group consisting of rheumatoid arthritis (RA), Crohn's disease, ankylosing spondylitis, psoriatic arthritis, plaque psoriasis, ulcerative colitis, vasculitis; Wegener's granulomatosis; sarcoidosis; adult-onset Still's disease, polymyositis/dermatomyositis, and systemic lupus erythematosus (SLE).
27. The method of embodiment 26, wherein said inflammatory disease is rheumatoid arthritis.
28. The method of anyone of embodiments 16 to 24, wherein said CyTD treatment is a TBA treatment.

Having generally described this invention, a further understanding of characteristics and advantages of the invention can be obtained by reference to certain specific examples and figures which are provided herein for purposes of illustration only and are not intended to be limiting unless otherwise specified.

### DESCRIPTION OF THE FIGURES

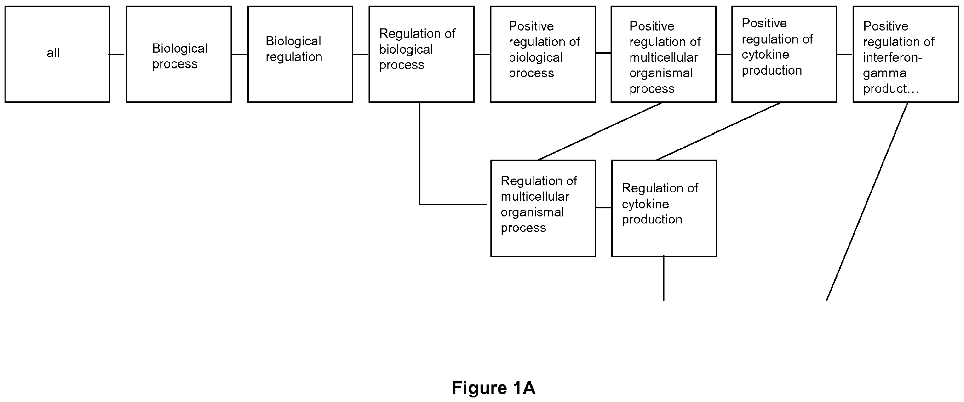

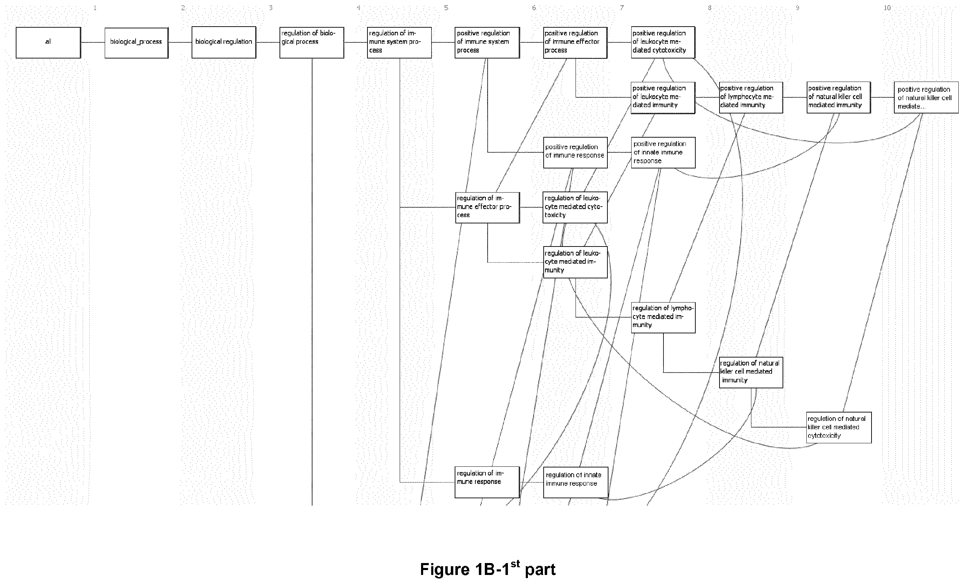

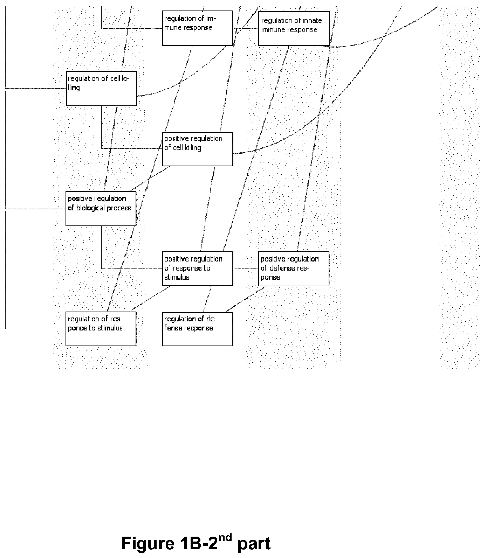

**Figure 1****:** The two most significantly enriched GO terms for the top differentially expressed 8 genes are: "Positive Regulation of interferon gamma production" (A) and "Positive regulation of natural killer cell mediated cytotoxicity" (B) (p-value <5x10⁻³).
**Figure 2****:** Pathway analysis for MAPK14 and S100A9 using PathGen
**Figure 3** **: The top differentially expressed 8 genes issued from Ingenuity® System analysis belong to a single biological network** (Cell-to-cell signalling and interaction, haematological system development and function, immune cell trafficking) In Figure 3, symbols represent different types of molecules;
   - upward triangles: phosphatase,
   - downward triangles: kinase,
   - horizontal ellipses: transcription regulator,
   - vertical ellipses: transmembrane receptor,
   - rhombuses: enzyme,
   - horizontal rectangles: ligand-dependent nuclear receptor,
   - vertical rectangles: g-protein coupled receptor,
   - simple circles: other types of molecules,
   - double circles: complex group,

Shaded shapes represent the molecules that are part of the 61 list.

The types of line represent the type of interaction:
- plain arrows: means the first molecule directly acts on the second one (such as but not limited to activation)
- dotted arrows: means the first molecule indirectly acts on the second one
- A line ending with a short segment indicates inhibition
- A simple line represents interaction (such a but not limited to protein-protein interaction)

### EXAMPLES

### Example 1: meta-analysis of two datasets (Bienkowska et al.(9) and Julia et al.(8))

### Materials and Methods

In this example, the materials and methodologies used in subsequent examples 1 to 3 are described.

*Data Identification and Data Extraction:* Studies were selected on the basis that they had been performed on RA patients naive to biologics who had started therapy with Infliximab and measurement of their response to treatment was available at 14 or 22 weeks. Large scale gene expression information had to be available at baseline (prior to treatment). Following the steps described in Ramasamy *et al.* (10), we identified four studies that matched our research criteria: Lequerre *et al.* (6), Sekiguchi *et al.* (7), Bienkowska *et al.* (9) and Julia *et al.* (8). The expression data, the phenotypes and the annotation data were all downloaded from GEO (GSE3592, GSE8350, GSE12051 and GSE15258 respectively).

All four studies identified "Gene expression signatures of response to anti-TNF therapy". Interestingly, however, no two publications used the same approach and this can partly explain the lack of overlap between the reported signatures. To make the four studies more comparable, we contacted the authors to obtain additional individual information such as the DAS28 at baseline and week 14 or week 22 to use a single definition of response (EULAR criteria - with "moderate" and "good" responders considered as responders) or detail of treatment to ensure that only Infliximab-treated patients were analyzed. We therefore reclassified patients as responders based on the EULAR definition at week 14 and week 22 and performed a binary analysis of good and moderate responders versus non-responders. This binary grouping is particularly suited for the identification of non responders. The final dataset is summarized in Table 1 and was the most homogeneous data we could obtain.

**Table 1: Summary of datasets after alignment of clinical criteria**

| **Study Reference** | **Bienkowska *et al.*** | **Julia *et al.*** | **Lequerre *et al.*** | **Sekiguchi *et al.*** |
|---|---|---|---|---|
| Sample type | whole blood | whole blood | PBMCs | whole blood |
| Microarrays | Affymetrix | Illumina | Custom | Custom |
| Number of probes | 54675 | 17454 | 13824 | 776 |
| Studied response *criteria* | EULAR @ 14wks | | | EULAR @22wks |
| Treatment | Infliximab (mono-therapy) | Infliximab (bi-therapy) | | |
| Number of R/NR | 20/8 | 37/7 | 9/4 | 15/3 |
| Mean age (R/NR) | NA | 52/57 | 56/57 | 55/53 |
| Mean RA duration (R/NR) | NA | 13/19 | 13/10 | 9.3/4.8 |
| Mean DAS28 at baseline (R/NR) | 5.4/5 | 6/5.8 | 6.1/6 | 6.2/6.2 |

Data Quality and Processing: Data from Bienkowska et al. was the only data for which we downloaded the raw .CEL files and processed them using our internal protocols (normalization was performed using GC-RMA in refiner array by GENEDATA Expressionist® (Genedata AG, Basel, Switzerland)). Six chips were flagged with quality issues due to increased distortion. However, due to the limited number of arrays available for Infliximab samples, we included them in our analysis.
Data from Lequerre et al., Julia et al. and Sekiguchi et al. were all downloaded as expression matrices, which correspond to expression values after normalization. The data by Lequerré et al. included technical replicates; we averaged the technical replicates and excluded the control samples from the analysis.

To translate probe information into gene information we used the NCBI's Geneld as provided in the respective annotation files found on GEO. When multiple probes were available, we selected probes with the same NCBI GI number across platforms. When probes differed in terms of GI, they were selected randomly. Therefore for each gene, only one probe contributed to the analysis.

Statistical Analysis: Because of the limited number of probes on the arrays used by Sekiguchi et al. and the major difference introduced by the sample source in the study by Lequerré et al. (PBMCs as opposed to whole blood), we initially performed a meta-analysis only on the Bienkowska et al. and Julia et al. data sets. We then performed the meta-analysis adding the data from Sekiguchi et al. and lastly, the data from Lequerré et al. Thus three meta-analyses were performed (examples 1, 2, and 3 respectively).
The statistical analysis was performed in R using the MetaArray package (D Ghosh and H Choi, 2009). This package implements the latent variable model described in (H Choi, 2005) as well as the integrative correlation (12). For the probability of expression (POE) estimation we used the EM estimation method. Probes were filtered on 100% presence and based on an average correlation >-0.2. Individual probes contribution was estimated using the t-test as implemented in the multtest library in R. We set the threshold for significance at 2.5 based on visual inspection of the distribution plots. The list of probes identified using this approach was then further evaluated in the individual data sets to assess their predictive performance. This was done in Genedata Expressionist® using the Fisher LDA predictor and leave-one-out cross validation. The assessment of biological processes enrichment was done in Genedata Expressionist® (Genedata AG, Basel, Switzerland) using the Gene Ontology Fisher's Exact Test function. The list of significant probes was compared to the list of probes that were tested (the common probes). Pathway analysis was performed using PathGen (available at http://dna.cs.byu.edu/pathgen).

### Results

The initial meta-analysis of the two datasets (i.e. Bienkowska *et al.* and Julia *et al.*) was performed on 4022 probes. Some heterogeneity between the two studies could be due to remaining clinical differences such as severity of disease, use of mono- versus bi-therapy or to probe differences due to the different platforms used (Affymetrix versus Illumina microarrays). Based on the t-test of the POE from the merged datasets, we ranked the genes based on their significance. **Table 2** provides the gene symbol and direction of the 61 most differentially expressed genes between responders and non responders. Interestingly this gene list overlaps only by two genes with the genes identified by Julia *et al.* and by one gene identified by Bienkowska *et al.* To assess the discriminatory performance of a group of genes we selected the top 8 genes and applied them to the individual data sets. The performance can be found in **Table 3.** This eight gene set performs extremely well in the dataset of Julia *et al.:* by LOO, 7 out of 44 samples were misclassified giving an overall error rate of 84%, the PPV was as high as 97%.

**Table 2: Ranked list of first 61 differentially expressed probes. The top genes are the most significant ones.**

| **Gene Symbol** | **Name** | **Accession Nb** | **Direction** |
|---|---|---|---|
| **IL2RB** | interleukin 2 receptor, beta | NM_000878.2 (SEQ ID NO :1) | Increased |
| **S100A9** | S100 calcium binding protein A9 | NM_002965.3 (SEQ ID NO :2) | Decreased |
| **KLRK1** | killer cell lectin-like receptor subfamily K, member 1 | NM_007360.2 (SEQ ID NO :3) | Increased |
| **HCK** | hemopoietic cell kinase | NM_001172129.1 (SEQ ID NO :4) | Decreased |
| **GNLY** | granulysin | NM_006433.3 (SEQ ID NO :5) | Increased |
| **CTSZ** | cathepsin Z | NM_001336.3 (SEQ ID NO :6) | Decreased |
| **ARF5** | ADP-ribosylation factor 5 | NM_001142272.1 (SEQ ID NO :7) | Decreased |
| **UTP14C** | U3 small nucleolar ribonucleoprotein, homolog C | NM_021645.5 (SEQ ID NO :8) | Increased |
| DRAP1 | DR1-associated protein 1 (negative cofactor 2 alpha) | NM_006442.3 (SEQ ID NO :9) | Decreased |
| SHKBP1 | SH3KBP1 binding protein 1 | NM_138392.3 (SEQ ID NO :10) | Decreased |
| IARS | isoleucyl-tRNA synthetase | NM_002161.4 (SEQ ID NO :11) | Increased |
| ATP6V1E1 | ATPase, H⁺ transporting, lysosomal 31kDa, V1 subunit E1 | NM_001039367.1 (SEQ ID NO :12) | Decreased |
| RPP21 | Ribonuclease P/MRP 21kDa subunit | NM_024839.1 (SEQ ID NO :13) | Increased |
| SFRS5 | splicing factor, arginine/serine-rich 5 | NM_001039465.1 (SEQ ID NO :14) | Increased |
| LOC285636 | chromosome 5 open reading frame 51 | NM_175921.4 (SEQ ID NO:15) | Increased |
| NTNG2 | netrin G2 | NM_032536.2 (SEQ ID NO :16) | Decreased |
| ASF1A | ASF1 anti-silencing function 1 homolog A | NM_014034.2 (SEQ ID NO :17) | Increased |
| DYSF | dysferlin | NM_001130455.1 (SEQ ID NO :18) | Decreased |
| EIF4H | eukaryotic translation initiation factor 4H | NM_031992.1 (SEQ ID NO :19) | Increased |
| USP39 | ubiquitin specific peptidase 39 | NM_006590.2 (SEQ ID NO :20) | Increased |
| ANAPC4 | Anaphase promoting complex subunit 4 | NM_013367.2 (SEQ ID NO :21) | Increased |
| NUDT15 | nudix | NM_018283.1 (SEQ ID NO :22) | Decreased |
| TRAPPC3 | trafficking protein particle complex 3 | NM_014408.3 (SEQ ID NO :23) | Increased |
| SRPRB | signal recognition particle receptor, B subunit | NM_021203.3 (SEQ ID NO :24) | Increased |
| UBE2Z | ubiquitin-conjugating enzyme E2Z | NM_023079.3 (SEQ ID NO :25) | Increased |
| RCN2 | reticulocalbin 2 | NM_002902.2 (SEQ ID NO :26) | Increased |
| RAB9A | member RAS oncogene family | NM_004251.3 (SEQ ID NO :27) | Increased |
| SBDS | Shwachman-Bodian-Diamond syndrome | NM_023248.1 (SEQ ID NO :28) | Increased |
| SYNE1 | spectrin repeat containing, nuclear envelope 1 | NM_015293.2 (SEQ ID NO :29) | Increased |
| | | NM_033071.2 (SEQ ID NO :30) | |
| | | NM_133650.2 (SEQ ID NO:31) | |
| | | NM_182961.2 (SEQ ID NO :32) | |
| PGLYRP1 | peptidoglycan recognition protein 1 | NM_005091.1 (SEQ ID NO :33) | Decreased |
| FLJ10769 | carbohydrate kinase domain containing | NM_018210.2 (SEQ ID NO :34) | Increased |
| MFAP1 | microfibrillar-associated protein 1 | NM_005926.2 (SEQ ID NO :35) | Increased |
| HK3 | hexokinase 3 | NM_002115.2 (SEQ ID NO :36) | Decreased |
| MLLT11 | myeloid/lymphoid or mixed-lineage leukemia (trithorax homolog, Drosophila); translocated to, 11 | NM_006818.3 (SEQ ID NO :37) | Increased |
| GPR137B | G protein-coupled receptor 137B | NM_003272.3 (SEQ ID NO :38) | Increased |
| CD63 | CD63 | NM_001040034.1 (SEQ ID NO :39) | Decreased |
| | | NM_001780.4 (SEQ ID NO :40) | |
| TARSL2 | threonyl-tRNA synthetase-like 2 | NM_152334.2 (SEQ ID NO :41) | Increased |
| TTYH2 | tweety homolog 2 | NM_032646.5 (SEQ ID NO :42) | Increased |
| BRP44L | brain protein 44-like | NM_016098.2 (SEQ ID NO :43) | Increased |
| MTERFD1 | MTERF domain containing 1 | NM_015942.3 (SEQ ID NO :44) | Increased |
| CASP5 | caspase 5 | NM_001136111.1 (SEQ ID NO :45) | Decreased |
| RIOK1 | RIO kinase 1 | NM_153005.1 (SEQ ID NO :46) | Increased |
| | | NM_031480.2 (SEQ ID NO :47) | |
| CLN5 | ceroid-lipofuscinosis, neuronal 5 | NM_006493.2 (SEQ ID NO :48) | Increased |
| ZC3H7A | zinc finger CCCH-type containing 7A | NM_014153.2 (SEQ ID NO :49) | Increased |
| NARG2 | NMDA receptor regulated 2 | NM_024611.4 (SEQ ID NO :50) | Increased |
| | | NM_001018089.1 (SEQ ID NO :51) | |
| TMEM85 | transmembrane protein 85 | NM_016454.2 (SEQ ID NO :52) | Increased |
| COBRA1 | cofactor of BRCA1 | NM_015456.3 (SEQ ID NO :53) | Increased |
| KEAP1 | kelch-like ECH-associated protein 1 | NM_012289.3 (SEQ ID NO :54) | Decreased |
| | | NM_203500.1 (SEQ ID NO :55) | |
| LRCH3 | leucine-rich repeats and calponin homology (CH) domain containing 3 | NM_032773.2 (SEQ ID NO :56) | Increased |
| C19orf12 | chromosome 19 open reading frame 12 | NM_001031726.2 (SEQ ID NO :57) | Increased |
| | | NM_031448.3 (SEQ ID NO :58) | |
| PIGC | phosphatidylinositol glycan anchor biosynthesis, class C | NM_002642.3 (SEQ ID NO :59) | Increased |
| | | NM_153747.1 (SEQ ID NO :60) | |
| DGAT2 | diacylglycerol O-acyltransferase 2 | NM_032564.3 (SEQ ID NO :61) | Decreased |
| DDX56 | DEAD box polypeptide 56 | NM_019082.2 (SEQ ID NO :62) | Increased |
| NIP30 | NEFA-interacting nuclear protein | NM_024946.2 (SEQ ID NO :63) | Increased |
| MAPK14 | mitogen-activated protein kinase 14 | NM_001315.2 (SEQ ID NO :64) | Decreased |
| | | NM_139012.2 (SEQ ID NO :65) | |
| | | NM_139013.2 (SEQ ID NO :66) | |
| | | NM_139014.2 (SEQ ID NO :67) | |
| SLC39A10 | solute carrier family 39 (zinc transporter), member 10 | NM_001127257.1 (SEQ ID NO :68) | Increased |
| | | NM_020342.2 (SEQ ID NO :69) | |
| ADH5 | alcohol dehydrogenase 5 | NM_000671.3 (SEQ ID NO :70) | Increased |
| KIAA0947 | KIAA0947 | NM_015325.1 (SEQ ID NO :71) | Increased |
| GLTSCR2 | glioma tumor suppressor candidate region gene 2 | NM_015710.4 (SEQ ID NO :72) | Increased |
| DNAJA3 | DnaJ (Hsp40) homolog, subfamily A, member 3 | NM_001135110.1 (SEQ ID NO :73) | Increased |
| | | NM_005147.4 (SEQ ID NO :74) | |
| FAM134C | family with sequence similarity 134, member C | NM 178126.3 (SEQ ID NO :75) | Increased |

**Table 3: Discriminatory performance of the gene set based on the top 8 genes in the data of Julià et al.**

| | True R (Julià *et al*.) | True NR (*Julià et al*.) | Sum | Correct [%] |
|---|---|---|---|---|
| Predicted R (Julià *et al.)* | 31 | 1 | 32 | 96.88 |
| Predicted NR (Julià *et al.)* | 6 | 6 | 12 | 50 |
| Sum | 37 | 7 | - | - |
| Correct [%] | 83.78 | 85.71 | - | - |

The top 8 most significantly differentially expressed genes (IL2RB, S100A9, KLRK1, HCK, GNLY, CTSZ, ARF5, and UTP14C) have all been associated to the same biological network ("Cell-to-cell signalling and interaction, haematological system development and function, immune cell trafficking") by an Ingenuity® Systems analysis (Figure 3).

Furthermore, the top 5 most significantly differentially expressed genes (IL2RB, S100A9, KLRK1, HCK, GNLY) are particularly relevant to RA physiopathology.

### IL2RB (Interleukin-2 receptor subunit beta)

IL2RB gene encodes the beta subunit of the IL2R, which is present in the moderate and high affinity forms of the receptor required for signal transduction from IL2. Several studies showed that SNPs in IL2RB gene (especially rs743777) are significantly associated with RA (13).

It has been recently proposed that thymic selection of an auto-reactive T-cell repertoire is an important risk factor for rheumatoid arthritis (RA) (14).

The IL2RB gene is thus an attractive candidate for RA because of the key role played by IL2 in T cell activation and regulation.

### S100A9 (S100 calcium-binding protein A9)

S100A9 gene codes for the S100A9 protein also called myeloid-related protein 14 (MRP14) or calgranulin B that belongs to the S100 family of proteins. S100 proteins are known to be associated with several pathological conditions such as cystic fibrosis (15), cancer (16) and inflammatory diseases (17).

S100A9 has also been shown to induce neutrophil degranulation (which may account for the tissue damage in RA) via a MAPK-dependent mechanism (18).

A recently published study showed that S100 proteins are associated with RA inflammation and auto-antibody production (19).

### KLRK1

KLRK1 gene encodes the natural killer group 2D (NKG2D) protein which belongs to the family of activating NK cell receptors (NKRs).

In RA patients, a significant proportion of CD4⁺CD28⁻ T cells were shown to express NKG2D which stimulated auto-reactive responses against RA synoviocytes (20).

### HCK

HCK gene encodes the tyrosine-protein kinase HCK (hematopoietic cell kinase) predominantly expressed in hematopoietic cell types.

HCK has been shown to be involved in functional pro-inflammatory responses such as the FcR-mediated respiratory burst (21), neutrophil migration (22) and neutrophil degranulation (23) that play important roles in the early phases of RA physiopathology Furthermore, it has also been shown that HCK mediates IL-2 signaling in human monocytes (24).

### GNLY

GNLY gene codes for Granulysin, a saponin-like protein present in cytotoxic granules of cytolytic T cells and NK cells and released upon antigen stimulation.

Granulysin is a chemoattractant for T lymphocytes, monocytes and other inflammatory cells and induces the expression of a number of cytokines, including RANTES, MCP-1, MCP-3, MIP-1α, IL-10, IL-1, IL-6 and IFN-α (25).

Granulysin is also involved in several diseases including infection, cancer, transplantation, autoimmunity, skin and reproductive maladies (26).

### Example 2: meta-analysis of three datasets (Bienkowska et al. Julia et al, and Sekiguchi et al.)

A second meta-analysis was performed after merging the dataset of Sekiguchi *et al.* This dramatically reduced the number of genes being tested, bringing it down to 290. Keeping the same threshold for the test statistic as previously (2.5) only three of the 61 previously described (see Example 1) genes were significant: IL2RB, S100A9 and CASP5. However, these genes widen the prediction of the Infliximab treatment to a 22 weeks follow-up. Besides the relationship between IL2RB and S100A9 and physiopathology of RA (see above), CASP5 gene also has a relevant significance in this auto-immune disorder.

### CASP5

CASP5 gene encodes the Caspase 5 enzyme that proteolytically cleaves other proteins at an aspartic acid residue. It is an inflammatory caspase that plays a role in the immune system (27) and in the complex process of cellular apoptosis (28).

The aberrant decrease in apoptosis or increased cell cycle activity of fibroblast-like or macrophage-like synoviocytes is responsible for the synovial hyperplasia and contributes to the destruction of cartilage and bone in RA patients thus suggesting a potential role of apoptosis-related caspases in the physiopathology of RA (29).

### Example 3: meta-analysis of four datasets (Bienkowska et al. Julia et al, Sekiguchi et al., and Leguerr6 et al.)

Further adding the data by Lequerré *et al*., and thus leading to a total number of 103 samples analysed in 179 genes identified only two of the 61 previously described (see Example 1) genes that are significantly increased in non responders: MAPK14 et S100A9 (Figure 2). The combination of these two genes is thus able to predict response to Infliximab treatment on a biological sample (whole blood or PBMCs) at week 14 or week 22.

### MAPK14

MAPK14 gene encodes the mitogen-activated protein kinase 14, a member of the MAP kinase family. MAP kinases act as an integration point for multiple biochemical signals, and are involved in a wide variety of cellular processes such as proliferation, differentiation, transcription regulation and development. The MAP kinase pathway is particularly influencing the biosynthesis of pro-inflammatory cytokines TNFα and IL-1β both at a translational and a transcriptional level (30). This pathway might thus be a key anti-inflammatory target for RA treatment.

### Example 4: quantitative PCR confirmation of the efficiency of signatures obtained from meta-analysis of microarray data

### Patients and methods

### Patients

Peripheral blood samples from 40 patients, with rheumatoid arthritis and classified as responders and non responders to Infliximab treatment at week 14 according to the EULAR definition of response (Good and moderate responders were classified as responders and poor responders were classified as non responders), were identified and analyzed by RT-PCR. The clinical and demographic variables of the patients are summarized in Table 4.

**Table 4: Patient clinical characteristics**

| | |
|---|---|
| Number of Responders | 34 |
| Number of Non Responders | 6 |
| Average DAS28 value at baseline | 6 |
| Average DAS28 value at week 14 | 4 |
| Number of Males | 5 |
| Number of Females | 35 |
| Average ESR at baseline | 40 |
| Average CRP at baseline | 2 |

### Methods

Peripheral blood was collected into RNA PAXgene® tubes (PreAnalytix, Switzerland) and RNA from whole blood was extracted according to the methods described in Julia *et al.* (8). Samples underwent quality control and were dried using speed vacuum prior to the RT and PCR steps. The real-time PCR reactions was carried out on a total of 40 samples using the TaqMan Universal PCR Master Mix (Applied Biosystems). 6 µL cDNA was added to a 9 µL PCR mixture containing 7.5 µL TaqMan Universal PCR Master Mix, 0.75 µL of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition was performed using the ABI 7900HT Fast Real-Time PCR System (Applied Biosystems). The "ΔΔCT method" was used as a measure of gene expression. Post extraction steps and analyses were carried out at TcLand Expression ISO13485 laboratory.

For 7 out of the 11 genes, probes were custom designed and the sequence information can be found in Table 5a. For 3 out of the 11 genes, probes were ordered from Applied Biosystems directly and are referenced in Table 5b.

**Table 5a : Sequence information for the 7probes that were custom designed.**

| Gene Symbol | RefSeq | Forward Primer Sequence | Probe Sequence | Reverse Primer Sequence | Amplicon Sequence |
|---|---|---|---|---|---|
| **ARF5** | NM_001 662.3 | CTCCTGC CTGCATG TTCTCT (SEQ ID NO :76) | TGTTGTTG GAGCCTG G (SEQ ID NO :77) | ACAGAG GGGTCC ACTCTCC (SEQ ID NO :78) | CTCCTGCCTGCATGTTCTCT CTGTTGTTGGAGCCTGGAG CCTTGCTCTCTGGGCACAG AGGGGTCCACTCTCC (SEQ ID NO :79) |
| **CTSZ** | NM_001 336.3 | GAAACGA TGGGAC CTCAGTC TT (SEQ ID NO :80) | CTTCAGCA GAGGACT TG (SEQ ID NO :81) | TATTTTG TATTTGG CAACTGT GGGC (SEQ ID NO :82) | GAAACGATGGACCTCAGT CTTCTTCAGCAGAGGACTT GATATTTTGTATTTGGCAAC TGTGGGC (SEQ ID NO :83) |
| **HCK** | NM_002 110.3 | CCCCTTC CTACTCC CAGACA (SEQ ID NO :84) | CACCCTC GCTTCAG CC (SEQ ID NO :85) | CAGTTTC CTCATCT GTCCAGT GG (SEQ ID NO :86) | CCCCTTCCTACTCCCAGAC ACCCACCCTCGCTTCAGCC ACAGTTTCCTCATCTGTCCA GTGG (SEQ ID NO :87) |
| **KLRK1** | NM_007 360.2 | GCCTTCC CTGCCTG TGG (SEQ ID NO :88) | CCACTTTT AATGGGT CCTCC (SEQ ID NO :89) | CAACGG GGTCAG GGAGG (SEQ ID NO :90) | GCCTTCCCTGCCTGTGGGG GTCATGCTGCCACTTTTAAT GGGTCCTCCACCCAACGGG GTCAGGGAGG (SEQ ID NO :91) |
| **S100A8** | NM_002 964.3 | ACGTCTG GTTCAAA GAGTTG GATAT (SEQ ID NO :92) | TAACTTCC AGGAGTT CCTCAT (SEQ ID NO :93) | GTGATAA AGATGG GCGTGG C (SEQ ID NO :94) | ACGTCTGGTTCAAAGAGTT GGATATCAACACTGATGGT GCAGTTAACTTCCAGGAGT TCCTCATTCTGGTGATAAAG ATGGGCGTGGC (SEQ ID NO :95) |
| **S100A9** | NM_002 965.3 | GGCCAC CCTGCCT CTAC (SEQ ID NO :96) | TGTCAAAC TGTCTTGG CTG (SEQ ID NO :97) | GGCTAG GGGCTG GGG (SEQ ID NO :98) | GGCCACCCTGCCTCTACCC AACCAGGGCCCCGGGGCC TGTTATGTCAAACTGTCTTG GCTGTGGGGCTAGGGGCT GGGG (SEQ ID NO :99) |
| **UTP14C** | NM_021 645.5 | TGCAGAA CTTTCAG GATGACT ATTAATT C (SEQ ID NO :100) | TTGAGTG GTCCAAG CCTG (SEQ ID NO :101) | TGTTTTG AACCCAC AGCAGTG (SEQ ID NO :102) | TGCAGAACTTTCAGGATGA CTATTAATTCCTCTCAGATG TCATTTTTGAGTGGTCCAAG CCTGCTGTTTTGAACCCAC AGCAGTG (SEQ ID NO :103) |

**Table 5b: Probe information for the three genes for which probes were ordered from Applied Biosystems repositoryResults**

| Gene Symbol | Probe ID | Context Sequence |
|---|---|---|
| GNLY | Hs01120727_m1 | TACCTTCTACAGGTCCCCTCTGAGC (SEQ ID NO :104) |
| MAPK14 | Hs01051153_m1 | TGTTTCCTGGTACAGACCATATTAA (SEQ ID NO :105) |
| IL2RB | HS00386697 | GAACACCGGGCCATGGCTGAAGAAG (SEQ ID NO:106) |

Following the results from the meta-analysis, Taqman probes were ordered through applied or designed in house. After our internal quality control steps 8 out of the 11 genes of the present invention were tested on the RA samples. The 40 samples, that represent a subset of the original samples used for microarray analysis in the study of Julia *et al*., were analyzed by RT-PCR following our internal protocols. The following statistical analysis has been performed: Identification of individually differentially expressed genes between the two groups of responders versus non responders (Table 6). The selection criteria used was a significant t-test of at least 0.05. Additionally the discriminatory power of the combination of 1, 2, 5 and 8 genes listed in the invention was assessed using a logistic regression and classification rates were provided (Table 7).

**Table 6: P-values and ΔΔCT for Responder and Non Responder groups. Genes underlined and in italic were confirmed as being discriminant (P-value<0.05).**

| **Gene Symbol** | **P-Value** | **Average ΔΔCT value in non responder group (ΔΔCT_{NonRESP})** | **Average ΔΔCT value in responder group (AACT_{RESP})** |
|---|---|---|---|
| ARF5 | 0.9284 | 0.3438 | 0.3557 |
| *CTSZ* | 0.4426 | -0.6069 | -0.6848 |
| *GNLY* | *0.0017* | -4.253 | -3.2036 |
| *HCK* | 0.1963 | -3.3665 | -3.5577 |
| *IL2RB* | *0.0282* | -3.527 | -3.1646 |
| *KLRK1* | 0.0829 | -1.5087 | -0.9914 |
| *MAPK14* | *0.0003* | 1.364 | 0.831 |
| *S100A8* | 0.1768 | -4.8138 | -5.2706 |
| S100A9 | 0.0642 | -5.1389 | -5.6202 |

**Table 7: Classification performance using logistic regression of claimed list gene combinations.**

| **Model** | **Sensitivity** | **Specificity** | **Error Rate** |
|---|---|---|---|
| S100A9 | 0% | 100% | 16% |
| MAPK14, S100A9 | 83% | 96% | 5% |
| IL2RB, S100A9, KLRK1,HCK and GNLY | 83% | 96% | 6% |
| IL2RB, S100A9, KLRK1, HCK, GNLY, CTSZ, ARF5 and UTP14C | 100% | 100% | 0% |

### Conclusion

The above results confirm that the particular combinations (signatures) of genes identified as predicting the response to infliximab of RA-suffering patients based on meta-analysis of microarray data obtained in several independent studies are actually efficient (high sensititity and specificity, low error rate) for predicting the response to influximab treatment of a validation group of RA-suffering patients.

### Example 5: Prediction of the response to Infliximab at week 14 of included patients using different sub-combinations of genes

### Patients

The same patients as in Example 4 have been studied.

### Results

The identification of an optimal subgroup of genes that together best discriminate between the responders and non responders was then performed. A logistic regression was applied and the classification error was used to identify the optimal subset of genes.

The most discriminating gene on its own is MAPK14 (Table 7), the optimal two-gene combination is MAPK14 with GNLY.

**Table 7: Classification performance using logistic regression of claimed list gene combinations.**

| **Model** | **Sensitivity** | **Specificity** | **Error Rate** |
|---|---|---|---|
| MAPK14 | 50% | 97% | 11% |
| MAPK14, GNLY | 100% | 100% | 0% |

### Conclusion

These results show that MAPK14, already identified as significant in Example 3 by meta-analysis of microarray data obtained from 4 independent studies, is actually highly predictive, even alone, of response to Infliximab treatment in RA-suffering patients. Moreover, in addition to the combination of genes S100A9 and MAPK14, already identified as useful in Example 3 and confirmed by qPCR experiments in Example 4, the combination of genes MAPK14 and GNLY (GNLY being already identified as significant in Example 1) is also highly predictive of response to Infliximab in RA-suffering patients, all patients tested being correctly classified using this combination.

Globally, the results presented in Examples 1 to 5 support the tight correlation of two individual genes, MAPK14 and S100A9, or equivalent correlated genes, with the Infliximab responsive or non-responsive phenotype of RA-suffering subjects. In addition, combination of one or both of these genes with a small number of other genes found associated to Infliximab responsive or non-responsive phenotype of RA-suffering subjects (notably the 61 genes of Table 2, and more particularly genes GNLY, IL2RB, S100A9, KLRK1, HCK, CTSZ, ARF5, and UTP14C), permit a highly sensitive and specific (very low error rate) prediction of the Infliximab responsive or non-responsive phenotype of RA-suffering subjects.

Finally, we note that genes found to be highly correlated to the Infliximab responsive or non-responsive phenotype of RA-suffering subjects are not genes that might be involved in the metabolism of Infliximab, but rather genes that may be associated to the disease or its underlying dysfunctions themselves. In particular, many genes found to be highly correlated to the Infliximab responsive or non-responsive phenotype of RA-suffering subjects are known to be involved in inflammatory or immune processes. This clearly gives a rational for the extension of the methods of the invention for the prediction of the TBA responsive or non-responsive phenotype of subjects suffering from other inflammatory diseases, in particular those involving pathogenic TNFα secretion and even more particularly those for which TBA have been approved or have been shown in preliminary studies to be useful.

### Example 6: Correlation between gene expression and inflammatory response measured by DAS28, CRP and ESR in rheumatoid arthritis patients

Clinical parameters measuring inflammation at baseline such as DAS28, C-reactive protein (CRP) and erythrocyte sedimentation rate (ESR) do not discriminate between responders and non responders (respective p-values for t-test are 0.7792, 0.4839 and 0.1755). To assess whether the gene expression levels are correlated to the clinical parameters of inflammation, a correlation coefficient was computed (Table 8). Table 8 indicates that the genes in the signature are not correlated to the clinical parameters and thus add independent information. Only the gene expression levels of IL2RB correlate negatively to CRP (correlation coefficient = -0.35, p-value 0.03), please note this p-value is uncorrected for multiple testing.

**Table 8: Correlation coefficient of gene expression versus clinical parameters indicate lack of correlation between gene expression levels and clinical parameters of inflammation.**

| | **DAS28_w0** | **CRP** | **ESR** |
|---|---|---|---|
| **MAPK14** | 0.06 | 0.17 | 0.17 |
| **S100A9** | -0.07 | 0.09 | 0 |
| **S100A8** | 0.1 | 0.09 | 0.13 |
| **IL2RB** | -0.19 | -0.35 | -0.25 |
| **KLRK1** | -0.17 | -0.19 | -0.15 |
| **HCK** | -0.23 | -0.09 | -0.21 |
| **GNLY** | -0.17 | -0.25 | -0.23 |
| **CTSZ** | 0.13 | 0.17 | -0.05 |
| **ARF5** | 0 | 0.1 | -0.08 |
| **UTP14C** | 0.11 | -0.08 | 0.16 |

These results support the claim that our gene expression signature provides independant discriminatory power over clinical variables.

### REFERENCES

1. Lee DM, Weinblatt ME. Rheumatoid arthritis. Lancet. 2001 Sep 15;358(9285):903-11.
2. Choy EH, Panayi GS. Cytokine pathways and joint inflammation in rheumatoid arthritis. N Engl J Med. 2001 Mar 22;344(12):907-16.
3. Kooloos WM, de Jong DJ, Huizinga TW, Guchelaar HJ. Potential role of pharmacogenetics in anti-TNF treatment of rheumatoid arthritis and Crohn's disease. Drug Discovery Today. 2007;12(3-4):125-31.
4. Isaacs JD. Antibody engineering to develop new antirheumatic therapies. Arthritis Res Ther. 2009;11 (3):225.
5. Hetland ML, Christensen IJ, Tarp U, Dreyer L, Hansen A, Hansen IT, et al. Direct comparison of treatment responses, remission rates, and drug adherence in patients with rheumatoid arthritis treated with adalimumab, etanercept, or infliximab: results from eight years of surveillance of clinical practice in the nationwide Danish DANBIO registry. Arthritis Rheum. 2010 Jan;62(1):22-32.
6. Lequerre T, Gauthier-Jauneau AC, Bansard C, Derambure C, Hiron M, Vittecoq O, et al. Gene profiling in white blood cells predicts infliximab responsiveness in rheumatoid arthritis. Arthritis Res Ther. 2006;8(4):R105.
7. Sekiguchi N, Kawauchi S, Furuya T, Inaba N, Matsuda K, Ando S, et al. Messenger ribonucleic acid expression profile in peripheral blood cells from RA patients following treatment with an anti-TNF-alpha monoclonal antibody, infliximab. Rheumatology (Oxford). 2008 Jun;47(6):780-8.
8. Julia A, Erra A, Palacio C, Tomas C, Sans X, Barcelo P, et al. An eight-gene blood expression profile predicts the response to infliximab in rheumatoid arthritis. PLoS One. 2009;4(10):e7556.
9. Bienkowska JR, Dalgin GS, Batliwalla F, Allaire N, Roubenoff R, Gregersen PK, et al. Convergent Random Forest predictor: methodology for predicting drug response from genome-scale data applied to anti-TNF response. Genomics. 2009 Dec;94(6):423-32.
10. Ramasamy A, Mondry A, Holmes CC, Altman DG. Key issues in conducting a meta-analysis of gene expression microarray datasets. PLoS Med. 2008 Sep 30;5(9):e184.
11. Arnett FC, Edworthy SM, Bloch DA, McShane DJ, Fries JF, Cooper NS, et al. The American Rheumatism Association 1987 revised criteria for the classification of rheumatoid arthritis. Arthritis Rheum. 1988 Mar;31(3):315-24.
12. Parmigiani G, Garrett-Mayer ES, Anbazhagan R, Gabrielson E. A cross-study comparison of gene expression studies for the molecular classification of lung cancer. Clin Cancer Res. 2004 May 1;10(9):2922-7.
13. Barton A, Thomson W, Ke X, Eyre S, Hinks A, Bowes J, et al. Rheumatoid arthritis susceptibility loci at chromosomes 10p15, 12q13 and 22q13. Nat Genet. 2008 Oct;40(10):1156-9.
14. Goronzy JJ, Weyand CM. Developments in the scientific understanding of rheumatoid arthritis. Arthritis Res Ther. 2009;11(5):249.
15. Lorenz E, Muhlebach MS, Tessier PA, Alexis NE, Duncan Hite R, Seeds MC, et al. Different expression ratio of S100A8/A9 and S100A12 in acute and chronic lung diseases. Respir Med. 2008 Apr;102(4):567-73.
16. Cheng P, Corzo CA, Luetteke N, Yu B, Nagaraj S, Bui MM, et al. Inhibition of dendritic cell differentiation and accumulation of myeloid-derived suppressor cells in cancer is regulated by S100A9 protein. J Exp Med. 2008 Sep 29;205(10):2235-49.
17. Lim SY, Raftery M, Goyette J, Hsu K, Geczy CL. Oxidative modifications of S100 proteins: functional regulation by redox. J Leukoc Biol. 2009 86(3): 577-87.
18. Simard JC, Girard D, Tessier PA. Induction of neutrophil degranulation by S100A9 via a MAPK-dependent mechanism. J Leukoc Biol. 2010 Jan 26.
19. Chen YS, Yan W, Geczy CL, Brown MA, Thomas R. Serum levels of soluble receptor for advanced glycation end products and of S100 proteins are associated with inflammatory, autoantibody, and classical risk markers of joint and vascular damage in rheumatoid arthritis. Arthritis Res Ther. 2009;11(2):R39.
20. Groh V, Bruhl A, El-Gabalawy H, Nelson JL, Spies T. Stimulation of T cell autoreactivity by anomalous expression of NKG2D and its MIC ligands in rheumatoid arthritis. Proc Natl Acad Sci USA. 2003 Aug 5;100(16):9452-7.
21. Paul R, Obermaier B, Van Ziffle J, Angele B, Pfister HW, Lowell CA, et al. Myeloid Src kinases regulate phagocytosis and oxidative burst in pneumococcal meningitis by activating NADPH oxidase. J Leukoc Biol. 2008 Oct;84(4):1141-50.
22. Fumagalli L, Zhang H, Baruzzi A, Lowell CA, Berton G. The Src family kinases Hck and Fgr regulate neutrophil responses to N-formyl-methionyl-leucyl-phenylalanine. J Immunol. 2007 Mar 15;178(6):3874-85.
23. Mocsai A, Ligeti E, Lowell CA, Berton G. Adhesion-dependent degranulation of neutrophils requires the Src family kinases Fgr and Hck. J Immunol. 1999 Jan 15;162(2):1120-6.
24. Bosco MC, Curiel RE, Zea AH, Malabarba MG, Ortaldo JR, Espinoza-Delgado I. IL-2 signaling in human monocytes involves the phosphorylation and activation of p59hck. J Immunol. 2000 May 1;164(9):4575-85.
25. Deng A, Chen S, Li Q, Lyu SC, Clayberger C, Krensky AM. Granulysin, a cytolytic molecule, is also a chemoattractant and proinflammatory activator. J Immunol. 2005 May 1;174(9):5243-8.
26. Krensky AM, Clayberger C. Biology and clinical relevance of granulysin. Tissue Antigens. 2009 Mar;73(3):193-8.
27. Martinon F, Tschopp J. Inflammatory caspases and inflammasomes: master switches of inflammation. Cell Death Differ. 2007 Jan;14(1):10-22.
28. Kurokawa M, Kornbluth S. Caspases and kinases in a death grip. Cell. 2009 Sep 4;138(5):838-54.
29. Morel J, Audo R, Hahne M, Combe B. Tumor necrosis factor-related apoptosis-inducing ligand (TRAIL) induces rheumatoid arthritis synovial fibroblast proliferation through mitogen-activated protein kinases and phosphatidylinositol 3-kinase/Akt. J Biol Chem. 2005 Apr 22;280(16):15709-18.
30. Korb A, Tohidast-Akrad M, Cetin E, Axmann R, Smolen J, Schett G. Differential tissue expression and activation of p38 MAPK alpha, beta, gamma, and delta isoforms in rheumatoid arthritis. Arthritis Rheum. 2006 Sep;54(9):2745-
31. Fransen J, van Riel PLCM. The Disease Activity Score and the EULAR response criteria. Clin Exp Rheumatol. 2005 23(5 Suppl 39): S93-9.
32. Lorenz HM et al. Arthritis Res. 2002;4 Suppl 3:S17-24
33. Atzeni F et al. Autoimmun Rev. 2007 Sep;6(8):529-36.

## Claims

1. A method for the in vitro diagnosis or prognosis of a TNF-alpha blocking agent (TBA) responding or non-responding phenotype, comprising:
(a) determining from a blood sample of a subject suffering from an inflammatory disease an expression profile comprising or consisting of:
(i) the gene S100A9; or
(ii) the genes S100A9, IL2RB, and CASP5; or
(iii) the genes S100A9, IL2RB, KLRK1, HCK, and GNLY; or
(iv) the genes S100A9, IL2RB, KLRK1, HCK, GNLY, CTSZ, ARF5, and UTP14C;
and optionally one or more housekeeping gene(s)
(b) comparing the obtained expression profile with at least one reference responding expression profile and at least one reference non-responding expression profile using statistical models or machine learning technologies, and
(c) determining the responding or non-responding phenotype from said comparison.

2. A method for designing a TBA treatment for a subject suffering from an inflammatory disease, said method comprising:
(a) determining from a blood sample of said subject an expression profile comprising or consisting of:
(i) the gene S100A9; or
(ii) the genes S100A9, IL2RB, and CASP5; or
(iii) the genes S100A9, IL2RB, KLRK1, HCK, and GNLY; or
(iv) the genes S100A9, IL2RB, KLRK1, HCK, GNLY, CTSZ, ARF5, and UTP14C;
and optionally one or more housekeeping gene(s)
(b) comparing the obtained expression profile with at least one reference responding expression profile and at least one reference non-responding expression profile using statistical models or machine learning technologies,
(c) determining the responding or non-responding phenotype of said subject from said comparison, and
(d) designing a dose of TBA treatment according to the said identified responding or non-responding phenotype.

3. The method of claim 1 or claim 2, wherein the expression profile of step (a) comprises or consists of the genes S100A9, IL2RB, and CASP5.

4. The method of anyone of claims 1 to 3, wherein the expression profile is determined by measuring the amount of nucleic acid transcripts of said gene(s).

5. The method of claim 4, wherein the expression profile is determined using quantitative PCR or an oligonucleotide microarray.

6. The method of anyone of claims claims 1 to 3, wherein the expression profile is determined using a genomic microarray or a proteic microarray.

7. The method of anyone of claims 1 to 6, wherein the expression profile of step (a) is determined at 14 or 22 weeks after the beginning of the CyTD treatment.

8. The method of anyone of claims 1 to 7, wherein said TBA is selected in the group consisting of Etanercept (Enbrel (Registered trademark), Infliximab (Remicade (Registered trademark), Adalimumab (Humira (Registered trademark), and Certolizumab pegol (Cimzia (Registered trademark).

9. The method according to anyone of claims 1 to 8, wherein said inflammatory disease is selected from the group consisting of rheumatoid arthritis (RA), Crohn's disease, ankylosing spondylitis, psoriatic arthritis, plaque psoriasis, ulcerative colitis, vasculitis; Wegener's granulomatosis; sarcoidosis; adult-onset Still's disease, polymyositis/dermatomyositis, and systemic lupus erythematosus (SLE).

10. The method of claim 9, wherein said inflammatory disease is rheumatoid arthritis.

11. The method according to claim 10, further comprising determining at least one additional parameter, said additional parameter being determined by a test selected from the Antinuclear Antibody test (ANA test), C-Reactive Protein test (CRP test), Cyclic Citrullinated Peptide Antibody test (CCP test), and the Rheumatoid Factor test.

12. Use of a kit comprising at least one reagent for the determination of an expression profile comprising or consisting of:
(i) the gene S100A9; or
(ii) the genes S100A9, IL2RB, and CASP5; or
(iii) the genes S100A9, IL2RB, KLRK1, HCK, and GNLY; or
(iv) the genes S100A9, IL2RB, KLRK1, HCK, GNLY, CTSZ, ARF5, and UTP14C;
and optionally one or more housekeeping gene(s),
for the in vitro diagnosis of a TBA responding or non-responding phenotype.

13. The use of claim 12, wherein said kit further comprises at least one reagent for determining at least one additional parameter, said reagent selected from the Antinuclear Antibody test (ANA test), C-Reactive Protein test (CRP test), Cyclic Citrullinated Peptide Antibody test (CCP test), and the Rheumatoid Factor test.

14. Use of a nucleic acid microarray, comprising nucleic acids specific for:
(i) the gene S100A9; or
(ii) the genes S100A9, IL2RB, and CASP5; or
(iii) the genes S100A9, IL2RB, KLRK1, HCK, and GNLY; or
(iv) the genes S100A9, IL2RB, KLRK1, HCK, GNLY, CTSZ, ARF5, and UTP14C;
and optionally one or more housekeeping gene(s),
for the in vitro diagnosis of a TBA responding or non-responding phenotype.

15. The use of claim 14, wherein said nucleic acid microarray is an oligonucleotide microarray.
